(19) Europäisches Patentamt
European Patent Office
Office européen des brevets

(11) EP 0 800 519 B1

(12) EUROPEAN PATENT SPECIFICATION

(45) Date of publication and mention of the grant of the patent:
**22.10.2003 Bulletin 2003/43**

(21) Application number: **95944089.2**

(22) Date of filing: **13.12.1995**

(51) Int Cl.[7]: **C07D 215/06**, A61K 31/47, C07D 417/04, C07D 413/04, C07D 401/04, C07D 221/18, C07D 493/04, C07D 471/04

(86) International application number:
**PCT/US95/16096**

(87) International publication number:
**WO 96/019458 (27.06.1996 Gazette 1996/29)**

# (54) STEROID RECEPTOR MODULATOR COMPOUNDS AND METHODS

STEROIDREZEPTOR-MODULATOR VERBINDUNGEN UND METHODEN

COMPOSES MODULATEURS DES RECEPTEURS DES STEROIDES ET PROCEDES D'UTILISATION

(84) Designated Contracting States:
**AT BE CH DE DK ES FR GB GR IE IT LI LU MC NL PT SE**

(30) Priority:
**22.12.1994 US 363529**
**05.06.1995 US 464541**
**05.06.1995 US 463231**
**05.06.1995 US 464546**
**05.06.1995 US 465429**
**05.06.1995 US 464360**
**05.06.1995 US 462643**
**05.06.1995 US 465556**

(43) Date of publication of application:
**15.10.1997 Bulletin 1997/42**

(60) Divisional application:
**00113829.6 / 1 043 325**
**00113830.4 / 1 043 326**
**00113914.6 / 1 041 071**
**00113915.3 / 1 041 066**
**00113916.1 / 1 043 315**

(73) Proprietor: **LIGAND PHARMACEUTICALS, INC.**
**San Diego, CA 92121 (US)**

(72) Inventors:
- **JONES, Todd, K. c/o Ligand Pharmaceuticals,Inc.**
  **San Diego, CA 92129 (US)**
- **GOLDMAN, Mark, E.**
  **San Diego, CA 92130 (US)**
- **POOLEY, Charlotte, L., F.**
  **San Diego, CA 92109 (US)**
- **WINN, David, T.**
  **Boulder, Colorado, 80301 (US)**
- **EDWARDS, James, E.**
  **San Diego, CA 92129 (US)**
- **WEST, Sarah, J.**
  **San Diego, CA 92122 (US)**
- **TEGLEY, Christopher, M. c/o Ligand**
  **San Diego, CA 92129 (US)**
- **ZHI, Lin**
  **San Diego, CA 92129 (US)**
- **HAMANN, Lawrence, G.**
  **San Diego, CA 92122 (US)**
- **FARMER, Luc, J.**
  **La Jolla, CA 92037 (US)**
- **DAVIS, Robert, J.**
  **Santee, CA 92071 (US)**

(74) Representative:
**Fürniss, Peter, Dr.rer.nat.Dipl.Chem**
**Winter, Brandl, Fürniss, Hübner, Röss, Kaiser, Polte Partnerschaft**
**Patent- und Rechtsanwaltskanzlei**
**Alois-Steinecker-Strasse 22**
**85354 Freising (DE)**

(56) References cited:
**EP-A- 0 260 744**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- DATABASE CROSSFIRE Beilstein Informationssysteme GmbH, Frankfurt, DE XP002002687 & J. ORG. CHEM. USSR, vol. 9, 1973, pages 2571-2576,
- DATABASE CROSSFIRE Beilstein XP002002688 & JOURNAL OF ORGANIC CHEMISTRY, vol. 30, 1965, EASTON US, pages 3560-3561,
- DATABASE CROSSFIRE Beilstein XP002002689 & YAKUGAKU ZASSHI, vol. 57, 1937, pages 312-316, UEDA:
- DATABASE CROSSFIRE Beilstein XP002002690 & ANAL. CHEM., vol. 27, 1955, pages 100-101,
- DATABASE CROSSFIRE Beilstein XP002002691 & CHEMISCHE BERICHTE, vol. 35, 1902, WEINHEIM DE, page 3278
- DATABASE CROSSFIRE Beilstein XP002002692 & JOURNAL OF THE AMERICAN CHEMICAL SOCIETY, vol. 57, 1935, DC US, page 902
- DATABASE CROSSFIRE Beilstein XP002002693 & JOURNAL OF ORGANIC CHEMISTRY, vol. 43, 1978, EASTON US, pages 1975-1980,
- DATABASE CROSSFIRE Beilstein XP002002694 & BULL. ACAD. SCI. USSR DIV. CHEM. SCI. (ENGL. TRANSL.), vol. 25, 1976, pages 2069-2071,
- DATABASE CROSSFIRE Beilstein XP002002695 & JOURNAL OF MEDICINAL CHEMISTRY, vol. 34, no. 11, 1991, WASHINGTON US, pages 3261-3267,
- DATABASE CROSSFIRE Beilstein XP002002696 & DE,A,20 15 351
- DATABASE CROSSFIRE Beilstein XP002002697 & US,A,3 907 507
- DATABASE CROSSFIRE Beilstein XP002002698 & JOURNAL OF THE CHEMICAL SOCIETY, 1964, LETCHWORTH GB, pages 3132-3140,
- DATABASE CROSSFIRE Beilstein XP002002699 & JOURNAL OF MEDICINAL CHEMISTRY, vol. 15, 1972, WASHINGTON US, page 1177
- DATABASE CROSSFIRE Beilstein XP002002700 & HELVETICA CHIMICA ACTA, vol. 60, 1977, BASEL CH, pages 978-1021,
- DATABASE CROSSFIRE Beilstein XP002002701 & JOURNAL OF THE AMERICAN CHEMICAL SOCIETY, vol. 66, 1944, DC US, page 1927

## Description

### Field of the Invention

This invention relates to non-steroidal compounds that are modulators (i.e. agonists and antagonists) of steroid receptors (e.g., progesterone receptor, androgen receptor, estrogen receptor, glucocorticoid receptor and mineralocorticoid receptor), and to methods for the making and use of such compounds.

### Background of the Invention

Intracellular receptors (IRs) form a class of structurally-related genetic regulators scientists have named "ligand dependent transcription factors." R.M. Evans, 240 *Science,* 889 (1988). Steroid receptors are a recognized subset of the IRs, including the progesterone receptor (PR), androgen receptor (AR), estrogen receptor (ER), glucocorticoid receptor (GR) and mineralocorticoid receptor (MR). Regulation of a gene by such factors requires both the IR itself and a corresponding ligand which has the ability to selectively bind to the IR in a way that affects gene transcription.

Ligands to the IRs can include low molecular weight native molecules, such as the hormones progesterone, estrogen and testosterone, as well as synthetic derivative compounds such as medroxyprogesterone acetate, diethylstilbesterol and 19-nortestosterone. These ligands, when present in the fluid surrounding a cell, pass through the outer cell membrane by passive diffusion and bind to specific IR proteins to create a ligand/receptor complex. This complex then translocates to the cell's nucleus, where it binds to a specific gene or genes present in the cell's DNA. Once bound to DNA, the complex modulates the production of the protein encoded by that gene. In this regard, a compound which binds an IR and mimics the effect of the native ligand is referred to as an "agonist", while a compound that inhibits the effect of the native ligand is called an "antagonist."

Ligands to the steroid receptors are known to play an important role in health of both women and men. For example, the native female ligand, progesterone, as well as synthetic analogues, such as norgestrel (18-homonorethisterone) and norethisterone (17$\alpha$-ethinyl-19-nortestosterone), are used in birth control formulations, typically in combination with the female hormone estrogen or synthetic estrogen analogues, as effective modulators of both PR and ER. On the other hand, antagonists to PR are potentially useful in treating chronic disorders, such as certain hormone dependent cancers of the breast, ovaries, and uterus, and in treating non-malignant conditions such as uterine fibroids and endometriosis, a leading cause of infertility in women. Similarly, AR antagonists, such as cyproterone acetate and flutamide have proved useful in the treatment of hyperplasia and cancer of the prostate.

The effectiveness of known modulators of steroid receptors is often tempered by their undesired side-effect profile, particularly during long-term administration. For example, the effectiveness of progesterone and estrogen agonists, such as norgestrel and diethylstilbesterol respectively, as female birth control agents must be weighed against the increased risk of breast cancer and heart disease to women taking such agents. Similarly, the progesterone antagonist, mifepristone (RU486), if administered for chronic indications, such as uterine fibroids, endometriosis and certain hormone-dependent cancers, could lead to homeostatic imbalances in a patient due to its inherent cross-reactivity as a GR antagonist. Accordingly, identification of compounds which have good specificity for one or more steroid receptors, but which have reduced or no cross-reactivity for other steroid or intracellular receptors, would be of significant value in the treatment of male and female hormone responsive diseases.

A group of quinoline analogs having an adjacent polynucleic ring system of the indene or fluorene series or an adjacent polynucleic heterocyclic ring system with substituents having a nonionic character have been described as photoconductive reducing agents, stabilizers, laser dyes and antioxidants. See e.g., U.S. Patent Nos. 3,798,031; 3,830,647; 3,832,171; 3,928,686; 3,979,394; 4,943,502 and 5,147,844 as well as Soviet Patent No. 555,119; R.L. Atkins and D.E. Bliss, "Substituted Coumarins and Azacoumarins: Synthesis and Fluorescent Properties", 43 *J. Org. Chem.*, 1975 (1978), E.R. Bissell et al., "Synthesis and Chemistry of 7-Amino-4-(trifluoromethyl)coumarin and Its Amino Acid and Peptide Derivatives", 45 *J. Org. Chem.*, 2283 (1980) and G.N. Gromova and K.B. Piotrovskii, "Relative Volatility of Stabilizers for Polymer Materials," 43 *Khim. Prom-st.*, 97 (Moscow, 1967). However, no biological activity of any kind has been ascribed to these compounds.

The European patent application EP 0 260 744 A2 discloses novel imidazole derivatives of the formula

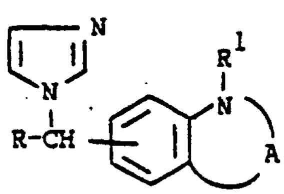

that have the following pharmacological properties:

They inhibit androgen formation from $C_{21}$-steroids, such as pregnenolone and prostagens, and can hence be used in the treatment of androgen dependant disorders. Further, some of the compounds of the invention show the capability to increase the excretion of ureic acid, thus causing a decrease of the ureic acid levels in the plasma and as such utility is indicated in various diseases which are related to increased levels of ureic acid, e. g. gout.

Compounds of the formula

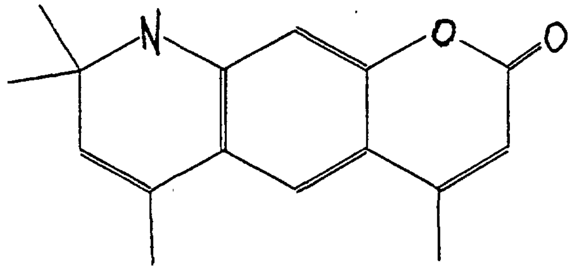

can be prepared from 3-amino-phenol and acetoacetic acid ethyl ester at 150 °C as it is disclosed in Atkins, R. L.; Bliss, D. E., JOCEAH, J. Org. Chem., EN, 43 [1978], 1975-1980.

## Summary of the Invention

The present invention is directed to compounds, pharmaceutical compositions, and methods for modulating processes mediated by steroid receptors. More particularly, the invention relates to non-steroidal compounds and compositions which are high affinity, high specificity agonists, partial agonists and antagonists for the AR, steroid receptors. Also provided are methods of making such compounds and pharmaceutical compositions, as well as critical intermediates used in their synthesis.

These and various other advantages and features of novelty which characterize the invention are pointed out with particularity in the claims annexed hereto and forming a part hereof. However, for a better understanding of the invention, its advantages, and objects obtained by its use, reference should be had to the accompanying drawings and descriptive matter, in which there is illustrated and described preferred embodiments of the invention.

## Definitions and Nomenclature

As used herein, the following terms are defined with the following meanings, unless explicitly stated otherwise. Furthermore, in an effort to maintain consistency in the naming of compounds of similar structure but differing substituents, the compounds described herein are named according to the following general guidelines. The numbering system for the location of substituents on such compounds is also provided.

The term alkyl, alkenyl, alkynyl and allyl includes straight-chain, branched-chain, cyclic, saturated and/or unsaturated structures, and combinations thereof.

The term aryl refers to an optionally substituted six-membered aromatic ring, including polyaromatic rings.

The term heteroaryl refers to an optionally substituted five-membered heterocyclic ring containing one or more heteroatoms selected from the group consisting of carbon, oxygen, nitrogen and sulfur, including polycyclic rings, or a six-membered heterocyclic ring containing one or more heteroatoms selected from the group consisting of carbon and nitrogen, including polycyclic rings.

A **quinoline** is defined by the following structure, and may be recognized as a benzannulated pyridine. Compounds of structures **4, 5, 13, 79, 83** and **86** herein are named as quinolines.

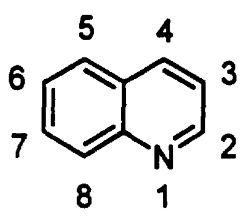

An **indeno[1,2-*g*]quinoline** is defined by the following structure. Compounds of structures **16** (X=C) and **20** herein are named as indeno[1,2-*g*]quinolines.

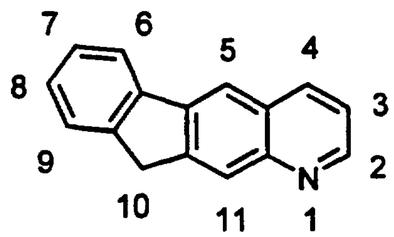

An **indeno[2,1-*f*]quinoline** is defined by the following structure. Compounds of structure **17** (X=C) herein are named as indeno[1,2-*f*]quinolines.

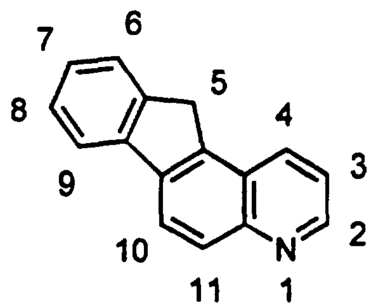

A **benzo[b]furano[3,2-*g*]quinoline** is defined by the following structure. Compounds of structure **16** (X=O) herein are named as benzo[b]furano[3,2-*g*]quinolines.

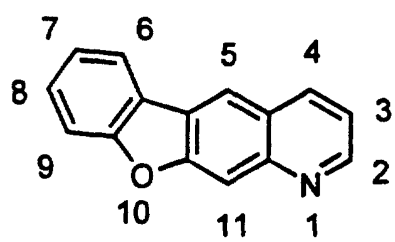

A **benzo[b]furano[2,3-*f*]quinoline** is defined by the following structure. Compounds of structure **17** (X=O) herein are named as benzo[b]furano[2,3-*f*]quinolines.

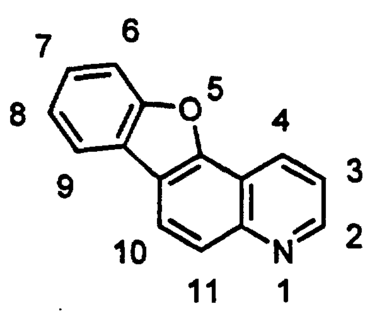

An **indolo[3,2-*g*]quinoline** is defined by the following structure. Compounds of structure **16** (X=N) herein are named as indolo[3,2-*g*]quinolines.

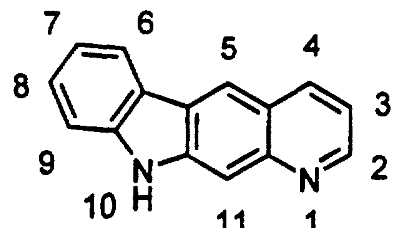

An **indolo[2,3-*f*]quinoline** is defined by the following structure. Compounds of structures **17** (X=N) and **29** herein are named as indolo[2,3-*f*]quinolines.

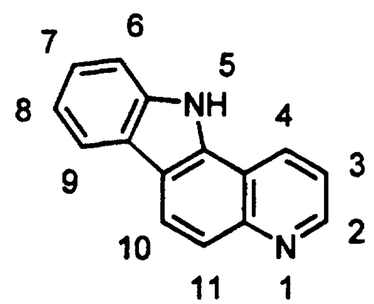

A **coumarino[3,4-*f*]quinoline** is defined by the following structure. Compound **159** and compounds of structures **41** and **88** herein are named as coumarino[3,4-*f*]quinolines.

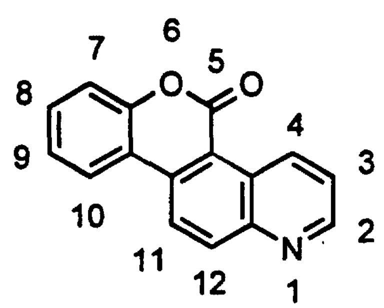

A ***5H*-chromeno[3,4-*f*]quinoline** is defined by the following structure. Compounds of structures **34, 35, 42, 45 to 54, 93, 95, 97 to 99, 1A, 4A, 7A to 11A, 17A to 19A and 25A to 27A** herein are named as coumarino[3,4-*f*] quinolines.

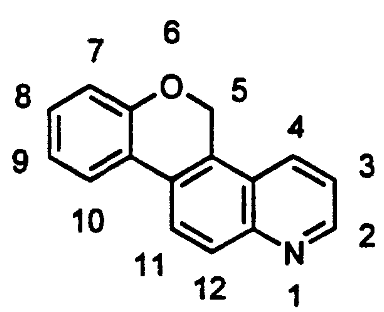

An **8-pyranono[5,6-*g*]quinoline** is defined by the following structure. Compounds of structures **57** (Y=O), **60** (Y=O), **63** (Y=O), **69** (Y=O), **73** (Y=O), **28A** (Y=O), **33A, 34A, 37A** (X=O), **38A** (X=O), **40A** (X=O), **41A** (X=O), **45A, 65A** (X=O) **and 67A** (X=O) herein are named as 8-pyranono[5,6-*g*]quinolines.

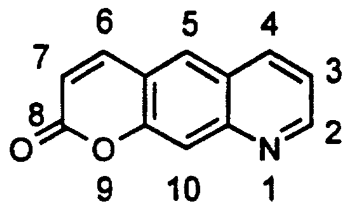

A **10-isocoumarino[4,3-*g*]quinoline** is defined by the following structure. Compounds of structures **57** ($R^2$=$R^3$=benzo, Y=O), **60** ($R^2$=$R^3$=benzo, Y=O), and **63** ($R^2$=$R^3$=benzo, Y=O) herein are named as 10-isocoumarino [4,3-g]quinolines.

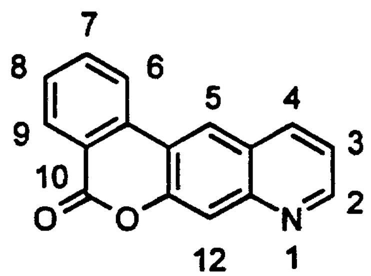

A **10-isoquinolino[4,3-*g*]quinoline** is defined by the following structure. Compounds of structures **57** ($R^2$=$R^3$=benzo, Y=NH), **60** ($R^2$=$R^3$=benzo, Y=NH), and **63** ($R^2$=$R^3$=benzo, Y=NH) herein are named as 10-isoquinolino [4,3-*g*]quinolines.

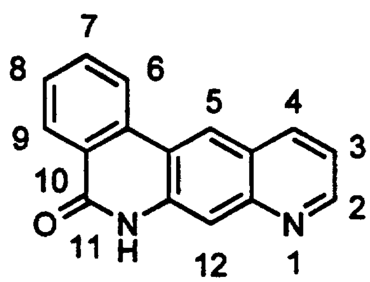

An **8-pyridono[5,6-*g*]quinoline** is defined by the following structure. Compounds of structures **57** (Y=N), **60** (Y=N), **63** (Y=N), **69** (Y=N), **73** (Y=N), **28A** (Y=N), **37A** (X=N), **38A** (X=N), **40A** (X=N), **41A** (X=N), **47A, 53A, 62A, 63A, 65A** (X=N), **67A** (X=N), **70A, 72A, 74A, 79A, 80A, 81A and 84A** herein are named as 8-pyridono[5,6-g]quinolines.

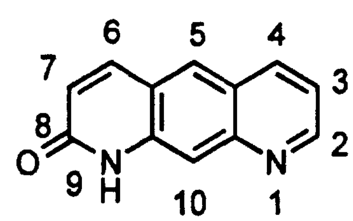

A **10*H*-isochromeno[4,3-*g*]quinoline** is defined by the following structure. Compounds of structures **61** ($R^2$=$R^3$=benzo, Y=O) and **62** ($R^2$=$R^3$=benzo, Y=O) herein are named as 10*H*-isochromeno[4,3-*g*]quinolines.

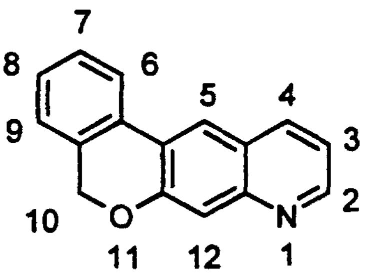

An **8*H*-pyrano[3,2-*g*]quinoline** is defined by the following structure. Compounds of structures **61** (Y=O) and **62** (Y=-O) herein are named as 8*H*-pyrano[3,2-*g*]quinolines.

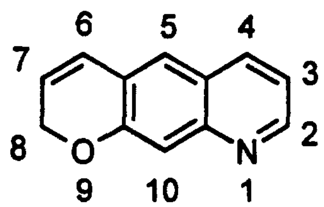

A **10-thioisoquinolino[4,3-*g*]quinoline** is defined by the following structure. Compounds of structures **58** ($R^2$=$R^3$=benzo, Y=NH) and **76** ($R^2$=$R^3$=benzo, Y=NH) herein are named as 10-thioisoquinolino[4,3-g]quinolines.

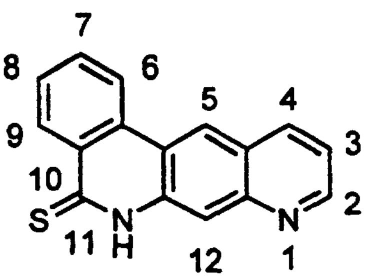

A **9-pyrido[3,2-*g*]quinoline** is defined by the following structure. Compounds of structures **71** (Y=N) and **75** (Y=N) herein are named as 9-pyrido[3,2-g]quinolines.

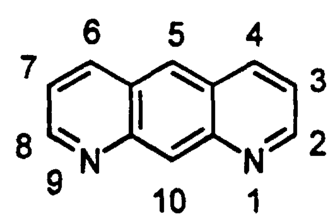

An **8-thiopyranono[5,6-*g*]quinoline** is defined by the following structure. Compounds of structures **58** (Y=O), **76** (Y=O) **and 29A** (Y=O) herein are named as 8-thiopyranono[5,6-*g*]quinolines.

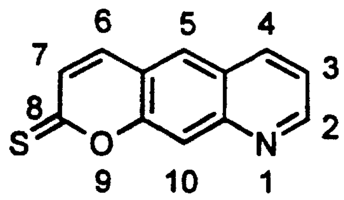

An **6-pyridono[5,6-*g*]quinoline** is defined by the following structure. Compounds of structures **70** (Y=N) and **74** (Y=N) herein are named as 6-pyridono[5,6-*g*]quinolines.

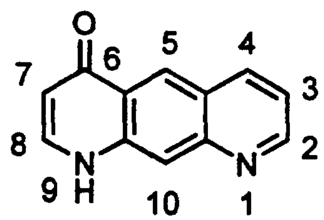

A **9-thiopyran-8-ono[5,6-*g*]quinoline** is defined by the following structure. Compounds of structure **57** (Y=S), **28A** (Y=S), **37A** (X=S), **38A** (X=S), **40A** (X=S), **41A** (X=S), **65A** (X=S) and **67A** (X=S) herein are named as 9-thiopyran-8-ono[5,6-*g*]quinolines.

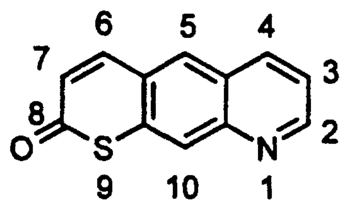

An **7-pyridono[5,6-*f*]indoline** is defined by the following structure. Compounds of structures **49A, 50A, 57A,** and **83A** are named as 7-pyridono[5,6-*f*]indolines.

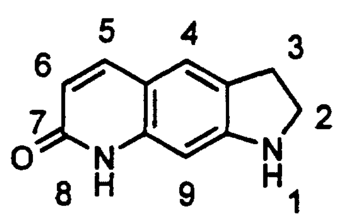

An **5H-isochromeno[3,4-*f*]quinoline** is defined by the following structure. Compounds of structures **22A, 23A** and **24A** are named as 5H-isochromeno[3,4-*f*]quinolines.

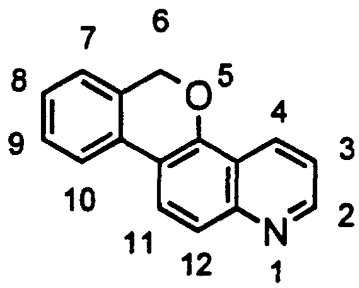

## Detailed Description of Embodiments of the Invention

Compounds of the present invention are defined as those having the formulae:

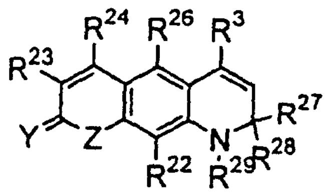

(IX)

OR

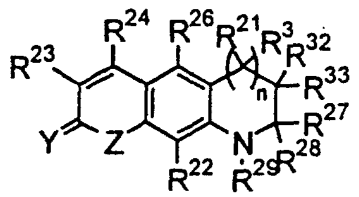

(XVII)

OR

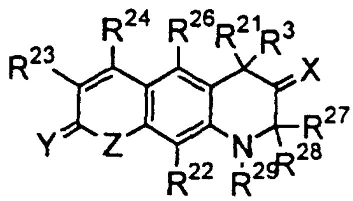

(XVIII)

wherein:

$R^3$ is hydrogen, a $C_1$ - $C_4$ alkyl or perfluoroalkyl, hydroxymethyl,

$R^7$ is hydrogen, a $C_1$ - $C_4$ alkyl or perfluoroalkyl, $OR^8$ or $NHR^8$, where $R^8$ is hydrogen, a $C_1$ - $C_6$ alkyl or perfluoroalkyl,

$SO_2R^2$ or $S(O)R^2$;

X is $CH_2$, O, S or $NR^7$, where $R^7$ has the definition given above;

$R^{21}$ is hydrogen, or a $C_1$ - $C_4$ alkyl

$R^{22}$ is hydrogen, a $C_1$ - $C_4$ alkyl, F, Cl, Br, I, $OR^2$, $NR^2R^7$ or $SR^2$, where $R^2$ and $R^7$ have the definitions given above;

$R^{23}$ is hydrogen, Cl, Br, F, $OR^8$, $NR^2R^7$ or a $C_1$ - $C_4$ alkyl or perhaloalkyl,

$R^{24}$ is hydrogen, F, Br, Cl, a $C_1$ - $C_4$ alkyl or perhaloalkyl, a phenyl ring or a five-membered heterocyclic ring containing one or more heteroatoms selected from the group consisting of oxygen, nitrogen and sulfur, or a six-membered heterocyclic ring containing one or more nitrogen heteroatoms, $CF_3$, $CF_2OR^{25}$, $CH_2OR^{25}$, or $OR^{25}$, where $R^{25}$ is a $C_1$ - $C_4$ alkyl, except that $R^{24}$ cannot be $CH_3$ when Z is O, $R^{22}$, $R^{23}$, and $R^{29}$ are all hydrogen and $R^3$, $R^{27}$ and $R^{28}$ all are $CH_3$;

$R^{27}$ and $R^{28}$ each independently are hydrogen or, a $C_1$ - $C_4$ alkyl or perfluoroalkyl,

$R^{29}$ is hydrogen, or a $C_1$ - $C_6$ alkyl

$R^{32}$ and $R^{33}$ each independently are hydrogen or, a $C_1$ - $C_4$ alkyl

n is 0 or 1;

Y is O or S;

Z is O, S, NH, $NR^2$ or $NCOR^2$, where $R^2$ has the same definition given above;

Preferably, the compounds of formulae IX, XVII and XVIII comprise AR modulators (i.e., both AR agonists and antagonists). More preferably, the compounds of formulae IX and XVII comprise AR antagonists.

The present invention provides a pharmaceutical composition comprising an effective amount of a steroid receptor modulating compound of the formulae:

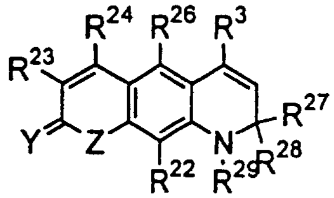

(IX)

OR

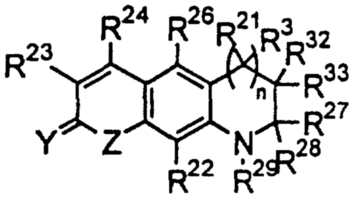

(XVII)

OR

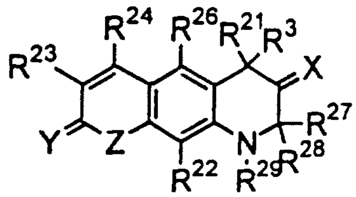

(XVIII)

wherein:

$R^2$ is hydrogen, a $C_1$ - $C_4$ alkyl or perfluoroalkyl,

$R^3$ is hydrogen, a $C_1$ - $C_4$ alkyl or perfluoroalkyl, hydroxymethyl,

$R^7$ is hydrogen, a $C_1$ - $C_4$ alkyl or perfluoroalkyl, $OR^8$ or $NHR^8$, where $R^8$ is hydrogen, a $C_1$ - $C_6$ alkyl or perfluoroalkyl, aryl, heteroaryl or optionally substituted allyl, arylmethyl, $SO_2R^2$ or $S(O)R^2$;

X is $CH_2$, O, S or $NR^7$, where $R^7$ has the definition given above;

$R^{21}$ is hydrogen, a $C_1$ - $C_4$ alkyl

$R^{22}$ is hydrogen, a $C_1$ - $C_4$ alkyl, F, Cl, Br, I, $OR^2$, $NR^2R^7$ or $SR^2$, where $R^2$ and $R^7$ have the definitions given above;

$R^{23}$ is hydrogen, Cl, Br, F, $OR^8$, $NR^2R^7$, a $C_1$ - $C_4$ alkyl or perhaloalkyl, where $R^2$, $R^7$ and $R^8$ have the definitions given above;

$R^{24}$ is hydrogen, F, Br, Cl, a $C_1$ - $C_4$ alkyl or perhaloalkyl, a phenyl ring or a five-membered heterocyclic ring containing one or more heteroatoms selected from the group consisting of oxygen, nitrogen and sulfur, or a six-membered heterocyclic ring containing one or more nitrogen heteroatoms, $CF_3$, $CF_2OR^{25}$, $CH_2OR^{25}$, or $OR^{25}$, where $R^{25}$ is a $C_1$ - $C_4$ alkyl, except that $R^{24}$ cannot be $CH_3$ when Z is O, $R^{22}$, $R^{23}$, and $R^{29}$ are all hydrogen and $R^3$, $R^{27}$ and $R^{28}$ all are $CH_3$;

$R^{27}$ and $R^{28}$ each independently are hydrogen, a $C_1$ - $C_4$ alkyl or perfluoroalkyl, heteroaryl,

$R^{29}$ is hydrogen, a $C_1$ - $C_6$ alkyl

$R^{32}$ and $R^{33}$ each independently are hydrogen, a $C_1$ - $C_4$ alkyl

n is 0 or 1;

Y is O or S;

Z is O, S, NH, $NR^2$ or $NCOR^2$, where $R^2$ has the same definition given above;

a pharmaceutically acceptable carrier.

Preferably, the compounds of formulae IX, XVII and XVIII comprise AR modulators (i.e., both AR agonists and antagonists). More preferably, the compounds of formulae IX and XVII comprise AR antagonists.

In a further preferred aspect, the present invention comprises a method of modulating processes mediated by steroid receptors comprising administering to a patient an effective amount of a compound of the formulae IX, XVII or XVIII shown above, wherein $R^2$ through $R^{33}$, W, X, Y and Z all have the same definitions as those given above for the preferred pharmaceutical composition of the present invention.

Any of the compounds of the present invention can be synthesized as pharmaceutically acceptable salts for incorporation into various pharmaceutical compositions. As used herein, pharmaceutically acceptable salts include, but are not limited to, hydrochloric, hydrobromic, hydroiodic, hydrofluoric, sulfuric, citric, maleic, acetic, lactic, nicotinic,

succinic, oxalic, phosphoric, malonic, salicylic, phenylacetic, stearic, pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and tris(hydoxymethyl)aminomethane. Additional pharmaceutically acceptable salts are known to those skilled in the art.

AR agonist, partial agonist and antagonist compounds of the present invention will prove useful in the treatment of acne, male-pattern baldness, male hormone replacement therapy, wasting diseases, hirsutism, stimulation of hematopoiesis, hypogonadism, prostatic hyperplasia, various hormone-dependent cancers, including, without limitation, prostate and breast cancer and as anabolic agents.

It will be understood by those skilled in the art that while the compounds of the present invention will typically be employed as a selective agonists, partial agonists or antagonists, that there may be instances where a compound with a mixed steroid receptor profile is preferred. For example, use of a PR agonist (i.e., progestin) in female contraception often leads to the undesired effects of increased water retention and acne flare ups. In this instance, a compound that is primarily a PR agonist, but also displays some AR and MR modulating activity, may prove useful. Specifically, the mixed MR effects would be useful to control water balance in the body, while the AR effects would help to control any acne flare ups that occur.

Furthermore, it will be understood by those skilled in the art that the compounds of the present invention, including pharmaceutical compositions and formulations containing these compounds, can be used in a wide variety of combination therapies to treat the conditions and diseases described above. Thus, the compounds of the present invention can be used in combination with other hormones and other therapies, including, without limitation, chemotherapeutic agents such as cytostatic and cytotoxic agents, immunological modifiers such as interferons, interleukins, growth hormones and other cytokines, hormone therapies, surgery and radiation therapy.

Representative PR antagonist compounds according to the present invention include:

Representative AR modulator compounds (i.e., agonists and antagonists) according to the present invention include: 1,2-Dihydro-2,2,4-trimethyl-6-methoxymethyl-8-pyranono[5,6-*g*]quinoline (Compound 237); 1,2-Dihydro-2,2,4-trimethyl-6-trifluoromethyl-8-pyranono[5,6-g]quinoline (Compound 238); 1,2-Dihydro-2,2,4-trimethyl-10-isocoumarino[4,3-*g*]quinoline (Compound 239); 1,2-Dihydro-2,2,4-trimethyl-10-isoquinolono[4,3-*g*]quinoline(Compound 240); 1,2-Dihydro-2,2,4,6-tetramethyl-8-pyridono[5,6-*g*]quinoline (Compound 241); 1,2-Dihydro-10-hydroxy-2,2,4-trimethyl-10*H*-isochromeno[4,3-*g*]quinoline (Compound 242); 1, 2-Dihydro-2,2,4,6-tetramethyl-8H-pyrano[3,2-*g*]quinoline (Compound 243); (*R*/*S*)-1,2,3,4-Tetrahydro-2,2,4-trimethyl-10-isoquinolono[4,3-*g*]quinoline (Compound 244); 1,2-Dihydro-2,2,4-trimethyl-10-thioisoquinolono[4,3-*g*]quinoline (Compound 245); (+)-1,2,3,4-Tetrahydro-2,2,4-trimethyl-10-isoquinolono[4,3-*g*]quinoline (Compound 246); 1,2-Dihydro-2,2,4-trimethyl-6-trifluoromethyl-8-pyridono[5,6-*g*] quinoline (Compound 247); (*R*/*S*)-1,2,3,4-Tetrahydro-2,2,4-trimethyl-6-trifluoromethyl-8-pyranono[5,6-*g*]quinoline (Compound 250); 1,2-Dihydro-2,2,4-trimethyl-6-trifluoromethyl-8-thiopyranono[5,6-g]quinoline (Compound 251); (*R*/*S*)-1,2,3,4-Tetrahydro-2,2,4-trimethyl-6-trifluoromethyl-8-thiopyranono[5,6-*g*]quinoline (Compound 252); 6-Chloro(difluoro)methyl-1,2-dihydro-2,2,4-trimethyl-8-pyranono[5,6-*g*]quinoline (Compound 253); 9-Acetyl-1,2-dihydro-2,2,4-trimethyl-6-trifluoromethyl-8-pyridono[5,6-*g*]quinoline (Compound 254); 1,2-Dihydro-2,2,4,10-tetramethyl-6-trifluoromethyl-8-pyridono[5,6-*g*]quinoline (Compound 255); 1,2-Dihydro-2,2,4-trimethyl-6-(1,1,2,2,2-pentafluoroethyl)-8-pyranono[5,6-g]quinoline (Compound 256); (*R*/*S*)-6-Chloro(difluoro)methyl-1,2,3,4-tetrahydro-2,2,4-trimethyl-8-pyranono [5,6-g]quinoline (Compound 257); 7-Chloro-1,2-dihydro-2,2,4-trimethyl-6-trifluoromethyl-8-pyranono[5,6-*g*]quinoline (Compound 258); (*R*/*S*)-7-Chloro-1,2,3,4-tetrahydro-2,2,4-trimethyl-6-trifluoromethyl-8-pyranono[5,6-*g*]quinoline (Compound 259); 1,2,3,4-Tetrahydro-2,2,4-trimethyl-6-trifluoromethyl-8-pyridono[5,6-*g*]quinoline (Compound 260); 1,2-Dihydro-2,2,4,9-tetramethyl-6-trifluoromethyl-8-pyridono[5,6-*g*]quinoline (Compound 261); 1,2-Dihydro-2,2,4-trimethyl-8-trifluoromethyl-6-pyridono[5,6-*g*]quinoline (Compound 262); 6-[Dichloro(ethoxy)methyl]-1,2-dihydro-2,2,4-trimethyl-8-pyranono[5,6-*g*]quinoline (Compound 263); 5-(3-Furyl)-1,2-dihydro-2,2,4-trimethyl-8-pyranono [5,6-*g*]quinoline (Compound 264); 1,2-Dihydro-1,2,2,4-tetramethyl-6-trifluoromethyl-8-pyranono[5,6-g]quinoline (Compound 265); 1,2-Dihydro-6-trifluoromethyl-2,2,4-trimethyl-9-thiopyran-8-ono[5,6-*g*] quinoline (Compound 266); 1,2-Dihydro-1,2,2,4,9-pentamethyl-6-trifluoromethyl-8-pyridono[5,6-*g*]quinoline (Compound 267); 7-Chloro-1,2-dihydro-2,2,4-trimethyl-6-trifluoromethyl-8-pyridono[5,6-*g*]quinoline (Compound 268); and 6-Chloro(difluoro)methyl-1,2-dihydro-2,2,4-trimethyl-8-pyridono[5,6-*g*]quinoline (Compound 269); (*R*/*S*)-1,2,3,4-Tetrahydro-1,2,2,4-tetramethyl-6-trifluoromethyl-8-pyranono[5,6-g]quinoline (Compound 404); (*R*/*S*)-5-(3-Furyl)-1,2,3,4-tetrahydro-2,2,4-trimethyl-8-pyranono[5,6-*g*]quinoline (Compound 405); 5-(3-Furyl)-1,2-dihydro-1,2,2,4-tetramethyl-8-pyranono[5,6-g]quinoline (Compound 406); 5-(3-Furyl)-1,2-dihydro-1,2,2,4-tetramethyl-8-thiopyranono[5,6-*g*]quinoline (Compound 407); 6-Chloro-5-(3-furyl)-1,2-dihydro-1,2,2,4-tetramethyl-8-pyranono[5,6-*g*]quinoline (Compound 408); 1,2,3,4-Tetrahydro-2,2,4,10-tetramethyl-6-trifluoromethyl-8-pyridono[5,6-*g*]quinoline (Compound 409); (*R*/*S*)-1,2,3,4-Tetrahydro-4-methyl-6-trifluoromethyl-8-pyranono[5,6-*g*]quinoline (Compound 410); 1,2-Dihydro-2,2-dimethyl-6-trifluoromethyl-8-pyranono[5,6-*g*]quinoline (Compound 411); 1,2,3,4-Tetrahydro-2,2-dimethyl-6-trifluoromethyl-8-pyranono[5,6-*g*]quinoline (Compound 412); 1,2,3,4-Tetrahydro-6-trifluoromethyl-8-pyranono[5,6-*g*]quinoline (Compound 413); (*R*/*S*)-4-Ethyl-1,2,3,4-tetrahydro-6-trifluoromethyl-8-pyranono[5,6-*g*]quinoline (Compound 414); (*R*/*S*)-1,2,3,4-Tetrahydro-1,4-dimethyl-8-pyranono[5,6-*g*]quinoline (Compound 415); (*R*/*S*)-4-Ethyl-1,2,3,4-tetrahydro-1-methyl-8-pyranono [5,6-*g*]quinoline (Compound 416); 2,2-Dimethyl-1,2,3,4-tetrahydro-6-trifloromethyl-8-pyridono[5,6-*f*]quinoline (Compound 417); (*R*/*S*)-1,2,3,4-tetrahydro-6-trifluoromethyl-2,2,4-trimethyl-8-pyridono[5,6-*f*]-3-quinolinone (Compound 418); 5-Trifluoromethyl-7-pyridono[5,6-*e*]indoline (Compound 419); 8-(4-Chlorobenzoyl)-5-trifluoromethyl-7-pyridono [5,6-*e*]indoline (Compound 420); 7-*tert*-Butyloxycarbamoyl-1,2-dihydro-2,2,8-trimethylquinoline (Compound 421); 1,2,3,4-Tetrahydro-6-trifluoromethyl-8-pyridono[5,6-*f*]quinoline (Compound 422); 1,2-Dihydro-6-trifluoromethyl-1,2,2,4-tetramethyl-8-pyridono[5,6-*f*]quinoline (Compound 423); 3,3-Dimethyl-5-trifluoromethyl-7-pyridono[5,6-*e*]indoline (Compound 424); (*R*/*S*)-1,2,3,4-Tetrahydro-4-methyl-6-(trifluoromethyl)-8-pyridono[5,6-*g*]quinoline (Compound 425); (*R*/*S*)-1,2,3,4-Tetrahydro-4-methyl-6-(trifluoromethyl)-8-pyridono[5,6-*g*]quinoline (Compound 426); 1,2,2,-Trimethyl-1,2,3,4-tetrahydro-6-trifluromethyl-8-pyranono[5,6-g]quinoline (Compound 427); (*R*/*S*)-1,2,3,4-Tetrahydro-4-propyl-6-trifluoromethyl-8-pyranono[5,6-*g*]quinoline (Compound 428); 1,2,3,4-Tetrahydro-2,2,4-trimethyl-6-trifluoromethyl-9-thiopyran-8-ono[5,6-*g*]quinoline (Compound 429); 1,2-Dihydro-1,2,2,4-tetramethyl-6-trifluoromethyl-9-thiopyran-8-ono[5,6-*g*]quinoline (Compound 430); 1,2,3,4-Tetrahydro-1,2,2-trimethyl-6-trifluoromethyl-8-pyridono[5,6-*g*] quinoline (Compound 431); 1,2,3,4-Tetrahydro-1-methyl-4-propyl-6-trifluoromethyl-8-pyranono[5,6-*g*]quinoline (Compound 432); 1,2,3,4-Tetrahydro-10-hydroxymethyl-2,2,4-trimethyl-6-trifluoromethyl-8-pyridono[5,6-*g*]quinoline (Compound 433); 1,2,3,4-Tetrahydro-1,2,2,4-tetramethyl-6-trifluoromethyl-9-thiopyran-8-ono[5,6-*g*]quinoline (Compound 434); 1,2,3,4-Tetrahydro-2,2,9-trimethyl-6-trifluoromethyl-8-pyridono[5,6-*g*]quinoline (Compound 435); (*R*/*S*)-1,2,3,4-Tetrahydro-3-methyl-6-trifluoromethyl-8-pyridono[5,6-*g*]quinoline (Compound 436); 1,2,3,4-Tetrahydro-3,3-dimethyl-6-trifluoromethyl-8-pyridono[5,6-*g*]quinoline (Compound 437); (*R*/*S*)1,2,3,4-Tetrahydro-2,2,3-trimethyl-6-trifluoromethyl-8-pyridono[5,6-g]quinoline (Compound 438); (*R*/*S*-2*l*,4*u*)-1,2,3,4-Tetrahydro-2,4-dimethyl-6-trifluoromethyl-8-pyridono[5,6-*g*]quinoline (Compound 439); (*R*/*S*-2*l*,4*u*)-4-Ethyl-1,2,3,4-tetrahydro-2-methyl-6-trifluoromethyl-8-pyranono[5,6-*g*]quinoline (Compound 440); (*R*/*S*-2*l*,3*u*)-1,2,3,4-Tetrahydro-2,3-dimethyl-6-trifluoromethyl-8-pyridono[5,6-*g*]quinoline (Compound 441); (*R*/*S*-2*l*,3*l*)-1,2,3,4-Tetrahydro-2,3-dimethyl-6-trifluoromethyl-8-pyridono [5,6-*g*]quinoline (Compound 442); (*R*/*S*)-1,2,3,4-Tetrahydro-2,3,3-trimethyl-6-trifluoromethyl-8-pyridono[5,6-*g*]quinoline (Compound 443); (*R*/*S*)-1,2,3,4-Tetrahydro-2-methyl-6-trifluoromethyl-8-pyridono[5,6-*g*]quinoline (Compound 444); (*R*/*S*)-4-Ethyl-1,2,3,4-tetrahydro-6-trifluoromethyl-8-pyridono[5,6-*g*]quinoline (Compound 445); (*R*/*S*-2*l*, 3*u*)-1,2,3,4-Tetrahydro-2,3,9-trimethyl-6-trifluoromethyl-8-pyridono[5,6-*g*]quinoline (Compound 446); (*R*/*S*)-1,2,3,4-Tetrahydro-4-propyl-6-trifluoromethyl-8-pyridono[5,6-g]quinoline (Compound 447); (*R*/*S*)-3-Ethyl-1,2,3,4-tetrahydro-2,2-dimethyl-6-trifluoromethyl-8-pyridono[5,6-*g*]quinoline (Compound 448); (*R*/*S*)-1,2,3,4-Tetrahydro-2,2-dimethyl-6-trifluoromethyl-3-propyl-8-pyridono[5,6-*g*]quinoline (Compound 449); and 1-Methyl-5-trifluoromethyl-7-pyridono [5,6-*f*]indoline (Compound 450).

Compounds of the present invention, comprising classes of quinoline compounds and their derivatives, that can be obtained by routine chemical synthesis by those skilled in the art, e.g., by modification of the quinoline compounds disclosed or by a total synthesis approach.

The sequence of steps for several general schemes to synthesize the compounds of the present invention are shown below. In each of the Schemes the R groups (e.g., $R^1$, $R^2$, etc...) correspond to the specific substitution patterns noted in the Examples. However, it will be understood by those skilled in the art that other functionalities disclosed herein at the indicated positions of compounds of formulas IX, XVII and XVIII also comprise potential substituents for the analogous positions on the structures within the Schemes.

**Scheme XVI**

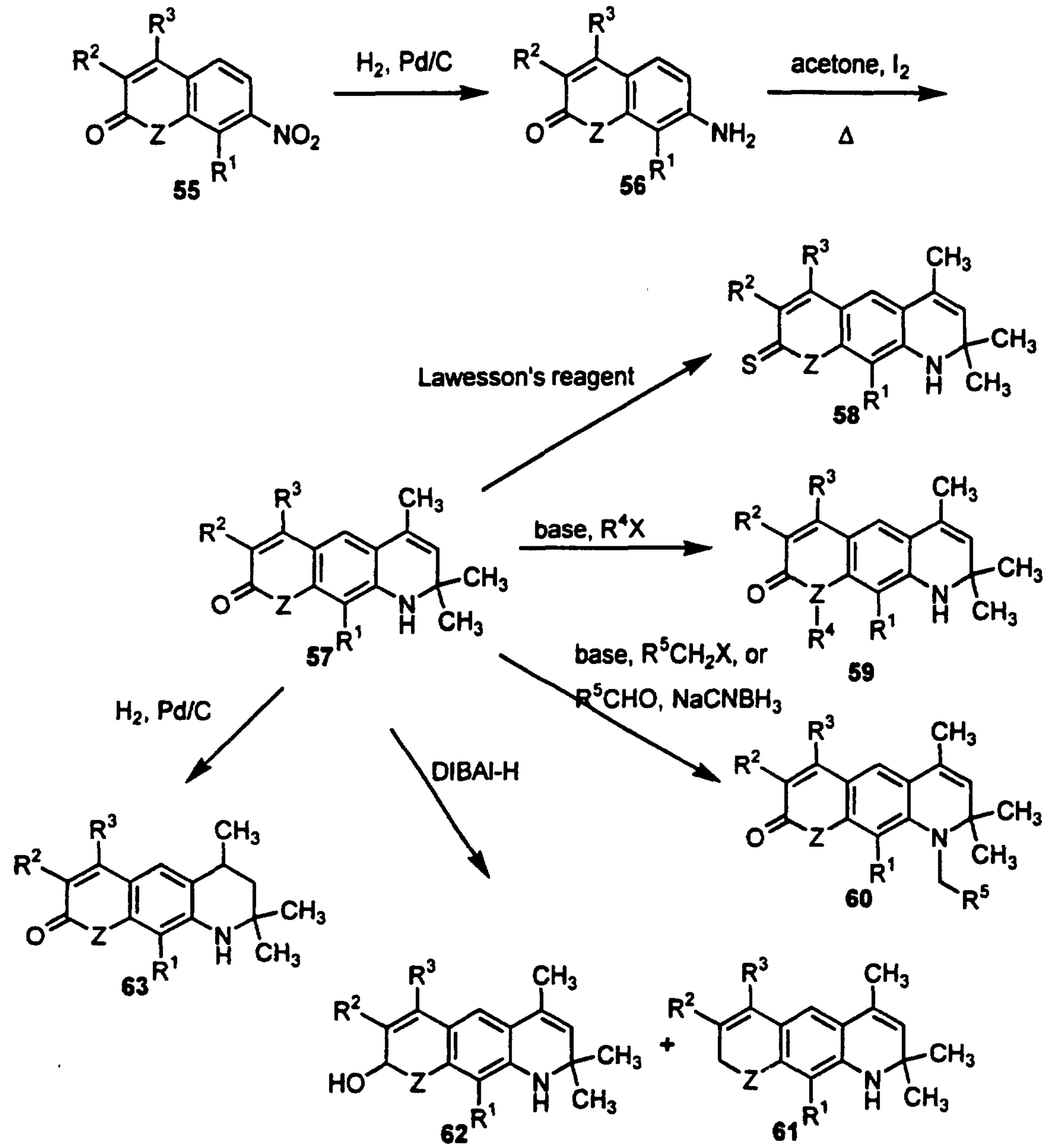


The process of **Scheme XVI** begins with the reduction of a nitro aromatic compound of structure **55** with, for example, hydrogen over a metal catalyst such as palladium on carbon. Treatment of an aniline of structure **56** with acetone and a catalyst such as iodine affords a compound of structure **57**. A compound of structure **57** may be converted to the corresponding thio-compound (structure **58**) by treatment with Lawesson's reagent [2,4-bis(4-methoxyphenyl)-1,3-dithia-2,4-diphosphetane-2,4-disulfide]. See B.S. Pedersen, S. Scheibye, K. Clausen and S.O. Lawesson, "Studies on Organophosphorus Compounds. XXII. The Dimer of *p*-Methoxyphenylthionophos-phine sulfide as Thiation Reagent. A New Route to *O*-Substituted Thioesters and Dithioesters", *Bull. Soc. Chim. Belg.* **1978,** *87*, 293, the disclosure of which is herein incorporated by reference.

Alternatively, N(9) of a compound of structure **57** (Y=N) may be alkylated by deprotonation with a strong base, for example, sodium hydride, followed by alkylation with an alkylating agent such as iodomethane.

Alternatively, N(1) of a compound of structure **57** (Y=O) may be alkylated by deprotonation with a strong base, for example, sodium hydride, followed by alkylation with an alkylating agent, for example, iodomethane, to afford a compound of structure **60**. In addition, N(1) of a compound of structure **57** (Y=O) may be alkylated by treatment with an aldehyde or paraformaldehyde in the presence of sodium cyanoborohydride and acetic acid. See R.O. Hutchins and N.R. Natale, "Cyanoborohydride. Utility and Applications in Organic Synthesis. A Review", *Org. Prep. Proced. Int.*

**1979**, *11*, 201, the disclosure of which is herein incorporated by reference.

Alternatively, the C(8) ester group of a compound of structure **57** (Y=O) may be reduced with a metal hydride, for example, diisobutylaluminum hydride, to afford one or both of two compounds (structures **61** and **62**).

Alternatively, the C(3)-C(4) olefin of a compound of structure **57** may be reduced with, for example, hydrogen over a metal catalyst such as palladium on carbon, to afford the 1,2,3,4-tetrahydroquinoline (structure **63**).

**Scheme XVII**

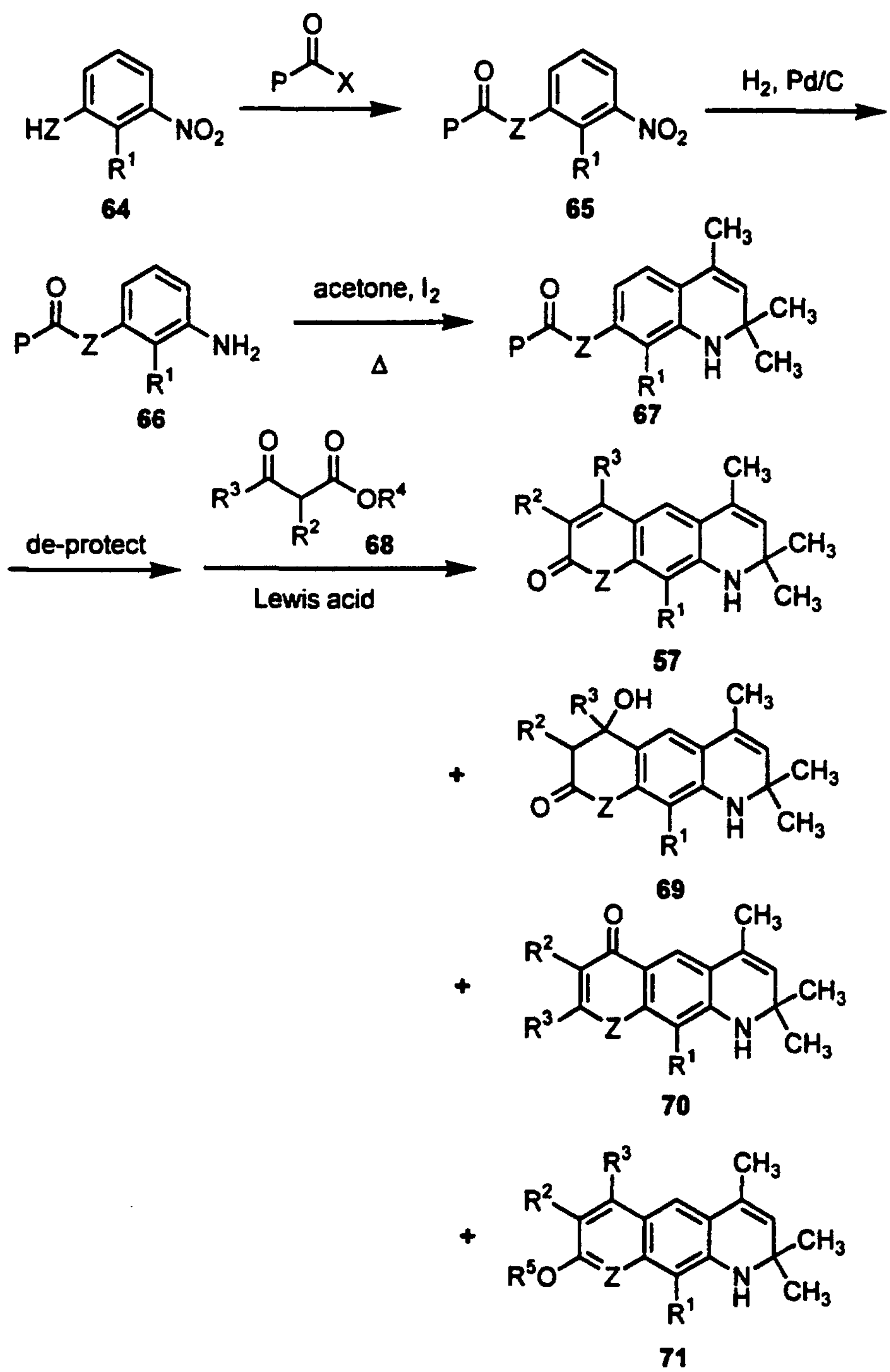


**64** **65** **66** **67** **57** **69** **70** **71**

The process of **Scheme XVII** begins with the acylation of a 3-nitrophenol (structure **64,** Y=O) or 3-nitroaniline (structure **64,** Y=NH) with an acylating agent, for example, di-*tert*-butyl dicarbonate or trimethylacetyl chloride, to afford a compound of structure **65.** Reduction of the nitro group with, for example, hydrogen over a metal catalyst such as palladium on carbon, affords the corresponding aniline (structure **66**). Treatment of a compound of structure **66** with

acetone and a catalyst such as iodine affords a compound of structure **67**. Deprotection by either acid or base, followed by treatment of the corresponding aniline or phenol with a β-keto ester (structure **68**) in the presence of a Lewis acid such as zinc chloride, affords one or more of four compounds (structures **57, 69, 70,** and **71**). The cyclization of a phenol as described above is known as a Pechmann reaction. See S. Sethna and R. Phadke, "The Pechmann Reaction", *Organic Reactions* **1953**, 7, 1, the disclosure of which is herein incorporated by reference. The cyclization of an aniline as described above is known as a Knorr cylization. See G. Jones, "Pyridines and their Benzo Derivatives: (v) Synthesis". In *Comprehensive Heterocyclic Chemistry,* Katritzky, A. R.; Rees, C. W., eds. Pergamon, New York, 1984. Vol. 2, chap. 2.08, pp 421-426, the disclosure of which is herein incorporated by reference. A compound of structure **69** may be converted to a compound of structure **57** by treatment with an acid, for example, *para*-toluenesulphonic acid. In addition, a compound of structure **71** may be converted to a compound of structure **57** by treatment with, for example, *para*-chlorophenol.

**Scheme XVIII**

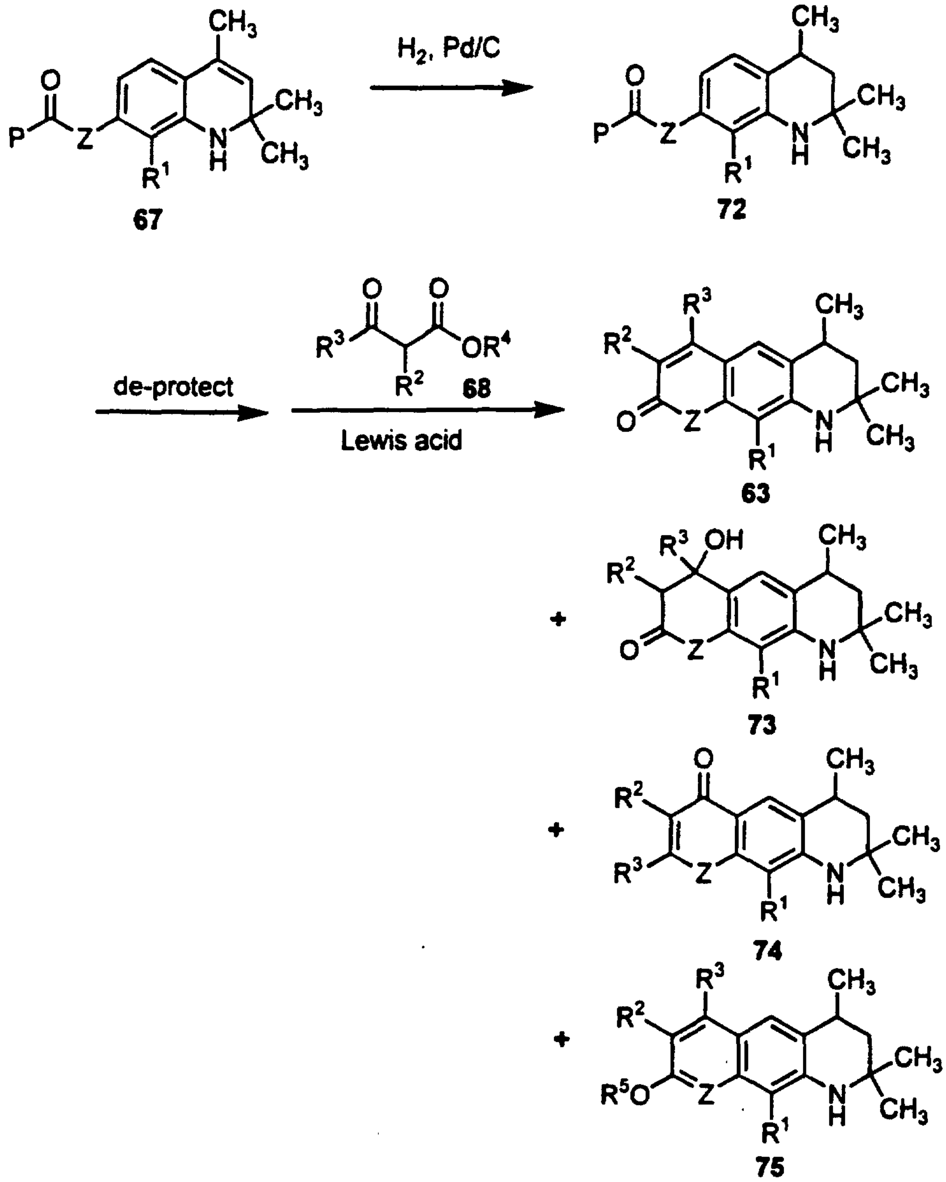


67

72

68

63

73

74

75

The process of **Scheme XVIII** begins with the reduction of a compound of structure **67** with, for example, hydrogen over a metal catalyst such as palladium on carbon. Deprotection by either acid or base, followed by treatment of the corresponding aniline or phenol with a β-keto ester (structure **68**) in the presence of a Lewis acid such as zinc chloride, as described above in **Scheme XVII,** affords one or more of four compounds (structures **63, 73, 74,** and **75**).

## Scheme XIX

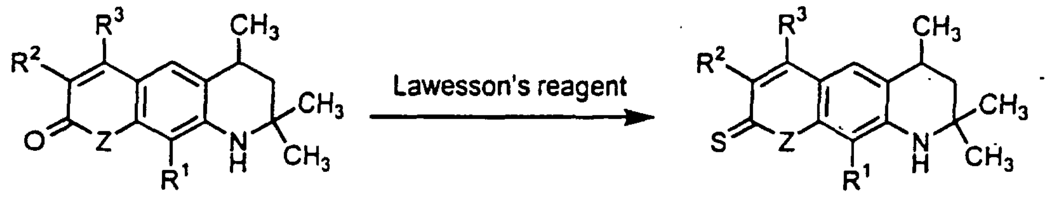


63 76

The process of **Scheme XIX** involves the conversion of a compound of structure **63** to the corresponding thio-compound (structure **78**) by treatment with Lawesson's reagent [2,4-bis(4-methoxyphenyl)-1,3-dithia-2,4-diphosphetane-2,4-disulfide].

## Scheme XXXVIII

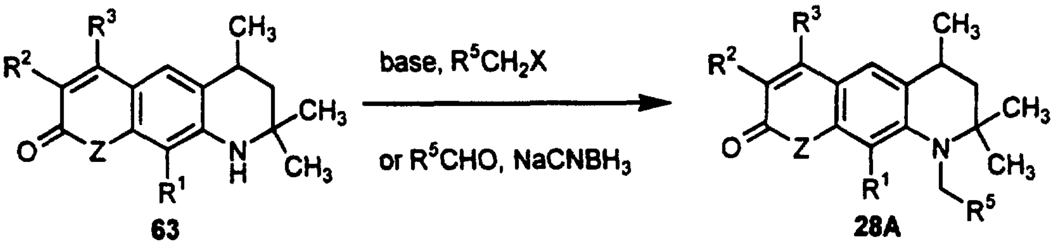


63 28A

The process of **Scheme XXXVIII** involves the alkylation of N(1) of a compound of structure **63**, which can be accomplished in one of two ways. Treatment of a compound of structure **63** with a base, such as sodium hydride, and an alkylating agent, such as benzyl bromide, affords a compound of structure **28A**. Alternatively, treatment of a compound of structure **63** with an aldehyde, for example acetaldehyde or para-formaldehyde, in the presence of a reducing agent, for example sodium cyanoboro-hydride or sodium (triacetoxy)borohydride, affords a compound of structure **28A.**

## Scheme XXXIX

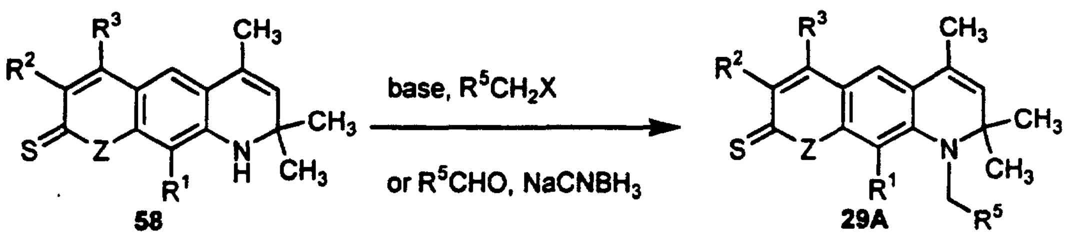


58 29A

The process of **Scheme XXXIX** involves the alkylation of N(1) of a compound of structure **58**, which can be accomplished in one of two ways. Treatment of a compound of structure **58** with a base, such as sodium hydride, and an alkylating agent, such as benzyl bromide, affords a compound of structure **29A**. Alternatively, treatment of a compound of structure **58** with an aldehyde, for example acetaldehyde or para-formaldehyde, in the presence of a reducing agent, for example sodium cyanoboro-hydride or sodium (triacetoxy)borohydride, affords a compound of structure **29A**.

**Scheme XL**

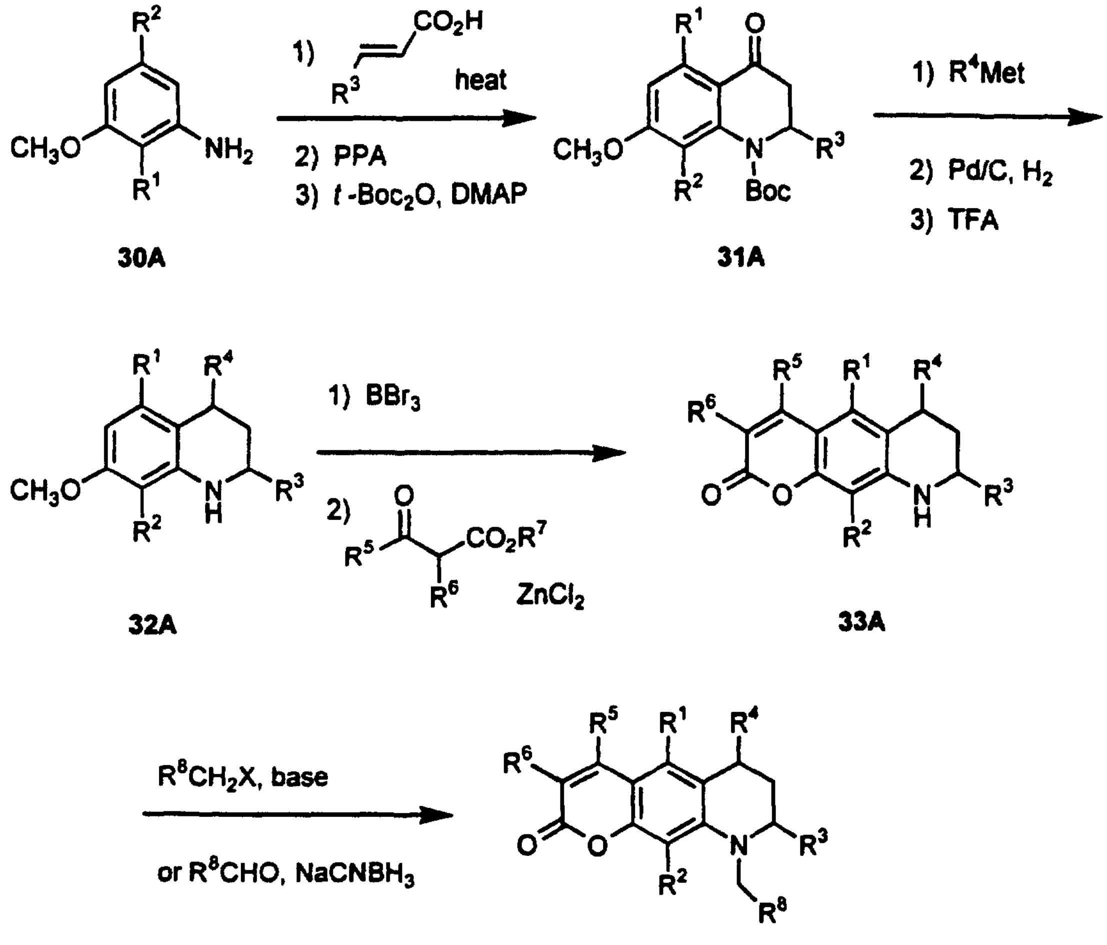


34A

The process of **Scheme XL** begins with reaction of a 3-methoxyaniline (a compound of structure **30A**) with an acrylic acid, for example, crotonic acid, followed by treatment with an acid such as polyphosphoric acid to afford a 4-quinolone. Protection of the nitrogen atom by treatment with a base, for example *n*-butyllithium, followed by the addition of an acylating agent such as di-*tert*-butyldicarbonate, affords a compound of structure **31A.** Addition of an organomagnesium or organolithium reagent ($R^4$ = alkyl, aryl, etc.), or a reducing agent such as sodium borohydride ($R^4$ = hydrogen), affords an alcohol. Reduction of the alcohol with, for example hydrogen over palladium on carbon, followed by deprotection of the nitrogen atom, affords a compound of structure **32A**. Demethylation of the methyl ether with, for example, boron tribromide, followed by a Pechman cyclization with a β-keto ester effected by, for example, zinc chloride, affords a compound of structure **33A**. A compound of structure **33A** may further be transformed to a compound of structure **34A** by alkylation of the nitrogen atom, which can be accomplished in one of two ways. Treatment of a compound of structure **33A** with a base, such as sodium hydride, and an alkylating agent, such as benzyl bromide, affords a compound of structure **34A.** Alternatively, treatment of a compound of structure **33A** with an aldehyde, for example acetaldehyde or paraformaldehyde, in the presence of a reducing agent, for example sodium cyanoborohydride or sodium (triacetoxy)borohydride, affords a compound of structure **34A.**

**Scheme XLI**

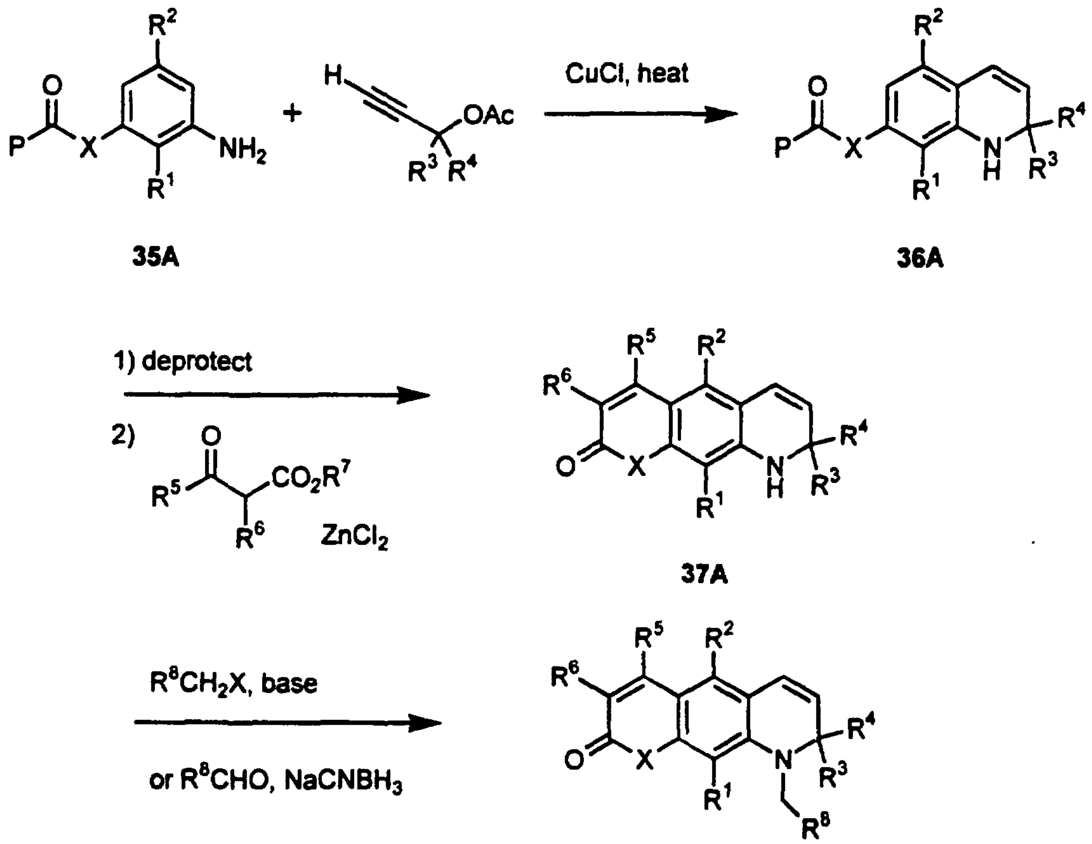


35A

36A

37A

38A

The process of **Scheme XLI** begins with the reaction of an aniline of structure **35A** with a propargyl acetate in the presence of a copper salt such as copper(I) chloride to afford a compound of structure **36A**. Deprotection of the heteroatom with, for example ethanolic potassium hydroxide, followed by a Pechman cyclization (X = O or S) or Knorr cyclization (X = NH) with a β-keto ester effected by, for example, zinc chloride, affords a compound of structure **37A**. A compound of structure **37A** may further be transformed to a compound of structure **38A** by alkylation of the nitrogen atom, which can be accomplished in one of two ways. Treatment of a compound of structure **37A** with a base, such as sodium hydride, and an alkylating agent, such as benzyl bromide, affords a compound of structure **38A**. Alternatively, treatment of a compound of structure **37A** with an aldehyde, for example acetaldehyde or paraformaldehyde, in the presence of a reducing agent, for example sodium cyanoborohydride or sodium (triacetoxy)borohydride, affords a compound of structure **38A**.

**Scheme XLII**

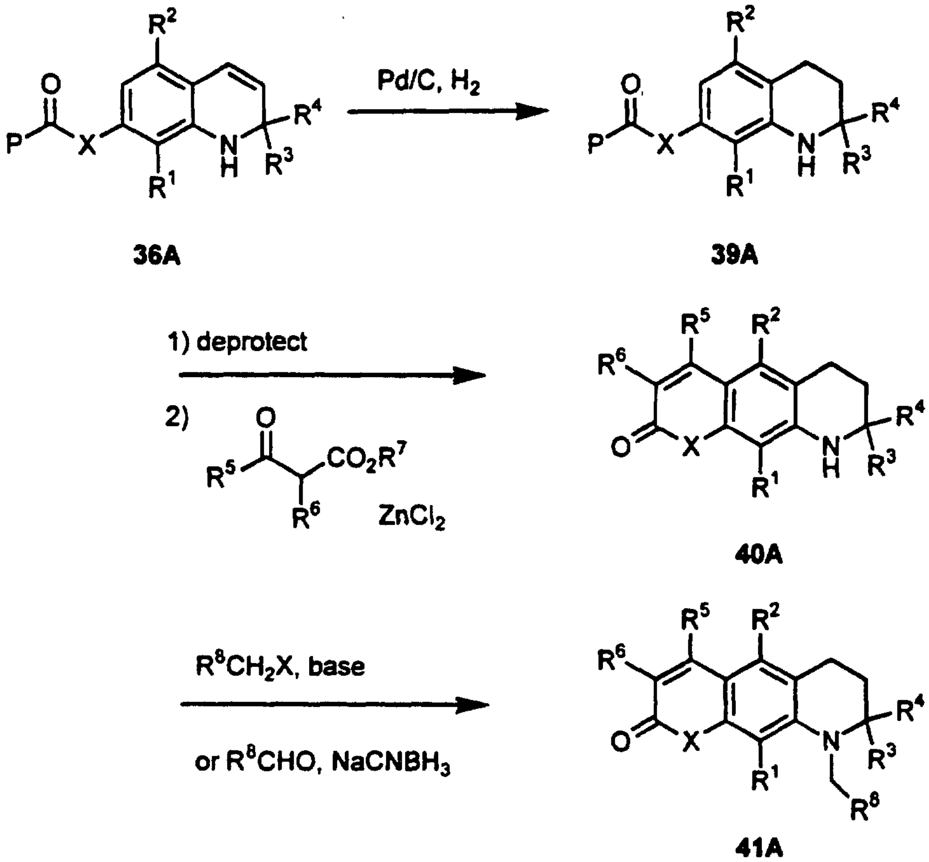


36A 39A 40A 41A

The process of **Scheme XLII** begins with the reduction of a compound of structure **36A** with, for example, hydrogen over palladium on carbon. Deprotection of the heteroatom with, for example ethanolic potassium hydroxide, followed by a Pechman cyclization (X = O or S) or Knorr cyclization (X = NH) with a β-keto ester effected by, for example, zinc chloride, affords a compound of structure **39A**. A compound of structure **39A** may further be transformed to a compound of structure **40A** by alkylation of the nitrogen atom, which can be accomplished in one of two ways. Treatment of a compound of structure **39A** with a base, such as sodium hydride, and an alkylating agent, such as benzyl bromide, affords a compound of structure **40A**. Alternatively, treatment of a compound of structure **39A** with an aldehyde, for example acetaldehyde or paraformaldehyde, in the presence of a reducing agent, for example sodium cyanoborohydride or sodium (triacetoxy)borohydride, affords a compound of structure **40A**.

## Scheme XLIII

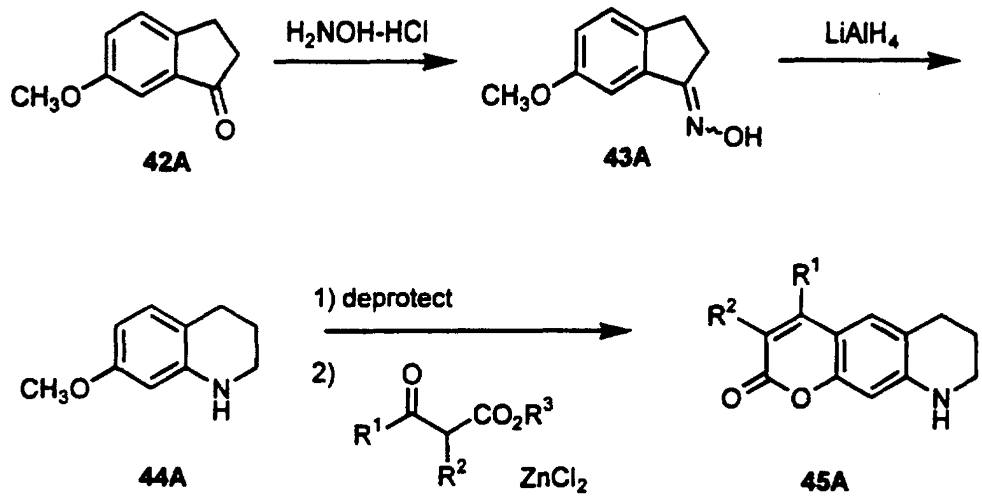


42A 43A 44A 45A

The process of **Scheme XLIII** begins with 6-methoxy-1-tetralone (Compound **42A**) which is treated with hydroxylamine hydrochloride to afford the corresponding oxime, Compound **43A**. A reductive Beckman rearrangement effected by, for example, lithium aluminum hydride, affords Compound **44A**. Demethylation of the methyl ether with, for example, boron tribromide, followed by a Pechman cyclization with a β-keto ester effected by, for example, zinc chloride, affords a compound of structure **45A**.

## Scheme XLIV

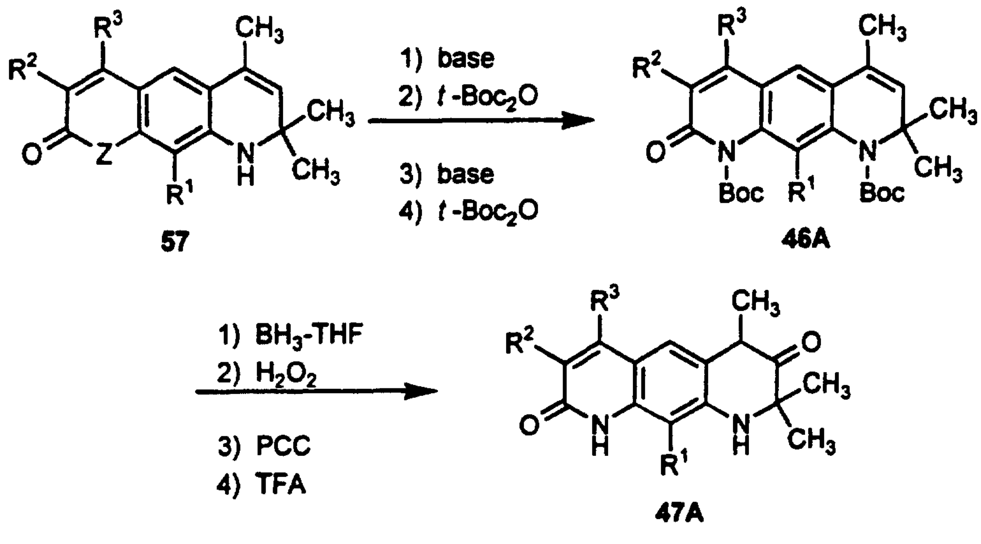


57 46A 47A

The process of **Scheme XLIV** begins with the protection of both nitrogen atoms of a compound of structure **57** (Z=NH) by two sequential treatments with a base, for example *n*-butyllithium, followed by an acylating agent, for example di-*tert*-butyldicarbonate, to afford a compound of structure **46A**. Hydroboration with a borane species, for example, boranetetrahydrofuran, followed by an oxidative work-up using, for example, basic hydrogen peroxide, affords a 3-hydroxyltetrahydroquinoline, which is oxidized with, for example, pyridinium chlorochromate, to afford the 3-ketotetrahydroquinoline. The 3-ketotetrahydroquinoline may subsequently be deprotected with, for example, trifluroacetic acid, to afford a compound of structure **47A**.

**Scheme XLV**

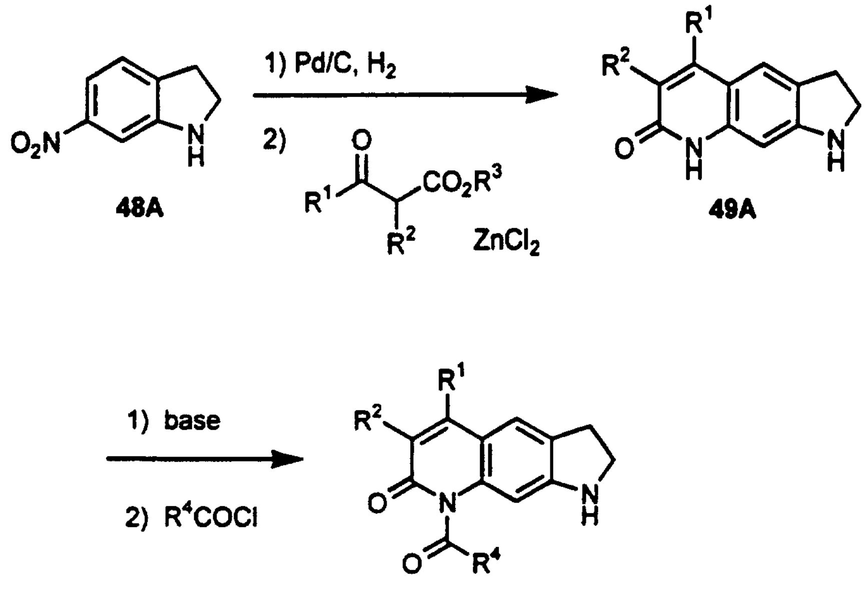


48A 49A

50A

The process of **Scheme XLV** begins with the reduction of 6-nitroindoline (Compound **48A**) with, for example, hydrogen over palladium on carbon. A Pechman cyclization with a β-keto ester effected by, for example, zinc chloride, affords a compound of structure **49A**. A compound of structure **49A** may further be transformed to a compound of structure **50A** by acylation of the quinolone nitrogen atom, which may be effected by deprotonation with, for example, sodium hydride, followed by the addition of an acylating agent, such as 3-nitrobenzoyl chloride.

**Scheme XLVI**

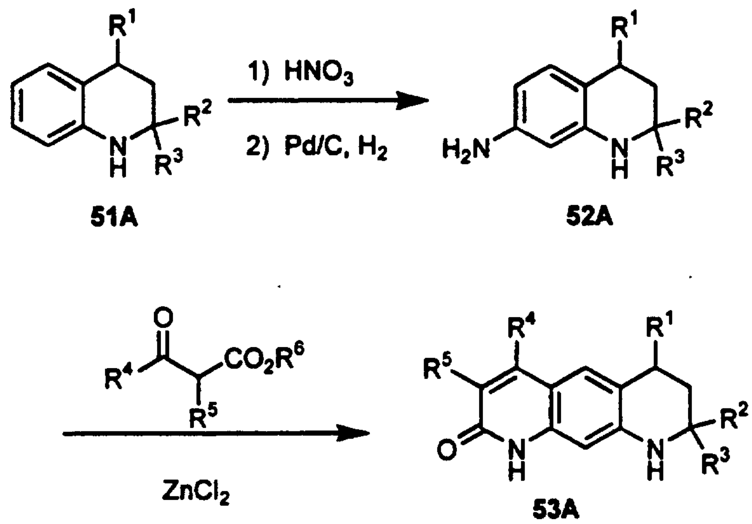


51A 52A

53A

The process of **Scheme XLVI** begins with the nitration of a 1,2,3,4-tetrahydroquinoline (a compound of structure **51A**) by the action of nitric acid in the presence of, for example, sulfuric acid. Reduction of the nitro group with, for example, hydrogen over palladium on carbon, affords a 7-amino-1,2,3,4-tetrahydroquinoline of structure **52A**. A Knorr cyclization with a β-keto ester effected by, for example, zinc chloride, affords a compound of structure **53A**.

**Scheme XLVII**

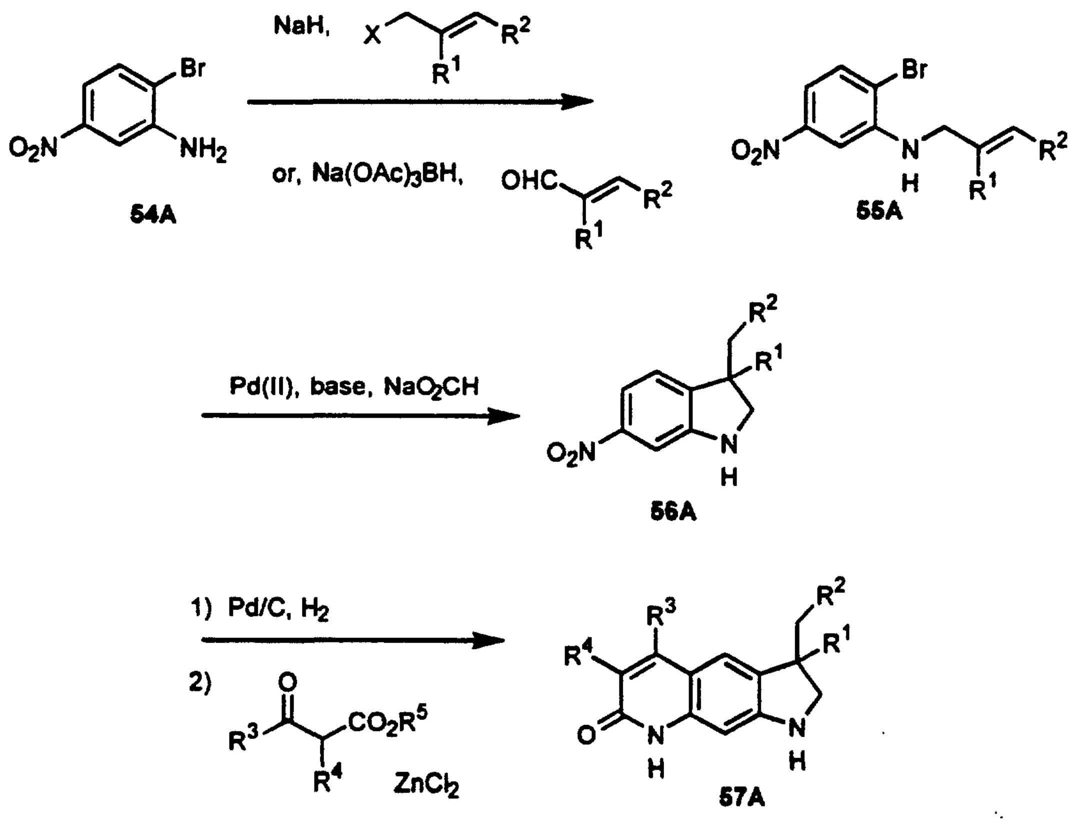


54A 55A 56A 57A

The process of **Scheme XLVII** begins with the alkylation of 2-bromo-5-nitroaniline (Compound **54A**) which may be accomplished in one of two ways. Treatment of Compound **54A** with a base such as sodium hydride and an allylating agent, for example, 1-bromo-3-methyl-2-butene, affords a compound of structure **55A**. Alternatively, Compound **54A** may be treated with an α,β-unsaturated aldehyde, for example, cinnamaldehyde, in the presence of a reducing agent such as sodium triacetoxyborohydride to afford a compound of structure 55A. A palladium-catalyzed cyclization reaction catalyzed by, for example, palladium(II) acetate, affords a compound of structure **56A.** Reduction of the nitro group with, for example, hydrogen over palladium on carbon, affords the aniline, and a Knorr cyclization with a β-keto ester effected by, for example, zinc chloride, affords a compound of structure **57A**.

**Scheme XLVIII**

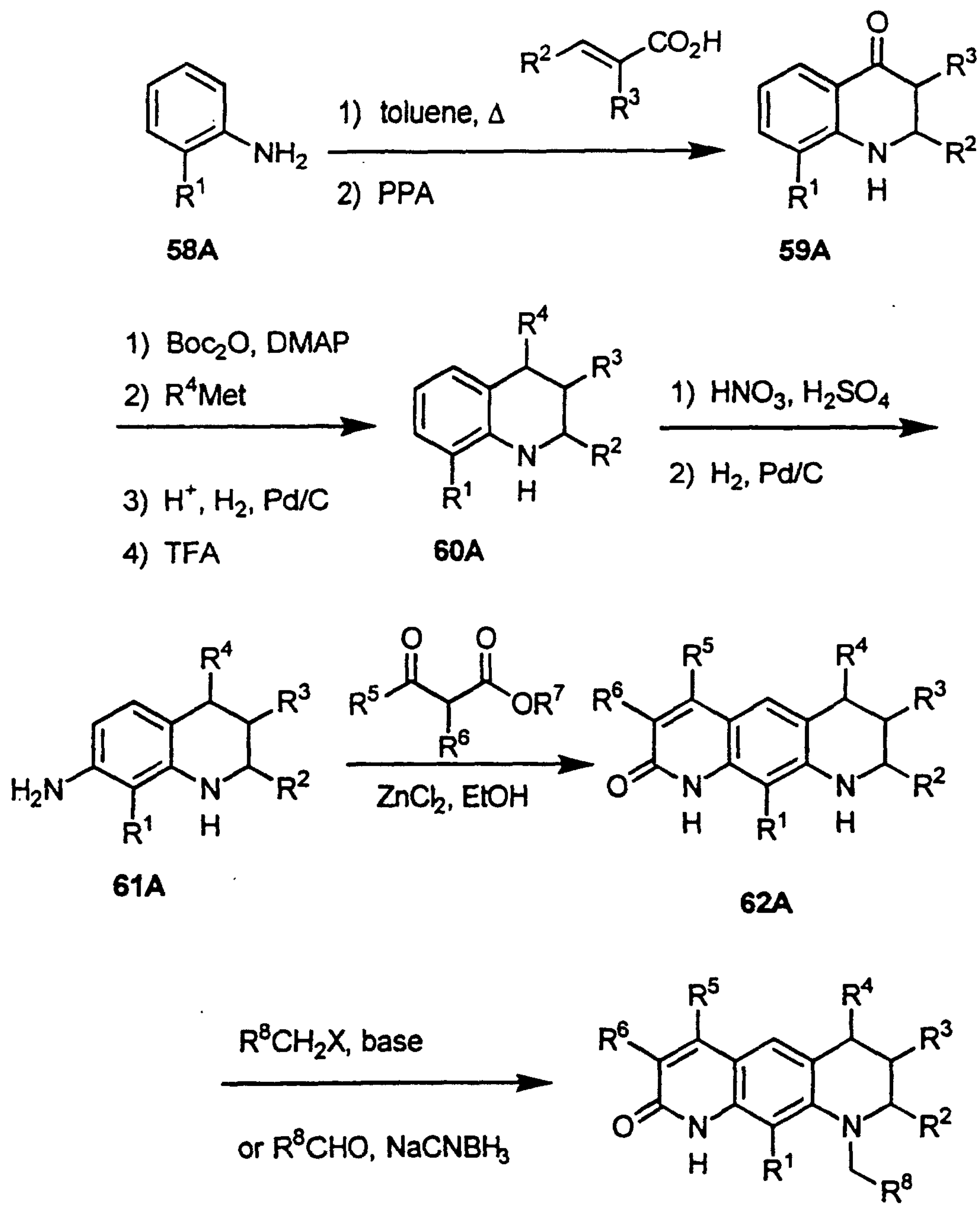


58A 59A 60A 61A 62A 63A

The process of **Scheme XLVIII** begins with the reaction of an aniline (structure **58A**) with an acrylic acid, for example crotonic acid, followed by a cyclization reaction mediated by, for example, polyphosphoric acid to afford a 4-quinolinone of structure **59A**. The nitrogen atom is then protected by treatment with a base, for example n-butyllithium, followed by the addition of an acylating agent such as di-*tert*-butyldicarbonate. Addition of an organomagnesium or organolithium reagent ($R^4$ = alkyl, aryl, etc.), or a reducing agent such as sodium borohydride ($R^4$ = hydrogen), affords an alcohol. Reduction of the alcohol with, for example hydrogen over palladium on carbon, followed by deprotection of the nitrogen atom, affords a compound of structure **60A.** Nitration of a compound of structure **60A** by the action of

nitric acid in the presence of, for example, sulfuric acid, followed by reduction of the nitro group with, for example, hydrogen over palladium on carbon, affords a 7-amino-1,2,3,4-tetrahydroquinoline of structure **61A**. A Knorr cyclization with a β-keto ester effected by, for example, zinc chloride, affords a compound of structure **62A**. A compound of structure **62A** may be further transformed into a compound of structure **63A** by alkylation of the nitrogen atom, which can be accomplished in one of two ways. Treatment of a compound of structure **62A** with a base, such as sodium hydride, and an alkylating agent, such as benzyl bromide, affords a compound of structure **63A.** Alternatively, treatment of a compound of structure **62A** with an aldehyde, for example acetaldehyde or paraformaldehyde, in the presence of a reducing agent, for example sodium cyanoborohydride or sodium (triacetoxy)borohydride, affords a compound of structure **63A**.

## Scheme XLIX

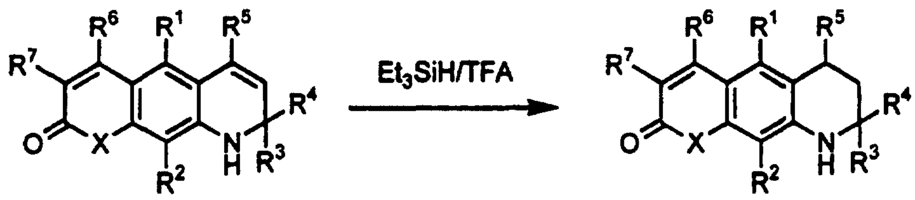


**64A** **65A**

The process of **Scheme XLIX** involves the reduction of a compound of structure **64A** by treatment with, for example, triethylsilane in the presence of trifluoroacetic acid, to afford a compound of structure **65A**.

## Scheme L

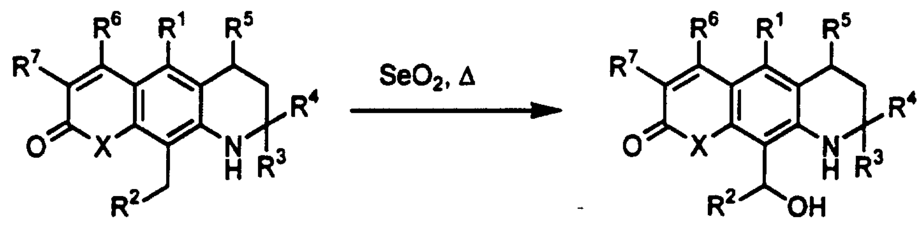


**66A** **67A**

The process of **Scheme L** involves the oxidation of benzylic substituent of a compound of structure **66A** by treatment with, for example, selenium dioxide, to afford a compound of structure **67A**.

## Scheme LI

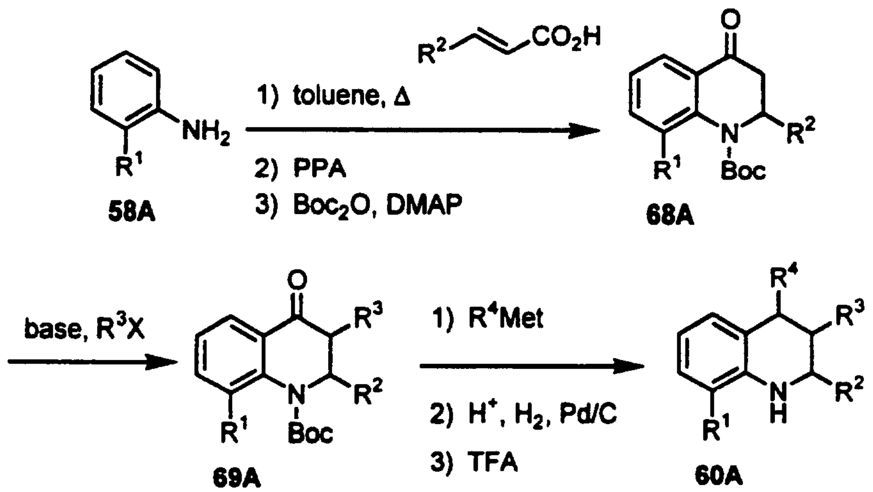


58A 68A 69A 60A

The process of **Scheme LI** begins with the reaction of an aniline (structure **58A**) with an acrylic acid, for example crotonic acid, followed by a cyclization reaction mediated by, for example, polyphosphoric acid to afford a 4-quinolinone. The nitrogen atom is then protected by treatment with a base, for example, 4-dimethylaminopyridine, followed by the addition of an acylating agent such as di-*tert*-butyldicarbonate to afford a compound of structure **68A.** The 4-quinolone is then deprotonated with a base, for example, sodium hydride, and treated with an alkylating agent such as iodomethane, to afford a compound of structure **69A.** Addition of an organomagnesium or organolithium reagent ($R^4$ = alkyl, aryl, etc.), or a reducing agent such as sodium borohydride ($R^4$ = hydrogen), affords an alcohol. Reduction of the alcohol with, for example hydrogen over palladium on carbon, followed by deprotection of the nitrogen atom, affords a compound of structure **60A**. Compounds of structure **60A** may be transformed into compounds of structure **62A** as described in **Scheme XLVIII**.

## Scheme LII

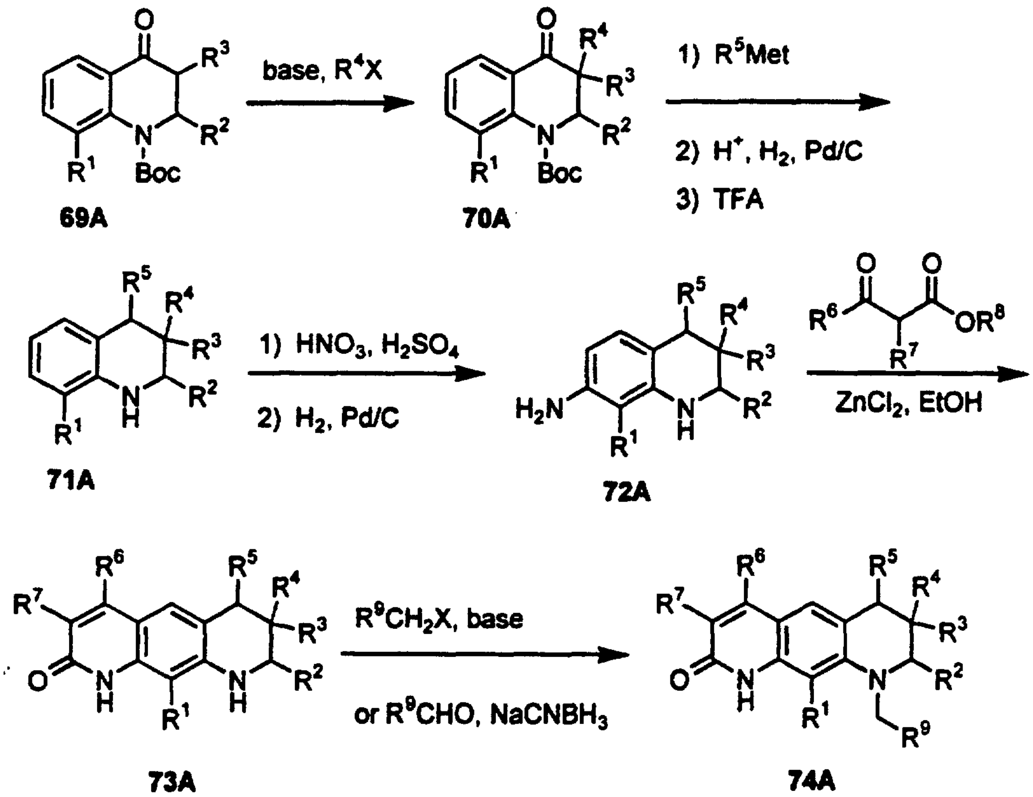


69A 70A 71A 72A 73A 74A

The process of **Scheme LII** begins with the deprotonation of a compound of structure **69A** with a base, for example, sodium hydride, and treatment with an alkylating agent such as iodomethane, to afford a compound of structure **70A.** Addition of an organomagnesium or organolithium reagent ($R^5$ = alkyl, aryl, etc.), or a reducing agent such as sodium borohydride ($R^5$ = hydrogen), affords an alcohol. Reduction of the alcohol with, for example, hydrogen over palladium on carbon, followed by deprotection of the nitrogen atom, affords a compound of structure **71A.** Nitration of a compound of structure **71A** by the action of nitric acid in the presence of, for example, sulfuric acid, followed by reduction of the nitro group with, for example, hydrogen over palladium on carbon, affords a 7-amino-1,2,3,4-tetrahydroquinoline of structure **72A**. A Knorr cyclization with a β-keto ester effected by, for example, zinc chloride, affords a compound of structure **73A**. A compound of structure **73A** may be further transformed into a compound of structure **74A** by alkylation of the nitrogen atom, which can be accomplished in one of two ways. Treatment of a compound of structure **73A** with a base, such as sodium hydride, and an alkylating agent, such as benzyl bromide, affords a compound of structure **74A.** Alternatively, treatment of a compound of structure **73A** with an aldehyde, for example acetaldehyde or paraformaldehyde, in the presence of a reducing agent, for example sodium cyanoborohydride or sodium (triacetoxy)borohydride, affords a compound of structure **74A**.

## Scheme LIII

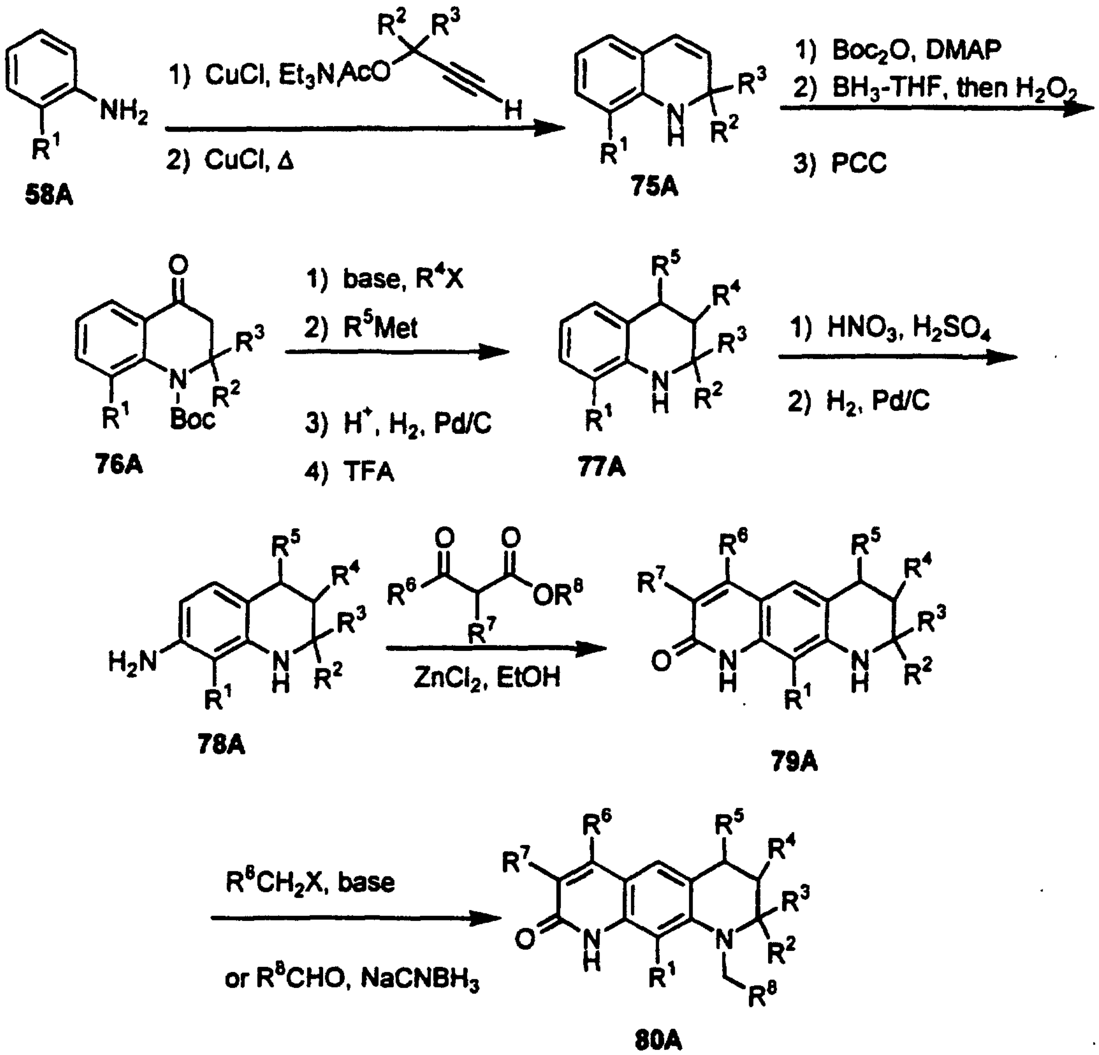


The process of **Scheme LIII** begins with the reaction of an aniline (structure **58A**) with a propargyl acetate in the presence of a copper salt such as copper(I) chloride to afford a compound of structure **75A**. The nitrogen atom is then protected by treatment with a base, for example 4-dimethylaminopyridine, followed by the addition of an acylating agent such as di-*tert*-butyldicarbonate. Hydroboration of the olefin with, for example, boranetetrahydrofuran, followed by an oxidative work-up with, for example, basic hydrogen peroxide, affords the 4-hydroxytetrahydroquinoline, which may be oxidized with, for example, pyridinium chlorochromate, to afford a compound of structure **76A**. A compound of structure **76A** may then be deprotonated with a base, for example, sodium hydride, and treated with an alkylating agent such as iodomethane. Addition of an organomagnesium or organolithium reagent ($R^5$ = alkyl, aryl, etc.), or a reducing agent such as sodium borohydride ($R^5$ = hydrogen), affords an alcohol. Reduction of the alcohol with, for example, hydrogen over palladium on carbon, followed by deprotection of the nitrogen atom, affords a compound of structure **77A**. Nitration of a compound of structure **77A** by the action of nitric acid in the presence of, for example, sulfuric acid, followed by reduction of the nitro group with, for example, hydrogen over palladium on carbon, affords 7-amino-1,2,3,4-tetrahydroquinolines of structure **78A.** A Knorr cyclization with a β-keto ester effected by, for example, zinc chloride, affords a compound of structure **79A**. A compound of structure **79A** may be further transformed into a compound of structure **80A** by alkylation of the nitrogen atom, which can be accomplished in one of two ways. Treatment of a compound of structure **79A** with a base, such as sodium hydride, and an alkylating agent, such as benzyl bromide, affords a compound of structure **80A**. Alternatively, treatment of a compound of structure **79A** with an aldehyde, for example acetaldehyde or paraformaldehyde, in the presence of a reducing agent, for example sodium cyanoborohydride or sodium (triacetoxy)borohydride, affords a compound of structure **80A**.

## Scheme LIV

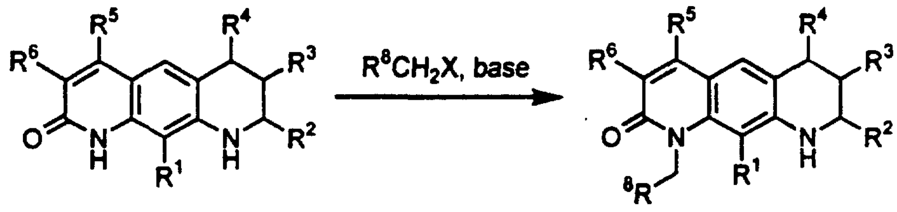


**62A** **81A**

The process of **Scheme LIV** involves the deprotonation of a compound of structure **62A** with, for example, sodium hydride, followed by treatment with an alkylating agent such as iodomethane to afford a compound of structure **81A**.

## Scheme LV

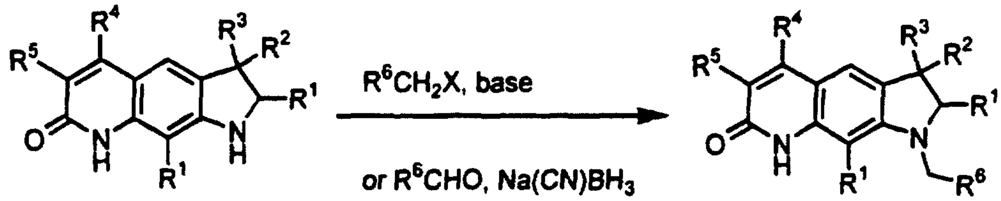


**82A** **83A**

The process of **Scheme LV** involves the conversion of a compound of structure **82A** into a compound of structure **83A** by alkylation of the nitrogen atom, which can be accomplished in one of two ways. Treatment of a compound of structure **82A** with a base, such as sodium hydride, and an alkylating agent, such as benzyl bromide, affords a compound of structure **83A**. Alternatively, treatment of a compound of structure **82A** with an aldehyde, for example acetaldehyde or paraformaldehyde, in the presence of a reducing agent, for example sodium cyanoborohydride or sodium (triacetoxy)borohydride, affords a compound of structure **83A**.

## Scheme LVI

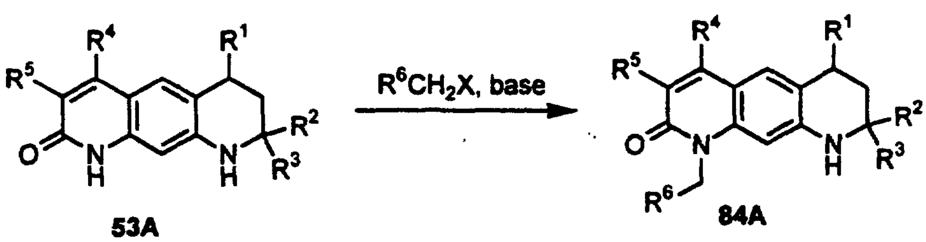


**53A** **84A**

The process of **Scheme LVI** involves the deprotonation of a compound of structure **53A** with, for example, sodium hydride, followed by treatment with an alkylating agent such as iodomethane to afford a compound of structure **84A**.

It will be understood by those skilled in the art that certain modifications can be made to the above-described methods that remain within the scope of the present invention.

In a further aspect, the present invention provides several novel processes for the preparation of the compounds of the present invention. Each of these processes is illustrated in one or more of the Schemes shown above, and is described with particularity as follows.

**Process 5** is depicted in **Scheme XVII** and begins with the acylation of a 3-nitroaryl, e.g., a 3-nitrophenol (structure **64,** Y=O), 3-nitroaniline (structure **64,** Y=NH), or 3-nitrothiophenol (structure **64,** Y=S), with an acylating agent (for example, di-*tert*-butyl dicarbonate or trimethylacetyl chloride), either with or without the addition of a base (for example, 4-dimethylaminopyridine, triethylamine, pyridine) in an inert solvent (typical solvents include dichloromethane, THF, toluene) at -100°C to 200°C, to afford the 5-protected 3-nitroaryl compound of structure **65**. Reduction of the nitro group with, for example, 1-200 atmospheres of hydrogen over a metal catalyst (for example, Pd/C, $PtO_2$), affords the corresponding 5-protected 3-aminoaryl (structure **66**). Treatment of a compound of structure **66** with acetone and a catalyst such as iodine and addition of a 1,2-dihydroquinoline affords the 5-protected 1,2-dihydroquinoline compound of structure **67**, as described above in **Process 1**. Deprotection, for example, by either acid (for example, hydrochloric acid, trifluoroacetic acid, sulfuric acid) or base (for example, sodium hydroxide) at -40°C to 300°C, followed by treatment of a solution (typical solvents include ethanol, toluene, methanol) of the corresponding aniline or phenol with a β-keto ester (structure 68) in the presence of a Lewis acid (for example, zinc chloride, boron trifluoride, aluminum trichloride) at -40°C to 300°C, affords one or more of the four linear tricyclic 1,2-dihydroquinoline compounds (structures **57, 69, 70,** and **71**). A compound of structure **69** may be converted to a compound of structure 57 by treatment of a solution (typical solvents include toluene, dichloromethane) of a compound of structure **69** with an acid (for example, *para*-toluenesulphonic acid, hydrochloric acid) at -40°C to 300°C. In addition, a compound of structure **71** may be converted to a compound of structure **57** by treatment of a solution (typical solvents include toluene, dichloromethane) of a compound of structure **71** with, for example, *para*-chlorophenol.

**Process 6** is a modification of **Process 5**. Thus, a solution (typical solvents include ether, THF, toluene) of a 3-aminoaryl, preferably 3-amino thioaryl, is treated with a strong base (for example, sodium hydride, *n*-butyllithium) at -100°C to 100°C, followed by the addition of an acylating agent (typical acylating agents include di-*t*-butyl dicarbonate, trimethylacetyl chloride, acetic anhydride) at -100°C to 200°C, to afford the corresponding the corresponding 5-protected 3-aminoaryl compound of structure **66** (Y=S). The conversion of a compound of structure **66** (Y=S) to the linear tricyclic 1,2-dihydroquinoline compounds of structures **57, 69, 70** and **71** (Y=S) is accomplished as described above in **Process 5.**

**Process 7** is depicted in **Scheme XLVI**, and also is included as parts of **Schemes XLVIII, LII**, and **LIII. Process 7** begins with the nitration of a 1,2,3,4-tetrahydroquinoline (for example, a compound of structure **51A** in **Scheme XLVI**, or of structure **60A** in **Scheme XLVIII**, etc) with a nitrating agent. For example a mixture of sulfuric acid and nitric acid is added to a solution of the tetrahydroquinoline in sulfuric acid or sulfuric acid and a second, inert solvent such as nitromethane, at -80 °C to +40 °C. The nitro group of the resulting 7-nitro-1,2,3,4-tetrahydroquinoline is then reduced by hydrogenation over a metal catalyst (for example, Pd/C, $PtO_2$) under 1-200 atmosperes of hydrogen, to afford the corresponding aniline (a compound of structure **52A** in **Scheme XLVI** or of structure **72A** in **Scheme LII**, for example). Treatment of a solution (typical solvents include ethanol, toluene, methanol) of the aniline with a b-keto ester (structure **68**) in the presence of a Lewis acid (for example, zinc chloride, boron trifluoride, aluminum trichloride) at -40 °C to +300 °C, affords the desired quinoline, a compound of structure **53A** in **Scheme XLVI,** or of structure **73A** in **Scheme LII,** etc.

In yet another aspect, the present invention provides novel intermediates useful in the preparation of the steroid modulator compounds of the present invention. The intermediates of the present invention are defined as those having the formulae:

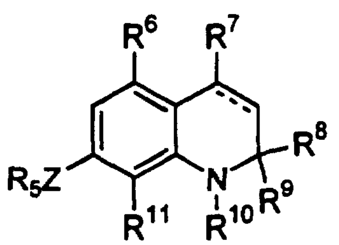

**(I)**

OR

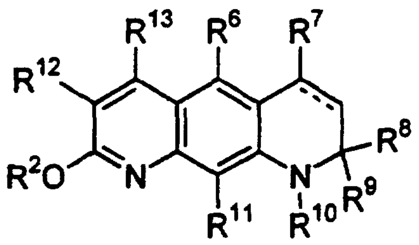

(II)

OR

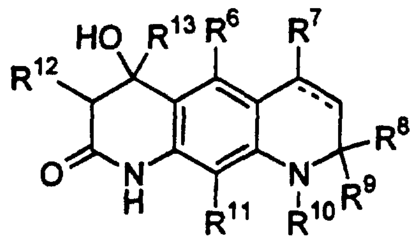

(III)

OR

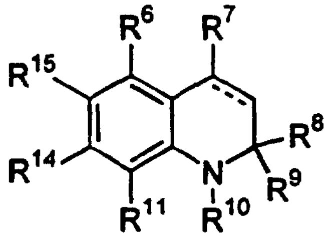

(IV)

wherein:

Z is O, S, or $NR^1$, where $R^1$ is hydrogen, $R^2C{=}O$, $R^2C{=}S$, $R^3OC{=}O$, $R^3SC{=}O$, $R^3OC{=}S$, $R^3SC{=}S$ or $R^3R^4NC{=}O$, where $R^2$ is hydrogen, a $C_1$ - $C_6$ alkyl or perfluoroalkyl, optionally substituted allyl or aryl methyl alkenyl, alkynyl, aryl or heteroaryl, and where $R^3$ and $R^4$ each independently are hydrogen, a $C_1$ - $C_6$ alkyl, optionally substituted allyl, arylmethyl, aryl or heteroaryl;

$R^5$ is hydrogen, $R^2C{=}O$, $R^2C{=}S$, $R^3OC{=}O$, $R^3SC{=}O$, $R^3OC{=}S$, $R^3SC{=}S$, or $R^3R^4NC{=}O$, where $R^2$, $R^3$ and $R^4$ have the same definitions as given above;

$R^6$ is hydrogen, a $C_1$ - $C_6$ alkyl, optionally substituted allyl, aryl methyl, alkenyl, alkynyl, aryl, heteroaryl, $R^3O$, $HOCH_2$, $R^3OCH_2$, F, Cl, Br, I, cyano, $R^3R^4N$ or perfluoroalkyl, where $R^3$ and $R^4$ have the same definitions as given above;

$R^7$ through $R^9$ each independently are hydrogen, a $C_1$ - $C_6$ alkyl, allyl or optionally substituted allyl, arylmethyl, alkynyl, alkenyl, aryl, or heteroaryl, or

$R^8$ and $R^9$ taken together form a three- to seven-membered carbocylic or heterocyclic ring;

$R^{10}$ is hydrogen, a $C_1$ - $C_6$ alkyl, optionally substituted allyl, arylmethyl, aryl, or heteroaryl, $R^2C{=}O$, $R^2C{=}S$, $R^3OC{=}O$, $R^3SC{=}O$, $R^3OC{=}S$, $R^3SC{=}S$ or $R^3R^4NC{=}O$, where $R^2$ through $R^4$ have the same definitions as given above;

$R^{11}$ and $R^{12}$ each independently represent hydrogen, a $C_1$ - $C_6$ alkyl, optionally substituted allyl, aryl methyl, alkenyl, alkynyl, aryl, heteroaryl, $R^3O$, $HOCH_2$, $R^3OCH_2$, F, Cl, Br, I, cyano, $R^3R^4N$ or perfluoroalkyl, where $R^3$ and $R^4$ have

the same definitions as given above;

$R^{13}$ is hydrogen, a $C_1$ - $C_6$ alkyl, optionally substituted allyl, aryl methyl, alkenyl, alkynyl, aryl, heteroaryl, $R^3O$, $HOCH_2$, $R^3OCH_2$, $R^3R^4N$, $CF_2Cl$, $CF_2OR^3$ or perfluoroalkyl, where $R^3$ and $R^4$ have the same definitions as given above;

$R^{14}$ is hydrogen, a $C_1$ - $C_6$ alkyl, optionally substituted allyl, aryl methyl, alkenyl, alkynyl, aryl, heteroaryl, $R^3O$, $HOCH_2$, $R^3OCH_2$, F, Cl, Br, I, cyano, $R^3R^4N$ or perfluoroalkyl where $R^3$ and $R^4$ have the same definitions as given above; and

$R^{15}$ is F, Cl, Br, I, $B(OR^{16})_2$, $SnR^{17}R^{18}R^{19}$ or $OSO_2R^{20}$, where $R^{16}$ is hydrogen or a $C_1$ - $C_6$ alkyl, $R^{17}$ through $R^{19}$ each independently represent a $C_1$ - $C_6$ alkyl, $R^2O$ or heteroaryl, $R^{20}$ is a $C_1$ - $C_6$ alkyl, perfluoroalkyl, aryl, or heteroaryl, and $R^2$ has the same definition as given above.

The compounds of the present invention also includes racemate, stereoisomers and mixtures of said compounds, including isotopically-labeled and radio-labeled compounds. Such isomers can be isolated by standard resolution techniques, including fractional crystallization and chiral column chromatography.

As noted above, any of the steroid modulator compounds of the present invention can be combined in a mixture with a pharmaceutically acceptable carrier to provide pharmaceutical compositions useful for treating the biological conditions or disorders noted herein in mammalian, and more preferably, in human patients. The particular carrier employed in these pharmaceutical compositions may take a wide variety of forms depending upon the type of administration desired, e.g., intravenous, oral, topical, suppository or parenteral.

In preparing the compositions in oral liquid dosage forms (e.g., suspensions, elixirs and solutions), typical pharmaceutical media, such as water, glycols, oils, alcohols, flavoring agents, preservatives, coloring agents and the like can be employed. Similarly, when preparing oral solid dosage forms (e.g., powders, tablets and capsules), carriers such as starches, sugars, diluents, granulating agents, lubricants, binders, disintegrating agents and the like will be employed. Due to their ease of administration, tablets and capsules represent the most advantageous oral dosage form for the pharmaceutical compositions of the present invention.

For parenteral administration, the carrier will typically comprise sterile water, although other ingredients that aid in solubility or serve as preservatives, may also be included. Furthermore, injectable suspensions may also be prepared, in which case appropriate liquid carriers, suspending agents and the like will be employed.

For topical administration, the compounds of the present invention may be formulated using bland, moisturizing bases, such as ointments or creams. Examples of suitable ointment bases are petrolatum, petrolatum plus volatile silicones, lanolin, and water in oil emulsions such as Eucerin™ (Beiersdorf). Examples of suitable cream bases are Nivea™ Cream (Beiersdorf), cold cream (USP), Purpose Cream™ (Johnson & Johnson) hydrophilic ointment (USP), and Lubriderm™ (Warner-Lambert).

The pharmaceutical compositions and compounds of the present invention will generally be administered in the form of a dosage unit (e.g., tablet, capsule etc.) at from about 1 µg/kg of body weight to about 500 mg/kg of body weight, more preferably from about 10 µg/kg to about 250 mg/kg, and most preferably from about 20 µg/kg to about 100 mg/kg. As recognized by those skilled in the art, the particular quantity of pharmaceutical composition according to the present invention administered to a patient will depend upon a number of factors, including, without limitation, the biological activity desired, the condition of the patient, and tolerance for the drug.

The compounds of this invention also have utility when radio- or isotopically-labeled as ligands for use in assays to determine the presence of PR, AR, ER, GR or MR in a cell background or extract. They are particularly useful due to their ability to selectively activate progesterone and androgen receptors, and can therefore be used to determine the presence of such receptors in the presence of other steroid receptors or related intracellular receptors.

Due to the selective specificity of the compounds of this invention for steroid receptors, these compounds can be used to purify samples of steroid receptors *in vitro*. Such purification can be carried out by mixing samples containing steroid receptors with one or more of the compounds of the present invention so that the compounds bind to the receptors of choice, and then separating out the bound ligand/receptor combination by separation techniques which are known to those of skill in the art. These techniques include column separation, filtration, centrifugation, tagging and physical separation, and antibody complexing, among others.

The compounds and pharmaceutical compositions of the present invention can advantageously be used in the treatment of the diseases and conditions described herein. In this regard, the compounds and compositions of the present invention will prove particularly useful as modulators of human fertility, and in the treatment of female and male sex steroid-dependent diseases and conditions such as hormone replacement therapy, dysfunctional uterine bleeding, endometriosis, leiomyomas, acne, male-pattern baldness, osteoporosis, prostatic hyperplasia and various hormone-dependent cancers, such as cancers of the breast, ovaries, endometrium and prostate. The GR and MR active compounds and compositions of the present invention will also prove useful as affectors of carbohydrate, protein and lipid metabolism, electrolyte and water balance, as well as modulators of the functions of the cardiovascular, kidney, central nervous, immune, skeletal muscle and other organ and tissue systems.

The compounds and pharmaceutical compositions of the present invention possess a number of advantages over previously identified steroidal and non-steroidal compounds.

Furthermore, the compounds and pharmaceutical compositions of the present invention possess a number of advantages over previously identified steroid modulator compounds. For example, the compounds are extremely potent activators of PR and AR, preferably displaying 50% maximal activation of PR and/or AR at a concentration of less than 100 nM, more preferably at a concentration of less than 50 nM, more preferably yet at a concentration of less than 20 nM, and most preferably at a concentration of 10 nM or less. Also, the selective compounds of the present invention generally do not display undesired cross-reactivity with other steroid receptors, as is seen with the compound mifepristone (RU486; Roussel Uclaf), a known PR antagonist that displays an undesirable cross reactivity on GR and AR, thereby limiting its use in long-term, chronic administration. In addition, the compounds of the present invention, as small organic molecules, are easier to synthesize, provide greater stability and can be more easily administered in oral dosage forms than other known steroidal compounds.

The invention will be further illustrated by reference to the following non-limiting Examples.

## EXAMPLE 137

1,2-Dihydro-2,2,4-trimethyl-6-methoxymethyl-8-pyranono[5,6-*g*]quinoline (Compound **237**, structure **57** of **Scheme XVI**, where $R^1$=$R^2$=H, $R^3$=methoxylmethyl, Y=O)

General Method 7 : 1,2-Dihydro-2,2,4-trimethylquinolines (Compounds of structure **57** or **67**) from anilines (Compounds of structure **56** or **66**); ambient pressure version In an r.b. flask equivuipped with a reflux condensor, a solution of the aniline (a compound of structure **56** or **66**) in acetone (0.05-0.20 M) was treated with iodine (5-20 mol%) and heated to reflux for 1-3 days. Addition of Celite™ followed by concentration afforded a fluffy orange powder which was purified by silica gel chromatography to afford the desired dihydroquinoline (compound of structure **57** or **67**).

1,2-Dihydro-2,2,4-trimethyl-6-methoxymethyl-8-pyranono[5,6-*g*]quinoline (Compound **237,** structure **57** of **Scheme XVI,** where $R^1$=$R^2$=H, $R^3$=methoxylmethyl, Z=O) This compound was prepared by General Method 7 from 7-amino-4-methoxymethylcoumarin (structure **56** of **Scheme XVI,** where $R^1$=$R^2$=H, $R^3$=methoxymethyl) (1.0 g, 4.87 mmol) to afford 82 mg (6%) of Compound **237** as a light yellow solid in addition to 487 mg (35%) of 1,2-dihydro-2,2,4-trimethyl-8-methoxymethyl-6-pyranono[6,5-*f*]quinoline. Data for Compound **237**: $R_f$ 0.23 (silca gel, hexanes/EtOAc, 2:1); **$^1$H NMR** (400 MHz, $C_6D_6$) 7.01 (s, 1 H), 6.24 (s, 1 H), 6.18 (s, 1 H), 5.02 (s, 1 H), 3.97 (s, 2 H), 3.74 (br s, 1 H), 2.92 (s, 3 H), 1.78 (d, J = 1.0, 3 H), 0.98 (s, 6 H).

## EXAMPLE 138

1,2-Dihydro-2,2,4-trimethyl-6-trifluoromethyl-8-pyranono[5,6-*g*]quinoline (Compound **238,** structure **57** of **Scheme XVII,** where $R^1$=$R^2$=H, $R^3$=trifluoromethyl, Z=O) This compound was prepared as depicted in **Scheme XVII** and as described below.

*O*-Pivaloyl-3-nitrophenol (structure **65** of **Scheme XVII,** where $R^1$=H, P=*t*-butyl, Z=O) To 300 mL of $CH_2Cl_2$ was added 3-nitrophenol (structure **64** of **Scheme XVII**, where $R^1$=H, Y=O) (15 g, 0.11 mol), pyridine (20 mL) and DMAP (10 mg). To this cooled solution (0°C) was slowly added trimethylacetyl chloride (18 mL, 146 mmol, 1.4 equivuiv). The solution was allowed to warm to rt and stirred for 3 h. To the amber colored solution was added sat'd $NH_4Cl$ (300 mL). The organic layer was washed with IN HCl (2 x 150 mL), 10% $CuSO_4 \cdot 5\ H_2O$ (2 x 100 mL), and brine (2 x 100 mL). The extract was dried ($Na_2SO_4$) and concentrated *in vacuo* to give 22.5 g (94%) of O-pivaloyl-3-nitrophenol as a white solid. Data for O-pivaloyl-3-nitrophenol: $R_f$ 0.55 (silica gel, hexanes/EtOAc, 3:1); **$^1$H NMR** (400 MHz, $CDCl_3$) 8.11 (dd, *J* = 4.2, 1.3, 1 H), 7.96 (t, *J* = 2.2, 1 H), 7.56 (dd, *J* = 8.4, 8.2, 1 H), 7.42 (dd, *J* = 6.5, 1.3, 1 H), 1.35 (s, 9 H).

*O*-Pivaloyl-3-aminophenol (structure **66** of **Scheme XVII,** where $R^1$=H, P=*t*-butyl, Z=O) To 60 mL anhydrous $CH_2Cl_2$ was added *O*-pivaloyl-3-nitrophenol (5.0 g, 22.4 mmol) and a catalytic amount (50 mg) of 10% Pd on C. The flask was repeatedly evacuated and flushed with $N_2$. The reaction flask was again evacuated and $H_2$ was introduced by balloon. After stirring under an atmosphere of $H_2$ for 3 h, the reaction flask was flushed twice with $N_2$. The suspension was then filtered through a bed of Celite™ and concentrated to give 4.15 g (96%) of *O*-pivaloyl-3-aminophenol as a viscous amber oil. Data for *O*-pivaloyl-3-aminophenol: $R_f$ 0.21 (silica gel, hexanes/EtOAc, 3:1); **$^1$H NMR** (400 MHz, $CDCl_3$) 7.12 (dd, *J* = 8.0, 8.0, 1 H), 6.52 (dd, *J* = 7.8, 2.7, 1 H), 6.44 (ddd, *J* = 8.0, 2.4, 1.4, 1H), 6.38 (t, *J* = 2.2, 1 H), 3.81 (br s, 2 H), 1.34 (s, 9 H).

General Method 8, 1,2-Dihydro-2,2,4-trimethylquinolines (Compounds of structure **57** or **67**) from anilines (Compounds of structure **56** or **66**); pressure tube version In a threaded resealable pressure tube, a solution of the aniline (a compound of structure **56** or **66**) in acetone (0.05-0.20M) was treated with iodine (5-20 mol%) and heated to 100-120 °C for 1-3 days. The reaction vessel was allowed to cool to rt and transferred to a r.b. flask. Addition of Celite™ followed by concentration afforded a fluffy orange powder which was purified by silica gel chromatography to afford the desired dihydroquinoline (Compound of structure **57** or **67**).

1,2-Dihydro-2,2,4-trimethyl-7-(1,1,1-trimethylacetoxy)quinoline (structure **67** of Scheme **XVII**, where $R^1$=H, P=*t*-butyl, Z=O This compound was prepared by General Method 8 from *O*-pivaloyl-3-aminophenol (structure **66** of **Scheme XVII**, where $R^1$=H, P=*t*-butyl, Y=O) (1.26 g, 6.53 mmol) to afford 1.06 g (60%) of 1,2-dihydro-2,2,4-trimethyl-7-(1,1,1-trimethylacetoxy)quinoline as a light brown solid. Data for 1,2-dihydro-2,2,4-trimethyl-7-(1,1,1-trimethylacetoxy)quinoline: $R_f$ 0.23 (silica gel, hexanes/EtOAc, 3:1); **$^1$H NMR** (400 MHz, $CDCl_3$) 7.00 (d, *J* = 8.3, 1 H), 6.28 (dd, *J* = 5.2, 2.3, 1 H), 5.25 (s, 1 H), 3.69 (s, 1 H,), 1.96 (d, *J* = 1.2, 3 H), 1.32 (s, 9 H), 1.26 (s, 6 H).

1,2-Dihydro-7-hydroxy-2,2,4-trimethylquinoline To 70 mL 85% ethanol was added 1,2-dihydro-2,2,4-trimethyl-7-(1,1,1-trimethylacetoxy)quinoline (1.03 g, 3.77 mmol) and 20% NaOH(aq) (3 mL) to give a clear colorless solution. The reaction was followed by TLC (hexanes/EtOAc, 3:1). After 3 h the resulting purple solution was quenched with sat'd $NH_4Cl$ (200 mL) and extracted with ethyl acetate (2 x 100 mL). The combined organic layers were washed with brine (2 x 75 mL), dried ($Na_2SO_4$), and concentrated *in vacuo* to give a dark purple oil. The oil was dissolved in a minimal amount of hexanes / ethyl acetate (3:1), and filtered though a plug of silica rinsing with a solution of hexanes / ethyl acetate (3:1). The washes were concentrated *in vacuo* to afford 710 mg (99%) of 1,2-dihydro-7-hydroxy-2,2,4-trimethylquinoline as a dark yellow oil. Data for 1,2-dihydro-7-hydroxy-2,2,4-trimethylquinoline: $R_f$ 0.30 (silica gel, hexanes/EtOAc, 3:1); **$^1$H NMR** (400 MHz, DMSO-$d_6$) 8.90 (s, 1 H), 6.70 (d, *J* = 8.2, 1 H), 5.89 (d, *J* = 2.3, 1 H), 5.85 (dd, *J* = 8.3, 2.4, 1 H), 5.65 (s, 1 H), 5,04 (s, 1 H), 1.8 (d, *J* = 1.1, 3 H), 1.14 (s, 6 H).

1,2-Dihydro-2,2,4-trimethyl-6-trifluoromethyl-8-pyranono[5,6-*g*]quinoline(Compound **238,** structure **57** of **Scheme XVII,** where $R^1$=$R^2$=H, $R^3$=trifluoromethyl, Z=O)
General Method 9: Preparation of Compounds of structure **56** or **57** from phenols. To a solution of 1,2-dihydro-7-hydroxy-2,2,4-trimethylquinoline (0.1-0.5 M) in absolute EtOH was added a β-keto ester (a compound of structure **68**) (1-3 equivuiv) in a 4 x 13.5 cm pressure tube equivuipped with a magnetic stir bar and a threaded Teflon stopcock. To this solution was added $ZnCl_2$ (1-6 equivuiv). The sealed pressure tube was heated in a oil bath at 80-120 °C for 6-72 h. The cooled solution was diluted with sat'd $NH_4Cl$ and extracted with ethyl acetate. The combined organics were concentrated on Celite™ under reduced pressure to give a free flowing powder, which was purified by flash column chromatography (silica gel 60, hexanes / ethyl acetate, 5:1) to give the desired product. Further purification could be effected by recrystallization from hexanes / toluene.

1,2-Dihydro-2,2,4-trimethyl-6-trifluoromethyl-8-pyranono[5,6-*g*]quinoline(Compound **238**, structure **55** of **Scheme XVII**, where $R^1$=$R^2$=H, $R^3$=trifluoromethyl, Z=O) This compound was prepared by General Method 9 from 1,2-dihydro-7-hydroxy-2,2,4-trimethylquinoline (1.58 g, 8.5 mmol) and ethyl 4,4,4-trifluoroacetoacetate (3.00 g, 16.8 mmol, 2.0 equivuiv) to afford 1.7 g (66%) of Compound **238** as a light yellow powder. Data for Compound **238**: $R_f$ 0.32 ( silica gel, hexanes/EtOAc, 3:1); **$^1$H NMR** (400 MHz, $C_6D_6$) 7.22 (s, 1 H), 6.15 (s, 1H), 5.97 (s, 1 H), 4.93 (s, 1 H), 3.23 (br s, 1 H), 1.66 (d, *J* = 1.1, 3 H), 0.98 (s, 6 H).

## EXAMPLE 139

1,2-Dihydro-2,2,4-trimethyl-10-isocoumarino[4,3-*g*]quinoline (Compound **239**, structure **57** of **Scheme XVI**, where $R^1$H, $R^2$=$R^3$=benzo, Z=O)
This compound was prepared by General Method 8 (EXAMPLE 138) from 7-amino-3,4-benzocoumarin (structure **56** of **Scheme XVI**, where $R^1$=H, $R^2$=$R^3$=benzo, Z=O) (180 mg, 0.85 mmol) to afford 75 mg (30%) of Compound **239** along with 150 mg (60%) of 1,2-dihydro-2,2,4-trimethyl-10-isocoumarino[3,4-*f*]quinoline as yellow solids. Data for Compound **239**: mp 246-248 °C; **$^1$H NMR** (400 MHz, $CDCl_3$) 8.18 (d, *J* = 7.6, 1 H), 8.16 (d, *J* = 7.6, 1 H), 7.80 (s, 1 H), 7.78 (t, *J* = 7.6, 1 H), 7.43 (t, *J* = 7.6, 1 H), 6.39 (s, 1 H), 5.45 (s, 1 H), 2.11 (s, 3 H), 1.33 (s, 6 H), **$^{13}$C NMR** (100 MHz, $CDCl_3$) 162.2, 152.7, 146.1, 136.4, 134.9, 130.7, 129.1, 127.2, 126.5, 120.5, 119.4, 119.0, 117.8, 107.6, 99.8, 52.7, 31.8, 19.0; Anal. Calcd for $C_{19}H_{17}NO_2$: C, 78.33; H, 5.88; N, 4.81. Found: C, 77.99; H, 5.79; N, 4.72.

## EXAMPLE 140

1,2-Dihydro-2,2,4-trimethyl-10-isoquinolono[4,3-*g*]quinoline(Compound **240,** structure **57** of **Scheme XVI,** where $R^1$=H, $R^2$=$R^3$=benzo, Z=NH)
3-Amino-6(5*H*)-phenanthridinone (structure **56** of **Scheme XVI,** where $R^1$=H, $R^2$=$R^3$=benzo, Z=NH) A mixture of 3-nitro-6(5*H*)-phenanthridinone (structure **55** of **Scheme XVI,** where $R^1$=H, $R^2$=$R^3$=benzo, Z=NH) (480 mg, 1.5 mmol) and 50 mg of 10% Pd/C in 60 mL of DMF was stirred under an atomsphere of $H_2$ for 2 h. The mixture was filtered through a Celite™ pad and the filtrate was concentrated to give 0.4 g of the crude aniline as a yellow solid. This material was used without further purification.

1,2-Dihydro-2,2,4-trimethyl-10-isoquinolono[4,3-*g*]quinoline (Compound **240,** structure **57** of **Scheme XVI,** where $R^1$=H, $R^2$=$R^3$=benzo, Z=NH) This compound was prepared by General Method 8 (EXAMPLE 238) from 3-amino-6(5*H*)-phenanthridinone (0.4 g), iodine (150 mg, 0.6 mmol), acetone (16 mL) and DMF (14 mL) to afford 220 mg (51%) of Compound **240** as a yellow solid. Data for Compound **240:** mp 301-302 °C; IR (KBr, cm-1) 3300, 3010, 1670, 1450,

1300; **$^1$H NMR** (400 MHz, $CDCl_3$) 8.28 (d, *J* = 7.6, 1 H), 8.25 (d, *J* = 7.6, 1 H), 7.90 (s, 1 H), 7.70 (t, *J* = 7.6, 1 H), 7.39 (t, *J* = 7.6, 1 H), 6.48 (s, 1 H), 5.78 (br s, 1 H), 5.42 (s, 1 H), 2.13 (s, 3 H), 1.33 (s, 6 H); **$^{13}$C NMR** (100 MHz, acetone-$d_6$) 162.4, 147.1, 139.2, 137.0, 133.3, 129.2, 128.7, 128.6, 125.8, 125.0, 121.8, 118.9, 118.4 108.5, 98.1, 52.8, 31.6, 19.0.

## EXAMPLE 141

1,2-Dihydro-2,2,4,6-tetramethyl-8-pyridono[5,6-*g*]quinoline (Compound **241,** structure **57** of **Scheme XVI,** where $R^1$=$R^2$=H, $R^3$=methyl, Z=NH)
This compound was prepared by General Method 8 (EXAMPLE 238) from Carbostyril 124 (structure **56** of **Scheme XVI**, where $R^1$=$R^2$=H, $R^3$=methyl, Z=NH) (500 mg, 2.8 mmol) to afford 175 mg (25%) of Compound **241** as a pale yellow solid. Data for Compound **241**: mp 282-284 °C; **IR** (KBr, $cm^{-1}$) 2966, 2918, 1658, 1641, 1425, 1257; **$^1$H NMR** (400 MHz, $CDCl_3$) 7.24 (s, 1 H), 6.34 (s, 1 H), 6.23 (s, 1 H), 5.37 (s, 1 H), 2.41 (s, 3 H), 2.04 (s, 3 H), 1.29 (s, 6 H); **$^{13}$C NMR** (100 MHz, $CDCl_3$) 165.0, 149.8, 146.5, 140.3, 129.2, 127.6, 119.1, 118.5, 114.9, 112.5, 97.2, 52.4, 31.8, 19.3, 18.9.

## EXAMPLE 142

1,2-Dihydro-10-hydroxy-2,2,4-trimethyl-10*H*-isochromeno[4,3-*g*]quinoline (Compound **242**, structure **62** of **Scheme XVI**, where $R^1$=H, $R^2$=$R^3$=benzo, Z=O)
To a yellow solution of Compound **239** (EXAMPLE 139) (10 mg, 0.033 mmol) in 0.5 mL of toluene at -78 °C was added 0.050 mL of DIBALH (1.5 M in toluene , 0.075 mmol), and the resulting solution was stirred at -50 ± 10 °C for 20 min. The reaction was quenched with water (1 mL) and extracted with ethyl acetate (2 x 5 mL). Removal of solvent and chromatography of the crude residue (silica gel, 20% ethyl acetate/hexanes) afforded 6 mg (63%) of Compound **242** as a colorless oil. Data for Compound **242: $^1$H** NMR (400 MHz, acetone-$d_6$) 7.74 (d, *J* = 7.8, 1 H), 7.52 (s, 1 H), 7.37 (t, *J* = 7.8, 1 H), 7.31 (d, *J* = 7.8, 1 H), 7.19 (t, *J* = 7.8, 1 H), 6.26 (d, *J* = 6.5, 1 H), 6.17 (s, 1 H), 5.97 (d, *J* = 6.5, 1 H), 5.40 (br s, 1 H), 5.29 (s, 1 H), 2.05 (s, 3 H), 1.27 (s, 6 H).

## EXAMPLE 143

1,2-Dihydro-2,2,4,6-tetramethyl-8*H*-pyrano[3,2-*g*]quinoline (Compound **243,** structure **61** of **Scheme XVI**, where $R^1$=$R^2$=H, $R^3$=methyl, Z=O)
1,2-Dihydro-2,2,4,6-tetramethyl-8-pyranono[5,6-*g*]quinoline (structure **57** of **Scheme XVI**, where $R^1$=$R^2$=H, $R^3$=methyl, Z=O) To a solution of 7-nitro-4-methylcoumarin (structure **55** of **Scheme XVI,** where $R^1$=$R^2$=H, $R^3$=methyl, Z=O) (0.61 g, 1.75 mmol) was added 50 mg of 10% Pd/C. The reaction mixture was stirred under an atmosphere of $H_2$ for 2 h. The mixture was filtered through a pad of Celite™ and the filtrate was concentrated to give 0.5 g of the crude amino compound as a yellow solid. This material was used without further purification, and was submitted to General Method 3 to afford 90 mg (20%) of 1,2-dihydro-2,2,4,6-tetramethyl-8-pyranono[5,6-*g*]quinoline as a yellow solid. Data for 1,2-dihydro-2,2,4,6-tetramethyl-8-pyranono[5,6-*g*]quinoline: mp 258-260 °C; **IR** (KBr) 3300, 2955, 1720, 1630, 1505, 1390, 1250; **$^1$H NMR** (400 MHz, $CDCl_3$) 7.27 (s, 1 H), 6.30 (s, 1 H), 6.12 (br s, 1 H), 5.84 (s, 1 H), 5.44 (s, 1 H), 2.37 (s, 3 H), 2.05 (s, 3 H), 1.32 (s, 6 H); **$^{13}$C NMR** (100 MHz, $CDCl_3$) 161.9, 155.4, 153.1, 147.1, 128.8, 127.0, 119.2, 110.3, 109.0, 98.6, 52.6, 31.8, 18.6.
1, 2-Dihydro-2.2.4.6-tetramethyl-8*H*-pyrano[3,2-*g*]quinoline (Compound **243**, structure **61** of **Scheme XVI,** where $R^1$=$R^2$=H, $R^3$=methyl, Z=O) To a solution of 1,2-dihydro-2,2,4,6-tetramethyl-8-pyranono[5,6-*g*]quinoline (15 mg, 0.06 mmol) in 1 mL of toluene at -78°C was added DIBA1-H (0.5 M in toluene, 0.24 mL, 0.12 mmol) and the resulting mixture was allowed to stir at -50°C for 60 min, generating a clear brown solution. The reaction was quenched with water (1 mL) and was extracted with ethyl acetate (2 x 10 mL). The organic extract was concentrated and was chromatographed (silica gel, 4:1 hexanes /ethyl acetate) to afford 1 mg (5%) of Compound **243** as a colorless oil. Data for Compound **243: $^1$H NMR** (400 MHz, acetone-$d_6$) 6.84 (s, 1 H), 5.96 (s, 1 H), 5.33 (t, *J* = 3.5, 1 H), 5.26 (s, 1 H), 5.21 (s, 1 H), 4.59 (d, *J* = 3.5, 2 H), 1.96 (s, 3 H), 1.93 (s, 3 H), 1.24 (s, 6 H).

## EXAMPLE 144

(*R*/*S*)-1,2,3,4-Tetrahydro-2,2,4-trimethyl-10-isoquinolono[4,3-*g*]quinoline (Compound **244,** structure **63** of **Scheme XVI,** where $R^1$=H, $R^2$=$R^3$=benzo, Z=O) Hydrogenation of Compound **240** (550 mg, 1.9 mmol) over 10% Pd/C (200 mg) in 250 mL of ethyl acetate for 14 h at rt afforded 510 mg (92%) of Compound **244** as a yellow solid. Data for Compound **244**: mp 263-264 °C; **IR** (KBr) 3304, 2960, 2928, 1658, 1606, 1467, 1267 $cm^{-1}$; **$^1$H NMR** (400 MHz, $CDCl_3$) 9.67 (br s, 1 H), 8.45 (d, *J* = 8.0, 1 H), 8.11 (d, *J* = 8.0, 1 H), 7.94 (s, 1 H), 7.69 (t, *J* = 8.0, 1 H), 7.41 (t, *J* = 8.0, 1 H), 6.25 (s, 1 H), 4.08 (br s, 1 H), 3.02 (m, 1 H), 1.81 (dd, *J* = 12.8, 5.2, 1 H), 1.49 (t, *J* = 12.8, 1 H), 1.46 (d, *J* = 6.7,

3 H), 1.29 (s, 3 H) and 1.23 (s, 3 H).

EXAMPLE 145

1,2-Dihydro-2,2,4-trimethyl-10-thioisoquinolono[4,3-*g*]quinoline (Compound **245,** structure **58** of **Scheme XVI,** where $R^1$=H, $R^2$=$R^3$=benzo, Z=O)
A mixture of Compound **240** (9 mg, 0.03 mmol) and Lawesson's reagent (41 mg, 0.1 mmol) in 2 mL of THF was stirred at 80°C for 3 h, generating a bright yellow solution. Removal of the solvent and chromatography of the crude mixture (silica gel, 1:1 ethyl acetate/hexanes) afforded 8.2 mg (90%) of Compound **245** as a yellow oil. Data for Compound **245: $^1$H NMR** (400 MHz, acetone-d6) 8.93 (d, *J* = 8.1,1 H), 8.33 (d, *J* = 8.1, 1 H), 8.01 (s, 1 H), 7.75 (t, *J* = 8.1, 1 H), 7.44 (t, *J* = 8.1, 1 H), 6.73 (s, 1 H), 5.97 (br s, 1 H), 5.51 (s, 1 H), 2.15 (s, 3 H), 1.35 (s, 6H).

EXAMPLE 146

(+)-1,2,3,4-Tetrahydro-2,2,4-trimethyl-10-isoquinolono[4,3-*g*]quinoline (Compound **246**, structure **63** of **Scheme XVI**, where $R^1$=H, $R^2$=$R^3$=benzo, Z=O)
This compound was prepared by a HPLC separation of the enantiomers of Compound **244** using a Chiracel OD-R column, using a 4:1 mixture of methanol and water as the mobile phase. The optical purity of Compound **246** was determined by HPLC to be > 99% e.e.; $[\alpha]^{20}D$ = + 106 (MeOH).

EXAMPLE 147

1,2-Dihydro-2,2,4-trimethyl-6-trifluoromethyl-8-pyridono[5,6-*g*]quinoline (Compound **247,** structure **57** of **Scheme XVII**, where $R^1$=$R^2$=H, $R^3$=trifluoromethyl, Z=NH) This compound was prepared as depicted in **Scheme XVII** and as described below.
1-*tert*-Butyloxycarbamoyl-3-nitrobenzene (structure **65** of **Scheme XVII**, where$R^1$=H, P=*t*-butyloxycarbonyl, Z=NH). General Method 10. *N*-Boc-Protection of Nitroanilines To a flame-dried 500 mL r.b. flask containing 3-nitroaniline (structure **64** of **Scheme XVII**, where $R^1$=H, Z=NH) (20.0 g, 144.8 mmol) in 150 mL THF was added di-*tert*-butyl dicarbonate (31.60 g, 144.8 mmol, 1.00 equivuiv), and the mixture was cooled to 0°C. 4-*N,N*-Dimethylaminopyridine (19.46 g, 159.3 mmol, 1.10 equivuiv) was added portion-wise, and the mixture was allowed to warm to rt overnight. Ethyl acetate (400 mL) was added, and the mixture was washed with 1M $NaHSO_4$(aq) (2 x 200 mL) and brine (200 mL), dried ($Na_2SO_4$), and concentrated under reduced pressure. Purification by flash column chromatography (silica gel, hexanes/ethyl acetate, 9:1) afforded 31.4 g (91%) of 1-*tert*-butyloxycarbamoyl-3-nitrobenzene as a white solid. Data for 1-*tert*-butyloxycarbamoyl-3-nitrobenzene: **$^1$H NMR** (400 MHz, $CDCl_3$) 8.31 (dd, 1H, *J* = 2.2, 2.2, 1H, 2-H), 7.88 (dd, *J* = 7.9, 1.5, 1H, 4-H), 7.69 (br d, *J* ≈ 7.8, 1H, 6-H), 7.44 (dd, *J* = 8.3, 8.1, 1H, 5-H), 6.74 (br s, 1H, N*H*), 1.54 [s, 9H, $(CH_3)_3CO$)].

3-*tert*-Butyloxycarbamoylaniline (structure **66** of **Scheme XVII,** where$R^1$=H, P=*t*-butyloxycarbonyl, Z=NH) To an oven-dried 1-L r.b. flask containing 1-*tert*-butyloxycarbamoyl-3-nitrobenzene (20.0 g, 83.9 mmol) in 500 mL 1:1 ethyl acetate/ethanol at rt was added 10% Pd on C (approx 1 mol%), and the mixture was stirred under an atmosphere of $H_2$ gas for 6 h. The reaction mixture was then filtered, and concentrated under diminished pressure to give 17.4 g (quant of 3-*tert*-butyloxycarbamoylaniline as a white oily solid. Data for 3-*tert*-butyloxycarbamoylaniline: **$^1$H NMR** (400 MHz, $CDCl_3$) 7.04 (t, *J* = 8.0, 8.0, 1H, 5-H), 6.98 (br s, 1H, N*H*), 6.53 (dd, *J* = 7.9, 1.8, 1H, 4-H), 6.36 (m, 2H, 6,2-H), 3.66 (br s, 2H, $NH_2$), 1.51 [s, 9H, $(CH_3)_3CO$)].

7-*tert*-Butyloxycarbamoyl-1,2-dihydro-2,2,4-trimethylquinoline (structure **67** of **Scheme XVII**, where$R^1$=H, P=*t*-butyloxycarbonyl, Z=NH) General Method 11: Skraup Cyclization of *tert*-Butyloxycarbamoylanilines To an oven-dried 1 L r.b. flask containing 3-*tert*-butyloxycarbamoylaniline (17.4 g, 83.5 mmol), $MgSO_4$ (50 g, 5 equivuiv), and 4-*tert*-butylcatechol (420 mg, 3 mol%) in 120 mL acetone (approx 0.75 M in the aniline) was added iodine (1.07 g, 5 mol%), and the mixture was heated to reflux for 8 h. The crude reaction mixture was then cooled to rt, filtered through a bed of Celite™ on a fritted-glass funnel, rinsing with ethyl acetate, dried ($Na_2SO_4$), and concentrated under reduced pressure. Purification by flash column chromatography (silica gel, hexanes/ethyl acetate, gradient elution) afforded 19.9 g (82%) of 7-*tert*-butyloxycarbamoyl-1,2-dihydro-2,2,4-trimethylquinoline as a white solid, which was further purified by recrystallization from acetonitrile to give white needles. Data for 7-*tert*-butyloxycarbamoyl-1,2-dihydro-2,2,4-trimethylquinoline: **$^1$H NMR** (400 MHz, $CDCl_3$) 6.93 (d, *J* = 8.3, 1H, 5-H), 6.81 (br s, 1H, *H*NBoc), 6.34 (m, 2H, 6,8-H), 5.21 (d, *J* = 0.9, 1H, 3-H), 3.71 (br s, 1H, N*H*), 1.94 (d, *J* = 1.0, 3H, 4-$CH_3$), 1.50 [s, 9H, $(CH_3)_3CO$)], 1.24 [s, 6H, 2-$(CH_3)_2$].
7-Amino-1,2-dihydro-2.2.4-trimethylguinoline General Method 12: Removal of Boc Protective Group from Compounds of structure **67** of **Scheme XVII**, where P=*t*-butyloxycarbonyl, Z=NH) To an oven-dried 25 mL r.b. flask containing 7-*tert*-butyloxycarbamoyl-1,2-dihydro-2,2,4-trimethylquinoline (400 mg, 1.38 mmol) in 2 mL dichloromethane at 0°C was added trifluoroacetic acid (1.06 mL, 10 equivuiv), and the mixture was allowed to warm to rt. After 3 h at rt, the reaction

mixture was diluted with 50 mL dichloromethane, transferring to a 125 mL erlynmeyer flask, and cooled to 0°C before neutralization to pH 8 with sat'd aqueous $NaHCO_3$. The biphasic mixture was transferred to a separatory funnel, the layers were separated, and the organic phase was dried ($Na_2SO_4$), and concentrated under reduced pressure to afford a light reddish oil. The crude material thus obtained was of greater than 98% purity by $^1H$ NMR, and was carried on to the next step without further purification. While the 7-amino-quinoline obtained decomposed appreciably within a few hours upon standing at rt, ethanolic solutions could be stored at -20°C for 2-3 days without substantial adverse effect on the subsequivuent reaction outcome. Typically however, the material was stored in bulk as the crystalline Boc-protected amine, and portions were hydrolysed as needed. Data for 7-amino-1,2-dihydro-2,2,4-trimethylquinoline: **$^1$H NMR** (400 MHz, $CDCl_3$) 6.86 (d, *J* = 8.2, 1H, 5-H), 5.99 (dd, *J* = 8.0, 2.3, 1H, 6-H), 5.79 (d, *J* = 2.0, 1H, 8-H), 5.12 (d, *J* = 1.4, 1H, 3-H), 3.53 (br s, 3H, N$H_2$, N*H*), 1.93 (d, *J* = 1.2, 3H, 4-C$H_3$), 1.24 [s, 6H, 2-(C$H_3$)$_2$].

1,2-Dihydro-2.2.4-trimethyl-6-trifluoromethyl-8-pyridono[5,6-*g*]quinoline (Compound **247**, structure **57** of **Scheme XVII**, where $R^1$=$R^2$=H, $R^3$=trifluoromethyl, Z=NH) General Method 13: Knorr Cyclization of 7-amino-1,2-dihydro-2,2,4-trimethylquinolines with a β-Keto Ester To an oven-dried 10 mL r.b. flask containing 7-amino-1,2-dihydro-2,2,4-trimethylquinoline (100 mg, 0.53 mmol) and ethyl 4,4,4-trifluoroacetoacetate (85.4 mL, 0.58 mmol, 1.1 equivuiv) in 2.5 mL absolute ethanol was added $ZnCl_2$ (110 mg, 0.81 mmol, 1.5 equivuiv) and the mixture was heated to reflux for 3 h. Upon cooling to rt, the reaction mixture was diluted with 40 mL ethyl acetate, and the organic solution was washed with sat'd aqueous $NH_4Cl$, dried ($Na_2SO_4$), and concentrated under reduced pressure. Purification by flash column chromatography (silica gel, hexanes/ethyl acetate, gradient elution) afforded 72 mg (44%) of Compound **247** as a bright fluorescent-yellow solid, in addition to 70 mg (40%) of Compound **248** (EXAMPLE 148) as a pale yellow crystalline solid, and 10.4 mg (6%) of Compound **249** (EXAMPLE 149) as a white solid. Data for Compound **247: $^1$H NMR** (400 MHz, $CDCl_3$) 11.45 (br s, 1H, CON*H*), 7.38 (s, 1H, 5-H), 6.66 (s, 1H, 7-H), 6.27 (s, 1H, 10-H), 5.42 (s, 1H, 3-H), 4.35 [br s, 1H, $(CH_3)_2$CN*H*], 2.03 (s, 3H, 4-C$H_3$), 1.33 [s, 6H, 2-(C$H_3$)$_2$].

## EXAMPLE 148

8-Ethoxy-1,2-dihydro-2,2,4-trimethyl-6-trifluoromethyl-8-pyrido[5,6-*g*quinoline (Compound **248,** structure **71** of **Scheme XVII,** where $R^1$=$R^2$=H, $R^3$=trifluoromethyl, $R^5$=ethyl, Z=N) This compound was obtained along with Compounds **247** and **249** as described above (EXAMPLE 147). Data for Compound **248: $^1$H NMR** (400 MHz, $CDCl_3$) 7.56 (d, 1H, *J* = 1.8, 5-H), 6.84 (s, 1H, 7-H), 6.74 (s, 1H, 10-H), 5.52 (s, 1H, 3-H), 4.47 (q, 2H, *J* = 7.0, $CH_3$C$H_2$O), 4.12 [br s, 1H, $(CH_3)_2$CN*H*], 2.09 (d, 3H, *J* = 1.3, 4-C$H_3$), 1.42 (t, 3H, *J* = 7.0, C$H_3$C$H_2$O), 1.34 [s, 6H, 2-(C$H_3$)$_2$]. This product was readily converted to the 2-quinolone isomer Compound **247** by heating neat with 10 equivuiv p-chlorophenol at 180°C for 3 h, giving Compound **247** in >80% yield.

## EXAMPLE 149

(*R,S*)-1,2,6,7-Tetrahydro-6-hydroxy-2,2,4-trimethyl-6-trifluoromethyl-8-pyridono[5,6-*g*]quinoline (Compound **249,** structure **69** of **Scheme XVII,** where $R^1$=$R^2$=H, $R^3$=trifluoromethyl, Z=NH) This compound was obtained along with Compounds **247** and **248** as described above (EXAMPLE 147). Data for Compound **249: $^1$H NMR** (400 MHz, DMSO-$d_6$) 10.16 (s, 1H, CON*H*), 7.09 (s, 1H, 5-H), 6.61 (s, 1H, O*H*), 6.24 (s, 1H, 10-H), 6.01 [s, 1H, $(CH_3)_2$CN*H*], 5.21 (s, 1H, 3-H), 2.80 and 2.72 (ABq, 2H, $J_{AB}$ = 16.4, 7-H), 1.86 (s, 3H, 4-C$H_3$), 1.19 and 1.17 [2s, 2 x 3H, 2-(C$H_3$)$_2$]. This product was readily converted to the 2-quinolone isomer Compound **247** by heating to 60°C in benzene or toluene with a catalytic amount of *p*-TsOH for 2 h, giving Compound **247** in >95% yield.

## EXAMPLE 150

(*R*/*S*)-1,2,3,4-Tetrahydro-2,2,4-trimethyl-6-trifluoromethyl-8-pyranono[5,6-*g*]quinoline (Compound **250**, structure **63** of **Scheme XVIII**, where $R^1$=$R^2$=H, $R^3$=trifluoromethyl, Z=O) (*R*/*S*)-1,2,3,4-Tetrahydro-2,2,4-trimethyl-7-(1,1,1-trimethylacetoxy)quinoline (structure **72** of **Scheme XVIII**, where $R^1$=H, P=*t*-butyl, Z=O) In a dry r.b. flask equivuipped with a magnetic stir bar was suspended 1,2-dihydro-2,2,4-trimethyl-7-(1,1,1-trimethylacetoxy)quinoline (structure **67** of **Scheme XVIII**, where $R^1$=H, P=*t*-butyl, Z=O; EXAMPLE 138) (1.01 g, 3.37 mmol) and 10% Pd/C (200 mg) in $CH_2Cl_2$. The flask was charged with $H_2$ gas and allowed to react for 12h with constant stirring. The suspension was filtered though a bed of Celite™, washed with EtOAc (2 x 50 mL) and concentrated *in vacuo* to afford 996 mg (98%) of (*R*/*S*)-1,2,3,4-tetrahydro-2,2,4-trimethyl-7-(1,1,1-trimethylacetoxy) quinoline as a light brownish-red solid. Data for (*R*/*S*)-1,2,3,4-tetrahydro-2,2,4-trimethyl-7-(1,1,1-trimethylacetoxy) quinoline: **$^1$H NMR** (400 MHz, $CDCl_3$) 7.10 (dd, *J* = 8.5, 0.9, 1 H), 6.30 (dd, *J* = 8.4, 2.4, 1 H), 6.13 (d, *J* = 2.2, 1 H), 3.62 (br s, 1 H), 2.87 (m, 1 H), 1.71 (dd, *J* = 13, 5.4, 1 H), 1.41 (apparent t, *J* = 13, 1 H), 1.31 (m, 10 H), 1.22 (s, 3 H), 1.16 (s, 3 H).

(*R*/*S*)-1,2,3,4-Tetrahydro-7-hydroxy-2,2,4-trimethylquinoline This compound was prepared as described above for 1,2-dihydro-7-hydroxy-2,2,4-trimethylquinoline (EXAMPLE 138) from (*R*/*S*)-1,2,3,4-tetrahydro-2,2,4-trimethyl-7-(1,1,1-trimethylacetoxy)quinoline (230 mg, 0.845 mmol) to afford (*R*/*S*)-1,2,3,4-tetrahydro-7-hydroxy-2,2,4-trimethylquinoline, which was used in the following reaction without further purification.

(*R*/*S*)-1,2,3,4-Tetrahydro-2,2,4-trimethyl-6-trifluoromethyl-8-pyranono[5,6-*g*]quinoline (Compound **250,** structure **63** of **Scheme XVIII,** where $R^1$=$R^2$=H, $R^3$=trifluoromethyl, Z=O) This compound was prepared by General Method 9 (EXAMPLE 238) from crude (*R*/*S*)-1,2,3,4-tetrahydro-7-hydroxy-2,2,4-trimethylquinoline and ethyl 4,4,4-trifluoroacetoacetate (310 mg, 1.69 mmol, 2 equivuiv) to afford 160 mg (61% overall) of Compound **250** as a yellow solid. Data for Compound **250:** $R_f$ 0.4 (hex/EtOAc, 3:1); **$^1$H NMR** (400 MHz, $CDCl_3$) 7.41 (s, 1 H), 6.37 (s, 1 H), 6.33 (s, 1 H), 4.46 (s, 1 H), 2.92 (m, 1 H), 1.80 (dd, *J* = 13, 5.0, 1 H), 1.42 (dd, *J* = 13, 13, 1 H), 1.38 (d, *J* = 6.0, 3 H), 1.31 (s, 3 H), 1.25 (s, 3 H).

## EXAMPLE 151

1,2-Dihydro-2,2,4-trimethyl-6-trifluoromethyl-8-thiopyranono[5,6-*g*]quinoline (Compound **251**, structure **58** of **Scheme XVI**, where $R^1$=$R^2$=H, $R^3$=trifluoromethyl, Z=O) In a dry pressure tube equivuipped with a magnetic stir bar was dissolved Compound **238** (EXAMPLE 138) (50 mg, 0.159 mmol) and Lawesson's reagent (320 mg, 0.79 mmol, 5 equivuiv) in 15 mL toluene. The resulting solution was heated at 100°C for 20 h. The cooled solution was concentrated on Celite ™ to give a free flowing powder which was purified by flash column chromotography (silica gel, hexanes/EtOAc, 5:1) to give 40 mg (78%) of Compound **251** as a bright red solid. Data for Compound **251**: $R_f$ 0.36 (silica gel, hex/EtOAc, 3:1); **$^1$H NMR** (400 MHz, acetone-$d_6$) 7.25 (s, 1 H,), 4.03 (s, 1 H), 6.89 (br s, 1 H), 6.53 (s, 1 H), 5.62 (s, 1 H), 2.77 (d, *J* = 1.1,3 H), 1.39 (s, 6 H).

## EXAMPLE 152

(*R*/*S*)-1,2,3,4-Tetrahydro-2,2,4-trimethyl-6-trifluoromethyl-8-thiopyranono[5,6-*g*]quinoline (Compound **252**, structure **76** of **Scheme XIX**, where $R^1$=$R^2$=H, $R^3$=trifluoromethyl, Z=O) In a dry pressure tube equivuipped with a magnetic stir bar was dissolved Compound **250** (EXAMPLE 150) (26 mg, 0.0836 mmol) and Lawesson's reagent (60 mg, 0.41 mmol, 5 equivuiv) in 15 mL toluene. The resulting solution was heated at 100°C for 20 h. The cooled solution was concentrated on Celite™ to give a free flowing powder which was purified by flash column chromotography (silica gel, hexanes/EtOAc, 5:1) to afford 19.2 mg (71%) of Compound **252** as a bright orange solid. Data for Compound **252**: Rf 0.37 (silica gel, hex/EtOAc, 3:1); **$^1$H NMR** (400 MHz, $CDCl_3$) 7.43 (s, 1 H), 7.16 (s, 1 H), 6.45 (s, 1 H), 4.59 (br s, 1 H), 2.93 (m, 1 H), 1.82 (dd, *J* = 13, 5.1, 1 H), 1.45 (app t, *J* = 13, 1 H), 1.39 (d, *J* = 6.6, 3 H), 1.34 (s, 3 H), 1.27 (s, 3 H).

## EXAMPLE 153

6-Chloro(difluoro)methyl-1,2-dihydro-2,2,4-trimethyl-8-pyranono[5,6-*g*]quinoline (Compound **253**, structure **57** of **Scheme XVII**, where $R^1$=$R^2$=H, $R^3$=chlorodifluoromethyl, Z=O)

This compound was prepared by General Method 9 (EXAMPLE 238) from 1,2-dihydro-7-hydroxy-2,2,4-trimethylquinoline (EXAMPLE 138) (71 mg, 0.37 mmol) and methyl 4-chloro-4,4-difluoroacetoacetate (150 mg, 1.62 mmol, 2.2 equivuiv) to afford 17.6 mg (15 %) of Compound **253** as a light yellow solid. Data for Compound **253:** $R_f$ 0.35 (hex/EtOAc, 3:1); **$^1$H NMR** (400 MHz, $CDCl_3$) 7.40 (s, 1 H), 6.33 (s, 1 H), 6.31 (s, 1 H), 5.41 (s, 1 H), 4.42 (br s, 1 H), 2.02 (s, 3 H), 1.36 (s, 6 H).

## EXAMPLE 154

9-Acetyl-1,2-dihydro-2,2,4-trimethyl-6-trifluoromethyl-8-pyridono[5,6-*g*]quinoline (Compound **254,** structure **59** of **Scheme XVI**, where $R^1$=$R^2$=H, $R^3$=trifluoromethyl, $R^4$=acetyl, Z=N)

To an oven-dried 10-mL r.b. flask containing Compound **247** (15 mg, 0.049 mmol) in 1 mL dichloromethane at rt was added acetic anhydride (0.10 mL, xs) and 4-*N,N*-dimethylaminopyridine (6.5 mg, 0.054 mmol, 1.1 equivuiv), and the mixture was stirred 10 min. Dichloromethane (20 mL) was added, and the solution was washed with 1M pH 7 potassium phosphate buffer, dried ($Na_2SO_4$), and concentrated under reduced pressure. Purification by flash column chromatography (silica gel, hexanes/ethyl acetate, gradient elution) afforded 16 mg (92%) of Compound **254** as a yellow oily solid. Data for Compound **254**: **$^1$H NMR** (400 MHz, $CDCl_3$) 7.66 (s, 1H, 5-H), 7.08 (s, 1H, 7-H), 6.83 (s, 1H, 10-H), 5.63 (s, 1H, 3-H), 4.31 [br s, 1H, $(CH_3)_2CNH$], 2.38 (s, 3H, $CH_3CON$), 2.12 (s, 3H, 4-$CH_3$), 1.48 [s, 6H, 2-$(CH_3)_2$].

**EXAMPLE 155**

1,2-Dihydro-2,2,4,10-tetramethyl-6-trifluoromethyl-8-pyridono[5,6-*g*]quinoline (Compound **255**, structure **57** of **Scheme XVII**, where $R^1$=methyl, $R^2$=H, $R^3$=trifluoromethyl, Z=NH) 6-*tert*-Butyloxycarbamoyl-2-nitrotoluene (structure **65** of **Scheme XVII**, where $R^1$=methyl, P=*t*-butyloxycarbonl, Z=NH) This intermediate was prepared from 2-methyl-3-nitroaniline (5.00 g, 32.8 mmol) by General Method 10 (EXAMPLE 147), affording 7.44 g (90%) of 6-*tert*-butyloxycarbamoyl-2-nitrotoluene as an off-white solid. Data for 6-*tert*-butyloxycarbamoyl-2-nitrotoluene: **$^1$H NMR** (400 MHz, $CDCl_3$) 7.98 (br d, *J* = 8.0, 1H, 5-H), 7.51 (br d, *J* = 8.1, 1H, 3-H), 7.28 (dd, *J* = 7.6, 3.4, 1H, 4-H), 6.58 (br s, 1H, N*H*), 2.34 (s, 3H, 1-C$H_3$), 1.53 [s, 9H, (C$H_3$)$_3$CO)].

2-Amino-6-*tert*-butyloxycarbamoyltoluene (structure **66** of **Scheme XVII,** where $R^1$=methyl, P=*t*-butyloxycarbonl, Z=NH) This compound was prepared from 6-*tert*-butyloxycarbamoyl-2-nitrotoluene (4.60 g, 18.2 mmol) in a manner similar to that described for 3-*tert*-butyloxycarbamoylaniline (EXAMPLE 147), affording 4.00 g (99%) of 2-amino-6-*tert*-butyloxycarbamoyltoluene as a colorless oil. Data for 2-amino-6-*tert*-butyloxycarbamoyltoluene: **$^1$H NMR** (400 MHz, $CDCl_3$) 7.04 (br d of ABq, $J_{AB}$ = 8.0, $J_A$ = 0, $J_B$ = 7.9, 2H, 5,4-H), 6.49 (d, *J* = 8.3, 1H. 3-H), 6.26 (br s, 1H, N*H*), 3.61 (br s, 2H, N$H_2$), 2.02 (s, 3H, 1-C$H_3$), 1.51 [s, 9H, (C$H_3$)$_3$CO)].

7-*tert*-Butyloxycarbamoyl-1,2-dihydro-2,2,4,8-tetramethylquinoline (structure **67** of **Scheme XVII,** where $R^1$=methyl, P=*t*-butyloxycarbonl, Z=NH) This compound was prepared from 2-amino-6-*tert*-butyloxycarbamoyltoluene (4.00 g, 18.0 mmol) according to General Method 11 (EXAMPLE 147), affording 4.56 g (84%) of 7-*tert*-butyloxycarbamoyl-1,2-dihydro-2,2,4,8-tetramethylquinoline as a white solid. Data for 7-*tert*-butyloxycarbamoyl-1,2-dihydro-2,2,4,8-tetramethylquinoline: **$^1$H NMR** (400 MHz, $CDCl_3$) 6.94 and 6.88 (br ABq, $J_{AB}$ = 8.3, 2H, 6,5-H), 6.16 (br s, 1H, *H*NBoc), 5.27 (s, 1H, 3-H), 3.61 (br s, 1H, (CH$_3$)$_2$CN*H*]) 2.04 (s, 3H, 8-C$H_3$), 1.97 (s, 3H, 4-C$H_3$), 1.50 (s, 9H, (C$H_3$)$_3$CO)]), 1.28 (s, 6H, 2-(C$H_3$)$_2$).

7-Amino-1,2-dihydro-2,2,4,8-tetramethylquinoline This compound was prepared by General Method 12 (EXAMPLE 147) from 7-*tert*-butyloxycarbamoyl-1,2-dihydro-2,2,4,8-tetramethylquinoline (400 mg, 1.32 mmol) affording 267 mg (quant) of 7-amino-1,2-dihydro-2,2,4,8-tetramethylquinoline as a light reddish oil. Data for 7-amino-1,2-dihydro-2,2,4,8-tetramethylquinoline: **$^1$H NMR** (400 MHz, $CDCl_3$) 6.82 (d, *J* = 8.2, 1H, 5-H), 6.08 (d, *J* = 8.1, 1H, 6-H), 5.15 (d, *J* = 1.2, 1H, 3-H), 3.56 (br s, 3H, N$H_2$, N*H*), 1.95 (d, *J* = 1.2, 3H, 4-C$H_3$), 1.91 (s, 3H, 8-C$H_3$), 1.27 [s, 6H, 2-(C$H_3$)$_2$].

1,2-Dihydro-2,2,4,10-tetramethyl-6-trifluoromethyl-8-pyridono[5,6-*g*]quinoline (Compound **255**, structure **57** of **Scheme XVII**, where $R^1$=methyl, $R^2$=H, $R^3$=trifluoromethyl, Z=NH) This compound was prepared by General Method 13 (EXAMPLE 147) from 7-amino-1,2-dihydro-2,2,4,8-tetramethylquinoline (100 mg, 0.49 mmol) and ethyl 4,4,4-trifluoroacetoacetate (107 mL, 0.73 mmol, 1.5 equivuiv) affording 75 mg (47%) of Compound **255** as a fluorescent-yellow solid. Data for Compound **255**: **$^1$H NMR** (400 MHz, $CDCl_3$) 9.23 (br s, 1H, CON*H*), 7.37 (s, 1H, 5-H), 6.67 (s, 1H, 7-H), 5.45 (s, 1H, 3-H), 4.14 [br s, 1H, (CH$_3$)$_2$CN*H*], 2.12 (s, 3H, 10-C$H_3$), 2.04 (d, *J* = 1.1, 3H, 4-C$H_3$), 1.37 [s, 6H, 2-(C$H_3$)$_2$].

**EXAMPLE 156**

1,2-Dihydro-2,2,4-trimethyl-6-(1,1,2,2,2-pentafluoroethyl)-8-pyranono[5,6-*g*]quinoline (Compound **256**, structure **57** of **Scheme XVII**, where $R^1$=$R^2$=H, $R^3$=pentafluoroethyl, Z=O) This compound was prepared by General Method 9 (EXAMPLE 238) from 1,2-dihydro-7-hydroxy-2,2,4-trimethylquinoline (EXAMPLE 138) (67 mg, 0.35 mmol) and ethyl 4,4,5,5,5- pentafluoropropionylacetate (179 mg, 0.76 mmol, 2.2 equivuiv) to afford 11.8 mg (10 %) of Compound **256** as a light yellow solid. Data for Compound **256**: **$^1$H NMR** (400 MHz, $CDCl_3$) 7.31 (s, 1 H), 6.35 (s, 1 H), 6.33 (s, 1 H), 5.40 (s, 1 H), 4.54 (s, 1 H), 1.99 (d, *J* = 1.1, 3 H), 1.35 (s, 6 H).

**EXAMPLE 157**

(*R*/*S*)-6-Chloro(difluoro)methyl-1,2,3,4-tetrahydro-2,2,4-trimethyl-8-pyranono[5,6-*g*]quinoline (Compound **257**, structure **63** of **Scheme XVIII**, where $R^1$=$R^2$=H, $R^3$=chlorodifluoromethyl, Z=O) This compound was prepared by General Method 9 (EXAMPLE 238) from (*R*/*S*)-1,2,3,4-tetrahydro-7-hydroxy-2,2,4-trimethylquinoline (EXAMPLE 150) (57 mg, 0.29 mmol) and methyl 4-chloro-4,4-difluoroacetoacetate (120 mg, 0.645 mmol, 2.2 equivuiv) to afford 35.6 mg (38 %) of Compound **257** as a light yellow solid. Data for Compound **257**: $R_f$ 0.37 (hex/EtOAc, 3:1); **$^1$H NMR** (400 MHz, $CDCl_3$) 7.55 (s, 1 H), 6.36 (s, 1 H), 6.32 (s, 1 H), 4.53 (brs, 1 H), 2.95 (m, 1 H ), 1.80 (ddd, *J* = 13, 5.1, 1.5, 1 H), 1.45 (apparent t, *J* = 13, 1 H), 1.39 (d, *J* = 6.7, 3 H), 1.32 (s, 3 H), 1.27 (s, 3 H).

**EXAMPLE 158**

7-Chloro-1,2-dihydro-2,2,4-trimethyl-6-trifluoromethyl-8-pyranono[5,6-*g*]quinoline (Compound **258**, structure **57** of **Scheme XVII**, where $R^1$=H, $R^2$=Cl, $R^3$=trifluoromethyl, Z=O) This compound was prepared by General Method 9 (EXAMPLE 238) from 1,2-dihydro-7-hydroxy-2,2,4-trimethylquinoline (EXAMPLE 138) (78 mg, 0.41 mmol) and ethyl

2-chloro-4,4,4-trifluoroacetoacetate (195 mg, 0.898 mmol, 2.2 equivuiv) to afford 7.2 mg (6%) of Compound **258** as a red solid. Data for Compound **258:** Rf 0.33 (hex/EtOAc, 3:1); **$^1$H NMR** (400 MHz, $CDCl_3$) 7.37 (s, 1 H), 6.32 (s, 1 H), 5.42 (s, 1 H), 4.54 (br s, I H), 2.01 (d, *J* = 1.0, 3 H), 1.31 (s, 6 H).

## EXAMPLE 159

(*R*/*S*)-7-Chloro-1,2,3,4-tetrahydro-2,2,4-trimethyl-6-trifluoromethyl-8-pyranono[5,6-*g*]quinoline (Compound **259**, structure **63** of **Scheme XVIII**, where $R^1$=H, $R^2$=Cl, $R^3$=trifluoromethyl, Z=O) This compound was prepared by General Method 9 (EXAMPLE 238) from (*R*/*S*)-1,2,3,4-tetrahydro-7-hydroxy-2,2,4-trimethylquinoline (EXAMPLE 150) (57 mg 0.29 mmol) and ethyl 2-chloro-4,4,4-trifluoroacetoacetate (140 mg, 0.645 mmol, 2.2 equivuiv) to afford 6.8 mg (7%) of Compound **259** as a yellow solid. Data for Compound **259**: $R_f$ 0.35 (hex/EtOAc, 3:1); **$^1$H NMR** (400 MHz, $CDCl_3$) 7.53 (s, 1 H), 6.32 (s, 1 H), 4.51 (br s, 1 H), 2.93 (m, 1 H), 1.81 (dd, *J* = 13, 3.7, 1 H), 1.44 (apparent t, *J* = 13, 3 H), 1.31 (s, 3 H), 1.25 (s, 3 H).

## EXAMPLE 160

(*R*/*S*)-1,2,3,4-Tetrahydro-2,2,4-trimethyl-6-trifluoromethyl-8-pyridono[5,6-*g*]quinoline (Compound **260**, structure **63** of **Scheme XVIII**, where $R^1$=$R^2$=H, $R^3$=trifluoromethyl, Z=NH)

(*R*/*S*)-7-*tert*-Butyloxycarbamoyl-1,2,3,4-tetrahydro-2,2,4-trimethylquinoline (structure **72** of **Scheme XVIII**, where $R^1$=H, P=*t*-butyloxycarbonyl, Y=NH) To an oven-dried 100 mL round-bottomed flask containing 7-*tert*-butyloxycarbamoyl-1,2-dihydro-2,2,4-trimethylquinoline (EXAMPLE 147) (200 mg, 0.69 mmol) in 50 mL 2:1 ethyl acetate/ethanol at rt was added 10% Pd on C (approx 1 mol%), and the mixture was stirred under an atmosphere of $H_2$ for 4 h. The reaction mixture was then filtered, and concentrated under diminished pressure to give 201 mg (quant) of 7-*tert*-butyloxycarbamoyl-1,2,3,4-tetrahydro-2,2,4-trimethylquinoline as a white oily solid. Data for (*R*/*S*)-7-*tert*-butyloxycarbamoyl-1,2,3,4-tetrahydro-2,2,4-trimethylquinoline: **$^1$H NMR** (400 MHz, $CDCl_3$) 7.02 (d, *J* = 8.7, 1H, 5-H), 6.73 (br s, 1H, *H*NBoc), 6.39 (dd, *J* = 8.3, 2.2, 1H, 6-H), 6.29 (br s, 1H, 8-H), 3.62 (br s, 1H, N*H*), 2.85 (ddq, *J* = 12.5, 12.3, 6.4, 1H, 4-H), 1.70 and 1.39 [d of ABq, $J_{AB}$ = 12.8, $J_A$ = 5.5 Hz (3-Hequiv), $J_B$ = 12.6 Hz (3-$H_{ax}$)2H], 1.49 [s, 9H, $(CH_3)_3CO$)], 1.29 (d, *J* = 6.7, 3H, 4-$CH_3$), 1.21 (s, 3H, 2-$CH_3$), 1-14 (s, 3H, 2-$CH_3$).

(*R*/*S*)-7-Amino-1,2,3,4-tetrahydro-2,2,4-trimethylquinoline This compound was prepared by General Method 12 (EXAMPLE 147) from 7-*tert*-butyloxycarbamoyl-1,2,3,4-tetrahydro-2,2,4-trimethylquinoline (150 mg, 0.51 mmol) to afford 98 mg (quant) of (*R*/*S*)-7-amino-1,2,3,4-tetrahydro-2,2,4-trimethylquinoline as a light reddish oil. Data for 7-amino-1,2,3,4-tetrahydro-2,2,4-trimethylquinoline: **$^1$H NMR** (400 MHz, $CDCl_3$) 6.92 (dd, *J* = 8.0, 0.8, 1H, 5-H), 6.02 (dd, *J* = 8.2, 2.3, 1H, 6-H), 5.77 (d, *J* = 2.3, 1H, 8-H), 3.39 (br s, 3H, $NH_2$, N*H*), 2.81 (ddq, *J* = 12.6, 12.3, 6.4, 1H, 4-H), 1.68 and 1.38 [d of ABq, $J_{AB}$ = 12.8, $J_A$ = 5.5 Hz (3-$H_{equiv}$), $J_B$ = 12.5 Hz (3-$H_{ax}$)2H], 1.26 (d, *J* = 6.7, 3H, 4-$CH_3$), 1.19 (s, 3H, 2-$CH_3$), 1.14 (s, 3H, 2-$CH_3$).

1,2,3,4-Tetrahydro-2,2,4-trimethyl-6-trifluoromethyl-8-pyridono[5,6-*g*]quinoline (Compound **260,** structure **63** of **Scheme XVIII,** where $R^1$=$R^2$=H, $R^3$=trifluoromethyl, Z=NH) This compound was prepared by General Method 13 (EXAMPLE 147) from (*R*/*S*)-7-amino-1,2,3,4-tetrahydro-2,2,4-trimethylquinoline (98 mg, 0.51 mmol) and ethyl 4,4,4-trifluoroacetoacetate (82 mL, 0.56 mmol, 1.1 equivuiv) to afford 66 mg (42%) of Compound **260** as a fluorescent-yellow solid. Data for Compound **260:** **$^1$H NMR** (400 MHz, $CDCl_3$) 11.32 (br s, 1H, CON*H*), 7.50 (s, 1H, 5-H), 6.64 (s, 1H, 7-H), 6.41 (s, 1H, 10-H), 4.55 [br s, 1H, $(CH_3)_2CNH$], 2.91 (ddq, *J* = 12.6, 12.4, 6.3, 1H, 4-H), 1.76 and 1.41 [d of ABq, $J_{AB}$ = 12.8, $J_A$ = 5.5 Hz (3-Hequiv), $J_B$ = 12.4 Hz (3-$H_{ax}$)2H], 1.37 (d, *J* = 6.8, 3H, 4-$CH_3$), 1.22 (s, 3H, 2-$CH_3$), 1.18 (s, 3H, 2-$CH_3$).

## EXAMPLE 161

1,2-Dihydro-2,2,4,9-tetramethyl-6-trifluoromethyl-8-pyridono[5,6-*g*]quinotine (Compound **261**, structure **57** of **Scheme XVI**, where $R^1$=$R^2$=H, $R^3$=trifluoromethyl, $R^4$=methyl) To an oven-dried 50-mL r.b. flask containing Compound **247** (500.0 mg, 1.62 mmol) in 5 mL THF at 0°C was added portion-wise sodium hydride (71.4 mg of a 60% dispersion in mineral oil, 1.78 mmol, 1.10 equivuiv). After 30 min, iodomethane (101 mL, 1.62 mmol, 1.00 equivuiv) was added, and the mixture was allowed to warm to rt, and after 4 h, the reaction mixture was cooled to 0°C, and water (5 mL) was added. The reaction mixture was then diluted with 100 mL ethyl acetate, and the organic solution was washed with 50 mL brine, dried ($Na_2SO_4$), and concentrated under reduced pressure. Purification by flash column chromatography (silica gel, hexanes/ethyl acetate, gradient elution) afforded 497 mg (95%) of Compound **261** as a bright fluorescent-yellow solid. Data for Compound **261:** **$^1$H NMR** (400 MHz, $CDCl_3$) 7.41 (d, *J* = 1.7, 1H, 5-H), 6.73 (s, 1H, 7-H), 6.28 (s, 1H, 10-H), 5.42 (s, 1H, 3-H), 4.36 [br s, 1H, $(CH_3)_2CNH$], 3.62 (s, 3H, $NCH_3$), 2.04 (d, J = 1.2, 3H, 4-$CH_3$), 1.33 [s, 6H, 2-$(CH_3)_2$].

## EXAMPLE 162

1,2-Dihydro-2,2,4-trimethyl-8-trifluoromethyl-6-pyridono[5,6-*g*]quinoline (Compound **262**, structure **70** of **Scheme XVII**, where $R^1$=$R^2$=H, $R^3$=trifluoromethyl, Z=NH) An alternative procedure for the Knorr reaction combined 7-amino-1,2-dihydro-2,2,4-trimethylquinoline (EXAMPLE 147) (131 mg, 0.70 mmol) and ethyl 4,4,4-trifluoroacetoacetate (154 mL, 1.05 mmol, 1.5 equivuiv) with 0.5 mL polyphosphoric acid (PPA) in a 10-mL r.b. flask and the mixture was heated to 100°C for 2 h. The cooled reaction mixture was diluted with 140 mL ethyl acetate, and the solution was washed with neutralized to pH 8 with 50 mL sat'd aqueous $NaHCO_3$. The layers were separated, and the organic phase was washed with 50 mL brine, dried ($Na_2SO_4$), and concentrated under reduced pressure. Purification by flash column chromatography (silica gel, hexanes/ethyl acetate, gradient elution) afforded 79 mg (37%) of Compound **247** along with 8 mg (4%) of Compound **262** as a fluorescent-yellow solid. Data for Compound **262:** **$^1$H NMR** (400 MHz, $CDCl_3$) 10.50 (br s, 1H, C=$CCF_3$N*H*), 7.33 (s, 1H, 5-H), 6.62 (s, 1H, 7-H), 6.17 (s, 1H, 10-H), 5.33 (s, 1H, 3-H), 4.21 [br s, 1H, $(CH_3)_2$CN*H*], 2.04 (s, 3H, 4-C$H_3$), 1.36 [s, 6H, 2-(C$H_3$)$_2$].

## EXAMPLE 163

6-[Dichloro(ethoxy)methyl]-1,2-dihydro-2,2,4-trimethyl-8-pyranono[5,6-*g*]quinoline (Compound **263,** structure **57** of **Scheme XVII,** where $R^1$=$R^2$=H, $R^3$=dichloro(ethoxy)methyl, Z=O)
This compound was prepared by General Method 9 (EXAMPLE 238) from 1,2-dihydro-7-hydroxy-2,2,4-trimethylquinoline (EXAMPLE 138) (67 mg, 0.35 mmol) and ethyl 4,4,4-trichloroacetoacetate (179 mg, 0.77 mmol, 2.2 equivuiv) to afford 30 mg (24%) of Compound 263 as a light orange solid. Data for Compound **263:** Rf 0.28 (hex/EtOAc, 3:1); **$^1$H NMR** (400 MHz, $CDCl_3$) 7.97 (s, 1 H), 6.51 (s, 1 H), 6.32 (s, 1 H), 4.42 (q, *J* = 7.2, 2 H), 2.92 (m, 1 H), 1.79 (dd, *J* = 13, 5.1, 1 H), 1.40 (m, 4 H), 1.38 (d, *J* = 6.6, 3 H), 1.30 (s, 3 H), 1.25 (s, 3 H).

## EXAMPLE 164

5-(3-Furyl)-1,2-dihydro-2,2,4-trimethyl-8-pyranono[5,6-*g*]quinoline (Compound **264,** structure **57** of **Scheme XVII,** where $R^1$=$R^2$=H, $R^3$=3-furyl, Z=O)
This compound was prepared by General Method 9 (EXAMPLE 238) from 1,2-dihydro-7-hydroxy-2,2,4-trimethylquinoline (EXAMPLE 138) (120 mg, 0.62 mmol) and ethyl β-oxo-3-furanpropionate (227 mg, 1.25 mmol, 2 equivuiv) to afford 6.4 mg (3%) of Compound **264** as a light yellow solid. Data for Compound **264:** $R_f$ 0.30 (hex/EtOAc, 3:1); **$^1$H NMR** (400 MHz, $CDCl_3$) 7.76 (s, 1 H), 7.76 (dd, *J* = 3.5, 1.8, 1 H), 7.34 (s, 1 H), 6. 66 (d, *J* = 1.7, 1 H), 6.35 (s, 1 H), 6.06 (s, 1 H), 5.36 (s, 1 H), 4.34 (s, 1 H), 1.95 (d, *J* = 1.1, 3 H), 1.34 (s, 6 H).

## EXAMPLE 165

1,2-Dihydro-1,2,2,4-tetramethyl-6-trifluoromethyl-8-pyranono[5,6-*g*]quinoline (Compound **265,** structure **60** of **Scheme XVI,** where $R^1$=$R^2$=$R^5$=H, $R^3$=trifluoromethyl, Z=O)
In a dry r.b. flask equivuipped with a magnetic stir bar was dissolved Compound **238** (50 mg, 0.162 mmol) and paraformaldehyde (48 mg, 1.62 mmol, 10 equivuiv) in glacial acetic acid (10 mL). To this bright yellow solution was added NaCNBH3 (50 mg, 0.81 mmol, 5 equivuiv). The solution stirred for 18 h under an atmosphere of $N_2$. In a separate flask was prepared a suspension of 100 g ice and 20 mL of 20% NaOH(aq). The reaction mixture was slowly poured over the NaOH solution, extracted with EtOAc (3 x 50 mL), washed with brine, dried ($Na_2SO_4$) and concentrated *in vacuo* to afford 50.6 mg (97%) of Compound **265** as a bright yellow solid. Data for Compound **265**: Rf 0.39 (hex/EtOAc, 3: 1); **$^1$H NMR** (400 MHz, $CDCl_3$) 7.20 (d, *J* = 1.8, 1 H), 6.36 (s, 2 H), 5.36 (d, *J* = 1.0, 1 H), 2.88 (s, 3 H), 2.00 (d, *J* = 1.1, 3 H), 1.39 (s, 6 H).

## EXAMPLE 166

1,2-Dihydro-6-trifluoromethyl-2,2,4-trimethyl-9-thiopyran-8-ono[5,6-*g*] quinoline (Compound **266**, structure **57** of **Scheme XVII**, where $R^1$=$R^2$=H, $R^3$=trifluoromethyl, Z=S)
3-Amino-*S*-*t*-butyloxycarbonyl thiophenol (structure **66** of **Scheme XVII**, where $R^1$=H, P=*t*-butyloxycarbonyl, Z=S) To a solution of 3-aminothiophenol (500 mg, 4.0 mmol) and di-*t*-butyl dicarbonate (872 mg, 4.0 mmol) in 10 mL of dry dichloromethane at 0°C was added dropwise, triethylamine (557 mL, 4.0 mmol). When the addition was complete, the reaction was allowed to warm to rt and the resulting mixture was stirred for 16 h. The reaction mixture was concentrated *in vacuo* and the residue was then diluted with 20 mL of ethyl acetate and washed with water (2x10 mL), dried ($Na_2SO_4$) and concentrated *in vacuo* to an oil that was subjected to flash chromatography (silica gel, hexanes/ethyl acetate, 7: 3) which gave 274 mg (30%) of 3-amino-*S*-*t*-butyloxycarbonyl thiophenol as a clear oil. Data for 3-amino-*S*-*t*-butyloxycarbonyl thiophenol: **$^1$H NMR** (400 MHz, $CDCl_3$) 7.12 (apparent t, *J* = 8.2, 1H), 6.90 (d, *J* = 8.2, 1H), 6.84 (d, *J* = 2.2, 1H), 6.68 (dd, *J* = 8.2, 2.2, 1H), 3.68 (br s, 2H), 1.56 (s, 9H).

7-*t*-Butyloxycarbonylthio-1,2-dihydro-2,2,4-trimethylquinoline (structure **67** of **Scheme XVII**, where $R^1$=H, P=*t*-butyloxycarbonyl, Z=S) This compound was prepared by General Method 13 (EXAMPLE 147) from 3-amino-S-t-butyloxycarbonyl thiophenol (274 mg, 1.2 mmol) to afford 148 mg (40%) of 7-*t*-butyloxycarbonylthio-1,2-dihydro-2,2,4-trimethylquinoline as a yellowish oil. Data for 7-*t*-butyloxycarbonylthio-1,2-dihydro-2,2,4-trimethylquinoline: **$^1$H NMR** (400 MHz, $CDCl_3$) 7.02 (d, *J* = 7.9, 1H), 6.74 (dd, *J* = 7.9, 1.6, 1H), 6.57 (d, *J* = 1.6, 1H), 5.31 (s, 1H), 3.73 (br s, 1H), 1.95 (s, 3H), 1.50 (s, 9H), 1.26 (s, 6H).

1,2-Dihydro-6-trifluoromethyl-2,2,4-trimethyl-9-thiopyran-8-ono[5,6-*g*] quinoline (Compound **266,** structure **57** of **Scheme XVII,** where $R^1$=$R^2$=H, $R^3$=trifluoromethyl, Z=S) Trifluoroacetic acid (744 mL, 0.0096 mol) was added all at once *via* a syringe to a solution of 7-*t*-butyloxycarbonylthio-1,2-dihydro-2,2,4-trimethylquinoline (0.14 g) in 1 mL of dry dichloromethane at 0 °C. After 10 min the ice bath was removed and the mixture was allowed to stir at rt for 45 minutes. It was then cooled to 0 °C and neutralized with sat'd $NaHCO_3$, extracted with dichloromethane (3 x 10 mL). The combined organic phases were washed with water (10 mL), dried ($Na_2SO_4$) and concentrated *in vacuo* to a crude product (50 mg) that was used directly in the next step. A solution of the crude material obtained above (50 mg) and zinc chloride (100 mg, 0.724 mmol) in 0.5 mL of absolute ethanol was heated in a sealed tube for 16 h at 80 °C. The reaction was quenched with sat'd $NH_4Cl$ (2 mL) and extracted with ethyl acetate (2 x 5 mL), dried ($Na_2SO_4$) and concentrated *in vacuo* to an orange solid residue that was subjected to flash chromatography (silica gel, hexanes/ethyl acetate, 7:3), followed by preparative TLC (500 µm, hexanes/ethyl acetate, 7:3) to afford 2.2 mg (3%) of Compound **266** as a yellow oil. Data for Compound **266**: **$^1$H NMR** (400 MHz, $CDCl_3$) 7.54 (s, 1H), 6.62 (s, 1H), 6.43 (s, 1H), 5.44 (s, 1H), 4.32 (br s, 1H), 2.03 (s, 3H), 1.29 (s, 6H).

## EXAMPLE 167

1,2-Dihydro-1,2,2,4,9-pentamethyl-6-trifluoromethyl-8-pyridono[5,6-*g*]quinoline (Compound **267**, structure **60** of **Scheme XVI**, where $R^1$=$R^2$=$R^5$=H, $R^3$=trifluoromethyl, Z=N-methyl)

To a 25-mL r.b. flask containing Compound **247** (EXAMPLE 147) (125.8 mg, 0.41 mmol) in 5 mL DMF at rt was added 200 mg (approx 10 equivuiv) solid KOH. After 30 min, iodomethane (129 ML, 2.04 mmol, 5.0 equivuiv) was then added, and the mixture was allowed to stir at rt overnight. Ethyl acetate (50 mL) was then added, the biphasic mixture was neutralized to pH 6 with sat'd aqueous $NH_4Cl$, and the layers were separated. The organic phase was washed with brine, dried over $Na_2SO_4$, and concentrated under reduced pressure. Purification by flash column chromatography (silica gel, hexanes/ethyl acetate, gradient elution) afforded 111 mg (81%) of Compound **267** as a bright fluorescent-yellow solid. Data for Compound **267**: **$^1$H NMR** (400 MHz, $CDCl_3$) 7.37 (s, 1H, 5-H), 6.74 ($_s$, 1H, 7-H), 6.21 (s, 1H, 10-H), 5.38 (s, 1H, 3-H), 3.69 [s, 3H, CONC*$H_3$*], 2.94 [s, 3H, $(CH_3)_2$CNC*$H_3$*], 2.03 (s, 3H, 4-C*$H_3$*), 1.40 [s, 6H, 2-(C*$H_3$*)$_2$].

## EXAMPLE 168

7-Chloro-1,2-dihydro-2,2,4-trimethyl-6-trifluoromethyl-8-pyridono[5,6-*g*]quinoline (Compound **268,** structure **57** of **Scheme XVII,** where $R^1$=H, $R^2$=Cl, $R^3$=trifluoromethyl, Z=NH)

This compound was prepared by General Method 13 (EXAMPLE 147) from 7-amino-1,2-dihydro-2,2,4-trimethylquinoline (EXAMPLE 147) (64 mg, 0.34 mmol) and ethyl 2-chloro-4,4,4-trifluoroacetoacetate (147 mg, 0.68 mmol, 2.0 equivuiv) to afford 36 mg (31%) of Compound **268** as a fluorescent-yellow solid. Data for Compound **268**: **$^1$H NMR** (400 MHz, $CDCl_3$) 7.52 (s, 1H, 5-H), 6.31 (s, 1H, 10-H), 5.43 (s, 1H, 3-H), 4.47 [br s, 1H, $(CH_3)_2$CN*H*], 2.03 (s, 3H, 4-C*$H_3$*), 1.33 [s, 6H, 2-(C*$H_3$*)$_2$].

## EXAMPLE 169

6-Chloro(difluoro)methyl-1,2-dihydro-2,2,4-trimethyl-8-pyridono[5,6-*g*]quinoline (Compound **269**, structure **57** of **Scheme XVII**, where $R^1$=$R^2$=H, $R^3$=chloro(difluoromethyl), Z=NH)

This compound was prepared by General Method 13 (EXAMPLE 147) from 7-amino-1,2-dihydro-2,2,4-trimethylquinoline (EXAMPLE 147) (60 mg, 0.33 mmol) and methyl 4-chloro-4,4-difluoroacetoacetate (92 mg, 0.49 mmol, 1.5 equivuiv) to afford 17 mg (16%) of Compound **269** as a fluorescent-yellow solid. Data for Compound 269: **$^1$H NMR** (400 MHz, $CDCl_3$) 12.50 (br s, 1H, CON*H*), 7.52 (s, 1H, 5-H), 6.62 (s, 1H, 7-H), 6.39 (s, 1H, 10-H), 5.42 (s, 1H, 3-H), 4.48 [br s, 1H, $(CH_3)_2$CN*H*], 2.04 (d, *J* = 1.0, 3H, 4-C*$H_3$*), 1.31 [s, 6H, 2-(C*$H_3$*)$_2$].

## EXAMPLE 305

(*R*/*S*)-5-(3-Furyl)-1,2,3,4-tetrahydro-2,2,4-trimethyl-8-pyranono[5,6-*g*]quinoline (Compound **405**, structure **63**

of **Scheme XVIII**, where $R^1$=$R^2$=H, $R^3$=3-furyl, Z=O)
In a oven dried pressure tube equivuipped with a magnetic stir bar was dissolved (*R*/*S*)-1,2,3,4-tetrahydro-2,2,4-trimethyl-7-hydroxyquinoline (EXAMPLE 150) (50.8 mg, 292 μmol), ethyl 3-keto-3-(3-furyl)propionate (0.10 mL, 642 μmol, 2.2 equiv) and $ZnCl_2$ (119 mg, 876 μmol, 3 equiv) in absolute ethanol (6 mL). The solution was heated at 105°C for 19 h. The cooled solution was concentrated on Celite™ to give a free flowing powder which was purified via silica gel flash column chromatography using a solvent system of hexanes/ethyl acetate (4:1) to 14.6 mg (16% ) of Compound **405** as a yellow oil. Data for Compound **405**: Rf = 0.26 (hexanes/EtOAc; 3:1); **$^1$H NMR** (400 MHz, $CDCl_3$) 7.76 (s, 1 H), 7.58 (dd, *J* = 1.4, 3.0, 1 H), 7.48 (s, 1 H), 6.66 (s, 1 H), 6.37 (s, 1 H), 6.06 (s, 1 H), 4.37 (br s, 1 H, NH), 2.91 (m, 1 H, C4-H), 1.78 (dd, *J* = 4.1, 13, 1 H, C3-H), 1.44 (dd, *J* = 13, 13, 1 H, C3-H), 1.33 (d, *J* = 6.7, 3 H, C4- $CH_3$), 1.31 (s, 3 H, C2- $CH_3$), 1.23 (s, 3 H, C2-$CH_3$).

**EXAMPLE 306**

5-(3-Furyl)-1,2-dihydro-1,2,2,4-tetramethyl-8-pyranono[5,6-*g*]quinoline (Compound **406**, structure **60** of **Scheme XVI**, where $R^1$=$R^2$=$R^5$=H, $R^3$=3-furyl, Z=O)
In a flame dried r.b. flask was dissolved Compound **264** (EXAMPLE 164) (1.1 mg, 3.58 μmol) in glacial acetic acid (3 mL). To the stirred solution was added *para*-formadehyde (1.2 mg, 36 μmol, 10 equiv). The cloudy yellow solution stirred for 10 min then $NaCNBH_3$ (1.1 mg, 17 μmol, 5 equiv) was added at once. Upon addition the solution emitted gas for approx 5 min then turned bright yellow. After stirring at rt under a blanket of $N_2$ for 20 h the solution was slowly poured over ice and quenched with NaOH (20%), extracted with EtOAc (2 x 10 mL), washed with brine (2 x 10 mL), dried over $Na_2SO_4$ and concentrated *in vacuo* to give a impure product that was further purified by preparatory plate chromatography (silica gel, 1000 μm) using a solvent system of 4:1 hexanes/EtOAc to afford 0.8 mg (70 % ) of Compound **406** as a yellow-green solid. Data for Compound **406:** Rf = 0.25 (hexanes/EtOAc; 3:1). **$^1$H NMR** (400 MHz, $CDCl_3$) 7.77 (s, 1 H), 7.57 (d, *J* = 1.7, 1 H), 7.30 (s, 1H), 6.66 (d, *J* = 1.7, 1 H), 6.40 (s, 1 H), 6.07 (s, 1 H), 5.33 (s, 1 H, C3-H), 2.89 (s, 3 H, N-C$\underline{H}_3$), 1.95 (d, *J* = 1.1, 3 H, C4-C$\underline{H}_3$), 1.38 (s, 6 H, (C$\underline{H}_3$)$_2$).

**EXAMPLE 307**

5-(3-Furyl)-1,2-dihydro-1,2,2,4-tetramethyl-8-thiopyranono[5,6-*g*]quinoline (Compound **407,** structure **29A** of **Scheme XXXIX,** where $R^1$=$R^2$=$R^5$=H, $R^3$=3-furyl, Z=O)
In a dry r.b. flask was dissolved LG12066X (5.2 mg, 16.1 μmol) in glacial acetic acid (5 mL). To the stirred solution was added *para*-formadehyde (5.4 mg, 160 μmol, 10 equiv). The cloudy red solution stirred for 10 min then $NaCNBH_3$ (5.1 mg, 80 μmol, 5 equiv) was added at once. After stirring under a blanket of $N_2$ for 12 h the solution was slowly poured over ice and quenched with NaOH (20%), extracted with EtOAc (2 x 20 mL), washed with brine (2 x 20 mL), dried over $Na_2SO_4$ and concentrated *in vacuo* to give an impure product that was further purified by prep TLC (silica gel, 1000 μm) using a solvent system of 4:1 hexanes/EtOAc to afford 3.2 mg (60%) of Compound **407** as a red solid. Data for Compound **407**: $R_f$ = 0.39 (hexanes/EtOAc; 3:1). **$^1$H NMR** (400 MHz, $CDCl_3$) 7.21 (d, *J* = 1.7, 1 H), 7.16 (s, 1 H), 6.51 (s, 1 H), 5.42 (s, 1 H, C3-$\underline{H}$), 2.90 (s, 3 H, N-C$\underline{H}_3$), 2.00 (d, *J* = 1.1, 3 H, C4-C$\underline{H}_3$), 1.41 (s, 6 H, (C$\underline{H}_3$)$_2$).

**EXAMPLE 308**

6-Chloro-5-(3-furyl)-1,2-dihydro-1,2,2,4-tetramethyl-8-pyranono[5,6-*g*]quinoline (Compound **408**, structure **60** of **Scheme XVI**, where $R^1$=$R^5$=H, $R^2$=chloro, $R^3$=trifluoromethyl, Z=O)
In a dry r.b. flask was dissolved Compound **258** (EXAMPLE 158) (5.9 mg, 17.2 μmol) in glacial acetic acid (5 mL). To the stirred solution was added *para*-formadehyde (5.5 mg, 172 μmol, 10 equiv). The cloudy yellow solution stirred for 10 min then $NaCNBH_3$ (5.8 mg, 86 μmol, 5 equiv) was added at once. After stirring under a blanket of $N_2$ for 12 h the solution was slowly poured over ice and quenched with NaOH (20%), extracted with EtOAc (2 x 20 mL), washed with brine (2 x 20 mL), dried over $Na_2SO_4$ and concentrated *in vacuo* to give an impure product that was further purified by 3 consecutive prep TLC's (silica gel, 1000 μm) using a solvent system of 4:1 hexanes/EtOAc to afford 2.5 mg (40%) of Compound **408** as a orange/yellow solid. Data for Compound **408:** $R_f$= 0.36 (hexanes/EtOAc; 3:1). **$^1$H NMR** (400 MHz, $CDCl_3$) 7.32 (d, *J* = 1.7, 1 H), 6.33 (s, 1 H), 5.38 (s, 1 H, C3-H), 2.88 (s, 3 H, N-CH3), 1,99 (d, *J* = 1.1, 3 H, C4-CH3), 1,39 (s, 6 H, (CH3)2).

**EXAMPLE 309**

1,2,3,4-Tetrahydro-2,2,4,10-tetramethyl-6-trifluoromethyl-8-pyridono[5,6-*g*]quinoline (Compound **409,** structure **63** of **Scheme XVIII,** where $R^1$=methyl, $R^2$=H, $R^3$=trifluoromethyl, Z=NH)
7-*tert*-Butyloxycarbamoyl-1,2,3,4-tetrahydro-2,2,4,8-tetramethylquinoline (structure 72 of **Scheme XVIII,** where

$R^1$=methyl, P=*t*-butoxy, Z=NH). This compound was prepared from 7-*tert*-butyloxycarbamoyl-1,2-dihydro-2,2,4,8-tetramethylquinoline (EXAMPLE 155) (4.50 g, 14.9 mmol) according to the general hydrogenation procedure previously described (EXAMPLE 160), affording 4.48 g (99%) of the desired tetrahydroquinoline as a white solid. Data for 7-*tert*-butyloxycarbamoyl-1,2,3,4-tetrahydro-2,2,4,8-tetramethylquinoline: **$^1$H NMR** (400 MHz, $CDCl_3$) 7.03 (d, 1H, $J$ = 8.3, 5-H), 6.81 (d, 1H, $J$= 8.2, 6-H), 6.13 (br s, 1H, BOCN*H*), 3.43 (br s, 2H, N$H_2$), 2.91 (ddq, 1H, $J$ = 19.0, 12.8, 6.6, 4-H), 1.96 (s, 3H, 8-C$H_3$), 1.73 and 1.40 (d of ABq, 2H, $J_{AB}$ = 12.8, $J_A$ = *5.6,* $J_B$ = 12.6, 3-H), 1.31 (d, 3H, $J$ = 6.7, 4-C$H_3$), 1.28 and 1.16 ppm [2s, 2 x 3H, 2-(C$H_3$)$_2$].

7-Amino-1,2-dihydro-2,2,4,8-tetramethylquinoline. This compound was prepared by General Method 12 (EXAMPLE 147) from 7-*tert*-butyloxycarbamoyl-1,2,3,4-tetrahydro-2,2,4,8-tetramethylquinoline (4.48 g, 14.9 mmol) to afford 2.92 g (96%) of the desired aniline as a light reddish oil. Data for 7-amino-1,2-dihydro-2,2,4,8-tetramethylquinoline: **$^1$H NMR** (400 MHz, $CDCl_3$) 6.89 (d, 1 H, $J$ = 8.1, 5-H), 6.14 (d, 1H, $J$ = 8.2, 6-H), 3.42 (br s, 3H, N$H_2$, N*H*), 2.87 (ddq, 1H, $J$ = 18.7, 12.7, 6.4, 4-H), 1.90 (s, 3H, 8-C$H_3$), 1.70 and 1.39 (d of ABq, 2H, $J_{AB}$ = 12.8, $J_A$ = 5.6, $J_B$ = 12.5, 3-H), 1.29 (d, 3H, $J$ = 6.7, 4-C$H_3$), 1.27 and 1.16 ppm [2s, 2 x 3H, 2-(C$H_3$)$_2$].

1,2,3,4-Tetrahydro-2,2,4,10-tetramethyl-6-trifluoromethyl-8-pyridono[5,6-*g*]quinoline. This compound was prepared by General Method 13 (EXAMPLE 147) from 7-amino-1,2,3,4-tetrahydro-2,2,4,8-tetramethylquinoline (2.92 g, 14.3 mmol) and ethyl 4,4,4-trifluoroacetoacetate (3.13 mL, 21.4 mmol, 1.5 equivuiv) to afford 2.04 g (44%) of Compound **409** as a pale fluorescent-yellow solid. Data for Compound **409:** mp 239-40 °C; **$^1$H NMR** (400 MHz, $CDCl_3$) 9.70 (br s, 1H, CON*H*), 7.50 (s, 1H, 5-H), 6.68 (s, 1H, 7-H), 4.13 [br s, 1H, $(CH_3)_2$CN*H*], 3.00 (ddq, 1H, $J$ = 12.9, 12.4, 6.3, 4-H), 2.15 (s, 3H, 10-$CH_3$), 1.83 and 1.46 [dd of ABq, 2H, $J_{AB}$ = 13.0, $J_A$ = 5.3, 1.6 Hz (3-$H_{equiv}$), $J_B$ = 12.9, 0 Hz (3-$H_{ax}$)], 1.40 (d, 3H, $J$ = 6.6, 4-C$H_3$), 1.36 and 1.25 ppm [2s, 2 x 3H, 2-(C$H_3$)$_2$]. **$^{13}$C NMR** (100 MHz, $CDCl_3$) d 162.5, 144.9, 139.1, 137.1, 124.3, 122.7, 120.9, 113.8, 105.7, 101.6, 50.2, 43.5, 31.8, 28.9, 27.6, 20.1, 9.7 ppm. **Anal.** Calcd for $C_{17}H_{19}F_3N_2O$: C, 62.95; H, 5.90; N, 8.64. Found: C, 63.02; H, 6.01; N, 8.48.

## EXAMPLE 310

(*R*/*S*)-1,2,3,4-Tetrahydro-4-methyl-6-trifluoromethyl-8-pyranono[5,6-*g*]quinoline (Compound **410**, structure **33A** of **Scheme XL**, where $R^{1-3}$=$R^6$=H, $R^4$=methyl, $R^5$=trifluoromethyl)

3-(3-Methoxyanilino)propionic acid. To a oven dried 500 mL rb flask equivuipped with a magnetic stir bar and a water cooled reflux condenser was dissolved anisidine (5 mL, 44.6 mmol) in toluene (70 mL). The stirred solution was heated to reflux and acrylic acid (3.0 mL, 44.1 mmol, 1 equiv) was dripped in over a 10 min period to give a clear colorless solution. After heating at reflux for 3 h the dark red solution was cooled to rt and concentrated *in vacuo* to remove both the unreacted acrylic acid and toluene to give a 6.4 g of a 1:1 mixture of the desired amino acid and anisidine as a red viscous oil. Data for 3-(3-Methoxyanilino)proionic acid: $R_f$ = 0.1 (hexanes/EtOAc, 3:1); **$^1$H NMR** (400 MHz, $CDCl_3$) 9.08 (br s, 1 H, NH), 7.28 (d, $J$ = 4.3, 1 H), 7.21 (dd, $J$ = 8.1, 8.1, 1 H), 7.03 (dd, $J$ = 1.3, 7.9, 1 H), 6.67 (dd, $J$ = 2.3, 8.4, 1 H).3.80 (s, 3 H), 3.58 (s, 2 H), 2.33 (s, 3 H).

1,2,3,4-Tetrahydro-7-methoxy-4-quinolone. To a oven dried 500 mL rb flask equivuipped with a magnetic stir bar and a $N_2$ gas inlet, the material obtained above was dissolved in PPA (~150 mL). The resulting red viscous solution was heated at 100°C with constant stirring under a blanket of $N_2$ for 12 h. The still warm solution was carefully poured over ice (1 L) and while vigorously stirring the iced solution with a metal stir rod the reaction was quenched by slow addition of a sat. $K_2CO_3$ solution. The near neutral solution was extracted with $CHCl_3$ (5 x 150 mL), washed with brine (2 x 100 mL), dried over $Na_2SO_4$ and concentrated in vacuo to give an impure solid. The solid was purified by taking up in EtOAc and concentrating the liquor on Celite™ to give a free flowing powder which was purified *via* flash column chromatography (silica gel, $CH_2Cl_2$ /MeOH, 95:5) to give 1.2 g (62%) of 1,2,3,4-tetrahydro-7-methoxy-4-quinolone as a yellow solid. Data for 1,2,3,4-tetrahydro-7-methoxy-4-quinolone: $R_f$ = 0.43 ($CH_2Cl_2$/MeOH, 95:5); **$^1$H NMR** (400 MHz, $CDCl_3$) 7.79 (d, $J$ = 8.8, 1 H), 6.31 (dd, $J$ = 2.0, 8.9, 1 H), 6.08 (d, $J$ = 2.0, 1 H), 4.54 (br s, 1 H, NH), 3.78 (s, 3 H, OCH3), 3.54 (td, $J$ = 1.7, 8.0, 2 H), 2.63 (t, $J$ = 7.0, 2 H).

1-*t*-Butoxycarbonyl-1,2,3,4-tetrahydro-7-methoxy-4-quinolone (structure **31A** of **Scheme XL,** where $R^{1-3}$=H). To a flame dried 250 mL rb flask equivuipped with a magnetic stir bar and a N2 gas inlet was dissolved 1,2,3,4-tetrahydro-7-methoxy-4-quinolone (1.18 g, 6.67 mmol) and BOC anhydride (2.03 g, 9.33 mmol, 1.33 equiv) in anhydrous THF (60 mL). The solution was cooled to 0°C and N,N-dimethyl-4-aminopyridine (DMAP) (1.30 g, 10.7 mmol, 1.6 equiv) was added with constant stirring. After stirring over $N_2$ for 16 h the reaction was carefully quenched with 10% $NaHSO_4$ solution (20 mL). The biphasic solution was extracted with EtOAc (3 x 50 mL), washed with brine (2 x 50 mL), dried over $Na_2SO_4$ and concentrated *in vacuo* to give 1.65 g (90%) of 1-*t*-butoxycarbonyl-1,2,3,4-tetrahydro-7-methoxy-4-quinolone as an off white solid. Data for 1-*t*-butoxycarbonyl-1,2,3,4-tetrahydro-7-methoxy-4-quinolone: $R_f$ = 0.31 (hexanes/EtOAc, 3:1); **$^1$H NMR** (400 MHz, $CDCl_3$) 7.27 (d, $J$ = 1.6, 1 H), 7.06 (d, $J$ = 8.5, 1 H), 6.61 (dd, $J$ = 2.4, 8.5, 1 H), 3.78 (s, 3 H), 3.71 (t, $J$ = 6.0, 2 H), 2.82 (m, 1 H), 2.02 (m, 1 H), 1.57 (m, 1 H), 1.27 (d, $J$ = 7.0, 3 H).

(*R*/*S*)-1-*t*-Butoxycarbonyl-1,2,3,4-tetrahydro-4-hydroxy-4-methyl-7-methoxyquinoline. To a flame dried 250 mL 3-necked rb flask equivuipped with a magnetic stir bar was added Ce(III)Cl·7 $H_2O$ (2.74 g, 7.35 mmol, 2 equiv).

The flask was heated in a 140°C oil bath under reduced pressure (~ 1 torr) for 2.5 h. The flask was cooled to rt and slowly filled with $N_2$ g. The white powder was suspended in dry THF (30 mL), stirred at rt for 1 h and then cooled to -78°C. To the white suspension was added a 1.4 M solution of methyl lithium (MeLi) in $Et_2O$ (5.25 mL, 7.35 mmol, 2 equiv) by syringe. The dark yellow/brown solution stirred at -78°C for 1 h and then 1-*t*-butoxycarbonyl-1,2,3,4-tetrahydro-7-methoxy-4-quinolone dissolved in 3 mL THF was added. The solution stirred at -78 °C for 3 h and was warmed to 0°C for 2 h. The reaction did not go to completion and starting material was observed by TLC (silica gel, hexane/EtOAc, 3:1). The reaction was quenched with $H_2O$ (1 mL) and allowed to warm to rt. The solution was neutralized with sat $NH_4Cl$ solution (5 mL), extracted with EtOAc (3 x 100 mL), washed with brine (1 x 100 mL), dried over $Na_2SO_4$ and concentrated in vacuo to give a mix of the desired alcohol and starting material. The mixture was taken up in EtOAc and concentrated on Celite™ to give a free flowing powder which was purified via flash column chromatography (silica gel, hexanes/EtOAc, 3:1) to give 796 mg (74%) of the desired product as a vicious clear colorless oil. Data for 1-*t*-butoxycarbonyl-1,2,3,4-tetrahydro-4-hydroxy-4-methyl-7-methoxy-quinoline: $R_f$ = 0.20 (hexanes/EtOAc, 3:1); **$^1$H NMR** (400 MHz, $CDCl_3$) 7.42 (d, *J* = 8.7, 1 H), 7.33 (d, *J* = 2.5, 1 H), 6.66 (dd, *J* = 2.5, 8.6, 1 H), 3.98 (m, 1 H), 3.79 (s, 3 H, $OCH_3$), 3.61 (m, 1 H), 1.98 (m, 2 H), 1.58 (s, 3 H), 1.53 (s, 9 H).

(*R*/*S*)-1-*t*-Butoxycarbonyl-1,2,3,4-tetrahydro-4-methyl-7-methoxy-quinoline

To a oven dried 250 mL rb flask equivuipped with a magnetic stir bar was dissolved 1-*t* -butoxycarbonyl-1,2,3,4-tetrahydro-4-hydroxy-4-methyl-7-methoxy-quinoline (44 mg, 150 μmol) in EtOAc (15 mL). The flask was repeatedly evacuated and flushed with $N_2$ then a catalytic amount of 10% Pd on C (~5 mg) was added. The flask was again evacuated and flushed with $N_2$ several times and then $H_2$ was introduced by balloon. The solution was stirred under $H_2$ for 12 h. The flask was again evacuated and flushed with $N_2$ several times to remove any residual $H_2$ and the solution was filtered through a pad of Celite™ and concentrated *in vacuo* to give the desired amine (37.0 mg, 133 μmol, 90% yield) as a clear colorless oil. Data for 1-*t*-butoxycarbonyl-1,2,3,4-tetrahydro-4-methyl-7-methoxy-quinoline: $R_f$ = 0.59 (hexanes/EtOAc, 3:1); **$^1$H NMR** (400 MHz, $CDCl_3$) 7.28 (d, *J* =2.3, 1 H, Ar-8), 7.07 (d, *J =8.5,* 1 H, Ar-5), 6.61 (dd, *J* =2.5, 8.5, 1 H, Ar-6), 3.78 (s, 3 H, OMe), 3.71 (dd, *J* =6.1, 12.2, 2 H, C2-H), 2,83 (m, 1 H, C4-H), 2.00 (m, 1 H, C3-H), 1.58 (m, 1 H, C3-H), 1.53, (s, 9 H, $(CH_3)_3$), 1.27 (d, *J* =7.0 Hz. 3 H, C4-$C\underline{H}_3$).

(*R*/*S*)-1,2,3,4-Tetrahydro-4-methyl-7-methoxyquinoline. To a oven dried 250 mL r.b. flask equivuipped with a magnetic stir bar and a $N_2$ gas inlet was dissolved 1-*t*-butoxycarbonyl-1,2,3,4-tetrahydro-4-methyl-7-methoxy-quinoline (678 mg, 2.44 mmol) in $CH_2Cl_2$ (15 mL). To the stirred solution was added trifluoroacetic acid (TFA) (2 mL) at rt. The solution stirred under $N_2$ for 2 h and then quenched with sat $NaHCO_3$ solution (25 mL), extracted with $CH_2Cl_2$ (3 x 20 mL), washed with brine (1 x 30 mL), dried over $Na_2SO_4$ and concentrated in vacuo to give 370 mg (77%) of the desired quinoline as a clear colorless oil. Data for 1,2,3,4-tetrahydro-4-methyl-7-methoxyquinoline: $R_f$ = 0.32 (hexanes/EtOAc, 3:1); **$^1$H NMR** (400 MHz, $CDCl_3$) 6.95 (d, *J* =8.1, 1 H, Ar-5), 6.22 (dd, *J* =2.5, 8.3, 1 H, Ar-6), 6.03 (d, *J* =2,6, 1 H, Ar-8), 3.86 (br s, 1 H, NH), 3.73 (s, 3 H, OMe), 3.28 (m, 2 H, C2-H), 2,85 (m, 1 H, C4-H), 1.95 (m, 1 H, C3-H), 1.64 (m, 1 H, C3-H), 1.25 (d, *J* =6.9, 3H, C4-Me).

(*R*/*S*)-1,2,3,4-Tetrahydro-7-hydroxy-4-methylquinoline. To a flame dried 25 mL rb flask equivuipped with a magnetic stir bar and a $N_2$ gas inlet was dissolved 1,2,3,4-tetrahydro-4-methyl-7-methoxyquinoline (17.7 mg, 100 μmol) in $CH_2Cl_2$ (3 mL). The solution was cooled to 0°C under a blanket of $N_2$ and then 250 μL of a 1.0 M solution of $BBr_3$ in hexanes (250 μmol, 2.5 equiv) was added at once by syringe. The stirred solution was warmed to rt and allowed to react for 3 h. The reaction was quenched with $H_2O$ (1 mL), neutralized with sat. $NaHCO_3$ (4 mL) and extracted with $CH_2Cl_2$ (5 x 50 mL), dried over $Na_2SO_4$ and concentrated to give 12 mg (66%) of the desired phenolic quinoline as a light yellow oil. Data for (*R*/*S*)-1,2,3,4-tetrahydro-7-hydroxy-4-methylquinoline: $R_f$ = 0.15 (hexanes/EtOAc, 3:1); **$^1$H NMR** (400 MHz, $CDCl_3$) 6.91 (d, *J* = 8.7, 1 H), 6.12 (dd, *J* = 2.5, 8.3, 1 H), 5.97 (d, *J* = 2.5, 1 H), 3.27 (m, 2 H, C2-H), 2.84 (m, 1 H, C4-H), 1.95 (m, 1 H, C3-H), 1.66 (m, 1 H, C3-H), 1.25 (d, *J* = 6.9, 3 H, C4-$CH_3$).

(*R*/*S*)-1,2,3,4-Tetrahydro-4-methyl-6-trifluoromethyl-8-pyranono[5,6-*g*]quinoline (Compound **410**, structure **33A** of **Scheme XL**, where $R^{1-3}$=$R^6$=H, $R^4$=methyl, $R^5$=trifluoromethyl). In an oven dried pressure tube equivuipped with a magnetic stir bar was dissolved (*R*/*S*)-1,2,3,4-tetrahydro-7-hydroxy-4-methylquinoline (11.7 mg, 64.6 μmol) and trifluoromethyl ethyl acetoacetate (20 μL, 146 μmol, 2.2 equiv) and $ZnCl_2$ (20 mg) in 0.5 mL absolute ethanol. The light yellow solution was heated at 98 °C for 20 h and cooled to rt. The dark green solution was concentrated on Celite™ to give a free flowing powder which was purified *via* silica gel flash column chromatography using a solvent system of hexanes/ethyl acetate (4:1) to give 12.4 mg (66%) of Compound **410** as a yellow solid. Data for Compound **410:** $R_f$ = 0.19 (hexanes/EtOAc, 3:1); $^1$H NMR (400 MHz, $CDCl_3$) 7.29 (s, 1 H), 6.36 (m, 2 H), 4.70 (br s, 1 H, NH), 3.43 (m, 2 H, C2-H), 2.95 (m, 1 H, C4-H), 1.97 (m, 1 H, C3-H), 1.72 (m, 1 H, C3-H), 1.31 (d, *J* = 7.0, 3 H, C4-$CH_3$).

**EXAMPLE 311**

1,2-Dihydro-2,2-dimethyl-6-trifluoromethyl-8-pyranono[5,6-*g*]quinoline (Compound **411**, structure **37A** of **Scheme XLI**, where $R^{1-2}$=$R^6$=H, $R^3$=$R^4$=methyl, $R^5$=trifluoromethyl, X=O)

1,2-Dihydro-2.2-dimethyl-7-(1,1,1-trimethylacetoxy)quinoline (structure **36A** of **Scheme XLI**, where $R^1$=$R^2$=H,

$R^3$=$R^4$=methyl, P=*t*-butyl, X=O). In a oven dried pressure tube equivuipped with a magnetic stir bar was dissolved *O*-pivaloyl-3-aminophenol (EXAMPLE 138) (4.8 g, 25.0 mmol, 1.5 equiv) and 3-methyl-3-acetoxy-1-butyne (2.1 g, 16.7 mmol, 1 equiv) in dry THF (~5 mL). To the stirred solution was added CuCl (240 mg, 25 mmol, 0.15 equiv). The sealed pressure tube was heated at 98 °C for 5 h, cooled to rt and concentrated on Celite™ to give a free flowing powder which was purified via flash column chromatography (silica gel, hexanes/EtOAc, 5:1) to give 1.2 g (18%) of the desired product as an off white solid. Data for 1,2-dihydro-2,2-dimethyl-7-(1,1,1-trimethylacetoxy)quinoline: $R_f$ = 0.80 (hexanes/EtOAc, 3:1); **$^1$H NMR** (400 MHz, $CDCl_3$) 6.83 (d, *J* = 8.0, 1 H), 6.23 (m, 2 H), 6.12 (d, *J* = 2.1, 1 H), 5.42 (d, *J* = 9.7, 1 H), 3.67 (br s, 1 H), 1.31 (s, 9 H), 1.29 (s, 6H). **$^{13}$C NMR** (100 MHz, $CDCl_3$) 177, 151, 130, 127, 123, 117, 109, 105, 52, 49, 31, 27.

1,2-Dihydro-7-hydroxy-2.2-dimethylquinoline. To a oven dried rb flask was dissolved 1,2-dihydro-2,2-dimethyl-7-(1,1,1-trimethylacetoxy)quinoline (48 mg, 185 µmol) in absolute ethanol (5 mL) and $H_2O$ (1 mL). To the stirred solution was added a catalytic amount of 20% aqueous NaOH solution (-0.2 mL). After 1.5 h the dark purple solution was diluted with $H_2O$ (10 mL), EtOAc (15 mL) and quenched with sat. $NH_4Cl$ solution (5 mL). The biphasic solution was extracted with EtOAc (4 x 20 mL), washed with brine (2 x 30 mL), dried ($Na_2SO_4$) and concentrated *in vacuo* to give 31 mg (96%) of the desired phenolic amine, which was used without further purification.

1,2-Dihydro-2.2-dimethyl-6-trifluoromethyl-8-pyranono[5,6-*g*]quinoline (Compound **411**, structure **37A** of **Scheme XLI**, where $R^{1-2}$=$R^6$=H, $R^3$=$R^4$=methyl, $R^5$=trifluoromethyl, X=O). In a oven dried pressure tube equivuipped with a magnetic stir bar was dissolved 1,2-dihydro-7-hydroxy-2,2-dimethylquinoline (31 mg, 177 µmol), ethyl (4,4,4-trifluoroacetoacetate) (75 mg, 408 µmol, 2.2 equiv) and $ZnCl_2$ (75 mg, 550 µmol, 3 equiv) in absolute EtOH. Upon addition of the $ZnCl_2$ the solution went a dark brown. The sealed pressure tube was heated at 105 °C for 16 h, cooled to rt and concentrated on Celite™ to give a free flowing powder which was purified *via* flash column chromatography (silica gel, hexanes/EtOAc, 5:1) to give 2.3 mg (4.4%) of Compound **411** as a bright yellow solid. Data for Compound **411**: $R_f$ = 0.31 (hexanes/EtOAc, 3:1); **$^1$H NMR** (400 MHz, $CDCl_3$) 7.11 (s, 1 H), 6.41 (s, 1 H), 6.32 (s, 2 H), 5.58 (d, *J* = 8.0, 1 H), 4.39 (br s, 1 H), 1.55 (s, 6 H).

## EXAMPLE 312

1,2,3,4-Tetrahydro-2,2-dimethyl-6-trifluoromethyl-8-pyranono[5,6-*g*]quinoline (Compound **412,** structure **40A** of **Scheme XLII,** where $R^{1-2}$=$R^6$=H, $R^3$=$R^4$=methyl, $R^5$=trifluoromethyl, X=O).
1,2,3,4-Tetrahydro-2,2-dimethyl-7-(1,1,1-trimethylacetoxy)quinoline (structure **39A** of **Scheme XLII,** where $R^1$=$R^2$=H, $R^3$=$R^4$=methyl, P=*t*-butyl, X=O). To a oven dried 250 mL rb flask equivuipped with a magnetic stir bar and a $N_2$ gas inlet was dissolved 1,2-dihydro-2,2-dimethyl-7-(1,1,1-trimethylacetoxy)quinoline (EXAMPLE 311) (47 mg, 192 µmol) in dry EtOAc (5 mL). The flask was repeatedly evacuated and flushed with $N_2$ then a catalytic amount of 10% Pd on C (~10 mg) was added. The flask was again evacuated and flushed with $N_2$ several times and then $H_2$ was introduced by balloon. The solution was stirred under $H_2$ for 13 h. The flask was again evacuated and flushed with $N_2$ several times to remove any residual $H_2$ and the solution was filtered through a pad of Celite™ and concentrated *in vacuo* to give the desired amine (38.0 mg, 154 µmol, 81% yield) as an off white solid. Data for 1,2,3,4-tetrahydro-2,2-dimethyl-7-(1,1,1-trimethylacetoxy)quinoline: $R_f$ = 0.54 (hexanes/EtOAc, 3:1); $^1$H NMR (400 MHz, $CDCl_3$) 6.93 (d, *J* = 8.1, 1 H), 6.26 (dd, *J* = 2.3, 8.1, 1 H), 6.13 (d, *J* = 2.1, 1 H), 3.59 (br s, 1 H), 2.73 (t, *J* = 6.7, 2 H), 1.67 (t, *J* = 6.7, 2 H), 1.32 (s, 9 H), 1.18 (s, 6 H).

1,2,3,4-Tetrahydro-7-hydroxy-2,2-dimethylquinoline. To a oven dried rb flask was dissolved 1,2,3,4-tetrahydro-2,2-dimethyl-7-(1,1,1-trimethylacetoxy)quinoline (38 mg, 154 µmol) in absolute ethanol (5 mL) and H20 (1 mL). To the stirred solution was added a catalytic amount of 20% aqueous NaOH solution (~0.2 mL) and stirred under N2 at rt. After 3 h the dark purple solution was diluted with $H_2O$ (10 mL), EtOAc (15 mL) and quenched with sat. $NH_4Cl$ solution (5 mL). The bi-phasic solution was extracted with EtOAc (4 x 20 mL), washed with brine (2 x 30 mL), dried ($Na_2SO_4$) and concentrated *in vacuo* to give 25 mg (92%) of the desired phenolic amine as a light yellow oil. Data for 1,2,3,4-tetrahydro-7-hydroxy-2,2-dimethylquinoline: $R_f$ = 0.22 (hexanes/EtOAc, 3:1); **$^1$H NMR** (400 MHz, $CDCl_3$) 6.82 (d, *J* = 8.1, 1 H), 6.09 (dd, *J* = 2.6, 8.2, 1 H), 5.93 (d, *J* = 2.4, 1 H), 2.68 (t, *J* = 6.7, 2 H), 1.67 (t, *J* = 6.7, 2 H), 1.19 (s, 6 H).

1,2,3,4-Tetrahydro-2,2-dimethyl-6-trifluoromethyl-8-pyranono[5,6-*g*]quinoline (Compound **412**, structure **40A** of **Scheme XLII**, where $R^{1-2}$=$R^6$=$R^8$=H, $R^3$=$R^4$=methyl, $R^5$=trifluoromethyl, X=O,). In a oven dried pressure tube equivuipped with a magnetic stir bar was dissolved 1,2,3,4-tetrahydro-7-hydroxy-2,2-dimethylquinoline (25.1 mg, 142 µmol), TFEEA (62 mg, 338 µmol, 2.2 equiv) and $ZnCl_2$ (62 mg, 462 µmol, 3 equiv) in absolute EtOH. The sealed pressure tube was heated at 105 °C for 13 h, cooled to rt and concentrated on Celite™ to give a free flowing powder which was purified via flash column chromatography (silica gel, hexanes/EtOAc, 5:1) to 26.3 mg (60%) of Compound **412** as a bright yellow solid. Data for Compound **412**: $R_f$ = 0.31 (hexanes/EtOAc, 3:1); **$^1$H NMR** (400 MHz, $CDCl_3$) 7.26 (s, 1 H), 6.37 (m, 2 H), 4.52 (br s, 1 H)m, 2.83 (t, *J* = 6.6, 2 H), 1.74 (t, *J* = 6.6, 2 H), 1.28 (s, 6 H).

**EXAMPLE 313**

1,2,3,4-Tetrahydro-6-trifluoromethyl-8-pyranono[5,6-*g*]quinoline (Compound **413**, structure **45A** of **Scheme XLIII,** where $R^1$=H, $R^2$=trifluoromethyl).
3-Methoxy-*trans* indanone oxime (Compound **43A, Scheme XLIII**). To an oven dried 250 mL rb flask equivuipped with a magnetic stir bar, a $N_2$ gas inlet and a water cooled reflux condenser was dissolved 7-methoxyindanone (2.0 g, 12.3 mmol), $Et_3N$ (3.0 mL, 21.5 mmol, 1 equiv) and $NH_2OH{\cdot}HCl$ (1,48 g, 21.5 mmol, 1 equiv) in MeOH (50 mL). The clear colorless solution was heated at reflux for 12 h, cooled to rt and partially concentrated under reduced pressure to half the original volume. The liquor was diluted with $H_2O$ (25 mL) and extracted with EtOAc (4 x 50 mL), washed with brine (3 x 25 mL), dried ($Na_2SO_4$) and concentrated *in vacuo* to afford 2.14 g (99%) of the desired adduct as a white solid. Data for 3-methoxy-*trans*-indanone oxime: $R_f$ = 0.23 (hexanes/EtOAc, 3:1); **$^1$H NMR** (400 MHz, $CDCl_3$) 8.01 (br s, 1 H), 7.47 (d, *J* = 2.4, 1 H), 6.88 (dd, *J* = 2.4, 8.3, 1 H), 6.82 (d, *J* = 8.3, 1 H), 3.30 (s, 3 H), 2.79 (m, 2 H), 2.44 (t, *J* = 6.7, 2 H).
1,2,3,4-Tetrahydro-7-methoxyquinoline (Compound **44A, Scheme XLIII)**. In a flame dried 100 mL rb flask equivuipped with a magnetic stir bar, a $N_2$ gas inlet and a water cooled reflux condenser was dissolved 3-methoxy-*trans* indanone oxime (280 mg, 1.10 mmol) in dry THF. Under a blanket of $N_2$ the solution was cooled to 0°C and a 1.0 M solution of LAH in pentane (0.5 mL, 5.0 mmol, 4.3 equiv) was added via syringe. The solution was then heated to reflux for 4.5 h. The solution was cooled to rt and quenched with $H_2O$ (2 mL), extracted with EtOAc (3 x 25 mL), washed with brine (50 mL), dried over $Na_2SO_4$ and concentrated on Celite™ to give a free flowing powder which was purified *via* flash column chromatography (silica gel, hexanes/EtOAc, 3:1) to give 14 mg (8%) of the desired adduct as an off white solid. Data for 1,2,3,4-tetrahydro-7-methoxyquinoline: $R_f$ = 0.35 (hexanes/EtOAc, 3:1); **$^1$H NMR** (400 MHz, $CDCl_3$) 6.84 (d, *J* = 8.0, 1 H), 6.19 (dd, *J* = 2.5, 8.2, 1 H), 6.03 (d, *J* = 2.5, 1 H), 3.81 (br s, 1 H), 3.73 (s, 3 H), 3.27 (m, 2 H), 2.69 (t, *J* = 6.4, 2 H), 1.91 (m, 2 H).
1,2,3,4-tetrahydro-7-hydroxyquinoline. In a flame dried 100 mL rb flask equivuipped with a magnetic stir bar and a $N_2$ gas inlet was dissolved 1,2,3,4-tetrahydro-7-methoxyquinoline (14.0 mg, 85.8 μmol) in $CH_2Cl_2$ (~3 mL). The solution was cooled to -78°C under a blanket of $N_2$ and a 1.0 M solution of BBr3 in $CH_2Cl_2$ (0.25 mL, 250 μmol, 3 equiv) was added via syringe. The solution stirred at -78°C for 1h, warmed to 0°C for 1 h and rt for 2 h. The reaction was quenched with $H_2O$ (2 mL), extracted with $CH_2Cl_2$ (3 x 20 mL), washed with brine (2 x 20 mL), dried ($Na_2SO_4$) and concentrated *in vacuo* to afford 12 mg (88%) of the desired adduct as a yellow oil. Data for 1,2,3,4-tetrahydro-7-hydroxyquinoline: $R_f$= 0.21 (hexanes/EtOAc, 3:1); **$^1$H NMR** (400 MHz, $CDCl_3$) 6.79 (d, *J* = 8.2, 1 H), 6.12 (dd, *J* = 2.4, 8.0, 1 H), 6.04 (d, *J* = 2.3, 1 H), 4.78 (br s, 1 H), 3.27 (m, 2 H), 2.67 (m, 2 H), 1.91 (m, 2 H).
1,2,3,4-Tetrahydro-6-trifluoromethyl-8-pyranono[5,6-*g*]quinoline (Compound **413**, structure **45A** of **Scheme XLIII**, where $R^1$=H, $R^2$=trifluoromethyl). In a oven dried pressure tube equivuipped with a magnetic stir bar was dissolved 1,2,3,4-tetrahydro-7-hydroxyquinoline (11.7 mg, 78.5 μmol), TFEEA (>10 fold excess) and $ZnCl_2$ (>10 fold excess) in absolute EtOH (3 mL). The sealed pressure tube was heated at 110°C for 16 h, cooled to rt and concentrated on Celite™ to give a free flowing powder which was purified *via* flash column chromatography (silica gel, hexanes/EtOAc, 4:1) to give 8.6 mg, (41%) of Compound **413** as a bright yellow solid. Data for Compound **413:** $R_f$ = 0.31 (hexanes/EtOAc, 3:1); **$^1$H NMR** (400 MHz, $CDCl_3$) 7.21 (s, 1 H), 6.35 (m, 2 H), 4.66 (br s, 1 H), 3.40 (m, 2 H), 2.80 (t, *J* = 6.3, 2 H), 1.95 (m, 2 H).

**EXAMPLE 314**

(*R*/*S*)-4-Ethyl-1,2,3,4-tetrahydro-6-trifluoromethyl-8-pyranono[5,6-*g*]quinoline (Compound **414,** structure **33A** of **Scheme XL,** where $R^{1-3}$=$R^6$=H, $R^4$=ethyl, $R^5$=trifluoromethyl) (*R*/*S*)-1-*t*-Butoxycarbonyl-4-ethyl-1,2,3,4-tetrahydro-4-hydroxy-7-methoxyquinoline. To a flame dried 250 mL r.b. flask equivuipped with a magnetic stir bar and a $N_2$ gas inlet was dissolved (*R*/*S*)-1-*t*-Butoxycarbonyl-1,2,3,4-tetrahydro-7-methoxy-4-quinolone (106 mg, 390 μmol) in dry THF (8 mL). The solution was cooled to 0°C under a blanket of $N_2$ and a 1.0 M solution of ethyl magnesium bromide (EtMgBr) in ethyl ether (1.3 mL, 1.36 mmol, 3.5 equiv)was added via syringe. The solution was stirred at 0°C for 2 h and at rt for 3 h. The reaction did not go to completion and starting material was observed by TLC (silica gel, hexane/EtOAc, 3:1). The reaction was quenched with $H_2O$ (2 mL), extracted with EtOAc (4 x 25 mL), washed with brine (40 mL), dried over $Na_2SO_4$ and concentrated *in vacuo* to give 38 mg (32%) of the desired alcohol as a colorless oil. Data for (*R*/*S*)-1-*t*-butoxycarbonyl-4-ethyl-1,2,3,4-tetrahydro-4-hydroxy-7-methoxyquinoline: $R_f$ = 0.14 (hexanes/EtOAc, 3:1); **$^1$H NMR** (400 MHz, $CDCl_3$) 7.36 (d, *J* = 8.7, 1 H, Ar-5H), 7.34 (d, *J* = 2.5, 1 H, Ar-8H), 6.67 (dd, J = 2.5, 8.5, 1 H, Ar-6H), 4.08 (m, 1 H), 3.86 (br s, 1 H, OH), 3.79 (s, 3 H, OMe), 3.43 (m, 1 H), 1.87 (m, 3 H), 1.53 (s, 9 H, t-butyl), 0.859 (t, J = 7.4, 3 H, $-CH_3$).
(*R*/*S*)-1-*t*-Butoxycarbonyl-4-ethyl-1,2,3,4-tetrahydro-7-methoxyquinoline. To an oven dried 250 mL r.b. flask equivuipped with a magnetic stir bar and a N2 gas inlet was dissolved (*R*/*S*)-1-*t*-butoxycarbonyl-4-ethyl-1,2,3,4-tetrahydro-4-hydroxy-7-methoxyquinoline (37.6 mg, 123 μmol) in dry EtOAc (8 mL). The flask was repeatedly evacuated and flushed with N2 then a catalytic amount of 10% Pd on C (~10 mg) was added. The flask was again evacuated

and flushed with $N_2$ several times and then $H_2$ was introduced by balloon. The solution was stirred under $H_2$ for 14 h. The flask was again evacuated and flushed with $N_2$ several times to remove any residual $H_2$ and the solution was filtered through a pad of Celite™ and concentrated *in vacuo* to give 34 mg (95%) of the desired amine as a clear colorless oil. Data for (*R*/*S*)-1-*t*-butoxycarbonyl-4-ethyl-1,2,3,4-tetrahydro-7-methoxyquinoline: $R_f$ = 0.52 (hexanes/EtOAc, 3:1); **$^1$H NMR** (400 MHz, $CDCl_3$) 7.27 (d, *J* = 2.3, 1 H, Ar-8H), 7.02 (d, *J* = 8.5, 1 H, Ar-5H), 6.59 (dd, *J* = 2.7, 8.4, 1 H, Ar-6H), 3.78 (s, 3 H, OMe), 3.74 (m, partially obscured by OMe, 1 H), 3.58 (m, 1 H), 2,62 (m, 1 H), 1.95 (m, 1 H), 1.72 (m, 2 H), 1.53 (s, 9 H, t-butyl), 1.48 (m, partially obscured by t-butyl, 1 H), 0.949 (t, *J* = 7.4, 3 H, $-CH_3$).

(*R*/*S*)-4-Ethyl-1,2,3,4-tetrahydro-7-methoxyquinoline. In a oven dried 250 mL r.b. flask equivuipped with a magnetic stir bar and a $N_2$ gas inlet was dissolved (*R*/*S*)-1-*t*-butoxycarbonyl-4-ethyl-1,2,3,4-tetrahydro-7-methoxyquinoline (34.0 mg, 117 μmol) in dry $CH_2Cl_2$ (1 mL). To the stirred solution was added TFA (1.2 mL) at rt and was allowed to react for 2 h. The dark red solution was quenched with sat $NaHCO_3$ solution (10 mL), extracted with $CH_2Cl_2$ (3 x 25 mL), washed with brine (50 mL), dried ($Na_2SO_4$) and concentrated *in vacuo* to afford 21 mg (95%) of the desired amine as a clear light yellow oil. Data for (*R*/*S*)-4-ethyl-1,2,3,4-tetrahydro-7-methoxyquinoline: $R_f$ = 0.1 (hexanes/EtOAc, 3:1); **$^1$H NMR** (400 MHz, $CDCl_3$) 6.92 (d, *J* = 8.5, 1 H), 6.21 (dd, *J* = 2.5, 8.2, 1 H), 6.03 (d, *J* = 2.5, 1 H), 3.73 (s, 3 H), 3.27 (m, 2 H), 2.59 (m, 1 H), 1.86 (m, 1 H), 1.75 (m, 2 H), 1.48 (m, 1 H), 0.968 (t, *J* = 7.4, 3 H).

(*R*/*S*)-4-Ethyl-1,2,3,4-tetrahydro-7-hydroxyquinoline (structure **32A** of **Scheme XL**, where $R^{1-3}$=H, $R^4$=ethyl). In a flame dried 100 mL rb flask equivuipped with a magnetic stir bar and a N2 gas inlet was dissolved (*R*/*S*)-4-ethyl-1,2,3,4-tetrahydro-7-methoxyquinoline (21 mg, 109.9 μmol) in $CH_2Cl_2$ (4 mL). The solution was cooled to 0°C and a 1.0 M solution of $BBr_3$ in $CH_2Cl_2$ (0.33 mL, 320 μmol, 2.75 equiv) was added slowly by syringe. The solution was warmed to rt and stirred under a blanket of $N_2$ for 9 h. The reaction was quenched by addition of sat. $NaHCO_3$ solution (5 mL), extracted with CH2C12 (3 x 25 mL), washed with brine (2 x 20 mL), dried ($Na_2SO_4$) and concentrated *in vacuo* to give 19 mg (99%) of the desired phenolic amine as a clear yellow oil. Data for (*R*/*S*)-4-ethyl-1,2,3,4-tetrahydro-7-hydroxyquinoline: **$^1$H NMR** (400 MHz, $CDCl_3$) 6.85 (d, *J* = 8.1, 1 H), 6.11 (dd, *J* = 2.4, 8.3, 1 H), 5.98 (d, *J* = 2.4, 1 H), 3.25 (m, 2 H), 2.57 (m, 1 H), 1.85 (m, 1 H), 1.76 (m, 1 H), 1.67 (m, 1 H), 1.51 (m, 1 H), 0.940 (t, *J* = 7.4, 3 H).

(*R*/*S*)-4-Ethyl-1,2,3,4-tetrahydro-6-trifluoromethyl-8-pyranono[5,6-*g*]quinoline (Compound **414,** structure **33A** of **Scheme XL,** where $R^{1-3}$=$R^6$=H, $R^4$=ethyl, $R^5$=trifluoromethyl). In a oven dried pressure tube equivuipped with a magnetic stir bar was dissolved (*R*/*S*)-4-ethyl-1,2,3,4-tetrahydro-7-hydroxyquinoline (19 mg, 109 μmol) and TFEAA (excess) and $ZnCl_2$ (excess) in absolute EtOH (~3 mL). The sealed pressure tube was heated at 101°C for 10 h, cooled to rt and concentrated on Celite™ to give a free flowing powder which was purified *via* flash column chromatography (silica gel, hexanes/EtOAc, 3:1) to give 3 mg (47%) of Compound **414** as a bright yellow solid. Data for Compound **414**: $R_f$ = 0.19 (hexanes/EtOAc, 3:1); **$^1$H NMR** (400 MHz, $CDCl_3$) 7.25 (s, 1 H), 6.38 (s, 1 H), 6.36 (s, 1 H), 4.70 (br s, 1 H), 3.40 (m, 2 H), 2.70 (m, 1 H), 1.89 (m, 2 H), 1.67 n(m, 1 H), 1.55 (m, 1 H), 0.95 (t, *J* =7.4, 3 H).

## EXAMPLE 315

(*R*/*S*)-1,2,3,4-Tetrahydro-1,4-dimethyl-8-pyranono[5,6-*g*]quinoline (Compound **415**, structure **34A** of **Scheme XL**, where $R^{1-3}$=$R^6$=$R^8$=H, $R^4$=methyl, $R^5$=trifluoromethyl). In a flame dried 100 mL rb flask equivuipped with a magnetic stir bar was dissolved Compound **410** (EXAMPLE 310) (10.0 mg, 35.6 μmol) in glacial acetic acid (4 mL). To the stirred solution was added *para*-formadehyde (12 mg, 356 μmol, 10 equiv). The cloudy yellow solution stirred for 10 min then $NaCNBH_3$ (12 mg, 178 μmol, 5 equiv) was added at once. Upon addition the solution emitted gas for approx 5 min then turned bright yellow. After stirring for 12 h the solution was slowly poured over ice and quenched with NaOH (20%), extracted with EtOAc (2 x 25 mL), washed with brine (50 mL), dried ($Na_2SO_4$) and concentrated *in vacuo* to give 8.9 mg (86%) of Compound **415** as a yellow-green solid. Data for Compound **415**: $R_f$ = 0.22 (hexanes/EtOAc; 3:1); **$^1$H NMR** (400 MHz, $CDCl_3$) 7.25 (s, 1 H), 6.42 (s, 1 H), 6.35 (s, 1 H), 3.42 (m, 2 H), 3.00 (s, 3 H), 2.91 (m, 1 H), 2.00 (m, 1 H), 1.72 (m, 1 H), 1.28 (d, *J* = 6.8, 3 H).

## EXAMPLE 316

(*R*/*S*)-4-Ethyl-1,2,3,4-tetrahydro-1-methyl-8-pyranono[5,6-*g*]quinoline (Compound **416,** structure **34A** of **Scheme XL,** where $R^{1-3}$=$R^6$=$R^8$=H, $R^4$=ethyl, $R^5$=trifluoromethyl). In a flame dried 100 mL rb flask equivuipped with a magnetic stir bar was dissolved Compound **414** (8.0 mg, 27.1 μmol) in glacial acetic acid (3 mL). To the stirred solution was added *para*-formadehyde (8.0 mg, 271 μmol, 10 equiv). The cloudy yellow solution stirred for 10 min then $NaCNBH_3$ (8.0 mg, 135 μmol, 5 equiv) was added at once. Upon addition the solution emitted gas for approx. 5 min then turned bright yellow. After stirring for 12 h the solution was slowly poured over ice and quenched with NaOH (20%), extracted with EtOAc (2 x 25 mL), washed with brine (50 mL), dried ($Na_2SO_4$) and concentrated *in vacuo* to give 7.9 mg (94% yield) of Compound **416** as a bright yellow-green solid. Data for Compound **416**: $R_f$ = 0.23 (hexanes/EtOAc; 3:1); **$^1$H NMR** (400 MHz, $CDCl_3$) 7. 20 (s,1 H), 6.43 (s, 1 H), 6.35 (s, 1 H), 3.47 (m, 1 H), 3.30 (m, 1 H), 3.00 (s, 3 H), 2.68 (m, 1 H), 1.89 (m, 2 H), 1.56 (m, 4 H), 0.980 (d, *J*= 7.4, 3 H).

**EXAMPLE 317**

2,2-Dimethyl-1,2,3,4-tetrahydro-6-trifloromethyl-8-pyridono[5,6-*f*]quinoline (Compound **417,** structure **40A** of **Scheme XLII,** where $R^{1-2}$=$R^6$=H, $R^3$=$R^4$=methyl, $R^5$=trifluoromethyl) 7-*tert*-Butyloxycarbamoyl-1,2-dihydro-2,2-dimethylquinoline (structure **36A** of **Scheme XLII,** where $R^1$=$R^2$=H, $R^3$=$R^4$=methyl). To a flame-dried 200 mL r.b. flask containing 3-*tert*-butylcarbamoylaniline (EXAMPLE 147) (7.7 g, 0.037 mol) in 40 mL of anhydrous THF was added CuCl (183 mg, 1.8 mmol), triethylamine (5.15 mL, 0.037 mol) and 3-acetoxy-3-methyl-1-butyne (4.66 g, 0.037 mol). The reaction mixture was brought to reflux for 5 h then cooled to rt and filtered though a short pad of celite. Purification by flash column chromatography (silica gel, hexanes/ethyl acetate, 7:3) afforded 6.83 g (67%) of the desired propargyl intermediate that was used directly for the next step. The propargyl amine (6.5 g, 0.0237 mol) was dissolved in 40 mL of anhydrous THF, CuCl (234 mg, 0.0024 mol) was added and the mixture was heated to reflux for 16 h. The reaction mixture was diluted with ethyl acetate (200 mL), and washed with water and brine. The organic layer was dried ($Na_2SO_4$) and concentrated *in vacuo* to an oil that was subjected to chromatography (silica gel, hexanes/ethyl acetate, 9:1) which afforded 2.24 g (34%) of 7-*tert*-butyloxycarbamoyl-1,2-dihydro-2,2-dimethylquinoline along with 4.1g (63%) of the undesired regioisomer. Data for 7-*tert*-butyloxycarbamoyl-1,2-dihydro-2,2-dimethylquinoline: **$^1$H NMR** (400 MHz, $CDCl_3$) 6.80 (bs, 1H), 6.77 (d, *J* = 7.4, 1H), 6.33 (bs, 1H), 6.31 (bd, *J* =7.4, 1H),.6.18 (d, *J* = 9.7, 1H), 5.37 (d, *J* = 9.7, 1H), 3.70 (bs, 1 H), 1.49 (s, 9H), 1.27 (s, 6H).

7-Amino-1,2,3,4-tetrahydro-2,2-dimethylquinoline. A solution of 7-*tert*-butyloxycarbamoyl-1,2-dihydro-2,2-dimethylquinoline (3.4 g, 0.012 mol) in 150 mL of ethyl acetate was hydrogenated under an atmosphere of hydrogen with Pd-C 10% (340 mg) at rt for 7 h. Filtration over celite afforded 3.7 g (100%) of pure 7-*tert*-butyloxycarbamoyl-1,2,3,4-tetrahydro-2,2-dimethylquinoline. The title compound was prepared by General Method 12 (EXAMPLE 147) from 7-*tert*-butyloxycarbamoyl-1,2,3,4-tetrahydro-2,2-dimethylquinoline (3.7 g, 0.012 mol) to afford 2.35 g (100%) of 7-amino-1,2,3,4-tetrahydro-2,2-dimethylquinoline as a light reddish oil. Data for 7-amino-1,2,3,4-tetrahydro-2,2-dimethylquinoline: **$^1$H NMR** (400 MHz, $CDCl_3$) 6.77 (d, *J* = 7.9, 1H), 6.00 (dd, *J* = 7.9, 2.2, 1H), 5.81 (d, *J* = 2.2, 1H), 3.47 (bs, 1H), 3.40 (bs, 2H), 2.66 (t, *J* = 6.7, 2H), 1.65 (t, *J* = 6.7, 2H), 1.18 (s, 6H).

2,2-Dimethyl-1,2,3,4-tetrahydro-6-trifloromethyl-8-pyridono[5,6-*f*]quinoline (Compound **417**, structure **40A** of **Scheme XLII**, where $R^{1-2}$=$R^6$=H, $R^3$=$R^4$=methyl, $R^5$=trifluoromethyl). This compound was prepared by General Method 13 (EXAMPLE 147) from 7-amino-1,2,3,4-tetrahydro-2,2-dimethylquinoline (2.35 g, 0.012 mol), $ZnCl_2$ (2.74 g, 0.02 mol) and ethyl 4,4,4-trifluoroacetoacetate (2.15 mL, 0.013 mol) to afford 1.91 g (48%) of Compound **417.** Data for Compound **417:** $^1$H NMR (400 MHz, DMSO $d_6$) 11.70 (s, 1H), 7.18 (s, 1H), 6.85 (s, 1H), 6.35 (s, 1H), 2.65 (t, *J* = 6.6, 2H), 1.61 (t, *J* = 6.6, 2H), 1.17 (s, 6H).

**EXAMPLE 318**

(*R*/*S*)-1,2,3,4-tetrahydro-6-trifluoromethyl-2,2,4-trimethyl-8-pyridono[5,6-*f*]-3-quinolinone (Compound **418**, structure **47A** of **Scheme XLIV**, where $R^1$=$R^2$=H, $R^3$=trifluoromethyl) 1,9-di-*tert*-Butyloxycarbamoyl-1,2-dihydro-6-trifluoromethyl-2,2,4-trimethyl-8-pyridono[5,6-*f*]quinoline (structure **46A** of **Scheme XLIV**, where $R^1$=$R^2$=H, $R^3$=trifluoromethyl). To a suspension of NaH 60% in mineral oil (16 mg, 0.387 mmol) in 1 mL of anhydrous THF at 0°C, was added dropwise, a solution of Compound **247** (EXAMPLE 147) (100 mg, 0.32 mmol) and the resulting mixture was stirred at 0 °C for 10 min. A solution of t-$Boc_2O$ (78 mg, 0.355 mmol) in 1 mL of THF was added dropwise and the reaction mixture was stirred at rt for 1 h. The reaction mixture was quenched with water (1 mL), extracted with ethyl acetate (2 x 5 mL) and concentrated *in vacuo* to give 148 mg (100%) of a yellow solid that was used directly for the next step. To a solution of 9-*tert*-butyloxycarbamoyl-1,2-dihydro-2,2,4-trimethyl-8-pyridono[5,6-*f*]quinoline (130 mg, 0.32mmol) in 10 mL of anhydrous THF at -78°C was added *n*-BuLi 2.5 M in hexane (121 mL, 0.32 mmol) and the mixture stirred for 10 min. t-$Boc_2O$ (73 mg, 0.33 mmol) in 1 mL of THF was added and the reaction mixture stirred at -78°C for 6.5 h. The temperature was raised to 0 °C and the mixture quenched with water (3 mL), extracted with ethyl acetate (2 x 10 mL), dried ($Na_2SO_4$) and concentrated *in vacuo* to give a solid residue. Purification by flash column chromatography (silica gel, hexanes/ethyl acetate, 8:2) gave 79 mg (48%) of 1,9-di-*tert*-butyloxycarbamoyl-1,2-dihydro-2,2,4-trimethyl-8-pyridono[5,6-*f*]quinoline (15). **$^1$H NMR** (400 MHz, $CDCl_3$) 7.76 (s, 1H), 7.72 (s, 1H), 7.38 (s, 1H), 5.67 (s, 1H), 2.13 (s, 3H), 1.62 (s, 3H), 1.57 (s, 9H), 1.50 (s, 9H).

1-*tert*-butyloxycarbamoyl-1,2,3,4-tetrahydro-3-hydroxy-6-trifluoromethyl-2,2,4-trimethyl-8-pyridono[5,6-*f*]quinoline. A solution of 1,9-di-*tert*-butyloxycarbamoyl-1,2-dihydro-2,2,4-trimethyl-8-pyridono[5,6-*f*]quinoline (79 mg, 0.155 mmol) in 2 mL of anhydrous THF at rt was treated with 388 μL of $BH_3$.THF (1.0 M in THF, 0.388 mmol) for 3 h and was then quenched with 78 μL of $NaHCO_3$ satd't followed by 30% $H_2O_2$ (78 μL). The reaction mixture was stirred for 1 h, then 2 mL of water was added. The mixture was extracted with ethyl acetate (5 mL), dried ($Na_2SO_4$) and concentrated *in vacuo* to an oil that was subjected to flash column chromatography (silica gel, hexanes/ethyl acetate, 7:3) to give 21 mg (32%) of 1-*tert*-butyloxycarbamoyl-1,2,3,4-tetrahydro-3-hydroxy-2,2,4-trimethyl-8-pyridono[5,6-*f*]quinoline. Data for 1-*tert*-butyloxycarbamoyl-1,2,3,4-tetrahydro-3-hydroxy-2,2,4-trimethyl-8-pyridono[5,6-*f*]quinoline: **$^1$H NMR** (400 MHz, $CDCl_3$) 12.5 (bs, 1H), 7.51 (s, 1H), 7.28 (s, 1H), 6.85 (s, 1H), 3.19 (dd, *J* = 7.3, 5.2, 1H), 2.91 (m, 1H), 2.14 (d, *J*= 7.0, 1H), 1.65 (s, 3H), 1.55 (s, 9H), 1.52 (s, 3H), 1.46 (d, *J* = 6.1, 1H).

(*R*/*S*)-1,2,3,4-tetrahydro-6-trifluoromethyl-2,2,4-trimethyl-8-pyridono[5,6-*f*]-3-quinolinone (Compound **418**, structure **47A** of **Scheme XLIV**, where $R^1$=$R^2$=H, $R^3$=trifluoromethyl) To a suspension of PCC (50 mg, 0.23 mmol) in 2 mL of dichloromethane at rt was added 1-*tert*-butyloxycarbamoyl-1,2,3,4-tetrahydro-3-hydroxy-6-trifluoromethyl-2,2,4-trimethyl-8-pyridono[5,6-*f*]quinoline (16) (10 mg, 0.023 mmol) in 1 mL of dichloromethane. The reaction mixture was stirred at rt for 1.5 h, then it was filtered over celite and the solvent was removed *in vacuo* to give a dark oil that was subjected to flash column chromatography (silica gel, hexanes/ethyl acetate, 6:4) to give 5.5 mg (56%) of 1-*tert*-butyloxycarbamoyl-1,2,3,4-tetrahydro-6-trifluoromethyl-2,2,4-trimethyl-8-pyridono[5,6-*f*]-3-quinolinone that was used directly for the next step. The title compound was prepared by General Method 12 (EXAMPLE 147) from 1-*tert*-butyloxycarbamoyl-1,2,3,4-tetrahydro-6-trifluoromethyl-2,2,4-trimethyl-8-pyridono[5,6-*f*]-3-quinolinone (5.5 mg, 0.013 mmol) to afford 3 mg (71%) of Compound **418.** Data for Compound **418**: $^1$H NMR (400 MHz, $CDCl_3$) 12.3 (bs, 1H), 7.51 (s, 1H), 6.85 (s, 1H), 6.71 (s, 1H), 4.27 (s, 1H), 3.61 (q, *J* = 6.3, 1H), 1.55 (d, *J* = 6.3, 3H), 1.40 (s, 3H), 1.31 (s, 3H).

## EXAMPLE 319

5-Trifluoromethyl-7-pyridono[5,6-*e*]indoline (Compound **419**, structure **49A** of **Scheme XLV**, where $R^1$=trifluoromethyl, $R^2$=H)

6-Aminoindoline. A solution of 6-nitroindoline (1 g, 6.1 mmol) in 50 mL of ethyl acetate was hydrogenated under an atmosphere of hydrogen with Pd-C 10% (100 mg) at rt for 3 h. Filtration over celite afforded 1.0 g (98%) of 6-aminoindoline. Data for 6-aminoindoline: **$^1$H NMR** (400 MHz, $CDCl_3$) 7.40 (d, *J* = 7.4, 1H), 6.05 (d, *J* = 2.0, 1H), 6.03 ( d, *J* = 7.5, 1H), 3.67 (bs, 1H), 3.49 (t, *J* = 8.1, 2H), 3.48 (bs, 2H), 2.90 (t, *J* = 8.2, 2H).

5-Trifluoromethyl-7-pyridono[5,6-*e*]indoline (Compound **419**, structure **49A** of **Scheme XLV**, where $R^1$=trifluoromethyl, $R^2$=H) This compound was prepared by General Method 13 (EXAMPLE 147) from 6-aminoindoline (200 mg, 1.2 mmol), $ZnCl_2$ (262 mg, 1.93 mmol) and ethyl 4,4,4-trifluoroacetoacetate (194 mL, 1.32 mmol) to afford 100 mg (32%) of Compound **419**. Data for Compound **419**: **$^1$H NMR** (400 MHz, DMSO $d_6$) 12.1 (s, 1H), 7.31 (s, 1H), 6.82 (s, 1H), 6.49 (s, 1H), 6.40 (s, 1H), 3.59 (t, *J* = 8.1, 2H), 3.01 (t, *J* = 8.1, 2H).

## EXAMPLE 320

8-(4-Chlorobenzoyl)-5-trifluoromethyl-7-pyridono[5,6-*e*]indoline (Compound **420**, structure **50A** of **Scheme XLV**, where R $^1$=trifluromethyl, $R^2$=H, $R^3$=4-chlorophenyl). To a solution of Compound **419** (EXAMPLE 319) (13 mg, 0.05 mmol) in 2 mL of anhydrous THF at -78 °C was added *n*-BuLi 2.5 M in hexane (21 mL, 0.05 mmol) and the resulting mixture was stirred for 15 min. Then 4-chlorobenzoyl chloride (6.4 mL, 0.05 mmol) was added and the reaction mixture was slowly brough to rt over a period of 30 min. The reaction mixture was quenched with a saturated aqueous solution of $NH_4Cl$ (1 mL), extracted with ethyl acetate (5 mL) and concentrated *in vacuo* to an oil that was subjected to flash column chromatography (silica gel, hexaneslethyl acetate, 8:2) which afforded 3 mg (15%) of Compound **420**. Data for Compound **420**: **$^1$H NMR** (400 MHz, $CDCl_3$) 8.19 (d, *J* = 8.6, 2H), 7.77 (s, 1H), 7.51 (d, *J* = 8.6, 2H), 7.25 (s, 1H), 6.99 (s, 1H), 4.45 (s, 1H), 3.77 (t, *J* = 8.0, 2H), 3.28 (t, *J* = 8.0, 2H).

## EXAMPLE 321

7-*tert*-Butyloxycarbamoyl-1,2-dihydro-2,2,8-trimethylquinoline (structure **36A**, **Scheme XLII**, where $R^1$=$R^3$=$R^4$=methyl, $R^2$=H, P=*t*-butoxy, X=NH). To a flame-dried 10 mL r.b. flask containing 3-*tert*-butylcarbamoyl-2-methylaniline (EXAMPLE 155) (490 mg, 0.0022 mol) in 3 mL of anhydrous THF was added CuCl (11 mg, 0.1 mmol), triethylamine (307 mL, 0.0022 mol) and 3-acetoxy-3-methyl-1-butyne (278 mg, 0.0022 mol). The reaction mixture was brought to reflux for 5 h then cooled to rt and filtered through a short pad of celite. Purification by flash column chromatography (silica gel, hexanes/ethyl acetate, 7:3) afforded 290 mg (46%) of the desired propargyl intermediate that was used directly for the next step. The propargyl amine (290 mg, 0.001 mol) was dissolved in 5 mL of anhydrous THF, CuCl (5 mg, 0.05 mmol) was added and the mixture was heated to reflux for 16 h. The reaction mixture was diluted with ethyl acetate (10 mL), and washed with water then brine. The organic layer was dried ($Na_2SO_4$) and concentrated *in vacuo* to an oil that was subjected to chromatography (silica gel, hexanes/ethyl acetate, 9:1) which afforded 114 mg (40%) of 7-*tert*-butyloxycarbamoyl-1,2-dihydro-2,2,8-trimethylquinoline. Data for 7-*tert*-butyloxycarbamoyl-1,2-dihydro-2,2,8-trimethylquinoline: **$^1$H NMR** (400 MHz, $CDCl_3$) 6.85 (d, *J* = 7.4, 1H), 6.75 (d, *J* = 7.4, 1H), 6.23 (d, *J* = 9.5, 1H), 6.18 (bs, 1H), 5.42 (d, *J* = 9.5, 1H), 3.57 (bs, 1H), 1.92 (s, 3H), 1.45 (s, 9H), 1.29 (s, 6H).

7-Amino-1,2,3,4-tetrahydro-2,2,8-trimethylquinoline. A solution of 7-*tert*-butyloxycarbamoyl-1,2-dihydro-2,2,8-trimethylquinoline (114 mg, 0.39 mmol) in 4 mL of ethyl acetate was hydrogenated under an atmosphere of hydrogen with Pd-C 10% (11 mg) at rt for 7 h. Filtration over Celite afforded 60 mg (60%) of 7-*tert*-butyloxycarbamoyl-1,2,3,4-tetrahydro-2,2,8-trimethylquinoline. The title compound was prepared by General Method 12 (EXAMPLE 147) from 7-*tert*-butyloxycarbamoyl-1,2,3,4-tetrahydro-2,2,8-trimethylquinoline (60 mg, 0.206 mmol) to afford 30 mg (77%) of 7-amino-1,2,3,4-tetrahydro-2,2,8-trimethylquinoline as a light reddish oil. Data for 7-amino-1,2,3,4-tetrahydro-2,2,8-trimethylquinoline: **$^1$H NMR** (400 MHz, $CDCl_3$) 6.70 (d, *J* = 7.9, 1H), 6.09 (d, *J* = 7.9, 1H), 3.30 (bs, 3H), 2.71 (t, *J* = 6.7, 2H), 1.89 (s, 3H), 1.65 (t, *J* = 6.7, 2H), 1.21 (s, 6H).

2,2,10-Trimethyl-1,2,3,4-tetrahydro-6-trifloromethyl-8-pyridono[5,6-*f*]quinoline (Compound **421**, structure **40A** of **Scheme XLII**, where $R^1$=$R^3$=$R^4$=methyl, $R^2$=$R^6$=H, $R^5$=trifluoromethyl). This compound was prepared by General Method 13 (EXAMPLE 147) from 7-amino-1,2,3,4-tetrahydro-2,2,8-trimethylquinoline (30 mg, 0.159 mmol), $ZnCl_2$ (35 mg, 0.255 mmol) and ethyl 4,4,4-trifluoroacetoacetate (26 mL, 0.175 mmol) to afford 21 mg (42%) of Compound **421**. Data for Compound **421**: **$^1$H NMR** (400 MHz, $CDCl_3$) 9.13 (s, 1H), 7.34 (s, 1H), 6.67 (s, 1H), 4.10 (s, 1H), 2.88 (t, *J* = 6.7, 2H), 2.10 (s, 3H), 1.75 (t, *J* = 6.7, 2H), 1.30 (s, 6H).

## EXAMPLE 322

1,2,3,4-Tetrahydro-6-trifluoromethyl-8-pyridono[5,6-*f*]quinoline (Compound **422**, structure **53A** of **Scheme XLVI**, where $R^{1-3}$=$R^5$=H, $R^4$=trifluoromethyl)

7-Nitro-1,2,3,4-tetrahydroquinoline. 1,2,3,4-Tetrahydroquinoline (5 g, 0.0375 mol) was dissolved in 16 mL of sulfuric acid and the temperature lowered to 0°C, then 90% fuming nitric acid (1.67 mL, 0.0375 mol) was added slowly and the mixture strirred at 0°C for 30 min. It was then poured onto 100 g of ice and extracted with dichloromethane (2 x 100 mL). The organic phase was washed with saturated aqueous solution of $NaHCO_3$ (75 mL) and concentrated *in vacuo* to a reddish residue that was subjected to chromatography (silica gel, hexanes/ethyl acetate, 8:2) which afforded 4.1 g (61%) of 7-nitro-1,2,3,4-tetrahydroquinoline. Data for 7-nitro-1,2,3,4-tetrahydroquinoline: **$^1$H NMR** (400 MHz, $CDCl_3$) 7.39 (dd, *J* = 8.3, 2.2, 1H), 7.26 (d, *J* = 3.5, 1H), 7.01 (d, *J* = 8.3, 1H), 4.16 (bs, 1H), 3.35 (t, *J* = 5.0, 2H), 2.8 (t, *J* = 6.3, 2H), 1.95 (quintet, *J* = 6.1, 2H).

7-Amino-1,2,3,4-tetrahydroquinoline (structure **52A** of **Scheme XLVI,** where $R^{1-3}$=H). A solution of 7-nitro-1,2,3,4-tetrahydroquinoline (396 mg, 0.0022 mol) in 4 mL of ethyl acetate was hydrogenated under an atmosphere of hydrogen with PdC 10% (40 mg) at rt for 2 h. Filtration over celite afforded 330 mg (100%) of 7-amino-1,2,3,4-tetrahydroquinoline. Data for 7-amino-1,2,3,4-tetrahydroquinoline: **$^1$H NMR** (400 MHz, $CDCl_3$) 6.72 (d, *J* = 7.9, 1H), 6.00 (dd, *J* = 7.9, 2.3, 1H), 5.84 (d, *J* = 2.3, 1H), 3.67 (bs, 1H), 3.42 (bs, 2H), 3.24 (t, *J* = 5.0, 2H), 2.65 (t, *J* = 6.4, 2H), 1.91 (quintet, *J* = 6.0 Hz. 2H).

1,2,3,4-Tetrahydro-6-trifluoromethyl-8-pyridono[5,6-*f*]quinoline (Compound **422,** structure **53A** of **Scheme XLVI,** where $R^{1-3}$=$R^5$=H, $R^4$=trifluoromethyl) This compound was prepared by General Method 13 (EXAMPLE 147) from 7-amino-1,2,3,4-tetrahydroquinoline (330 mg, 0.0022 mol), $ZnCl_2$ (452 mg, 0.0033 mol) and ethyl 4,4,4-trifluoroacetoacetate (356 mL, 0.0024 mol) to afford 70 mg (11%) of Compound **422.** Data for Compound **422: $^1$H NMR** (400 MHz, DMSO $d_6$) 11.7 (bs, 1H), 7.11 (s, 1H), 6.92 (s, 1H), 6.35 (s, 2H), 3.22 (bs, 2H), 2.71 (t, *J* = 5.1, 2H), 1.93 (quintet, *J* = 6.1, 2H).

## EXAMPLE 323

1,2-Dihydro-6-trifluoromethyl-1,2,2,4-tetramethyl-8-pyridono[5,6-*f*]quinoline (Compound **423,** structure **60** of **Scheme XVI,** where $R^{1-2}$=$R^5$=H, $R^3$=trifluoromethyl, Z=NH).

To a stirred solution of Compound **247** (EXAMPLE 147) (100 mg, 0.323 mmol) and paraformaldehyde (98 mg, 3.23 mmol) in 3 mL of acetic acid at rt was added portionwise sodium cyanoborohydride (102 mg, 1.61 mmol). The resulting mixture was stirred at 25°C for 29 h then carefully poured into 20% aqueous NaOH (10 mL) and ice 10 g and the pH adjusted to ~7. The mixture was extracted with dichloromethane (25mL), dried ($Na_2SO_4$) and concentrated *in vacuo* to a fluorescent yellow solid that was subjected to flash column chromatography (silica gel, hexanes/ethyl acetate, 8:2) to give 92 mg (71%) of Compound **423**. Data for Compound **423**: **$^1$H NMR** (400 MHz, $CDCl_3$) 11.21 (bs, 1H), 7.33 (s, 1H), 6.67 (s, 1H), 6.23 (s, 1H), 5.39 (s, 1H), 2.92 (s, 3H), 2.02 (s, 3H), 1.38 (s, 6H).

## EXAMPLE 324

3,3-Dimethyl-5-trifluoromethyl-7-pyridono[5,6-e]indoline (Compound **424**, structure **57A** of **Scheme XLVII**, where $R^1$=methyl, $R^2$=$R^4$=H, $R^3$=trifluoromethyl).

2-bromo-*N*-(2-methyl-2-propenyl)-5-nitroaniline (structure **55A** of **Scheme XLVII**, where $R^1$=methyl, $R^2$=H) To a suspension of NaH 60% dispersion in oil (97 mg, 0.0023 mol) in 2 mL of anhydrous THF at 0°C was added 2-bromo-5-nitroaniline (500 mg, 0.0023 mol) in 2 mL of THF dropwise, the temperature was raised to rt to complete deprotonation then lowered to 0 °C. 3-bromo-2-methylpropene (232 mL, 0.0023 mol) was added very slowly and the reaction mixture was stirred at 0°C for 3 h then neutralized with water (5 mL). The mixture was extracted with ethyl acetate (2 x 10 mL),

dried ($Na_2SO_4$) and concentrated *in vacuo* to an oil that was subjected to flash column chromatography (silica gel, hexaneslethyl acetate, 8:2) to give 200 mg (32%) of 2-bromo-*N*-(2-methyl-2-propenyl)-5-nitroaniline (structure 55A of **Scheme XLVII,** where $R^1$=methyl, $R^2$=H). Data for 2-bromo-*N*-(2-methyl-2-propenyl)-5-nitroaniline: **$^1$H NMR** (400 MHz, $CDCl_3$) 7.55 (d, *J* = 8.5, 1H), 7.40 (dd, *J* = 8.5, 2.8, 1H), 7.39 (d, *J* = 2.8, 1H), 4.96 (s, 2H), 4.95 (bs, 1H), 3.82 (d, *J* = 5.9, 2H), 1.81 (s, 3H).

3,3-Dimethyl-6-nitroindoline (structure **56A** of **Scheme XLVII**, where $R^1$=methyl, $R^2$=H). A solution of 2-bromo-*N*-(2-methyl-2-propenyl)-5-nitroaniline (100 mg, 0.369 mmol), $Pd(OAc)_2$ (2 mg, 0.0073 mol), $Bu_4NBr$ (119 mg, 0.369 mmol) and triethylamine (129 mL, 0.922 mmol) in 1 mL of dry DMF under argon atmospere was heated at 80 °C for 1 h. Then sodium formate (25 mg, 0.369 mmol) was added to the reaction mixture with continued heating at 80 °C for 20 h. Water (2 mL) was added and the mixture was extracted with ethyl acetate (2 x 5 mL), dried ($Na_2SO_4$) and concentrated *in vacuo* to an oil that was subjected to flash column chromatography (silica gel, hexaneslethyl acetate, 8:2) to give 60 mg (80%) of 3,3-dimethyl-6-nitroindoline. Data for 3,3-dimethyl-6-nitroindoline: **$^1$H NMR** (400 MHz, $CDCl_3$) 7.60 (dd, *J*= 8.2, 2.0, 1H), 7.35 (d, *J* = 2.0, 1H), 7.08 (d, *J* = 8.2, 1H), 3.98 (bs, 1H), 3.41 (s, 2H), 1.33 (s, 6H).

6-Amino-3,3-dimethylindoline. A solution of 3,3-dimethyl-6-nitroindoline (60 mg, 0.31 mmol) in 3 mL of ethyl acetate was hydrogenated under an atmosphere of hydrogen with Pd-C 10% (10 mg) at rt for 3 h. Filtration over celite afforded 45 mg (90%) of 6-amino-3,3-dimethylindoline. Data for 6-amino-3,3-dimethylindoline: **$^1$H NMR** (400 MHz, $CDCl_3$)6.80 (d, *J* = 7.8, 1H), 6.08 (dd, *J* = 7.8, 2.1, 1H), 6.01 (d, *J* = 2.1, 1H), 3.60 (bs, 1H), 3.50 (bs, 2H), 3.26 (s, 2H), 1.25 (s, 6H).

3,3-Dimethyl-5-trifluoromethyl-7-pyridono[5,6-*e*]indoline (Compound **424**, structure **57A** of **Scheme XLVII**, where $R^1$=methyl, $R^2$=$R^4$=H, $R^3$=trifluoromethyl). This compound was prepared by General Method 13 (EXAMPLE 147) from 6-amino-3,3-dimethylindoline (45 mg, 0.277 mmol), $ZnCl_2$ (57 mg, 0.416 mmol) and ethyl 4,4,4-trifluoroacetoacetate (45 mL, 0.305 mmol) to afford 7.3 mg (9%) of 3,3-dimethyl-5-trifluoromethyl-7-pyridono[5,6-*e*]indoline (22). $^1$H NMR (400 MHz, $CDCl_3$) 12.4 (bs, 1H), 7.32 (s, 1H), 6.73 (s, 1H), 6.52 (s, 1H), 4.33 (s, 1H), 3.45 (s, 2H), 1.36 (s, 3H).

## EXAMPLE 325

(*R*/*S*)-1,2,3,4-Tetrahydro-4-methyl-6-(trifluoromethyl)-8-pyridono[5,6-*g*]quinoline (Compound **425**, structure **62A** of **Scheme XLVIII**, where $R^{1-3}$=$R^6$=H, $R^4$=methyl, $R^5$=trifluoromethyl).

1,2,3,4-Tetrahydro-4-quinolinone (structure **59A** of **Scheme XLVIII**, where $R^{1-3}$=H). In a 200 mL r.b. flask was introduced aniline (9.78 mL, 0.107 mol), acrylic acid (7.36 mL, 0.107 mol) and toluene (100 mL). The reaction mixture was stirred and heated at 100° C for 16 h, cooled to rt and the solvent was removed *in vacuo* to give 10.34 g (60%) of the desired intermediate carboxylic acid .that was used directly without further purification for the next step. In a 500 mL r.b. flask was introduced the acid (10.34 g, 0.064 mol) and polyphosphoric acid (200 mL). The reaction mixture was stirred and heated at 100° C for 16 h. The reaction mixture was cooled to rt, poured onto 700 mL of a 1:1 mixture of ice/water and neutralized slowly with NaOH. The aqueous phase was extracted with ethyl acetate (3 x 200 mL), dried ($Na_2SO_4$) and the solvent was removed *in vacuo* to give a solid residue that was subjected to flash chromatography (silica gel, hexanes/ethyl acetate, 6:1) to afford 6.97 g (76%) of 1,2,3,4-tetrahydro-4-quinolinone. Data for 1,2,3,4-tetrahydro-4-quinolinone: **$^1$H NMR** (400 MHz, $CDCl_3$) 7.84 (dd, *J* = 7.9, 1.1, 1H), 7.28 (ddd, *J* = 7.9, 7.9, 1.2, 1H), 6.72 (ddd, *J* = 8.1, 8.1, 0.8, 1H), 6.66 (d, *J* = 8.1, 1H), 4.49 (s, 1H), 3.56 (t, *J* = 6.9, 2H), 2.69 (t, *J* = 6.8, 2H).

1-*tert*-Butyloxycarbonyl-1,2,3,4-tetrahydro-4-quinolinone. To a stirred solution of $Boc_2O$ (10.05 g, 0.046 mol) and 1,2,3,4-tetrahydro-4-quinolinone (6.16 g, 0.042 mol) in THF (100 mL) at 0° C was added slowly DMAP (5.11 g, 0.042 mol) in 100 mL of THF. The reaction mixture was stirred overnight, then water (75 mL) was added and the mixture was extracted with ethyl acetate (2 x 200 mL). The organic phase was dried ($Na_2SO_4$) and the solvent was removed *in vacuo* to give a solid residue that was subjected to flash chromatography (silica gel, hexanes/ethyl acetate, 8:2) which afforded 8.5 g (82%) of 1-*tert*-butyloxycarbamoyl-1,2,3,4-tetrahydro-4-quinolinone. Data for 1-*tert*-butyloxycarbonyl-1,2,3,4-tetrahydro-4-quinolinone: **$^1$H NMR** (400 MHz, $CDCl_3$) 7.98 (dd, *J* = 7.9, 1.7, 1H), 7.76 (d, *J* = 8.4, 1H), 7.49 (ddd, *J* = 7.5, 7.5, 1.7, 1H), 7.15 (ddd, *J* = 8.0, 8.0, 0.9, 1H), 4.15 (t, *J* = 6.3, 2H), 2.76 (t, *J* = 6.6, 2H), 1.55 (s, 9H).

1-*tert*-Butyloxycarbonyl-1,2,3,4-tetrahydro-4-hydroxy-4-methylquinoline. To a solution of 1-*tert*-butyloxycarbonyl-1,2,3,4-tetrahydro-4-quinolinone (170 mg, 0.687 mmol) in THF (5 mL) at 0° C was added 3.0 M methylmagnesium bromide in ether (688 mL, 2.1 mmol). The reaction mixture was stirred at 0° C for 1 h then quenched with water (2 mL), extracted with ethyl acetate (2 x 10 mL), dried ($Na_2SO_4$) and the solvent was removed *in vacuo* to give an oil that was subjected to flash chromatography (silica gel, hexanes/ethyl acetate, 7:3) to afford 120 mg (66%) of 1-*tert*-butyloxycarbonyl-1,2,3,4-tetrahydro-4-hydroxy-4-methylquinoline. Data for 1-*tert*-butyloxycarbonyl-1,2,3,4-tetrahydro-4-hydroxy-4-methylquinoline: **$^1$H NMR** (400 MHz, DMSO-$d_6$) 7.54 (d, *J* = 7.7, 1H), 7.50 (dd, *J* = 7.7, 1.5, 1H), 7.14 (ddd, *J* = 7.3, 7.3, 1.7, 1H), 7.04 (ddd, *J* = 7.9, 7.9, 1.0, 1H), 5.14 (s, 1H), 3.69 (m, 2H), 1.87 (t, *J* = 6.5, 2H), 1.46 (s, 9H), 1.37 (s, 3H).

1-*tert*-Butyloxycarbonyl-1,2,3,4-tetrahydro-4-methylquinoline. A solution of 1-*tert*-butyloxycarbonyl-1,2,3,4-tetrahydro-4-hydroxy-4-methylquinoline (109 mg, 0.41 mmol) in ethyl acetate (3 mL) was hydrogenated under an atmosphere of hydrogen with 10% Pd/C (10 mg) and a trace of conc. $H_2SO_4$ at rt for 7 h. Filtration over Celite™ afforded 93 mg (92%) of 1-*tert*-butyloxycarbonyl-1,2,3,4-tetrahydro-4-methylquinoline. Data for 1-*tert*-butyloxycarbonyl-1,2,3,4-tetrahydro-4-methylquinoline: **$^1$H NMR** (400 MHz, $CDCl_3$) 7.62 (d, *J* = 8.1, 1H), 7.16 (d, *J* = 7.8, 1H), 7.11 (ddd, *J* = 7.8, 7.8, 1.6, 1H), 7.01 (ddd, *J* = 7.7, 7.5, 1.0, 1H), 3.71 (m, 2H), 2.87 (ddq, *J* = 6.8, 6.8, 6.8, 1H), 2.04 (dddd, *J* = 7.4, 7.4, 7.4, 6.1, 1H), 1.61 (m, 1H), 1.51 (s, 9H), 1.3 (d, *J* = 6.8, 3H).

1,2,3,4-tetrahydro-4-methylquinoline (structure **60A** of **Scheme XLVIII,** where $R^{1-3}$=H, $R^4$=methyl). This compound was prepared by General Method 12 (EXAMPLE 147) from 1-*tert*-butyloxycarbonyl-1,2,3,4-tetrahydro-4-methylquinoline (93 mg, 0.353 mmol) to afford 55 mg (95%) of 1,2,3,4-tetrahydro-4-methylquinoline as an oil which was used directly without purification for the next step.

7-Nitro-1,2,3,4-tetrahydro-4-methylquinoline. 1,2,3,4-Tetrahydro-4-methylquinoline (55 mg, 0.337 mmol) was dissolved in sulfuric acid (0.5 mL) and the temperature was lowered to 0° C. To this solution 90% fuming nitric acid (15 mL, 0.337 mmol) was added slowly and the mixture stirred at 0° C for 1 h, then warmed to rt. The reaction mixture was poured onto 1 g of ice and extracted with dichloromethane (2 x 5 mL). The organic phase was washed with sat. $NaHCO_3$ (1 x 3 mL) and concentrated *in vacuo* to a reddish residue that was subjected to chromatography (silica gel, hexanes/ethyl acetate, 8:2) which afforded 36 mg (52%) of 7-nitro-1,2,3,4-tetrahydro-4-methylquinoline. Data for 7-nitro-1,2,3,4-tetrahydro-4-methylquinoline: **$^1$H NMR** (400 MHz, $CDCl_3$) 7.41 (dd, *J* = 8.3, 2.2, 1H), 7.27 (d, *J* = 2.3, 1H), 7.11 (d, *J* = 8.3, 1H), 4.21 (s, 1H), 3.35 (m, 2H), 2.95 (m, 1H), 1.96 (m, 1H), 1.72 (m, 1H), 1.3 (d, *J* = 7.0, 3H).

1,2,3,4-Tetrahydro-4-methyl-6-trifluoromethyl-8-pyridono[5,6-*g*]quinoline (Compound **425**). A solution of 7-nitro-1,2,3,4-tetrahydro-4-methylquinoline (36 mg, 0.172 mmol) in ethyl acetate (3 mL) was hydrogenated under an atmosphere of hydrogen with 10% Pd/C (4 mg) at rt for 2 h. Filtration over Celite™ afforded 26 mg (85%) of 7-amino-1,2,3,4-tetrahydro-4-methylquinoline (structure **61A** of **Scheme XLVIII**, where $R^{1-3}$=H, $R^4$=methyl) that was used without further purification for the next step. The title compound was prepared by General Method 13 (EXAMPLE 147) from 7-amino-1,2,3,4-tetrahydro-4-methylquinoline (26 mg, 0.145 mmol), $ZnCl_2$ (30 mg, 0.218 mmol) and ethyl 4,4,4-trifluoroacetoacetate (21 mL, 0.145 mol) to afford 0.8 mg (2%) of 1,2,3,4-tetrahydro-4-methyl-6-trifluoromethyl-8-pyridono[5,6-*g*]quinoline (Compound **425**). Data for Compound **425**: **$^1$H NMR** (400 MHz, DMSO-$d_6$) 11.65 (bs, 1H), 7.20 (s, 1H), 6.96 (s, 1H), 6.37 (s, 2H), 3.25 (m, 2H), 2.90 (m, 1H), 1.84 (m, 1H), 1.59 (m, 1H), 1.20 (d, *J* = 6.9, 3H).

## EXAMPLE 326

1,2-Dihydro-2.2.4-trimethyl-6-methoxymethyl-8-pyridono[5,6-*g*]quinoline (Compound **426,** structure **57** of **Scheme XVII,** where $R^1$=$R^2$=H, $R^3$=methoxymethyl, Z=NH)

To a flame-dried 25-mL rb flask at rt was added ethanol (10 mL) and 7-amino-1,2-dihydro-2,2,4-trimethylquinoline (EXAMPLE 147)(600 mg, 3.5 mmol), and the mixture stirred at rt until the amine had completely dissolved. Methyl-4-methoxyacetoacetate (680 µL, 5.3 mmol, 1.5 equiv) was then added, followed by $ZnCl_2$ (960 mg, 7.0 mmol, 2.0 equiv). The reaction was stirred at rt under $N_2$ for 24 h. The solvent was removed under reduced pressure, and the solid residue was dissolved in EtOAc (10 mL). The organic phase was washed with sat'd. $NaHCO_3$ (adjusted to pH 9 with 3.0 M NaOH) (3 x 5 mL), dried ($Na_2SO_4$), and concentrated under reduced pressure. Purification by flash chromatography (silica gel, $CH_2Cl_2$/MeOH, 9:1), afforded 65 mg (7%) of Compound **426** as a dark yellow powder. Data for Compound **426:** $R_f$ 0.42 ($CH_2Cl_2$:MeOH, 9:1); **$^1$H NMR** (400 MHz, DMSO-$d_6$) 11.24 (s, 1H, 9-H), 7.12 (s, 1H, 5-H), 6.63 (s, 1H, 7-H), 6.26 (s, 1H, 10-H), 6.04 (s, 1H, 3-H ), 5.35 [s, 1H, $(CH_3)_2CNH$], 4.56 (s, 2H, $CH_2$), 3.37 (s, 3H, $OCH_3$), 1.93 (s, 3H, 4-$CH_3$), 1.21 [s, 6H, $C(CH_3)_2$].

## EXAMPLE 327

1,2,2,-Trimethyl-1,2,3,4-tetrahydro-6-trifluromethyl-8-pyranono[5,6-*g*]quinoline (Compound **427,** structure **41A** of **Scheme XLII,** where $R^{1-2}$=$R^6$=$R^8$=H, $R^{3-4}$=methyl, $R^5$=trifluoromethyl).

This compound was prepared in the manner similar to that described for Compound **416** (EXAMPLE 316) from Compound **412** (EXAMPLE 312) (5 mg) to give Compound **427** (4.2 mg, 93% yield) as a bright yellow solid. Data for Compound **427: $^1$H NMR** (400 MHz, $CDCl_3$) 7.19 (s, 1 H), 6.49 (s, 1 H), 6.36 (s, 1 H), 2.91 (s, 3 H), 2.78 (t, *J* = 6.5, 2 H), 1.84 (t, *J* = 6.5, 2 H), 1.31 (s, 6 H).

## EXAMPLE 328

(*R*/*S*)-1,2,3,4-Tetrahydro-4-propyl-6-trifluoromethyl-8-pyranono[5,6-*g*]quinoline (Compound **428,** structure **33A** of **Scheme XL,** where $R^{1-3}$, $R^6$=H, $R^4$=*n*-propyl, $R^5$=trifluoromethyl).

1-*tert*-Butoxycarbonyl-4-hydroxy-7-methoxy-4-propylquinoline. This compound was prepared in a manner similar to that described for 1-*tert*-butoxycarbonyl-4-ethyl-4-hydroxy-7-methoxyquinoline (EXAMPLE 314) from 1-*tert*-butoxycarbonyl-1,2,3,4-tetrahydro-7-methoxy-4-quinolinone (100 mg) to give the desired quinoline (51.2 mg, 40% yield) as an off-white solid. Data for 1-*tert*-butoxycarbonyl-4-hydroxy-7-methoxy-4-propyl-quinoline: **$^1$H NMR** (400 MHz, $CDCl_3$) 7.37 (d, *J* = 8.7, 1 H), 7.30 (d, *J* = 2.5, 1 H), 6.66 (dd, *J* = 8.9, 2.8, 1 H), 4.08 (m, 1 H), 3.86 (s, 3 H), 3.42 (m, 1 H), 2.04 (m, 1 H), 1.89 (m, 1 H), 1.81 (m, 2 H), 1.53 (s, 9 H), 1.26 (m, 2 H), 0.90 (t, *J* = 7.3, 3 H).

1-*tert*-Butoxycarbonyl-7-methoxy-4-propylquinoline. This compound was prepared in a manner similar to that described for 1-*tert*-butoxycarbonyl-4-ethyl-1,2,3,4-tetrahydro-7-methoxyquinoline (EXAMPLE 314) from 1-*tert*-butoxycarbonyl-1,2,3,4-tetrahydro-4-hydroxy-7-methoxy-4-propylquinoline (50 mg) to give the desired quinoline (44.3 mg, 94% yield) as a colorless oil. Data for 1-*tert*-butoxycarbonyl-7-methoxy-4-propylquinoline: **$^1$H NMR** (400 MHz, $CDCl_3$) 7.27 (d, *J* = 2.5, 1 H), 7.01 (d, *J* = 8.6, 1 H), 6.59 (dd, *J* = 8.5, 2.5, 1H), 3.78 (s, 3 H), 3.73 (m, 1 H), 3.58 (m, 1 H), 2.70 (m, 1 H), 1.94 (m, 1 H), 1.71 (m, 1 H), 1.63 (m, 1 H), 1.53 (s, 9H), 1.40 (m, 3 H), 0.93 (t, *J* = 7.2, 3 H)

1,2,3,4-Tetrahydro-7-methoxy-4-propylquinoline (structure **32A** of **Scheme XL**, where $R^{1-3}$ =H, $R^4$=*n*-propyl). This compound was prepared in a manner similar to that described for 4-ethyl-1,2,3,4-tetrahydro-7-methoxyquinoline (EXAMPLE 314) from 1-*tert*-butoxycarbonyl-1,2,3,4-tetrahydro-7-methoxy-4-propylquinoline (44 mg) to give the desired quinoline (28 mg, 98%) as a colorless oil. Data for 1,2,3,4-tetrahydro-7-methoxy-4-propylquinoline: **$^1$H NMR** (400 MHz, $CDCl_3$) 6.90 (d, *J* = 8.2, 1 H), 6.20 (dd, *J* = 8.4, 2.6, 1 H), 6.03 (d, *J = 2.5,* 1 H), 3.83 (br s, 1 H), 3.72 (s, 3 H), 3.28 (m, 2 H), 2.68 (m, 1 H), 1.89 (m, 1 H), 1.75 (m, 1 H), 1.60 (m, 1 H), 1.46 (m, 3 H), 0.94 (t, *J* = 7.1, 3 H)

1,2,3,4-Tetrahydro-7-hydroxy-4-propylquinoline. This compound was prepared in a manner similar to that described for 4-ethyl-1,2,3,4-tetrahydro-7-hydroxyquinoline (EXAMPLE 314) from 1,2,3,4-tetrahydro-7-methoxy-4-propylquinoline (28 mg) to give the desired quinoline as a colorless oil, which was used without further purification in the following reaction. Data for 1,2,3,4-tetrahydro-7-hydroxy-4-propylquinoline: **$^1$H NMR** (400 MHz, $CDCl_3$) 6.84 (d, *J* = 8.2, 1 H), 6.10 (dd, *J* = 8.2, 2.3, 1 H), 5.97 (d, *J* = 2.2, 1 H), 3.78 (br s, 1 H), 3.29 (m 1 H), 3.21 (m, 1 H), 2.66 (m, 1 H), 1.87 (m, 1 H), 1.75 (m, 1 H), 1.60 (m, 1 H), 1.45 (m, 3 H), 0.933 (t, *J* = 7.2, 3 H).

4-Propyl-1,2,3,4-tetrahydro-6-trifluromethyl-8-pyranono[5,6-*g*]quinoline (Compound **428**). This compound was prepared in a manner similar to that described for Compound **414** (EXAMPLE 314) from 1,2,3,4-tetrahydro-7-hydroxy-4-propylquinoline (23 mg) to give the Compound **428** (28.4 mg, 61 %) as a yellow solid. Data for Compound **428: $^1$H NMR** (400 MHz, $CDCl_3$) 7.23 (s, 1 H), 6.37 (s, 1 H), 6.36 (s, 1 H), 4.70 (br s, 1 H), 3.40 (m, 2 H), 2.81 (m, 1 H), 1.88 (m, 2 H), 1.47 (m, 3 H), 0.963 (t, *J* = 7.2, 3 H)

**EXAMPLE 329**

1,2,3,4-Tetrahydro-2,2,4-trimethyl-6-trifluoromethyl-9-thiopyran-8-ono[5,6-*g*]quinoline (Compound **429**, structure **65A** of **Scheme XLIX**, where $R^{1-2}$=$R^7$=H, $R^{3-5}$=methyl, $R^6$=trifluoromethyl, X=S).
To a solution of Compound **266** (EXAMPLE 166) (50 mg, 0.15 mmol) in dichloromethane (7 mL ) was added triethylsilane (0.23 mL, 1.5 mmol) and TFA (0.25 mL ) at rt. After 15 h, the reaction was complete according to TLC. The reaction mixture was quenched with a saturated $NaHCO_3$ solution (10 mL). This solution was extracted with EtOAc (20 mL ). The organic layer was washed with water and brine (3 x 5 mL each), dried ($Na_2SO_4$), and concentrated *in vacuo* to afford the crude product as an orange solid. The crude product was purified by prep TLC (20 x 20cm, 1000µm, 1:1 $CH_2Cl_2$:Hex.) to afford 49 mg (99%) of Compound **429** as a yellow solid. Data for Compound **429:** $R_f$ = 0.44 (silica gel, 25% EtOAc:Hex); **$^1$H NMR**(400 MHz, $CDCl_3$) 7.70 (s, 1 H), 6.62 (s, 1 H), 6.46 (s, 1 H), 4.41 (brs, 1 H), 2.95 (ddq, *J* = 12.9, 6.1, 1 H), 1.81 (dd, *J* = 12.9, 1.1, 1 H), 1.48 (d, *J* = 6.1, 1 H), 1.41 (d, *J* = 6.1, 3 H), 1.31 (s, 3 H), 1.24 (s, 3 H); **IR** (film, NaCl) 1134, 1177, 1200, 1235, 1269, 1368, 1365, 1420, 1451, 1476, 1520, 1634, 3351.

**EXAMPLE 330**

1,2-Dihydro-1,2,2,4-tetramethyl-6-trifluoromethyl-9-thiopyran-8-ono[5,6-*g*]quinoline (Compound **430,** structure **60** of **Scheme XVI,** where $R^{1-2}$=$R^5$=H, $R^3$=trifluoromethyl, Z=S).
To a stirred solution of Compound **266** (EXAMPLE 166) (100 mg, 0.30 mmol) and paraformaldehyde (93 mg, 3.0 mmol) in acetic acid (3 mL) at rt was added portionwise sodium cyanoborohydride (100 mg, 1.50 mmol). The resulting mixture was stirred at rt for 16 h, then carefully poured into 20% aqueous NaOH (10 mL) and ice (10 g) and the pH adjusted to ~7. The mixture was extracted with dichloromethane (25 mL), dried ($Na_2SO_4$) and concentrated *in vacuo* to a fluorescent yellow solid that was subjected to flash column chromatography (silica gel, hexanes/ethyl acetate, 9:1) to give 90 mg (88%) of Compound **430** as a fluorescent yellow solid. Data for Compound **430: $^1$H NMR** (400 MHz, $CDCl_3$) 7.48 (s, 1 H), 6.62 (s, 1 H), 6.45 (s, 1 H), 5.40 (s, 1 H), 2.89 (s, 3 H), 2.10 (s, 3 H), 1.39 (s, 6 H).

**EXAMPLE 331**

1,2,3,4-Tetrahlydro-1,2,2-trimethyl-6-trifluoromethyl-8-pyridono[5,6-*g*]quinoline (Compound **431**, structure **41A** of **Scheme XLII**, where $R^{1-2}$=$R^6$=$R^8$=H, $R^{3-4}$=methyl, $R^5$=trifluoromethyl, X=NH).

To a stirred solution of Compound **417** (EXAMPLE 317) (21 mg, 0.07 mmol) and paraformaldehyde (22 mg, 0.70 mmol) in acetic acid (1 mL) at rt was added portionwise sodium cyanoborohydride (22 mg, 0.35 mmol). The resulting mixture was stirred at rt for 16 h then carefully poured into 20% aqueous NaOH (2 mL) and ice (10 g) and the pH adjusted to ~7. The mixture was extracted with dichloromethane (2 x 10mL), dried ($Na_2SO_4$) and concentrated *in vacuo* to a fluorescent yellow solid that was subjected to flash column chromatography (silica gel, hexanes/ethyl acetate, 7:3) to give 16 mg (73%) of Compound **431** as a fluorescent yellow solid. Data for Compound **431**: **$^1$H NMR** (400 MHz, $CDCl_3$) 10.83 (bs, 1 H), 7.31 (s, 1 H), 6.66 (s, 1 H), 6.29 (s, 1 H), 2.93 (s, 3 H), 2.80 (t, *J* = 6.1, 2 H), 1.83 (t, J = 6.5, 2 H), 1.30 (s, 6 H).

## EXAMPLE 332

1,2,3,4-Tetrahydro-1-methyl-4-propyl-6-trifluoromethyl-8-pyranono[5,6-*g*]quinoline (Compound **432**, structure **34A** of **Scheme XL**, where $R^{1-3}$=$R^6$=$R^8$=H, $R^4$=*n*-propyl, $R^5$=trifluoromethyl).
This compound was prepared in a manner similar to that described for Compound **415** (EXAMPLE 315) from Compound **428** (EXAMPLE 328) (8.0 mg) to afford 7.9 mg (99%) of Compound **432** as a bright yellow solid. Data for Compound **432**: **$^1$H NMR** (400 MHz, $CDCl_3$) 7.18 (s, 1 H), 6.43 (s, 1 H), 6.35 (s, 1 H), 3.46 (m, 1 H), 3.33 (m, 1 H), 3.00 (s, 3 H), 1.92 (m, 1 H), 1.87 (m, 1 H), 1.49 (m, 4 H), 0.95 (d, *J* = 7.3, 3 H).

## EXAMPLE 333

1,2,3,4-Tetrahydro-10-hydroxymethyl-2,2,4-trimethyl-6-trifluoromethyl-8-pyridono[5,6-*g*]quinoline (Compound **433**, structure **67A** of **Scheme L**, where $R^{1-2}$=$R^7$=H, $R^{3-5}$ =methyl, $R^6$=trifluoromethyl, X=NH).
To an oven-dried 50-mL round-bottom flask containing Compound **409** (EXAMPLE 309) (125 mg, 0.39 mmol) in 1,4-dioxane (7 mL) was added selenium dioxide (107 mg, 0.96 mmol, 2.50 equiv), and the mixture was heated to reflux for 18 h. Upon cooling to rt, the solvent was removed under reduced pressure and the residue was purified by flash chromatography (silica gel, hexanes/ethyl acetate, 4:1 to 0:1 gradient), affording 15.6 mg (12%) of Compound **433** as a fluorescent yellow solid. Data for Compound **433: $^1$H NMR** (400 MHz, $CDCl_3$) 9.32 (br s, 1 H, CON*H*), 7.44 (s, 1 H, 5-H), 6.74 (s, 1 H, 7-H), 5.32 [br s, 1 H, $(CH_3)_2CNH$], 4.57 (d, 1 H, *J* = 9.7, O*H*), 5.02 and 4.93 (ABq, 2 H, $J_{AB}$ = 14.0, $CH_2OH$), 2.85 (ddq, 1 H, *J* = 12.9, 12.4, 5.5, 4-H), 1.84 and 1.54 [d of ABq, 2H, $J_{AB}$ = 13.1, $J_A$ = 4.3, (3-Heq), $J_B$ = 0 (3-$H_{ax}$)], 1.41 (d, 3H, *J* = 5.5 Hz, 4-$CH_3$), 1.39 and 1.26 [2s, 2 x 3H, 2-$(CH_3)_2$].

## EXAMPLE 334

1,2,3,4-Tetrahydro-1,2,2,4-tetramethyl-6-trifluoromethyl-9-thiopyran-8-ono[5,6-*g*]quinoline (Compound **434,** structure **28A** of **Scheme XXXVIII,** where $R^{1-2}$=$R^5$=H, $R^3$=trifluoromethyl, Z=S)
To a solution of Compound **429** (EXAMPLE 329) (10 mg, 0..03 mmol) in acetic acid (5 mL ) was added paraformaldehyde (10 mg, 0.3 mmol) and sodium cyanoborohydride (10 mg, 0.15 mmol) under nitrogen with stirring at rt. After 15 h, the reaction was complete according to $^1$H NMR. The reaction was quenched with saturated $NaHCO_3$ (10 mL). This solution was extracted with EtOAc (20 mL). The organic layer was washed with water and brine (3 x 5 mL each), dried ($Na_2SO_4$), and concentrated *in vacuo* to afford the crude product. The product was purified by prep TLC (5 x 20cm, 250µm, 1:1 $CH_2Cl_2$:hexanes) to afford 4.5 mg (44%) of Compound **434** as a yellow solid. Data for compound **434**: **$^1$H NMR**(400 MHz, $CDCl_3$) 7.59 (s, 1 H), 6.60 (s, 1 H), 6.53 (s, 1 H), 2.89 (s, 3 H), 2.85 (m, 1 H), 1.83 (dd, *J* = 13.2, 4.2, 1 H), 1.53 (d, *J* = 13.2, 1 H), 1.36 (d, *J* = 6.6, 3 H), 1.33 (s, 3 H), 1.23 (s, 3H); **IR** (film, NaCl) 1022, 1066, 1094, 1113, 1134, 1271, 1368, 1464, 1512, 1593, 2926.

## EXAMPLE 335

1,2,3,4-Tetrahydro-2,2,9-trimethyl-6-trifluoromethyl-8-pyridono[5,6-*g*]quinoline (Compound **435,** structure **84A** of **Scheme LVI**, where $R^1$=$R^{5-6}$=H, $R^{2-3}$=methyl, $R^4$=trifluoromethyl).
In a 25-mL r.b., a solution of Compound **417** (165 mg, 0.557 mmol) in THF (4 mL) was cooled to 0° C and treated with 60% NaH in mineral oil (23 mg, 0.58 mmol, 1.0 equivuiv). The reaction mixture was stirred 10 min. To this slurry, iodomethane (35 mL, 0.56 mmol, 1.0 equiv) was added via syringe. The reaction mixture was stirred 12 h, diluted with $H_2O$ (20 mL), and extracted with ethyl acetate (3 x 20 mL). The extracts were washed with brine (1 x 20 mL), combined, dried ($MgSO_4$), filtered, and concentrated. Purification by silica gel chromatography ($CH_2Cl_2$:MeOH, 50:1) afforded 134 mg (78%) of Compound 435 as a pale yellow powder. Data for Compound 435: **$^1$H NMR** (400 MHz, acetone-$d_6$): 7.35 (s, 1 H), 6.56 (s, 1 H), 6.51 (s, 1 H), 6.09 (br s, 1 H), 3.53 (s, 3 H), 2.87 (t, *J* = 6.7, 2 H), 1.76 (t, *J* = 6.7, 2 H), 1.29 (s, 6 H).

## EXAMPLE 336

(*R*/*S*)-1,2,3,4-Tetrahydro-3-methyl-6-trifluoromethyl-8-pyridono[5,6-*g*]quinoline (Compound **436**, structure **62A** of **Scheme XLVIII**, where $R^{1-2}$=$R^4$=$R^6$=H, $R^3$=methyl, $R^5$=trifluoromethyl).
1-*tert*-Butyloxycarbonyl-1,2,3,4-tetrahydro-3-methyl-4-quinolinone (structure **69A** of **Scheme LI**, where $R^{1-2}$=H, $R^3$=methyl). To a solution of 1-*tert*-butyloxycarbonyl-1,2,3,4-tetrahydro-4-quinolinone (structure **68A** of **Scheme LI**, where $R^{1-2}$=H) (EXAMPLE 325) (500 mg, 0.002 mol) in THF (5 mL) at -78° C was added 2.0 M LDA in THF (1.01 mL, 0.002 mol). The reaction mixture was stirred at -78° C for 15 min and iodomethane (126 mL, 0.002 mol) was added all at once. The temperature was raised to 0° C and the resulting mixture stirred for 4 h. The reaction was then quenched with sat'd $NH_4Cl$ (5mL), extracted with ethyl acetate (2 x 10 mL), dried ($Na_2SO_4$) and concentrated *in vacuo* to a solid residue that was subjected to flash column chromatography (silica gel, hexanes/ethyl acetate, 95:5) to give 117 mg (23%) of 1-*tert*-butyloxycarbonyl-1,2,3,4-tetrahydro-3-methyl-4-quinolinone (structure **69A** of **Scheme LI,** where $R^{1-2}$=H, $R^3$=methyl), 128 mg (23%) of 1-*tert*-butyloxycarbonyl-1,2,3,4-tetrahydro-3,3-dimethyl-4-quinolinone (structure **70A** of **Scheme LII,** where $R^{1-2}$=H, $R^{3-4}$=methyl) and 200 mg (40%) of recovered starting material. Data for 1-*tert*-butyloxycarbonyl-1,2,3,4-tetrahydro-3-methyl-4-quinolinone: **$^1$H NMR** (400 MHz, $CDCl_3$) 7.99 (dd, *J* =7.9, 1.7, 1 H), 7.77 (d, *J* = 8.4, 1 H), 7.48 (ddd, *J* =7.3, 7.3, 1.7, 1 H), 7.13 (dd, *J* = 7.4, 1.0, 1 H), 4.32 (dd, *J* = 13.4, 4.4, 1 H), 3.69 (dd, *J* = 13.3, 9.8, 1 H), 2.76 (ddq, *J* = 9.8, 7.0, 4.4, 1 H), 1.56 (s, 9 H), 1.24 (d, *J* = 7.0, 3 H).
1-*tert*-Butyloxycarbonyl-1,2,3,4-tetrahydro-3-methylquinoline. To a solution of 1-*tert* -butyloxycarbonyl-1,2,3,4-tetrahydro-3-methyl-4-quinolinone (117 mg, 0.45 mmol) in methanol (2 mL) at 0° C was added portionwise sodium borohydride (17 mg, 0.45 mmol) and the reaction mixture was stirred at 0° C for 3 h. The reaction was quenched with of sat'd $NH_4Cl$ (2 mL), extracted with ethyl acetate (2 x 5 mL), dried ($Na_2SO_4$) and concentrated *in vacuo* to give 116 mg (98%) of the alcohol that was used directly without purification for the next step. A solution of the alcohol intermediate (116 mg, 0.44 mmol) in ethyl acetate (3 mL) was hydrogenated under an atmosphere of hydrogen with 10% Pd/C (20 mg) and a trace of conc. $H_2SO_4$ at rt for 16 h. Filtration over Celite™ afforded 104 mg (95%) of 1-*tert*-butyloxycarbonyl-1,2,3,4-tetrahydro-3-methylquinoline. Data for 1-*tert*-butyloxycarbonyl-1,2,3,4-tetrahydro-3-methylquinoline: **$^1$H NMR** (400 MHz, $CDCl_3$) 7.65 (d, *J* = 8.3, 1 H), 7.11 (dd, *J* = 7.7, 7.7,1 H), 7.04 (d, *J* = 7.2, 1 H), 6.96 (dd, *J* = 7.4, 7.4, 1 H), 3.97 (ddd, *J* = 12.7, 4.2, 1.0, 1 H), 3.09 (dd, *J* = 11.8, 9.8, 1 H), 2.86 (dd, *J* = 16.2, 5.3, 1 H), 2.40 (dd, *J* = 16.1, 9.6, 1 H), 2.03 (m, 1 H), 1.52 (s, 9 H), 1.05 (d, *J* = 6.7, 3 H).

1,2,3,4-tetrahydro-3-methylquinoline (structure **60A** of **Scheme LI,** where $R^{1-2}$=$R^4$=H, $R^3$=methyl). This compound was prepared by General Method 12 (EXAMPLE 147) from 1-*tert*-butyloxycarbonyl-1,2,3,4-tetrahydro-3-methylquinoline (104 mg, 0.42 mmol) to afford 51 mg (83%) of 1,2,3,4-tetrahydro-3-methylquinoline as an oil which was used directly without purification for the next step.

7-Nitro-1,2,3,4-tetrahydro-3-methylquinoline. 1,2,3,4-Tetrahydro-3-methylquinoline (51 mg, 0.35 mmol) was dissolved in sulfuric acid (0.5 mL) and the temperature lowered to 0° C. To this solution 90% fuming nitric acid (15 mL, 0.35 mmol) was added slowly and the mixture stirred at 0° C for 1 h, then warmed to rt. The reaction mixture was then poured onto 1 g of ice and extracted with dichloromethane (2 x 5 mL). The organic phase was washed with saturated aqueous $NaHCO_3$ (3 mL) and concentrated *in vacuo* to a reddish residue that was subjected to chromatography (silica gel, hexanes/ethyl acetate, 85:15) which afforded 8.2 mg (12%) of 7-nitro-1,2,3,4-tetrahydro-3-methylquinoline. Data for 7-nitro-1,2,3,4-tetrahydro-3-methylquinoline: **$^1$H NMR** (400 MHz, $CDCl_3$) 7.39 (dd, *J* = 8.25, 2.2, 1 H), 7.27 (d, *J* = 2.3, 1 H), 7.01 (d, *J* = 8.3, 1 H), 4.19 (s, 1 H), 3.33 (m, 1 H), 2.94 (dd, *J* = 10.1, 10.1, 1 H), 2.86 (ddd, *J* = 13.8, 4.7, 1.7, 1 H), 2.46 (dd, *J* = 16.6, 10.0, 1 H), 2.05 (m, 1 H), 1.06 (d, *J* = 6.7, 3 H).

1,2,3,4-Tetrahydro-3-methyl-6-trifluoromethyl-8-pyridono[5,6-*g*]quinoline (Compound **436**). A solution of 7-nitro-1,2,3,4-tetrahydro-3-methylquinoline (8.2 mg, 0.042 mmol) in ethyl acetate (1 mL) was hydrogenated under an atmosphere of hydrogen with 10% Pd/C (4 mg) at rt for 2 h. Filtration over Celite™ afforded 6.2 mg (89%) of 7-amino-1,2,3,4-tetrahydro-3-methylquinoline (structure **61A** of **Scheme XLVIII**, where $R^{1-2}$=$R^4$=H, $R^3$=methyl) that was used without further purification for the next step. Compound **436** was prepared by General Method 13 (EXAMPLE 147) from 7-amino-1,2,3,4-tetrahydro-3-methylquinoline (6.2 mg, 0.038 mmol), $ZnCl_2$ (8.0 mg, 0.057 mmol) and ethyl 4,4,4-trifluoroacetoacetate (5.5 mL, 0.038 mol) to afford 5.8 mg (54%) of Compound **436** as a yellow solid. Data for Compound **436**: **$^1$H NMR** (400 MHz, DMSO-$d_6$) 11.80 (bs, 1 H), 7.11 (s, 1 H), 6.95 (s, 1 H), 6.37 (s, 2 H), 3.26 (m, 1 H), 2.83 (m, 2 H), 2.51 (dd, *J* = 15.7, 10.3, 1 H), 1.88 (s, 1 H), 0.97 (d, *J* = 6.6, 3 H).

## EXAMPLE 337

1,2,3,4-Tetrahydro-3,3-dimethyl-6-trifluoromethyl-8-pyridono[5,6-*g*]quinoline (Compound **437**, structure **73A** of **Scheme LII**, where $R^{1-2}$=$R^5$=$R^7$=H, $R^{3-4}$=methyl, $R^6$=trifluoromethyl).
1-*tert*-Butyloxycarbonyl-1,2,3,4-tetrahydro-3,3-dimethyl-4-quinolinone (structure **70A** of **Scheme LII**, where $R^{1-2}$=H, $R^{3-4}$=methyl). This compound was obtained along with 1-*tert*-butyloxycarbonyl-1,2,3,4-tetrahydro-3-methyl-4-quinolinone as described above (EXAMPLE 336). Data for 1-*tert*-butyloxycarbonyl-1,2,3,4-tetrahydro-3,3-dimethyl-4-quinolinone: **$^1$H NMR** (400 MHz, $CDCl_3$) 8.01 (dd, *J* = 7.9, 1.6, 1 H), 7.78 (d, *J* = 8.4, 1 H), 7.49 (ddd, *J* = 7.6, 7.6, 1.7, 1 H), 7.14 (ddd, *J* = 7.8, 7.8, 1.6, 1 H), 3.86 (s, 2 H), 1.56 (s, 9 H), 1.20 (s, 6 H).

1-*tert*-Butyloxycarbonyl-1,2,3,4-tetrahydro-3,3-dimethylquinoline. To a solution of 1-*tert*-butyloxycarbonyl-1,2,3,4-tetrahydro-3,3-dimethyl-4-quinolinone (128 mg, 0.47 mmol) in methanol (2 mL) at 0° C was added portionwise sodium borohydride (18 mg, 0.47 mmol) and the reaction mixture was stirred at 0° C for 3 h. The reaction mixture was then quenched with sat'd $NH_4Cl$ (2 mL), extracted with ethyl acetate (2 x 5 mL), dried ($Na_2SO_4$) and concentrated. A solution of this crude material in ethyl acetate (3 mL) was hydrogenated under an atmosphere of hydrogen with 10% Pd/C (20 mg) and a trace of conc. $H_2SO_4$ at rt for 16 h. Filtration over Celite™ afforded 100 mg (84%) of 1-*tert*-butyloxycarbonyl-1,2,3,4-tetrahydro-3,3-dimethylquinoline. Data for 1-*tert*-butyloxycarbonyl-1,2,3,4-tetrahydro-3,3-dimethylquinoline: **$^1$H NMR** (400 MHz, $CDCl_3$) 7.68 (d, *J* = 8.3, 1 H), 7.12 (ddd, *J* = 8.8, 8.8, 1.5, 1 H), 7.02 (d, *J* = 7.0, 1 H), 6.97 (ddd, *J* = 7.4, 7.4, 1.0, 1 H), 3.46 (s, 2 H), 2.58 (s, 2 H), 1.51 (s, 9 H), 1.01 (s, 6 H).

1,2,3,4-tetrahydro-3,3-dimethylquinoline (structure **71A** of **Scheme LII,** where $R^{1-2}$ =$R^5$=H, $R^{3-4}$=methyl). This compound was prepared by General Method 12 (EXAMPLE 147) from 1-*tert*-butyloxycarbonyl-1,2,3,4-tetrahydro-3,3-dimethylquinoline (100 mg, 0.38 mmol) to afford 51 mg (83%) of 1,2,3,4-tetrahydro-3,3-dimethylquinoline as an oil which was used directly without purification for the next step.

7-Nitro-1,2,3,4-tetrahydro-3,3-dimethylquinoline. 1,2,3,4-Tetrahydro-3,3-dimethylquinoline (51 mg, 0.32 mmol) was dissolved in sulfuric acid (0.5 mL) and the temperature lowered to 0° C. To this solution 90% fuming nitric acid (14 mL, 0.32 mmol) was added slowly and the mixture stirred at 0° C for 1 h, then warmed to rt. The reaction mixture was then poured onto 1 g of ice and extracted with dichloromethane (2 x 5 mL). The organic phase was washed with saturated aqueous $NaHCO_3$ (3 mL) and concentrated *in vacuo* to a reddish residue that was subjected to chromatography (silica gel, hexanes/ethyl acetate, 85:15) which afforded 39 mg (58%) of 7-nitro-1,2,3,4-tetrahydro-3,3-dimethylquinoline. Data for 7-nitro-1,2,3,4-tetrahydro-3,3-dimethylquinoline: **$^1$H NMR** (400 MHz, $CDCl_3$) 7.40 (dd, *J* = 8.3, 2.1, 1 H), 7.29 (d, *J* = 1.8, 1 H), 7.01 (d, *J* = 8.3 H, 1 H), 4.25 (s, 1 H), 2.98 (s, 2 H), 2.54 (s, 2 H), 1.01 (s, 6 H).

1,2,3,4-Tetrahydro-3,3-dimethyl-6-trifluoromethyl-8-pyridono[5,6-*g*]quinoline (Compound **437**). A solution of 7-nitro-1,2,3,4-tetrahydro-3,3-dimethylquinoline (39 mg, 0.187 mmol) in ethyl acetate (2 mL) was hydrogenated under an atmosphere of hydrogen with 10% Pd/C (4 mg) at rt for 2 h. Filtration over Celite™ afforded 30 mg (91%) of 7-amino-1,2,3,4-tetrahydro-3,3-dimethylquinoline (structure **72A** of **Scheme LII,** where $R^{1-2}$=$R^5$=H, $R^{3-4}$ =methyl) that was used without further purification in the next step. Compound **437** was prepared by General Method 13 (EXAMPLE 147) from 7-amino-1,2,3,4-tetrahydro-3,3-dimethylquinoline (30 mg, 0.17 mmol), $ZnCl_2$ (34 mg, 025 mmol) and ethyl 4,4,4-trifluoroacetoacetate (25 mL, 0.17 mol) to afford 13 mg (26%) of Compound **437** as a yellow solid. Data for Compound **437: $^1$H NMR** (400 MHz, DMSO-$d_6$) 11.71 (bs, 1 H), 7.11 (s, 1 H), 7.01 (s, 1 H), 6.40 (s, 1 H), 6.37 (s, 1 H), 2.89 (s, 2 H), 2.51 (s, 2 H), 0.93 (s, 6 H).

## EXAMPLE 338

(*R*/*S*)1,2,3,4-Tetrahydro-2,2,3-trimethyl-6-trifluoromethyl-8-pyridono[5,6-*g*]quinoline (Compound **438**, structure **79A** of **Scheme LIII**. where $R^1$=$R^5$=$R^7$=H, $R^{2-4}$=methyl, $R^6$=trifluoromethyl).

1-*tert*-Butoxycarbonyl-1,2,3,4-tetrahydro-2,2-dimethyl-4-quinolinone (structure **76A** of **Scheme LIII**, where $R^1$=H, $R^{2-3}$=methyl). A solution of aniline (19 mL, 0.20 mol), 3-acetoxy-3-methyl-1-butyne (26 g, 0.20 mol), CuCl (1.0 g, 10 mmol) and $Et_3N$ (28 mL, 0.20 mol) in THF (120 mL ) was heated at reflux for 5 h and was filtered through a pad of Celite™. Removal of solvent and chromatography of the crude mixture (silica gel, EtOAc/hexane, 3/7) afforded 21 g (67%) of 3-methyl-3-phenylamino-1-butyne. Treatment of the aminobutyne with CuCl (0.70 mg, 7.0 mmol) in THF (200 mL ) at 70° C for 16 h followed by chromatography (silica gel, EtOAc/hexane, 3/7) afforded 13 g (60%) of 1,2-dihydro-2,2-dimethylquinoline (structure **75A** of **Scheme LII**, where $R^1$=H, $R^{2-3}$=methyl). Treatment of the quinoline with di-tert-butyl dicarbonate (22 g, 0.10 mol) and DMAP (12 g, 0.10 mol) in THF (100 mL ) for 16 h followed by chromatography (silica gel, EtOAc/hexane, 2/8) afforded 15 g (71%) of 1-*tert*-butoxycarbonyl-1,2-dihydro-2,2-dimethylquinoline. 1-*tert*-Butoxycarbonyl-1,2-dihydro-2,2-dimethylquinoline (3.0 g, 11 mmol) in THF (30 mL ) was treated with 1.0 M $BH_3$-THF in THF (29 mL, 29 mmol) at rt for 3 h and was quenched with 3 M KOH (20 mL). To the above solution 30% $H_2O_2$ (5 mL) was added and the mixture was stirred for 60 min, then 5 mL of water was introduced. The mixture was extracted, washed with brine and concentrated. Chromatography of the crude mixture on a silica gel column using a 10-30% mixture of EtOAc/Hexane as eluents afforded a 2:1 mixture of two isomers (0.87 g, 3.1 mmol), which was oxidized with PCC (2.5 g, 11 mmol) in 60 mL of methylene chloride at rt for 60 min. Removal of solvent and chromatography of the black oil on a silica gel column using a 20% mixture of EtOAc and hexane as solvent afforded 0.58 g (68%) of 1-*tert*-butoxycarbonyl-1,2,3,4-tetrahydro-2,2-dimethyl-4-quinolinone as a white solid. Data for 1-*tert*-butoxycarbonyl-1,2,3,4-tetrahydro-2,2-dimethyl-4-quinolinone: **$^1$H NMR** (400 MHz, $CDCl_3$) 7.93 (d, *J* = 7.8, 1 H), 7.42 (t, *J* = 7.8, 1 H), 7.31 (d, *J* = 7.8, 1 H), 7.02 (t, *J* = 7.8, 1 H), 2.73 (s, 2 H), 1.56 (s, 9 H), 1.49 (s, 6 H).

1,2,3,4-tetrahydro-2,2,3-trimethylquinoline (structure **77A** of **Scheme LII,** where $R^1$=$R^5$=H, $R^{2-4}$=methyl). To a solution of 1-*tert*-butoxycarbonyl-1,2,3,4-tetrahydro-2,2-dimethyl-4-quinolinone (0.10 g, 0.36 mmol) and iodomethane (0.50 mL, 8.0 mmol) in DMF (4 mL) was added NaH (60 % in mineral oil, 20 mg, 0.50 mmol) and the resulting mixture was stirred at rt for 2 h. The reaction was quenched with water (5 mL) and was extracted with EtOAc (2 x 15 mL). Removal of solvent and chromatography of the crude residue on a silica gel column using a 10% mixture of EtOAc and hexane as solvents afforded 90 mg (86%) of 1-*tert*-butoxycarbonyl-1,2,3,4-tetrahydro-2,2,3-trimethyl-4-quinolinone as a colorless oil. The oil (90 mg, 0.32 mmol) was treated with $NaBH_4$ (50 mg, 1.3 mmol) in methanol (5 mL ) for 1 h and the reaction mixture was concentrated. Filtration from the inorganic material through a silica gel pad provided a colorless oil, which was then subjected to hydrogenation over 10 % Pd/C (10 mg) in EtOAc (5 mL ) under a hydrogen balloon for 15 h. Filtration from the catalyst through a Celite™ pad followed by removal of solvent gave 70 mg (82%) of 1-*tert*-butoxycarbonyl-1,2,3,4-tetrahydro-2,2,3-trimethylquinoline as a colorless oil. The crude oil (70 mg, 0.26 mmol) was treated with TFA (0.50 mL, 6.5 mmol) in $CH_2Cl_2$ for 30 min. and was quenched with 5% NaOH (6 mL). The mixture was extracted with EtOAc (2 x 15 mL) and was concentrated. Chromatography on silica gel using a 10% mixture of EtOAc and hexane afforded 1,2,3,4-tetrahydro-2,2,3-trimethylquinoline as a colorless oil (40 mg, 89%). Data for 1,2,3,4-tetrahydro-2,2,3-trimethylquinoline: **$^1$H NMR** (400 MHz, $CDCl_3$) 7.00-6.91 (m, 2 H), 6.60 (t, *J* = 7.3, 1 H), 6.45 (d, *J* = 7.3, 1 H), 3.61 (br s, 1 H), 2.74 (dd, *J* = 16.6, 5.3, 1 H), 2.47 (dd, *J* = 16.6, 10.3, 1 H), 1.82 (m, 1 H), 1.20 (s, 3 H), 1.05 (s, 3 H), 0.97 (d, *J* = 7.2, 3 H).

(*R*/*S*)1,2,3,4-Tetrahydro-2,2,3-trimethyl-6-trifluoromethyl-8-pyridono[5,6-*g*]quinoline (Compound **438**, structure ). The quinoline (20 mg, 0.11 mmol) was converted to Compound **438** according to the nitration-hydrogenation-Knorr procedure described above for Compound **436** (EXAMPLE 336) in a 12% yield as a yellow solid (4 mg). Data for Compound **436**: **$^1$H NMR** (400 MHz, $CDCl_3$) 11.46 (s, 1 H), 7.35 (s, 1 H), 6.66 (s, 1 H), 6.31 (s, 1 H), 4.40 (s, 1 H), 2.83 (dd, *J* = 16.6, 4.8, 1 H), 2.57 (dd, *J* = 16.6, 10.3, 1 H), 1.83 (m, 1 H), 1.25 (s, 3 H), 1.10 (s, 3 H), 0.99 (d, *J* = 6.9, 3 H).

## EXAMPLE 339

(*R*/*S*-2*l*,4*u*)-1,2,3,4-Tetrahydro-2,4-dimethyl-6-trifluoromethyl-8-pyridono[5,6-*g*]quinoline (Compound **439**, structure **62A** of **Scheme XLVIII**, where $R^1$=$R^3$=$R^6$=H, $R^2$=$R^4$=methyl, $R^5$=trifluoromethyl)

1-*tert*-Butoxycarbonyl-1,2,3,4-tetrahydro-2-methyl-4-quinolinone. A mixture of aniline (3.0 g, 32 mmol) and crotonic acid (2.0 g, 23 mmol) in toluene (20 mL ) was heated at reflux for 18 h. Removal of solvent and chromatography (silica gel, EtOAc/hexane, 9/1) of the crude material afforded 2.5 g (61%) of 3-phenylaminobutanoic acid. The acid was treated with PPA (20 mL ) at 110° C for 6 h and the reaction mixture was poured into ice water (50 mL) and then was neutralized with $Na_2CO_3$ to pH 7. Extraction with EtOAc (3 x 60 mL) followed by chromatography (silica gel, EtOAc/hexane, 4/6) afforded 1.0 g (44%) of 1,2,3,4-tetrahydro-2-methyl-4-quinolinone (structure **59A of Scheme XLVIII**, where $R^1$=$R^3$=H, $R^2$=methyl) as a yellow solid. The quinolinone was treated with di-*tert*-butyl dicarbonate (2.2 g, 10 mmol) and DMAP (0.84 g, 6.8 mmol) in THF (15 mL) for 16 h followed by chromatography (silica gel, EtOAc/hexane, 2/8) to afford 1.1 g (68%) of 1-*tert*-butoxycarbonyl-1,2,3,4-tetrahydro-2-methyl-4-quinolinone as a yellow oil. Data for 1-*tert*-butoxycarbonyl-1,2,3,4-tetrahydro-2-methyl-4-quinolinone: **$^1$H NMR** (400 MHz, $CDCl_3$) 7.99 (d, *J* = 7.5, 1 H), 7.78 (d, *J* = 7.5, 1 H), 7.50 (t, *J* = 7.5, 1 H), 7.12 (t, *J* = 7.5, 1 H), 5.10 (m, 1 H), 3.04 (dd, *J* = 17.3, 5.8, 1 H), 2.57 (dd, *J* = 17.3, 1.7, 1 H), 1.56 (s, 9 H), 1.22 (d, *J* = 6.9, 3 H).

(*R*/*S*-2*l*,4*u*)-1,2,3,4-Tetrahydro-2,4-dimethyl-6-trifluoromethyl-8-pyridono[5,6-*g*]quinoline (Compound **439)** To a solution of a 3.0 M ether solution of MeMgBr (1.0 mL, 3.0 mmol) was added 1-*tert*-butoxycarbonyl-1,2,3,4-tetrahydro-2-methyl-4-quinolinone (0.13 mg, 0.50 mmol) in THF (6 mL) and the reaction was allowed to stir at rt for 3 h, then was quenched with water (10 mL). Extraction with EtOAc (2 x 30 mL) followed by chromatography (silica gel, EtOAc/hexane, 3/7) afforded 50 mg (36%) of the adduct ,which was treated with 10% Pd/C (10 mg) and one drop of $H_2SO_4$ in EtOAc (15 mL) under a hydrogen atmosphere for 16 h. Filtration from the catalyst through Celite™ afforded the crude 1-*tert*-butoxycarbonyl-1,2,3,4-tetrahydro-2,4-dimethyl-4-quinoline, which was treated with TFA (0.4 mL) in methylene chloride (1 mL) for 30 min. The reaction was neutralized with 5 % NaOH to pH 10 and was extracted with EtOAc (2 x 20 mL). Chromatography (silica gel, EtOAc/hexane, 1/9) afforded 20 mg (69%) of (*R*/*S*-2*l*-4*u*)-1,2,3,4-tetrahydro-2,4-dimethyl-4-quinoline (structure **60A** of **Scheme LI,** where $R^1$=$R^3$=H, $R^2$=$R^4$=methyl) as a colorless oil. The quinoline was converted to the title compound according to the general nitration-hydrogenation-Knorr procedure described above for Compound **436** (EXAMPLE 336) in 14% three step yield as a yellow solid. Data for Compound **439**: **$^1$H NMR** (400 MHz, $CDCl_3$) 11.75 (s, 1 H), 7.47 (s, 1 H), 6.65 (s, 1 H), 6.33 (s, 1 H), 4.41 (s, 1 H), 3.59 (m, 1 H), 2.92 (m, 1 H), 1.94 (m, 1 H), 1.38 (d, *J* = 6.8, 3 H), 1.24 (m, 1 H), 1.22 (d, *J* = 6.4, 3 H).

## EXAMPLE 340

(*R*/*S*-2*l*,4*u*)-4-Ethyl-1,2,3,4-tetrahydro-2-methyl-6-trifluoromethyl-8-pyranono[5,6-*g*]quinoline (Compound **440**, structure **33A** of **Scheme XL**, where $R^{1-2}$=$R^6$=H, $R^3$=methyl, $R^4$=ethyl, $R^5$=trifluoromethyl).

(*R*/*S*)-1,2,3,4-Tetrahydro-7-methoxy-2-methyl-4-quinoline. This compound was prepared in a manner similar

to that described for 1,2,3,4-tetrahydro-7-methoxy-4-quinolone (EXAMPLE 310) from anisidine and crotonic acid to afford the quinolinone as a brown oil. Data for 1,2,3,4-tetrahydro-7-methoxy-2-methyl-4-quinolinone: **$^1$HNMR** (400 MHz, $CDCl_3$) 7.78 (d, *J* = 8.7, 1 H), 6.33 (dd, *J* = 6.2, 2.2, 1 H), 6.08 (d, *J* = 2.1, 1 H), 4.27 (br s, 1 H), 3.80 (s, 3 H), 2.59 (dd, *J* = 16, 3.7, 2 H), 2.42 (dd, *J* = 13, 12,2 H),

(*R*/*S*)-1-*tert*-Butoxycarbonyl-1,2,3,4-tetrahydro-7-methoxy-2-methyl-4-quinolone (structure **31A** of **Scheme XL,** where $R^{1-2}$=H, $R^3$=methyl). This compound was prepared in a manner similar to that described for 1-*tert*-butoxycarbonyl-1,2,3,4-tetrahydro-7-methoxy-4-quinolone (EXAMPLE 310) from 1,2,3,4-tetrahydro-7-methoxy-2-methyl-4-quinolinone (3.26 mg) to give 961 mg (62%) of the desired quinolone as an off-white solid. Data for 1-*tert*-butoxycarbonyl-1,2,3,4-tetrahydro-7-methoxy-2-methyl-4-quinolone: **$^1$H NMR** (400 MHz, $CDCl_3$) 7.94 (d, *J* = 8.9, 1 H), 7.35 (d, *J* = 2.4, 1 H), 6.67 (dd, *J* = 8.7, 2.4, 1 H), 5.08 (m, 1 H), 3.86 (s, 3 H), 2.99 (dd, *J* = 17, 5.8, 1 H), 2.48 (dd, *J* = 17, 1.7, 1 H), 1.57 (s, 9 H), 1.24 (d, *J* = 6.9, 3 H).

(*R*/*S*)-1-*tert*-Butoxycarbonyl-4-ethyl-1,2,3,4-tetrahydro-7-methoxy-2-methylquinoline (structure **32A** of **Scheme XL,** where $R^{1-2}$=H, $R^3$=methyl, $R^4$=ethyl). This compound was prepared in a manner similar to that described for 1-*tert*-butoxycarbonyl-4-ethyl-1,2,3,4-tetrahydro-7-methoxy-4-quinolone (EXAMPLE 314) from 1-*tert*-butoxycarbonyl-1,2,3,4-tetrahydro-7-methoxy-2-methyl-4-quinolone (100 mg) to give the desired quinoline (34 mg, 30%) as a mixture of diastereomers. Data for 1-*tert*-butoxycarbonyl-4-ethyl-1,2,3,4-tetrahydro-7-methoxy-2-methylquinoline: **$^1$H NMR** (400 MHz, $CDCl_3$) 7.05 (d, *J* = 8.6, 1 H), 6.97 (d, *J* = 2.5, 1 H), 6.66 (dd, *J* = 8.5, 2.5, 1 H), 4.38 (m, 1 H), 3.78 (s, 3 H), 2.39 (m, 1 H), 2.28 (m, 1 H), 2.04 (m, 2 H), 1.55 (m, 1 H), 1.49 (s, 9 H), 1.14 (d, *J* = 6.2, 3 H), 1.08 (t, *J* = 7.4, 3 H).

(*R*/*S*)-4-Ethyl-1,2,3,4-tetrahydro-7-hydroxy-2-methylquinoline. This compound was prepared in a manner similar to that described for 4-ethyl-1,2,3,4-tetrahydro-7-hydroxyquinoline (EXAMPLE 314) from 1-*tert*-butoxycarbonyl-4-ethyl-1,2,3,4-tetrahydro-7-methoxy-2-methylquinoline (34 mg) to give the desired quinoline as a colorless oil, which was used without further purification in the following reaction.

(*R*/*S*-2*l*,4*u*)-4-Ethyl-1,2,3,4-tetrahydro-2-methyl-6-trifluoromethyl-8-pyranono[5,6-*g*]quinoline (Compound **440**). This compound was prepared in a manner similar to that described for Compound **414** (EXAMPLE 314) to give the desired compound as a mix of diastereomers. Recrystallization of the diastereomeric mixture afforded a sample of Compound **440**. Data for Compound **440**: **$^1$H NMR** (400 MHz, $CDCl_3$) 7.38 (s, 1 H), 6.37 (s, 1 H), 6.35 (s, 1 H), 4.43 (br s, 1 H), 3.57 (m, 1 H), 2.79 (m, 1 H), 2.04 (m, 2 H), 1.61 (m, 1 H), 1.28 (d, *J* = 6.4, 3 H), 1.00 (t, *J* = 7.3, 3 H).

## EXAMPLE 341

(*R*/*S*-2*l*,3*u*)-1,2,3,4-Tetrahydro-2,3-dimethyl-6-trifluoromethyl-8-pyridono[5,6-*g*]quinoline (Compound **441**, structure **62A** of **Scheme XLVIII**, where $R^1$=$R^4$=$R^6$=H, $R^{2-3}$=methyl, $R^5$=trifluoromethyl).

To a solution of 1-*tert*-butoxycarbonyl-1,2,3,4-tetrahydro-2-methyl-4-quinolinone (EXAMPLE 339) (0.13 mg, 0.50 mmol) and iodomethane (0.50 mL, 8.0 mmol) in DMF (6 mL ) was added NaH in a 60% mineral oil (40 mg, 1.0 mmol). The reaction mixture was stirred at rt for 16 h and was quenched by water (10 mL ). Extraction of the mixture with EtOAc (2 x 30 mL ) followed by chromatography (silica gel, EtOAc/hexane, 1/9) afforded a mixture of three alkylated products (125 mg, 91%). The above mixture was treated with NaBH4 (38 mg, 1.0 mmol) in methanol (15 mL ) for 1 h and the alcohol intermediates were purified by chromatography (silica gel, EtOAc/hexane, 3/7) to afford a mixture of three alcohols (120 mg, 95%). The mixture of alcohol intermediates (120 mg, 0.43 mmol) was treated with 10% Pd/C (20 mg) and one drop of $H_2SO_4$ in EtOAc (15 mL ) under $H_2$ for 18 h. Filtration through a Celite™ pad provided the reduced products, which were directly treated with TFA (0.5 mL) in methylene chloride (1.0 m ) for 1 h. The reaction was quenched with 5% NaOH, brought to pH 10, and was extracted with EtOAc (2 x 20 mL). Chromatography (silica gel, EtOAc/hexane, 2/8) afforded a mixture of three products (30 mg, 43%), containing (*R*/*S*-21,3*u*)-1,2,3,4-tetrahydro-2,3-dimethylquinoline (structure **60A** of **Scheme LI,** where $R^1$=$R^4$=H, $R^{2-3}$=methyl); (*R*/*S*-2*l*,3*l*)-1,2,3,4-tetrahydro-2,3-dimethylquinoline (structure **60A** of **Scheme LI**, where $R^1$=$R^4$=H, $R^{2-3}$=methyl), and (*R*/*S*)-1,2,3,4-tetrahydro-2,3,3-trimethylquinoline (structure **71A** of **Scheme LII**, where $R^1$=$R^5$=H, $R^{2-4}$=methyl). The mixture of the quinolines (30 mg, 0.18 mmol) was subjected to the nitration-hydrogenation-Knorr procedure described above for Compound **436** (EXAMPLE 336) to afford a mixture of Compound **441**, **442**, and **443**, which was purified by HPLC (10 mm x 25 cm ODC column, 80% MeOH/20% $H_2O$, 3.0 mL /min.). Data for Compound **441**: **$^1$H NMR** (400 MHz, acetone-$d_6$) 10.68 (s, 1 H), 7.25 (s, 1 H), 6.48 (s, 1 H), 6.41 (s, 1 H), 6.09 (s, 1 H), 3.13 (m, 1 H), 2.80 (dd, *J* = 15.9, 4.3, 1 H), 2.53 (dd, *J* = 15.9, 12.0, 1 H), 1.61 (m, 1 H), 1.24 (d, *J* = 6.3, 3 H), 1.04 (d, *J* = 6.5, 3 H).

## EXAMPLE 342

(*R*/*S*-2*l*,3*l*)-1,2,3,4-Tetrahydro-2,3-dimethyl-6-trifluoromethyl-8-pyridono[5,6-*g*]quinoline (Compound **441**, structure **62A** of **Scheme XLVIII**, where $R^1$=$R^4$=$R^6$=H, $R^{2-3}$=methyl, $R^5$=trifluoromethyl)

Compound **442** was obtained along with Compounds **441** and **443** as described above (EXAMPLE 341). Data for Compound 442: **$^1$H NMR** (400 MHz, acetone-$d_6$) 10.80 (s, 1 H), 7.28 (s, 1 H), 6.49 (s, 1 H), 6.48 (s, 1 H), 6.15 (s, 1

H), 3.62 (m, 1 H), 2.91 (m, 1 H), 2.62 (dd, *J* = 16.3, 6.5, 1 H), 2.07 (m, 1 H), 1.15 (d, *J* = 6.5, 3 H), 0.93 (d, *J* = 6.8, 3 H).

## EXAMPLE 343

(*R*/*S*)-1,2,3,4-Tetrahydro-2,3,3-trimethyl-6-trifluoromethyl-8-pyridono[5,6-*g*]quinoline (Compound **443**, structure **73A** of **Scheme LII**, where $R^1$=$R^5$=$R^7$=H, $R^{2-4}$=methyl, $R^6$=trifluoromethyl).
Compound **443** was obtained along with Compounds **441** and **442** as described above (EXAMPLE 341). Data for Compound **443**: **$^1$H NMR** (400 MHz, acetone-$d_6$) 10.58 (s, 1 H), 7.23 (s, 1 H), 6.50 (s, 1 H), 6.41 (s, 1 H), 6.08 (s, 1 H), 3.28 (m, 1 H), 2.65 (d, *J* = 15.8, 1 H), 2.53 (d, *J* = 15.8, 1 H), 1.15 (d, *J* = 6.6, 3 H), 1.03 (s, 3 H), 0.84 (s, 3 H).

## EXAMPLE 344

(*R*/*S*)-1,2,3,4-Tetrahydro-2-methyl-6-trifluoromethyl-8-pyridono[5,6-*g*]quinoline (Compound **444,** structure **53A** of **Scheme XLVI,** where $R^{1-2}$=$R^5$=H, $R^3$=methyl, $R^4$=trifluoromethyl)
1,2,3,4-tetrahydro-2-methylquinoline (0.15 g, 1.0 mmol) was converted to Compound **444** according to the nitration-hydrogenation-Knorr procedure described for Compound **436** (EXAMPLE 336) to afford 35 mg (13%) of Compound **444** as a yellow solid. Data for Compound **444**: **$^1$H NMR** (400 MHz, acetone-$d_6$) 10.83 (s, 1 H), 7.29 (s, 1 H), 6.55 (s, 1 H), 6.50 (s, 1 H), 6.17 (s, 1 H), 3.57 (m, 1 H), 2.91-2.82 (m, 2 H), 2.03 (m, 1 H), 1.54 (m, 1 H), 1.25 (d, *J* = 6.4, 3 H).

## EXAMPLE 345

(*R*/*S*)-4-Ethyl-1,2,3,4-tetrahydro-6-trifluoromethyl-8-pyridono[5,6-*g*]quinoline (Compound **445**, structure **62A** of **Scheme XLVIII**, where $R^{1-3}$=$R^6$=H, $R^4$=ethyl, $R^5$=trifluoromethyl) (*R*/*S*)-1-*tert*-Butyloxycarbonyl-4-ethyl-1,2,3,4-tetrahydro-4-hydroxyquinoline. To a flame-dried 25-mL rb flask containing ethylmagnesium bromide (4.0 mL of a 3.0 M solution in $Et_2O$, 12.0 mmol, 3.0 equiv), at -10° C was added dropwise a solution of 1-*tert*-butyloxycarbonyl-1,2,3,4-tetrahydro-4-quinolone (1.0 g, 4.0 mmol) in $Et_2O$ (4 mL). The reaction mixture was stirred at -10° C for 15 min, then allowed to warm to rt over 10 min. A 1.0 M solution of $NaHSO_4$ (10 mL) was then rapidly added. The resulting biphasic mixture was extracted with EtOAc (3 x 10 mL), and the combined organic extracts were dried ($Na_2SO_4$) and concentrated under reduced pressure. The residue was purified by flash chromatography (silica gel, hexanes / EtOAc, 4:1), affording 800 mg (71%) of the desired product as a clear yellow oil ($R_f$ 0.14, hexanes / EtOAc, 4:1). Data for 1-tert-butoxycarbonyl-4-ethyl-1,2,3,4-tetrahydro-4-hydroxyquinoline: **$^1$H NMR** (400 MHz, $CDCl_3$) 7.68 (d, 1H, *J* = 8.4, 8-H), 7.47 (dd, 1H, *J* = 7.9, 1.7, 5-H), 7.21 (ddd, 1H, *J* = 7.4, 7.4, 1.6, 6-H), 7.09 (ddd, 1H, *J* = 7.8, 7.8, 1.1, 7-H), 4.03 (ddd, 1H, *J* = 12.9, 7.1, 4.7, 2-H), 3.47 (ddd, 1H, *J* = 13.1, 8.6, 4.3, 2-H), 2.11 (ddd, 1H, *J* = 13.5, 8.6, 4.8, 3-H), 1.86 (m, 3H, 3-H, $CH_2CH_3$), 1.52 [s, 9H, $C(CH_3)_3$], 0.89 (t, 3H, *J* = 7.5, $CH_3$).

(*R*/*S*)-4-Ethyl-1,2,3,4-tetrahydroquinoline (structure **60A** of **Scheme XLVIII**, where $R^{1-3}$ =H, $R^4$=ethyl). To a flame-dried 100-mL rb flask containing 1-*tert*-butyloxycarbonyl-4-ethyl-1,2,3,4-tetrahydro-4-hydroxyquinoline (800 mg, 2.88 mmol ) in a 1:1 solution of EtOAc / EtOH (20 mL) at rt was added 10% Pd/C (approx. 1 mol %). After evacuation and flushing of the vessel three times with nitrogen, one drop of trifluoroacetic acid was added, the vessel evacuated once more, and the mixture stirred under an atmosphere of hydrogen for 16 h. The reaction mixture was then filtered, and concentrated under reduced pressure. The residue was transferred to a 25-mL rb flask with $CH_2Cl_2$ (3 mL) and stirred at rt. TFA (1.2 mL) was added and the reaction was vented and stirred for 2 h at rt. A solution of sat'd. $NaHCO_3$ (adjusted to pH 9 with 3.0 M NaOH) was added until the aqueous phase was approximately pH 9. The resulting aqueous phase was extracted with $CH_2Cl_2$ (3 x 10 mL), and the combined organic extracts were dried ($Na_2SO_4$), and concentrated under reduced pressure to yield 351 mg (71%) of a colorless oil, which turned blue on exposure to air ($R_f$ 0.40, hexanes / EtOAc, 2:1). Data for (*R*/*S*)-4-ethyl-1,2,3,4-tetrahydroquinoline: **$^1$H NMR** (400 MHz, $CDCl_3$) 7.02 (d, 1H, *J* = 7.6, 8-H), 6.96 (ddd, 1H, *J* = 7.7, 7.7, 1.3, 7-H), 6.61 (ddd, 1H, *J* = 8.2, 8.2, 1.0, 6-H), 6.47 (d, 1H, *J* = 7.9, 5-H), 3.83 (br s, 1H, $CH_2NH$ ), 3.31 (ddd, 1H, *J* = 11.3, 11.3, 3.6, 2-H), 3.25 (ddd, 1H, *J* = 9.7, 9.7, 4.8, 2-H), 2.65 (dddd, 1H, *J* = 10.1, 5.1, 5.1, 5.1, 4-H), 1.92 (dddd, 1H, *J* = 9.6, 4.7, 4.7, 4.7, 3- H), 1.82 (m, 1H, 3-H), 1.74 (m, 1H, $CH_2CH_3$), 0.98 (t, 3H, *J* = 7.4, $CH_3$).

(*R*/*S*)-7-Amino-4-ethyl-1,2,3,4-tetrahydroquinoline (structure **61A** of **Scheme XLVIII,** where $R^{1-3}$=H, $R^4$=ethyl). A 25-mL rb flask containing (*R*/*S*)-4-ethyl-1,2,3,4-tetrahydroquinoline (340 mg, 2.1 mmol) was cooled to -10° C, and conc. $H_2SO_4$ (5 mL) was added slowly. The resulting solution was warmed to rt to effect complete dissolution of the quinoline, then cooled again to -10° C and stirred vigorously. Fuming $HNO_3$ (85 µL) was added dropwise, slowly, and the reaction mixture turned dark red. After 10 min, the reaction mixture was poured onto cracked ice and diluted with water (5 mL). Sat'd $NaHCO_3$ (80 mL) was added, and the pH was adjusted to pH 9 with 3.0 M NaOH. This aqueous phase was extracted with EtOAc (3 x 75 mL), and the combined extracts were dried ($Na_2SO_4$),and concentrated under reduced pressure to yield a dark red oil. This crude material was placed into a 250-mL rb flask with 1:1 EtOAc / EtOH (40 mL) and 10% Pd on C (approx. 1 mol %). The vessel was evacuated and flushed with nitrogen three times, the

stirred under an atmosphere of hydrogen for 16 h, filtered, and concentrated under reduced pressure to yield a yellow oil, which was purified by flash chromatography (silica gel, $CH_2Cl_2$ / methanol, 9:1), affording 210 mg (57 %) of the desired product as a dark yellow oil ($R_f$ 0.50, $CH_2Cl_2$ / MeOH, 9:1). Data for (*R*/*S*)-7-amino-4-ethyl-1,2,3,4-tetrahydroquinoline: **$^1$H NMR** (400 MHz, $CDCl_3$) 6.81 (d, 1H, *J* = 8.1, 5-H), 6.02 (dd, 1H, *J* = 8.0, 2.2, 6-H), 5.84 (d, 1H, *J* = 2.3, 8-H), 3.48 (s, 2H, N$H_2$), 3.27 (ddd, 1H, *J* = 11.1, 11.1, 3.5, 2-H), 3.20 (ddd, 1H, *J* = 9.8, 5.3, 4.5, 2-H), 2.55 (dddd, 1H, *J* = 10.2, 5.2, 5.2, 5.2, 4H), 1.90 (dddd, 1H, *J* = 9.6, 9.6, 9.6, 4.7, 3-H), 1.72 (m, 2H, 3-H, C$H_2$C$H_3$), 1.4 (m, 1H, C$H_2$C$H_3$), 0.96 (t, 3H, *J*= 7.4, C$H_3$).

(*R*/*S*)-4-Ethyl-1,2,3,4-tetrahydro-6-trifluoromethyl-8-pyridono[5,6-*g*]quinoline (Compound 445). To a flame-dried 100-mL rb flask containing 7-amino-4-ethyl-1,2,3,4-tetrahydroquinoline (210 mg, 1.19 mmol), in ethanol (20 mL), at rt, was added ethyl-4,4,4-trifluoroacetoacetate (190 µL, 1.31 mmol, 1.1 equiv) followed by $ZnCl_2$ (244 mg, 1.79 mmol, 1.5 equiv). The reaction mixture was heated to reflux for 6 h, at which point all starting material had been consumed (by TLC analysis). The reaction mixture was cooled t rt, and the solvent removed under reduced pressure. Dichloromethane (20 mL) was added and the organic phase washed with sat'd $NaHCO_3$ (2 x 10 mL) and brine (1 x 10 mL), then dried ($Na_2SO_4$), and concentrated under reduced presure. This crude product was purified by flash chromatography (silica gel, $CH_2Cl_2$ / MeOH, 15:1), affording 24.4 mg (7%) of the desired product as a yellow solid. Data for Compound **445**: $R_f$ 0.37, ($CH_2Cl_2$ / MeOH, 9:1); **$^1$H NMR** (400 MHz, $CD_3OD$) 7.31 (s, 1H, 5-H), 6.47 (s, 1H, 7-H), 6.37 (s, 1H, 10-H), 3.34 (m, 2H, 2-H), 2.70 (m, 1H, 4-H), 1.88 (m, 2H, 3-H), 1.62 (m, 2H, C$H_2$C$H_3$), 1.00 (t, 3H, *J* = 7.5, C$H_3$).

## EXAMPLE 346

(*R*/*S-2l*, 3*u*)-1,2,3,4-Tetrahydro-2,3,9-trimethyl-6-trifluoromethyl-8-pyridono[5,6-*g*]quinoline (Compound **446,** structure **81A** of **Scheme LIV**, where $R^1$=$R^4$=$R^6$=$R^8$=H, $R^{2\ 3}$=methyl, $R^5$=trifluoromethyl)
To a solution of Compound **441** (3.5 mg, 0.012 mmol) and iodomethane (0.10 mL , 1.6 mmol) in THF (2.0 mL ) was added NaH as a 60% in mineral oil (10 mg, 0.25 mmol) and the reaction was stirred at rt for 1 h, then, was quenched by water (10 mL ). Extraction with EtOAc (2 x 15 mL ) and chromatography (silica gel, EtOAc/hexane, 1/1) afforded 3.0 mg (81 %) of Compound **446** as a yellowish solid. Data for Compound **446: $^1$H NMR** (400 MHz, $CDCl_3$) 7.36 (s, 1 H), 6.72 (s, 1 H), 6.32 (s, 1 H), 4.40 (s, 1 H), 3.61 (s, 3 H), 3.14 (m, 1 H), 2.83 (dd, *J* = 16.0, 4.4, 1 H), 2.54 (dd, *J* = 16.0, 11.0, 1 H), 1.63 (m, 1 H), 1.26 (d, *J* = 6.3 , 3 H), 1.06 (d, *J* = 6.6, 3 H).

## EXAMPLE 347

(*R*/*S*)-1,2,3,4-Tetrahydro-4-propyl-6-trifluoromethyl-8-pyridono[5,6-g]quinoline (Compound **447,** structure **62A** of **Scheme XLVIII,** where $R^{1\text{-}3}$=$R^6$=H, $R^4$=*n*-propyl, $R^5$=trifluoromethyl)
1-*tert*-Butyloxycarbonyl-1,2,3,4-tetrahydro-4-hydroxy-4-propylquinoline. This compound was prepared from 1-*tert*-butyloxycarbamoyl-1,2,3,4-tetrahydro-4-quinolone (1.00 g, 4.00 mmol) in the manner previously described for 1-*tert*-butyloxycarbamoyl-4-ethyl-1,2,3,4-tetrahydro-4-hydroxyquinoline (EXAMPLE 345), affording 567 mg (48%) of the tertiary alcohol as a yellow oil (Rf 0.22, hexanes / EtOAc, 4:1). Data for 1-*tert*-butyloxycarbonyl-1,2,3,4-tetrahydro-4-hydroxy-4-propylquinoline: **$^1$H NMR** (400 MHz, $CDCl_3$) 7.67 (d, 1H, *J* = 8.2, 8-H), 7.48 (dd, 1H, *J* = 7.9, 1.7, 5-H), 7.20 (ddd, 1H, *J* = 8.6, 8.6, 1.4, 6-H), 7.08 (ddd, 1H, *J* = 7.6, 7.6, 1.1, 7-H), 4.03 (ddd, 1H, *J* = 12.8, 7.1, 4.8, 2-H), 3.46 (ddd, 1H, *J* = 13.0, 8.5, 4.4, 2-H), 2.11 (ddd, 1H, *J* = 13.5, 8.5, 4.8, 3-H), 1.89 (ddd, 1H, *J* = 13.6, 7.2, 4.4, 3-H), 1.78 (m, 2H, C$H_2$$C_2H_5$), 1.52 [s, 9H, C(C$H_3$)$_3$], 1.32 (m, 2H, $CH_2$C$H_2$$CH_3$), 0.90 (t, 3H, *J* = 7.3, $CH_2$C$H_3$).

(*R*/*S*)-1,2,3,4-Tetrahydro-4-propylquinoline (structure **60A** of **Scheme XLVIII,** where $R^{1\text{-}3}$ =H, $R^4$=*n*-propyl). This compound was prepared from 1-*tert*-butyloxycarbonyl-1,2,3,4-tetrahydro-4-hydroxy-4-propylquinoline (550 mg, 1.89 mmol) in the manner previously described for 4-ethyl-1,2,3,4-tetrahydroquinoline (EXAMPLE 345), affording 229 mg (66%) of the desired tetrahydroquinoline as a yellow oil (Rf 0.10, hexanes / EtOAc, 2:1). Data for (*R*/*S*)-1,2,3,4-tetrahydro-4-propylquinoline: **$^1$H NMR** (400 MHz, $CDCl_3$) 7.07 (d, 1H, *J* = 7.6, 5-H), 7.02 (ddd, 1H, *J* = 7.9, 7.9, 1.1, 7-H), 6.77 (dd, 1H, *J* = 7.5, 7.4, 6-H), 6.67 (d, 1H, *J* = 7.9, 8-H), 6.25 (br s, 1H, N*H*), 3.37 (ddd, 1H, *J* = 11.5, 11.5, 3.5, 2-H), 3.30 (m, 1H, 2-H), 2.78 (dddd, 1H, *J* = 10.0, 5.0, 5.0, 5.0, 4-H), 1.99 and 1.84 (2 x m, 2 x 1H, 3-H), 1.68 (m, 1H, C$H_2$$CH_2$$CH_3$), 1.47 (m, 3H, C$H_2$C$H_2$$CH_3$), 0.95 (t, 3H, *J* = 7.3, $CH_3$).

(*R*/*S*)-7-Amino-1,2,3,4-tetrahydro-4-propylquinoline (structure **61A** of **Scheme XLVIII,** where $R^{1\text{-}3}$=H, $R^4$=*n*-propyl). This compound was prepared from (*R*/*S*)-1,2,3,4-tetrahydro-4-propylquinoline (220 mg, 0.78 mmol) in the manner previously described for 7-amino-4-ethyl-1,2,3,4-tetrahydroquinoline (EXAMPLE 345), affording 114 mg (77%) of the product as a colorless oil (Rf 0.10, hexanes / EtOAc, 2:1). Data for (*R*/*S*)-7-amino-1,2,3,4-tetrahydro-4-propylquinoline: **$^1$H NMR** (400 MHz, $CDCl_3$) 6.80 (d, 1H, *J* = 8.0, 5-H), 6.01 (dd, 1H, *J* = 8.0, 2.3, 6-H), 5.83 (d, 1H, *J* = 2.2, 8-H), 3.74 (br s, 1H, N*H*), 3.41 (br s, 2H, N$H_2$), 3.28 (ddd, 1 H, *J* = 11.0, 11.0, 3.3, 2-H), 3.19 (ddd, 1H, *J* = 9.7, 4.7, 4.7, 2-H), 2.65 (dddd, 1H, *J* = 5.1, 5.1, 5.1, 5.1, 4-H), 1.89 (dddd, 1H, *J* = 9.7, 9.7, 9.7, 4.5, 3-H), 1.73 (dddd, 1H, *J* = 8.6, 8.6, 4.8, 4.8, 3-H), 1.61 (m, 1H, C$H_2$$CH_2$$CH_3$), 1.40 (m, 3H, C$H_2$C$H_2$$CH_3$), 0.93 (t, 3H, *J* = 7.0, C$H_3$).

(*R*/*S*)-1,2,3,4-Tetrahydro-4-propyl-6-trifluoromethyl-8-pyridono[5,6-*g*]quinoline (Compound **447**) This compound was prepared from 7-amino-1,2,3,4-tetrahydro-4-propylquinoline (110 mg, 0.58 mmol) in the manner previously described for Compound **445** (EXAMPLE 345), affording 8.9 mg (5%) of the desired product as a yellow powder (Rf 0.44, $CH_2Cl_2$ / MeOH, 9:1). **$^1$H NMR** (400 MHz, $CDCl_3$) 7.34 (s, 1H, 5-H), 6.65 (s, 1H, 7-H), 6.40 (s, 1H, 10-H), 4.65 [br s, 1H, $(CH_3)_2CNH$)], 3.42 (ddd, 1H, *J* = 11.2, 11.2, 4.0, 2-H), 3.34 (ddd, 1H, *J* = 7.9, 3.8, 3.8, 2-H), 2.82 (m, 1H, 4-H), 1.88 (m, 2H, 3-H), 1.52 (m, 4H, $CH_2CH_2CH_3$), 0.96 (t, 3H, *J* = 7.1, $CH_3$).

## EXAMPLE 348

(*R*/*S*)-3-Ethyl-1,2,3,4-tetrahydro-2,2-dimethyl-6-trifluoromethyl-8-pyridono[5,6-*g*]quinoline (Compound **448,** structure **79A** of **Scheme LIII,** where $R^1$=$R^5$=$R^7$=H, $R^{2-3}$=methyl, $R^4$=ethyl, $R^6$=trifluoromethyl)

(*R*/*S*)-3-Ethyl-1,2,3,4-tetrahydro-2,2-dimethylquinoline (structure **77A** of **Scheme LIII**, where $R^1$=$R^5$=H, $R^{2-3}$=methyl, $R^4$=ethyl). To a solution of 1-*tert*-butoxycarbonyl-1,2,3,4-tetrahydro-2,2-dimethyl-4-quinolinone (EXAMPLE 325) (0.10 g, 0.36 mmol) and iodoethane (0.50 mL , 6.3 mmol) in DMF (5 mL ) was added NaH (60 % in mineral oil, 40 mg, 1.0 mmol) and the resulting mixture was stirred at rt for 15 h. The reaction was quenched with water (5 mL ) and was extracted with EtOAc (2 x 15 mL ). Removal of solvent and chromatography of the crude residue on a silica gel column using a 10% mixture of EtOAc and hexane as solvents afforded a mixture of products, which was treated with TFA (0.50 mL ) in methylene chloride (1.0 mL ) for 3 h. The reaction was neutralized to pH 10 by 5 % NaOH and was extracted with EtOAc (2 x 20 mL ). Chromatography (silica gel, EtOAc/hexane, 3/7) afforded 30 mg (41%) of (*R*/*S*)-3-ethyl-1,2,3,4-tetrahydro-2,2-dimethyl-4-quinolinone (30 mg, 0.15 mmol) as a colorless oil. The quinolinone (30 mg, 0.15 mmol) was treated with $Et_3SiH$ (1.0 mL ) and $BF_3$-$OEt_2$ (0.05 mL, 0.4 mmol) in $CH_2Cl_2$ (1.0 mL) at 100° C for 15 h in a sealed tube. Purification of the crude product by chromatography (silica gel, EtOAc/hexane, 1/9) afforded 20 mg (71%) of (*R*/*S*)-3-ethyl-1,2,3,4-tetrahydro-2,2-dimethylquinoline. Data for (*R*/*S*)-3-ethyl-1,2,3,4-tetrahydro-2,2-dimethylquinoline: **$^1$H NMR** (400 MHz, $CDCl_3$) 6.98 (d, *J* = 7.5, 1 H), 6.96 (t, *J* = 7.5, 1 H), 6.61 (t, *J* = 7.5, 1 H), 6.44 (d, *J* = 7.5, 1 H), 3.60 (s, 1 H), 2.90 (dd, *J* = 16.7, 5.2, 1 H), 2.41 (dd, *J* = 16.7, 10.7, 1 H), 1.68 (m, 1 H), 1.52 (m, 1 H), 1.23 (m, 1 H), 1.22 (s, 3 H), 1.05 (s, 3 H).

(*R*/*S*)-3-Ethyl-1,2,3,4-tetrahydro-2,2-dimethyl-6-trifluoromethyl-8-pyridono[5,6-*g*]quinoline (Compound **448**). The quinoline prepared above (20 mg) was converted to Compound **448** according to the nitration-hydrogenation-Knorr procedure described for Compound **436** (EXAMPLE 336) to afford 2.0 mg (13%) of Compound **448** as a yellow solid. Data for Compound **448: $^1$H NMR** (400 MHz, acetone-$d_6$) 10.65 (s, 1 H), 7.31 (s, 1 H), 6.47 (s, 1 H), 6.41 (s, 1 H), 6.06 (s, 1 H), 3.01 (dd, *J* = 16.6, 4.8, 1 H), 2.53 (dd, *J* = 16.6, 11.0, 1 H), 1.72 (m, 1 H), 1.53 (m, 1 H), 1.30 (s, 1 H), 1.12 (s, 3 H), 1.10-1.00 (m, 4 H).

## EXAMPLE 349

(*R*/*S*)-1,2,3,4-Tetrahydro-2,2-dimethyl-6-trifluoromethyl-3-propyl-8-pyridono[5,6-*g*]quinoline (Compound **449**, structure **79A** of **Scheme LIII**, where $R^1$=$R^5$=$R^7$=H, $R^{2-3}$ =methyl, $R^4$=*n*-propyl, $R^6$=trifluoromethyl))

(*R*/*S*)-1,2,3,4-Tetrahydro-2.2-dimethyl-4-propylquinoline (structure **77A** of **Scheme LIII**, where $R^1$=$R^5$=H, $R^{2-3}$=methyl, $R^4$=*n*-propyl). This compound was prepared in a manner similar to that described for (*R*/*S*)-3-ethyl-1,2,3,4-tetrahydro-2,2-dimethylquinoline (EXAMPLE 348) but using iodopropane in place of iodoethane. (*R*/*S*)-1,2,3,4-Tetrahydro-2,2-dimethyl-4-propylquinoline was obtained in 16% overall yield as a colorless oil. Data for (*R*/*S*)-1,2,3,4-tetrahydro-2,2-dimethyl-4-propylquinoline: **$^1$H NMR** (400 MHz, $CDCl_3$) 6.98 (d, *J* = 7.4, 1 H), 6.96 (t, *J* = 7.4, 1 H), 6.61 (t, *J* = 7.4, 1 H), 6.45 (d, *J* = 7.4, 1 H), 3.60 (brs, 1 H), 2.87 (dd, *J* = 16.6, 5.2, 1 H), 2.42 (dd, *J* = 16.6, 10.7, 1 H), 1.66-1.49 (m, 3 H), 1.40-1.25 (m, 2 H), 1.21 (s, 3 H), 1.05 (s, 3 H), 0.92 (t, *J* = 7.1 , 3 H).

(*R*/*S*)-1,2,3,4-Tetrahydro-2,2-dimethyl-6-trifluoromethyl-3-propyl-8-pyridono[5,6-*g*]quinoline (Compound **449**). Compound **449** was prepared in manner similar to that described for Compound **448** (EXAMPLE 348), to afford Compound **449** in a 32% overall yield. Data for Compound **449:** $^1$H NMR (400 MHz, $CDCl_3$) 11.00 (s, 1 H), 7.32 (s, 1 H), 6.61 (s, 1 H), 6.42 (s, 1 H), 4.60 (brs, 1 H), 2.90 (dd, *J* = 16.6, 4.4, 1 H), 2.45 (dd, *J* = 16.6, 11.3, 1 H), 1.70-1.42 (m, 3 H), 1.36-1.24 (m, 2 H), 1.18 (s, 3 H), 1.02 (s, 3 H), 0.93 (t, *J*= 6.7, 3 H).

## EXAMPLE 350

1-Methyl-5-trifluoromethyl-7-pyridono[5,6-*f*]indoline (Compound **450**, structure **83A** of **Scheme LV,** where $R^{1-3}$=$R^5$=H, $R^4$=trifluoromethyl, $R^6$=methyl).

Compound **419** (10 mg, 0.0393 mmol) and paraformaldehyde (11 mg, 0.0393 mmol) were dissolved in glacial acetic acid (2.5 mL) and stirred for 10 min at rt. $NaBH_3CN$ (13 mg, 0.197 mmol) was added in one portion and allowed to stir at rt for 15 h. The reaction mixture was poured over ice and made basic with 10% NaOH. The aqueous layer was extracted with EtOAc (3 x 50 mL), dried ($Na_2SO_4$), filtered, and concentrated. The crude material was dissolved in 5% MeOH/$CHCl_3$ (0.5 mL) and loaded onto a 1000 μm reverse phase TLC plate (Whatman PLKC18F Silica Gel 150 Å).

The plate was eluted with 80% $MeOH/H_2O$ to afford 5.8 mg (55 %) of Compound **450** as a light yellow solid. Data for Compound **450:** $^1$**H NMR** (400 MHz, acetone-$d_6$) 7.29 (d, *J* = 1.6, 1 H), 6.54 (s, 1 H), 6.10 (s, 1 H), 3.50 (t, *J* = 8.1, 2 H), 3.01 (t, *J* = 8.0, 2 H), 2.83 (s, 3 H).

**Steroid Receptor Activity**

Utilizing the "cis-trans" or "co-transfection" assay described by Evans et al., Science, 240:889-95 (May 13, 1988), the disclosure of which is herein incorporated by reference, the compounds of the present invention were tested and found to have strong, specific activity as both agonists, partial agonists and antagonists of PR, AR, ER, GR and MR. This assay is described in further detail in U.S. Patent Nos. 4,981,784 and 5,071,773, the disclosures of which are incorporated herein by reference.

The co-transfection assay provides a method for identifying functional agonists and partial agonists which mimic, or antagonists which inhibit, the effect of native hormones, and quantifying their activity for responsive IR proteins. In this regard, the co-transfection assay mimics an *in vivo* system in the laboratory. Importantly, activity in the co-transfection assay correlates very well with known *in vivo* activity, such that the co-transfection assay functions as a qualitative and quantitative predictor of a tested compounds *in vivo* pharmacology. See, e.g., T. Berger et al. 41 *J.* Steroid Biochem. Molec. Biol. 773 (1992), the disclosure of which is herein incorporated by reference.

In the co-transfection assay, a cloned cDNA for an IR (e.g., human PR, AR or GR) under the control of a constitutive promoter (e.g., the SV 40 promoter) is introduced by transfection (a procedure to induce cells to take up foreign genes) into a background cell substantially devoid of endogenous IRs. This introduced gene directs the recipient cells to make the IR protein of interest. A second gene is also introduced (co-transfected) into the same cells in conjunction with the IR gene. This second gene, comprising the cDNA for a reporter protein, such as firefly luciferase (LUC), controlled by an appropriate hormone responsive promoter containing a hormone response element (HRE). This reporter plasmid functions as a reporter for the transcription-modulating activity of the target IR. Thus, the reporter acts as a surrogate for the products (mRNA then protein) normally expressed by a gene under control of the target receptor and its native hormone.

The co-transfection assay can detect small molecule agonists or antagonists of target IRs. Exposing the transfected cells to an agonist ligand compound increases reporter activity in the transfected cells. This activity can be conveniently measured, e.g., by increasing luciferase production, which reflects compound-dependent, IR-mediated increases in reporter transcription. To detect antagonists, the co-transfection assay is carried out in the presence of a constant concentration of an agonist to the target IR (e.g., progesterone for PR) known to induce a defined reporter signal. Increasing concentrations of a suspected antagonist will decrease the reporter signal (e.g., luciferase production). The co-transfection assay is therefore useful to detect both agonists and antagonists of specific IRs. Furthermore, it determines not only whether a compound interacts with a particular IR, but whether this interaction mimics (agonizes) or blocks (antagonizes) the effects of the native regulatory molecules on target gene expression, as well as the specificity and strength of this interaction.

The activity of selected steroid receptor modulator compounds of the present invention were evaluated utilizing the co-transfection assay, and in standard IR binding assays, according to the following illustrative Examples.

**EXAMPLE 358**

**Co-transfection assay**

CV-1 cells (African green monkey kidney fribroblasts) were cultured in the presence of Dulbecco's Modified Eagle Medium (DMEM) supplemented with 10% charcoal resin-stripped fetal bovine serum then transferred to 96-well microtiter plates one day prior to transfection.

To determine PR agonist and antagonist activity of the compounds of the present invention, the CV-1 cells were transiently transfected by calcium phosphate coprecipitation according to the procedure of Berger et al., 41 *J. Steroid Biochem. Mol. Biol.*, 733 (1992) with the following plasmids: pSVhPR-B (5 ng/well), MTV-LUC reporter (100 ng/well), pRS-β-Gal (50 ng/well) and filler DNA (pGEM; 45 ng/well). The receptor plasmid, pSVhPR-B, contains the human PR-B under constitutive control of the SV-40 promoter, and is more fully described in E. Vegeto et al., "The mechanism of RU 486 antagonism is dependent on the conformation of the carboxy-terminal tail of the human progesterone receptor", 69 *Cell,* 703 (1992), the disclosure of which is herein incorporated by reference. Similarly, the AR, ER, GR and MR agonist and antagonist activity of the compounds of the present invention were determined according to the same procedure described herein, except that the plasmids pRShAR, pRShER, pRShGR and pRShMR were substituted for the plasmid pSVhPR-B described above. Each of these plasmids are more fully described in J.A. Simental et al., "Transcriptional activation and nuclear targeting signals of the human androgen receptor", 266 *J. Biol. Chem.*, 510 (1991) (pRShAR), M.T. Tzukerman et al., "Human estrogen receptor transactivational capacity is determined by both cellular and promoter context and mediated by two functionally distinct intramolecular regions", 8 *Mol. Endocrinol.,* 21 (1994) (pRShER), V. Giguere et al., "Functional domains of the human glucocorticoid receptor", 46 *Cell,* 645 (1986) (pRShGR), and J.L. Arriza et al., "Cloning of human mineralocorticoid receptor complementary DNA: structural and functional kinship with glucocorticoid receptor", 237 *Science,* 268 (1987) (pRShMR), the disclosures of which are herein incorporated by reference.

The reporter plasmid, MTV-LUC, contains the cDNA for firefly luciferase (LUC) under control of the mouse mammary tumor virus (MTV) long terminal repeat, a conditional promoter containing a progesterone response element. This plasmid is more fully described in Berger et al. supra. In addition, for ER agonist and antagonist determinations, the reporter plasmid MTV-ERE5-LUC, which contains LUC under control of the mouse mammary tumor virus (MTV) long terminal repeat in which the glucocorticoid response elements have been deleted and replaced with five copies of a 33-base pair ERE as described in Tzukerman et al., supra, was substituted for the MTV-LUC plasmid described herein. pRS-β-Gal, coding for constitutive expression of E. coli β-galactosidase (β-Gal), was included as an internal control for evaluation of transfection efficiency and compound toxicity.

Six hours after transfection, media was removed and the cells were washed with phosphate-buffered saline (PBS). Media containing reference compounds (i.e. progesterone as a PR agonist, mifepristone ((11beta, 17beta)-11-[4-(dimethylamino)phenyl]-17-hydroxy-17-(1-propynyl)estra-4,9-dien-3-one: RU486; Roussel Uclaf) as a PR antagonist; dihydrotestosterone (DHT; Sigma Chemical) as an AR agonist and 2-OH-flutamide (the active metabolite of 2-methyl-N-[4-nitro-3-(trifluoromethyl)phenyl]pronanamide; Schering-Plough) as an AR antagonist; estradiol (Sigma) as an ER agonist and ICI 164,384 (N-butyl-3,17-dihydroxy-N-methyl-(7-alpha,17-beta)-estra-1,3,5(10)-triene-7-undecanamide; ICI Americas) as an ER antagonist; dexamethasone (Sigma) as a GR agonist and RU486 as a GR antagonist; and aldosterone (Sigma) as a MR agonist and spirolactone ((7-alpha-[acetylthio]-17-alpha-hydroxy-3-oxopregn-4-ene-21-carboxylic acid gamma-lactone; Sigma) as an MR antagonist) and/or the modulator compounds of the present invention in concentrations ranging from $10^{-12}$ to $10^{-5}$ M were added to the cells. Three to four replicates were used for each sample. Transfections and subsequivuent procedures were performed on a Biomek 1000 automated laboratory work station.

After 40 hours, the cells were washed with PBS, lysed with a Triton X-100-based buffer and assayed for LUC and β-Gal activities using a luminometer or spectrophotometer, respectively. For each replicate, the normalized response (NR) was calculated as:

$$\text{LUC response}/\beta\text{-Gal rate}$$

where β-Gal rate = β-Gal•1x$10^{-5}$/β-Gal incubation time.

The mean and standard error of the mean (SEM) of the NR were calculated. Data was plotted as the response of the compound compared to the reference compounds over the range of the dose-response curve. For agonist experiments, the effective concentration that produced 50% of the maximum response ($EC_{50}$) was quantified. Agonist efficacy was a function (%) of LUC expression relative to the maximum LUC production by the reference agonist for PR, AR, ER, GR or MR. Antagonist activity was determined by testing the amount of LUC expression in the presence of a fixed amount of progesterone as a PR agonist, DHT as an AR agonist, estradiol as an ER agonist, dexamethasone as a GR agonist, or aldosterone as an MR agonist at the $EC_{50}$ concentration. The concentration of test compound that inhibited 50% of LUC expression induced by the reference agonist was quantified ($IC_{50}$). In addition, the efficacy of antagonists was determined as a function (%) of maximal inhibition.

## IR Binding assay

**PR and GR Binding:** In addition, the binding of the compounds of the present invention to the steroid receptors was also investigated according to the following methodology for PR and GR. PR and GR proteins were prepared from Baculovirus extracts by incorporating the appropriate cDNAs for human progesterone receptor A form (PR-A; P. Kastner et al., 9 *EMBO*, 1603 (1990), the disclosure of which is herein incorporated by reference) and human glucocorticoid receptor alpha (GRα) into appropriate baculovirus the expression plasmids as described in E.A. Allegretto et al., 268 *J. Biol. Chem.,* 26625 (1993); G. Srinivasan and B. Thompson, 4 *Mol. Endo.,* 209 (1990); and D.R. O'Reilly et al., In, "Baculovirus Expression Vectors", D.R. O'Reilly et al., eds., W.H. Freeman, New York, NY, pp. 139-179 (1992), the disclosures of which are herein incorporated by reference. Assay buffers consisted of the following: PR, 10% glycerol, 10 mM Tris, 1 mM EDTA, 12 mM monothioglycerol (MTG) and 1mM PMSF, pH = 7.5 @ 4°C; GR, 10% glycerol, 25 mM sodium phosphate, 10 mM KF, 2mM DTT, 0.25 mM CHAPS, and 20 mM sodium molybdate, pH = 7.5.

The PR and GR steroid receptor binding assays were performed in the same manner. The final assay volume was 500 μL for PR and 250 μL for GR, and contained ~5 μg of extract protein for PR and -50 mg for GR, as well as 2-4 nM of the appropriate [$^3$H] steroid (e.g, [$^3$H] progesterone and [$^3$H] dexamethasone, respectively) and varying

concentrations of competing ligand at concentrations that ranged from 0 - $10^{-5}$ M. Incubations were carried out at 4°C for 16 hours.

Non-specific binding was defined as that binding remaining in the presence of 500 nM of the appropriate unlabelled steroid. At the end of the incubation period, bound from free ligand were separated by either charcoal (PR) or hydroxylapatite (GR). The amount of bound tritiated hormone was determined by liquid scintillation counting of an aliquot (700 mL) of the supernatant fluid or the hydroxylapatite pellet.

**AR Binding:** For the whole cell binding assay, COS-1 cells in 96-well microtiter plates containing DMEM-10% FBS were transfected as described above with the following plasmid DNA: pRShAR (2 ng/well), pRS-β-Gal (50 ng/well) and pGEM (48 ng/well). Six hours after transfection, media was removed, the cells were washed with PBS and fresh media was added. The next day, the media was changed to DMEM-serum free to remove any endogenous ligand that might be complexed with the receptor in the cells.

After 24 hours in serum-free media, either a saturation analysis to determine the Kd for tritiated dihydrotestosterone ($^3$H-DHT) on human AR or a competitive binding assay to evaluate the ability of test compounds to compete with $^3$H-DHT for AR was performed.

For the saturation analysis, media (DMEM-0.2% CA-FBS) containing $^3$H-DHT (in concentrations ranging from 12 nM to 0.24 nM) in the absence (total binding) or presence (non-specific binding) of a 100-fold molar excess of unlabeled DHT were added to the cells. For the competitive binding assay, media containing 1 nM $^3$H-DHT and test compounds in concentrations ranging from $10^{-10}$ to $10^{-6}$ M were added to the cells. Three replicates were used for each sample. After three hours at 37°C, an aliquot of the total binding media at each concentration of $^3$H-DHT was removed to estimate the amount of free $^3$H-DHT. The remaining media was removed, the cells were washed three times with PBS to remove unbound ligand, and cells were lysed with a Triton X-100-based buffer. The lysates were assayed for amount of bound $^3$H-DHT and β-Gal activity using a scintillation counter or spectrophotometer, respectively.

For the saturation analyses, the difference between the total binding and the nonspecific binding, normalized by the β-Gal rate, was defined as specific binding. The specific binding was evaluated by Scatchard analysis to determine the $K_d$ for $^3$H-DHT. See e.g., D. Rodbard, "Mathematics and statistics of ligand assays: an illustrated guide" In: *J*. Langon and *J.J*. Clapp, eds., *Ligand Assay*, Masson Publishing U.S.A., Inc., New York, pp. 45-99, (1981), the disclosure of which is herein incorporated by reference. For the competition studies, the data was plotted as the amount of $^3$H-DHT (% of control in the absence of test compound) remaining over the range of the dose-response curve for a given compound. The concentration of test compound that inhibited 50% of the amount of $^3$H-DHT bound in the absence of competing ligand was quantified ($IC_{50}$) after log-logit transformation. The $K_i$ values were determined by application of the Cheng-Prusoff equivuation to the $IC_{50}$ values, where:

$$K_i = \frac{IC_{50}}{(1+[^3H\text{-}DHT])/K_d \text{ for } ^3H\text{-}DHT}$$

To date, binding assays have not been performed utilizing ER or MR proteins.

After correcting for non-specific binding, $IC_{50}$ values were determined. The $IC_{50}$ value is defined as the concentration of competing ligand needed to reduce specific binding by 50%. The $IC_{50}$ value was determined graphically from a log-logit plot of the data. The $K_i$ values were determined by application of the Cheng-Prusoff equivuation to the $IC_{50}$ values, the labeled ligand concentration and the $K_d$ of the labeled ligand.

The agonist, antagonist and binding activity assay results of selected steroid receptor modulator compounds of present invention and the standard reference compounds on PR, AR, ER, GR and MR, as well as the cross-reactivity of selected compounds on all of these receptors, are shown in Tables 1-5 below. Efficacy is reported as the percent maximal response observed for each compound relative to the reference agonist and antagonist compounds indicated above. Also reported in Tables 2 for each compound is its antagonist potency or $IC_{50}$ (which is the concentration (nM), requivuired to reduce the maximal response by 50%), its agonist potency or $EC_{50}$ (nM). PR, AR and GR protein binding activity ($K_i$ in nM) is shown in Tables 2.

Table 2:

Agonist, antagonist and binding activity of selected steroid receptor modulator compounds of present invention and the reference agonist compound, dihydrotestosterone (**DHT**), and reference antagonist compound, 2-hydroxyflutamide (**Flut**), on AR.

| Cmpd | AR Agonist CV-1 Cells | | AR Antagonist CV-1 Cells | | AR Binding |
|---|---|---|---|---|---|
| **No.** | **Efficacy (%)** | **Potency (nM)** | **Efficacy (%)** | **Potency (nM)** | **$K_i$ (nM)** |
| **238@** | 96 | 10 | na | na | 44 |
| **247** | 23 | 2,400 | 69 | 34 | 864 |
| **255** | na | na | 82 | 25 | 675 |
| **256** | na | na | 91 | 62 | 4,500 |
| **260** | na | na | 53 | 24 | 435 |
| **265** | na | na | 78 | 56 | 23 |
| **405** | na | na | 89 | 77 | 6 |
| **414** | 118 | 1 | na | na | 0.3 |
| **416** | 88 | 340 | 24 | 5009 | 388 |
| **417** | na | na | 74 | 21 | 23 |
| **418** | na | na | 63 | 200 | 1000 |
| **419** | 29 | 1800 | 74 | 46 | 60 |
| **420** | 40 | 2100 | 80 | 32 | 346 |
| **437** | na | na | 72 | 13 | 38 |
| **445** | 74 | 7 | 32 | 8450 | 13 |
| **DHT** | 100 | 6 | na | na | 2 |
| **Flut** | na | na | 87 | 26 | 2085 |

**na = not active (i.e. efficacy of <20 and potency of >10,000)**
**@ profiles as an AR antagonist *in vivo***

Compounds 255, 260, 417 and 437 of the present invention shown equivual or better activity as AR antagonists than the known antagonist compound 2-OH-flutamide.

**EXAMPLE 361**

The activity of selected compounds of the present invention as AR antagonists was investigated in an immature castrated male rat model, a recognized test of the antiandrogen activity of a given compound, as described in L. G. Hershberger et al., 83 *Proc. Soc. Exptl. Biol. Med.,* 175 (1953); P.C. Walsh and R.F. Gittes, "Inhibition of extratesticular stimuli to prostatic growth in the castrated rat by antiandrogens", 86 *Endocrinology,* 624 (1970); and B.J. Furr et al., "ICI 176,334: A novel non-steroidal, peripherally selective antiandrogen", 113 *J. Endocrinol.,* R7-9 (1987), the disclosures of which are herein incorporated by reference.

The basis of this assay is the fact that the male sexual accessory organs, such as the prostate and seminal vesicles, play an important role in reproductive function. These glands are stimulated to grow and are maintained in size and secretory function by the continued presence of serum testosterone (T), which is the major serum androgen (>95%) produced by the Leydig cells in the testis under the control of the pituitary luteinizing hormone (LH) and follicle stimulating hormone (FSH). Testosterone is converted to the more active form, dihydrotestosterone (DHT), within the prostate by 5$\alpha$-reductase. Adrenal androgens also contribute about 20% of total DHT in the rat prostate, and about 40% of that in 65-year-old men. F. Labrie et al.. 16 *Clin. Invest. Med.*, 475-492 (1993). However, this is not a major pathway, since in both animals and humans, castration leads to almost complete involution of the prostate and seminal vesicles without concomitant adrenalectomy. Therefore, under normal conditions, the adrenals do not support significant growth of prostatic tissue. M.C. Luke and D.S. Coffey, "The Physiology of Reproduction" ed. by E. Knobil and *J*. D. Neill, *1*, 1435-1487 (1994). Since the male sex organs are the tissues most responsive to modulation of androgen activity, this model is used to determine the androgen-dependent growth of the sex accessory organs in immature castrated rats.

Male immature rats (60-70 g, 23-25-day-old, Sprague-Dawley, Harlan) were castrated under metofane anesthesia. Five days after surgery, animals groups were dosed for 3 days as follows:

(1) control vehicle
(2) Testosterone Propionate (TP)(0.1 mg/rat/day, sub cutaneous)
(3) TP plus flutamide, a recognized antiandrogen, as a reference compound, and/or a compound of the present invention (different doses, oral administration, daily) to demonstrate antagonist activity, or
(4) a compound of the present invention alone (different doses, oral administration daily) to demonstrate agonist activity

At the end of the 3-day treatment, the animals were sacrificed, and the ventral prostates (VP) and seminal vesicles (SV) were collected and weighed. To compare data from different experiments, the sexual organ weights were first standardized as mg per 100 g of body weight, and the increase in organ weight induced by TP was considered as the maximum increase (100%). Super-anova (one factor) was used for statistical analysis.

The gain and loss of sexual organ weights reflect the changes of cell number (DNA content) and cell mass (protein content), depending upon the serum androgen concentration. See Y. Okuda et al., 145 *J Urol*., 188-191 (1991), the disclosure of which is herein incorporated by reference.. Therefore, measurement of organ wet weights is sufficient to indicate the bioactivity of androgens and androgen antagonists. In immature castrated rats, replacement of exogenous androgens increased the weights of the ventral prostate (VP) and the seminal vesicles (SV) in a dose-dependent manner as shown in Table 8.

Table 8:

| TP-Induced Ventral Prostate and Seminal Vesicle Growth in castrated immature rats, with oral dosing once daily, for 3 days. | | | | |
|---|---|---|---|---|
| **Treatment (mg TP)** | **VP (wet wt)** | **%VP growth** | **SV (wet wt)** | **%SV growth** |
| 0 | 10.5±1.0 | 100 | 7.5±0.6 | 100 |
| 0.01 | 15.4±0.6 | 146.5 | 12.3±0.8 | 165.1 |
| 0.03 | 23.5±1.3 | 224.1 | 27.5±0.8 | 369.5 |
| 0.1 | 35.3±2.1 | 337.0 | 42.0±2.0 | 563.8 |
| 0.3 | 43.6±1.1 | 415.9 | 45.9±1.9 | 616.1 |
| 1 | 44.8±3.7 | 427.4 | 51.0±5.4 | 684.6 |

The maximum increase in organ wet weights was 4 to 5-fold when dosing 3 mg/rat/day of testosterone (T) or 1 mg/rat/day of testosterone propionate (TP) for 3 days. The $EC_{50}$ of T and TP were about 1 mg and 0.03 mg, respectively. The increase in the weights of VP and SV also correlated with the increase in the serum T and DHT concentrations. Although administration of T showed 5-times higher serum concentrations of T and DHT at 2 hours after subcutaneous injection than that of TP, thereafter, these high levels declined very rapidly. In contrast, the serum concentrations of T and DHT in TP-treated animals were fairly consistent during the 24 hours, and therefore, TP showed about 10-30-fold higher potency than free T.

In this immature castrated rat model, a known AR antagonist (flutamide) was also administered simultaneously with 0.1 mg of TP ($ED_{80}$), inhibiting the testosterone-mediated increases in the weights of VP and SV in a dose-dependent manner as shown in Table 9. The antagonist effects were similar when dosing orally or subcutaneously. Compounds 255 and 261 also exhibited AR antagonist activity by suppressing the testosterone-mediated increases in the weights of the VP and SV, as summarized in Table 9.

Table 9:

| Inhibition of TP-InducedVentral Prostate and Seminal Vesicle Growth in castrated immature rats at oral dosing, once daily, for 3 days of flutamide (flut), Compound 255 or Compound 261. | | | | |
|---|---|---|---|---|
| **Treatment** | **VP (wet wt)** | **VP wt (% of TP (0.1) control)** | **SV (wet wt)** | **SV wt (% of TP (0.1) control)** |
| **Control** | 9.8±1.2 | 36.2 | 9.9±0.9 | 21.7 |
| **TP (0.1)** | 25.5±1.2 | 100 | 33.6±4.0 | 100 |
| **TP+ flut (1.0)** | 12.4±1.1 | 49.9 | 8.5±0.6 | 30.3 |
| **TP+ flut (3.0)** | 9.5 ±0.4 | 37.4 | 9.8±0.5 | 29.3 |
| **TP+ 255 (0.3)** | 22.1±0.7 | 86.4 | 29.8±2.5 | 88.7 |
| **TP+ 255 (1.0)** | 20.0±4.5 | 78.2 | 24.8±9.0 | 73.9 |
| **TP+ 255 (3.0)** | 17.3±1.2 | 67.7 | 20.4±1.2 | 60.6 |
| **TP+ 261 (1.0)** | 21.0±1.7 | 84.4 | 23.8±1.8 | 85.0 |
| **TP+ 261 (3.0)** | 16.7±1.0 | 67.1 | 20.8±1.3 | 74.2 |

## Pharmacological and Other Applications

As will be discernible to those skilled in the art, the non-steroid modulator compounds of the present invention can be readily utilized in pharmacological applications
where PR, AR, ER, GR and/or MR antagonist or agonist activity is desired, and where it is desired to minimize cross reactivities with other steroid receptor related IRs. In vivo applications of the invention include administration of the disclosed compounds to mammalian subjects, and in particular to humans.

While in accordance with the patent statutes, description of the preferred embodiments and processing conditions have been provided, the scope of the invention is not to be limited thereto or thereby.

Consequently, for an understanding of the scope of the present invention, reference is made to the following claims.

## Claims

**1.** A compound having the formulae:

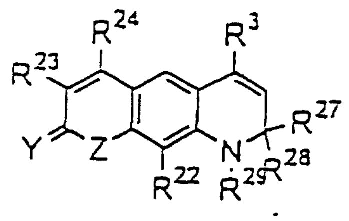

(IX)

OR

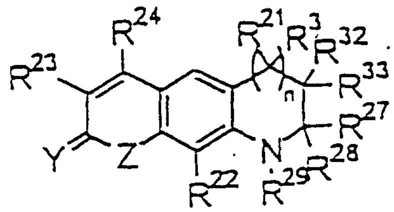

(XVII)

OR

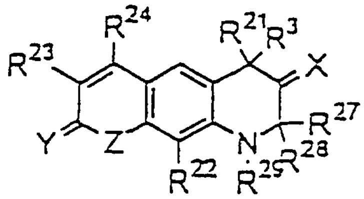

(XVIII)

wherein:

$R^2$ is hydrogen, a $C_1$ - $C_4$ alkyl or perfluoroalkyl;

$R^3$ is hydrogen, a $C_1$ - $C_4$ alkyl or perfluoroalkyl, or hydroxymethyl;

$R^7$ is hydrogen, a $C_1$ - $C_4$ alkyl or perfluoroalkyl, $OR^8$ or $NHR^8$, where $R^8$ is hydrogen, a $C_1$ - $C_6$ alkyl or perfluoroalkyl, $SO_2R^2$ or $S(O)R^2$, where $R^2$ has the definition given above;

X is $CH_2$, O, S or $NR^7$, where $R^7$ has the definition given above;

$R^{21}$ is hydrogen, or a $C_1$ - $C_4$ alkyl;

$R^{22}$ is hydrogen, a $C_1$ -$C_4$ alkyl, F, Cl, Br, I, $OR^2$, $NR^2R^7$ or $SR^2$, where $R^2$ and $R^7$ have the definitions given above;

$R^{23}$ is hydrogen, Cl, Br, F, or a $C_1$ - $C_4$ alkyl or perhaloalkyl;

$R^{24}$ is hydrogen, F, Br, Cl, a $C_1$ - $C_4$ alkyl or perhaloalkyl, a phenyl ring or a five-membered heterocyclic ring containing one or more heteroatoms selected from the group consisting of oxygen, nitrogen and sulfur, or a six-membered heterocyclic ring containing one or more nitrogen heteroatoms, $CF_3$, $CF_2OR^{25}$, $CH_2OR^{25}$, or $OR^{25}$, where $R^{25}$ is a $C_1$ - $C_4$ alkyl, except that $R^{24}$ cannot be $CH_3$ when Z is O, $R^{22}$, $R^{23}$, and $R^{29}$ are all hydrogen and $R^3$, $R^{27}$ and $R^{28}$ all are $CH_3$;

$R^{27}$ and $R^{28}$ each independently are hydrogen, or a $C_1$ - $C_4$ alkyl or perfluoroalkyl;

$R^{29}$ is hydrogen, or a $C_1$ - $C_6$ alkyl;

$R^{32}$ and $R^{33}$ each independently are hydrogen, or a $C_1$ - $C_4$ alkyl;

n is 0 or 1;

Y is O or S;

Z is O, S, NH, $NR^2$ or $NCOR^2$, where $R^2$ has the same definition given above.

**2.** A compound according to claim 1 selected from the group of compounds of general formula **IX** consisting of:

1,2-Dihydro-2,2,4-trimethyl-6-methoxymethyl-8-pyranono[5,6-*g*]quinoline (Compound 237);
1,2-Dihydro-2,2,4-trimethyl-6-trifluoromethyl-8-pyranone[5,6-*g*]quinoline (Compound 238) ;
1,2 Dihydro-2,2,4,6-tetramethyl-8-pyridono[5 ,6-*g*]quinoline (Compound 241);
1,2-Dihydro-2,2,4-trimethyl-6-trifluoromethyl-8-pyridono[5,6-*g*]quinoline (Compound 247);
1,2-Dihydro-2,2,4-trimethyl-6-trifluoromethyl-8-thiopyranono[5,6-*g*]quinoline (Compound 251);
6-Chloro(difluoro)methyl-1,2-dihydro-2,2,4-trimethyl-8-pyranono[5,6-*g*]quinoline (Compound 253);
9-Acetyl-1,2-dihydro-2,2,4-trimethyl-6-trifluoromethyl-8-pyridono[5,6-*g*]quinoline (Compound 254);
1,2-Dihydro-2,2,4,10-tetramethyl-6-trifluoromethyl-8-pyridono[5,6-*g*]quinoline (Compound 255);
1,2-Dihydro-2,2,4-trimethyl-6-(1,1,2,2,2-pentafluoroethyl)-8-pyranono[5,6-g] quinoline (Compound 256);
7-Chloro-1,2-dihydro-2,2,4-trimethyl-6-trifluoromethyl-8-pyranono[5,6-*g*]quinoline (Compound 258);
1,2-Dihydro-2,2,4,9-tetramethyl-6-trifluoromethyl-8-pyridono[5,6-*g*] quinoline (Compound 261);
6-[Dichloro(ethoxy)methyl]-1,2-dihydro-2,2,4-trimethyl-8-pyranono[5,6-*g*]quinoline (Compound 263);
6-(3-Furyl)-1,2-dihydro-2,2,4-trimethyl-8-pyranono[5,6-g]quinoline (Compound 264);
1,2-Dihydro-1,2,2,4-tetramethyl-6-trifluoromethyl-8-pyranono[5,6-*g*]quinoline (Compound 265);
1,2-Dihydro-6-trifluoromethyl-2,2,4-trimethyl-9-thiopyran-8-ono[5,6-*g*]quinoline (Compound 266);
1,2-Dihydro-1,2,2,4,9-pentamethyl-6-trifluoromethyl-8-pyridono[5,6-*g*]quinoline (Compound 267),
7-Chloro-1,2-dihydro-2,2,4 trimethyl-6-trifluoromethyl-8-pyridono[5,6-*g*]quinoline (Compound 268); and
6-Chloro(difluoro)methyl-1,2-dihydro-2,2,4-trimethyl-8-pyridono[5,6-*g*]quinoline (Compound 269);
6-(3-Furyl)-1,2-dihydro-1,2,2,4-tetramethyl-8-pyranono[5,6-*g*]quinoline (Compound 406);
6-(3-Furyl)-1,2-dihydro-1,2,2,4-tetramethyl-8-thiopyranono[5,6-*g*]quinoline (Compound 407);
7-Chloro-6-triflouromethyl-1,2-dihydro-1,2,2,4-tetramethyl-8-pyranono[5,6-*g*]quinoline (Compound 408);
1,2-Dihydro-2,2-dimethyl-6-trifluoromethyl-8-pyranono[5,6-*g*]quinoline (Compound 411);
1,2-Dihydro-6-trifluoromethyl-1,2,2,4-tetramethyl-8-pyridono[5,6-*f*]quinoline (Compound 423);
1,2-Dihydro-1,2,2,4-tetramethyl-6-trifluoromethyl-9-thiopyran-8-ono[5,6-*g*]quinoline (Compound 430);

the group of compounds of general formula **XVII** consisting of:

(*R*/*S*)-1,2,3,4-Tetrahydro-2,2,4-trimethyl-6-trifluoromethyl-8-pyranono[5,6-*g*]quinoline (Compound 250), (*R*/*S*)-1,2,3,4-Tetrahydro-2,2,4-trimethyl-6-trifluoromethyl-8-thiopyranono[5,6-*g*]quinoline (Compound 252) ; (*R*/*S*)-6-Chloro(difluoro)methyl-1,2,3,4-tetrahydro-2,2,4-trimethyl-8-pyranono[5,6-*g*]quinoline (Compound 257); (*R*/*S*)-7-Chloro-1,2,3,4-tetrahydro-2,2,4-trimethyl-6-trifluoromethyl-8-pyranono[5,6-*g*]quinoline (Compound 259) ;
1,2,3,4-Tetrahydro-2,2,4-trimethyl-6-trifluoromethyl-8-pyridono[5,6-*g*]quinoline (Compound 260);
(*R*/*S*)-1,2,3,4-Tetrahydro-1,2,2,4-tetramethyl-6-trifluoromethyl-8-pyranono[5,6-*g*]quinoline (Compound 404);
6-(3-Furyl)-1,2,3,4-tetrahydro-2,2,4-trimethyl-8-pyranono[5,6-*g*]quinoline (Compound 405);
1,2,3,4-Tetrahydro-2,2,4,10-tetramethyl-6-trifluoromethyl-8-pyridono[5,6-*g*]quinoline (Compound 409); (*R*/*S*)-1,2,3,4-Tetrahydro-4-methyl-6-trifluoromethyl-8-pyranono[5,6-*g*]quinoline (Compound 410);
1,2,3,4-Tetrahydro-2,2-dimethyl-6-trifluoromethyl-8-pyranono [5,6-*g*] quinoline (Compound 412);
1,2,3,4-Tetrahydro-6-trifluoromethyl-8-pyranono[5,6-*g*]quinoline (Compound 413);
(*R*/*S*)-4-Ethyl-1,2,3,4-tetrahydro-6-trifluoromethyl-8-pyranono[5,6-*g*]quinoline (Compound 414);
(*R*/*S*)-1,2,3,4-Tetrahydro-1,4-dimethyl-8-pyranono[5,6-*g*]quinoline (Compound 415);
(*R*/*S*)-4-Ethyl-1,2,3,4-tetrahydro-1-methyl-8-pyranono[5,6-*g*]quinoline (Compound 416);
2,2-Dimethyl-1,2,3,4-tetrahydro-6-trifluoromethyl-8-pyridono[5,6-*f*]quinoline (Compound 417);
5-Trifluoromethyl-7-pyridono[5,6-e]indoline (Compound 419) ;
2,2,10-Trimethyl-1,2,3,4-tetramethyl-6-trifluoromethyl-8-pyridono[5,6-*g*]quinoline (Compound 421);
1,2,3,4-Tetrahydro-6-trifluoromethyl-8-pyridono[5,6-*g*]quinoline (Compound 422);
3,3-Dimethyl-5-trifluoromethyl-7-pyridono[5,6-*f*]indoline (Compound 424);

(*R*/*S*)-1,2,3,4-Tetrahydro-4-methyl-6-(trifluoromethyl)-8-pyridono[5,6-*g*]quinoline (Compound 425); 1,2,2,-Trimethyl-1,2,3,4-tetrahydro-6-trifluoromethyl-8-pyranono[5,6-*g*]quinoline (Compound 427); (*R*/*S*)-1,2,3,4-Tetrahydro-4-propyl-6-trifluoromethyl-8-pyranono[5,6-*g*]quinoline (Compound 428); 1,2,3,4-Tetrahydro-2,2,4-trimethyl-6-trifluoromethyl-9-thiopyran-8-ono[5,6-*g*]quinoline (Compound 429); 1,2,3,4-Tetrahydro-1,2,2-trimethyl-6-trifluoromethyl-8-pyridono[5,6-*g*]quinoline (Compound 431); 1,2,3,4-Tetrahydro-1-methyl-4-propyl-6-trifluoromethyl-8-pyranono[5,6-*g*]quinoline (Compound 432); 1,2,3,4-Tetrahydro-1,2,2,4-tetramethyl-6-trifluoromethyl-9-thiopyran-8-ono[5,6-*g*]quinoline (Compound 434); 1,2,3,4-Tetrahydro-2,2,9-trimethyl-6-trifluoromethyl-8-pyridono[5,6-*g*]quinoline (Compound 435); (*R*/*S*)-1,2,3,4-Tetrahydro-3-methyl-6-trifluoromethyl-8-pyridono[5,6-*g*]quinoline (Compound 436); 1,2,3,4-Tetrahydro-3,3-dimethyl-6-trifluoromethyl-8-pyridono[5,6-*g*]quinoline (Compound 437); (*R*/*S*) 1,2,3,4-Tetrahydro-2,2,3-trimethyl-6-trifluoromethyl-8-pyridono[5,6-*g*]quinoline (Compound 438); (*R*/*S*-2*l*,4*u*)-1,2,3,4-Tetrahydro-2,4-dimethyl-6-trifluoromethyl-8-pyridono[5,6-*g*] quinoline (Compound 439); (*R*/*S*-2*l*,4*u*)-4-Ethyl-1,2,3,4-tetrahydro-2-methyl-6-trifluoromethyl-8-pyranono[5,6-*g*]quinoline (Compound 440); (*R*/*S*-2*l*, 3*u*)-1,2,3,4-Tetrahydro-2,3-dimethyl-6-trifluoromethyl-8-pyridono[5,6-*g*]quinoline (Compound 441); (*R*/*S*-2*l*,3*l*)-1,2,3,4-Tetrahydro-2,3-dimethyl-6-trifluoromethyl-8-pyridono[5,6-*g*]quinoline (Compound 442); (*R*/*S*)-1,2,3,4-Tetrahydro-2,3,3-trimethyl-6-trifluoromethyl-8-pyridono[5,6-*g*]quinoline (Compound 443); (*R*/*S*)-*1,2,3,4-Tetrahydro-2-methyl-6-trifluoromethyl-8*-pyridono[5,6-*g*]quinoline (Compound 444); (*R*/*S*)-4-Ethyl-1,2,3,4-tetrahydro-6-trifluoromethyl-8-pyridono[5,6-*g*]quinoline (Compound 445); (*R*/*S*-2*l*, 3*u*)-1,2,3,4-Tetrahydro-2,3,9-trimethyl-6-trifluoromethyl-8-pyridono[5,6-*g*]quinoline (Compound 446); (*R*/*S*)-1,2,3,4-Tetrahydro-4-propyl-6-trifluoromethyl-8-pyridono[5,6-*g*]quinoline (Compound 447); (*R*/*S*)-3-Ethyl-1,2,3,4-tetrahydro-2,2-dimethyl-6-trifluoromethyl-8-pyridono[5,6-*g*]quinoline (Compound 448); (*R*/*S*)-1,2,3,4-Tetrahydro-2,2-dimethyl-6-trifluoromethyl-3-propyl-8-pyridono[5,6-*g*]quinoline (Compound 449); and 1-Methyl-5-trifluoromethyl-7-pyridono[5,6-*f*]indoline (Compound 450); and

the compound of general formula **XVIII**
(*R*/*S*)-1,2,3,4-tetrahydro-6-trifluoromethyl-2,2,4-trimethyl-8-pyridono[5,6-*f*]-3-quinolinone (Compound 418).

**3.** A pharmaceutical composition comprising a compound of claim 1 and a pharmaceutically acceptable carrier.

**4.** A pharmaceutical composition according to claim 3, wherein the composition is formulated for oral, topical, intravenous, suppository or parental administration.

**5.** A pharmaceutical composition according to claim 3, wherein the compound is administered to a patient as a dosage unit at from about 1 μg/kg of body weight to 500 mg/kg of body weight.

**6.** A pharmaceutical composition according to claim 3, wherein the compound is administered to a patient as a dosage unit at from 10 μg/kg of body weight to 250 mg/kg of body weight.

**7.** A pharmaceutical composition according to claim 3, wherein the compound is administered to a patient as a dosage unit at from 20 μg/kg of body weight to 100 mg/kg of body weight.

**8.** Use of the compounds according to claim 1 for the preparation of a medicament for the treatment and/or modulation of human fertility, female hormone replacement, dysfunctional uterine bleeding, endometriosis, leiomyomas, acne, male-pattern baldness, osteoporosis, prostatic hyperplasia, cancer of the breast, cancer of the ovaries, endometrial cancer, prostate cancer, carbohydrate, protein and lipid metabolism, electrolyte and water balance, and functioning of the cardiovascular, kidney, central nervous, immune and skeletal muscle systems.

**9.** Use of a compound according to claim 1 for the preparation of a medicament for treating patients requiring androgen receptor therapy, wherein the compound is effective in treating and/or modulating human fertility, female hormone replacement, dysfunctional uterine bleeding, endometriosis, leiomyomas, acne, male-pattern baldness, osteoporosis, prostatic hyperplasia, cancer of the breast, cancer of the ovaries, endometrial cancer, prostate cancer, carbohydrate, protein and lipid metabolism, electrolyte and water balance, and functioning of the cardiovascular, kidney, central nervous, immune and skeletal muscle systems.

**10.** A method for determining the presence of one or more androgen receptors in a sample comprising combining a compound according to claim 1 with the sample containing one or more unknown androgen receptors and determining whether said compound binds to a receptor in the sample.

**11.** A ligand-androgen receptor complex formed by the binding of a compound according to claim 1 to a androgen receptor.

**12.** A method of purifying androgen receptors comprising combining a compound according to claim 1 with a sample containing androgen receptors, allowing said compound to bind said androgen receptors, and separating out the bound combination of said compound and said androgen receptors.

**13.** A compound selected from the group of
1,2-Dihydro-2,2,4-trimethyl-10-isocoumarino[4,3-*g*]quinoline (Compound 239);
1,2-Dihydro-2,2,4-trimethyl-10-isoquinolono[4,3-g]quinoline(Compound 240);
(*R*/*S*)-1,2,3,4-Tetrahydro-2,2,4-trimethyl-10-isoquinolono[4,3-*g*]quinoline (Compound 244);
1,2-Dihydro-2,2,4-trimethyl-10-thioisoquinolono[4,3-*g*]quinoline (Compound 245);
(+)- 1,2,3,4-Tetrahydro- 2,2,4-trimethyl-10-isoquinolono[4,3-*g*]quinoline (Compound 246);
1,2-Dihydro-10-hydroxy-2,2,4-trimethyl-10*H* -isochromeno[4,3-*g*]quinoline (Compound 242) ;
1,2-Dihydro-2,2,4,6-tetramethyl-8H-pyrano[3,2-*g*]quinoline (Compound 243); and 1,2-Dihydro-2,2,4-trimethyl-8-trifluoromethyl-6-pyridono[5,6-*g*]quinoline (Compound 262);
8-(4-Chlorobenzoyl)-5-trifluoromethyl-7-pyridono[5,6-e]indoline (Compound 420);
1,2-Dihydro-2,2,4-trimethyl-6-methoxymethyl-8-pyridono [5, 6-*g*] quinoline (Compound 426); and
1,2,3,4-Tetrahydro-10-hydroxymethyl-2,2,4-trimethyl-6-trifluoromethyl-8-pyridono[5,6-*g*]quinoline (Compound 433).

**Patentansprüche**

**1.** Verbindung mit der Formel:

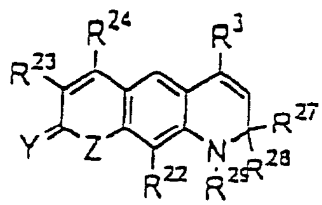

(IX)

oder

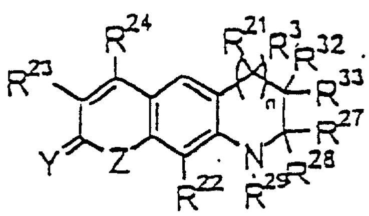

(XVII)

oder

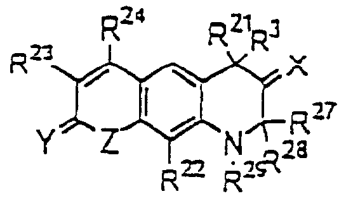

(XVIII)

wobei:

$R^2$ gleich Wasserstoff, ein $C_1$-$C_4$-Alkyl oder Perfluoralkyl ist;

$R^3$ gleich Wasserstoff, ein $C_1$-$C_4$-Alkyl oder Perfluoralkyl oder Hydroxymethyl ist;

$R^7$ gleich Wasserstoff, ein $C_1$-$C_4$-Alkyl oder Perfluoralkyl, $OR^8$ oder $NHR^8$, wobei $R^8$ gleich Wasserstoff, ein $C_1$-$C_6$-Alkyl oder Perfluoralkyl ist, $SO_2R^2$ oder $S(O)R^2$, wobei $R^2$ die oben angegebene Definition besitzt, ist;

X gleich $CH_2$, O, S oder $NR^7$ ist, wobei $R^7$ die oben angegebene Definition besitzt;

$R^{21}$ gleich Wasserstoff oder ein $C_1$-$C_4$-Alkyl ist;

$R^{22}$ gleich Wasserstoff, ein $C_1$-$C_4$-Alkyl, F, Cl, Br, I, $OR^2$, $NR^2R^7$ oder $SR^2$ ist, wobei $R^2$ und $R^7$ die oben angegebenen Definitionen besitzen;

$R^{23}$ gleich Wasserstoff, Cl, Br, F oder ein $C_1$-$C_4$-Alkyl oder Perhaloalkyl ist;

$R^{24}$ gleich Wasserstoff, F, Br, Cl ein $C_1$-$C_4$-Alkyl oder Perhaloalkyl, ein Phenylring oder ein fünfgliedriger heterocyclischer Ring, der ein oder mehrere aus der aus Sauerstoff, Stickstoff und Schwefel bestehenden Gruppe ausgewählte Heteroatome enthält, oder ein sechsgliedriger heterocyclischer Ring, der ein oder mehrere Stickstoffheteroatome enthält, $CF_3$, $CF_2OR^{25}$, $CH_2OR^{25}$ oder $OR^{25}$ ist, wobei $R^{25}$ ein $C_1$-$C_4$-Alkyl ist, mit der Ausnahme, dass $R^{24}$ nicht $CH_3$ sein kann, wenn Z gleich O ist, $R^{22}$, $R^{23}$, und $R^{29}$ alle Wasserstoff sind und $R^3$, $R^{27}$ und $R^{28}$ alle $CH_3$ sind;

$R^{27}$ und $R^{28}$ jeweils unabhängig Wasserstoff oder ein $C_1$-$C_4$-Alkyl oder Perfluoralkyl sind;

$R^{29}$ gleich Wasserstoff oder ein $C_1$-$C_6$-Alkyl ist;

$R^{32}$ und $R^{33}$ jeweils unabhängig Wasserstoff oder ein $C_1$-$C_4$-Alkyl sind;

n gleich 0 oder 1 ist;

Y gleich O oder S ist;

Z gleich O, S, NH, $NR^2$ oder $NCOR^2$ ist, wobei $R^2$ dieselbe wie oben angegebene Definition besitzt.

**2.** Verbindung nach Anspruch 1, die ausgewählt ist aus der Gruppe an Verbindungen der allgemeinen Formel IX, bestehend aus:

1,2-Dihydro-2,2,4-trimethyl-6-methoxymethyl-8-pyranono[5,6-*g*]chinolin (Verbindung 237);
1,2-Dihydro-2,2,4-trimethyl-6-trifluormethyl-8-pyranono[5,6-*g*]chinolin (Verbindung 238);
1,2-Dihydro-2,2,4,6-tetramethyl-8-pyridono[5,6-*g*]chinolin (Verbindung 241);
1,2-Dihydro-2,2,4-trimethyl-6-trifluormethyl-8-pyridono[5,6-*g*]chinolin (Verbindung 247);
1,2-Dihydro-2,2,4-trimethyl-6-trifluormethyl-8-thiopyranono[5,6-*g*]chinolin (Verbindung 251);
6-Chlor(difluor)methyl-1,2-dihydro-2,2,4-trimethyl-8-pyranono[5,6-*g*]chinolin (Verbindung 253);

9-Acetyl-1,2-dihydro-2,2,4-trimethyl-6-trifluormethyl-8-pyridono[5,6-*g*]chinolin (Verbindung 254);
1,2-Dihydro-2,2,4,10-tetramethyl-6-trifluormethyl-8-pyridono[5,6-*g*]chinolin (Verbindung 255);
1,2-Dihydro-2,2,4-trimethyl-6-(1,1,2,2,2-pentafluorethyl)-8-pyranono[5,6-*g*]chinolin (Verbindung 256);
7-Chlor-1,2-dihydro-2,2,4-trimethyl-6-trifluormethyl-8-pyranono[5,6-g]chinolin (Verbindung 258);
1,2-Dihydro-2,2,4,9-tetramethyl-6-trifluormethyl-8-pyridono[5,6-*g*]chinolin (Verbindung 261);
6-[Dichlor(ethoxy)methyl]-1,2-dihydro-2,2,4-trimethyl-8-pyranono[5,6-*g*]chinolin (Verbindung 263);
6-(3-Furyl)-1,2-dihydro-2,2,4-trimethyl-8-pyranono[5,6-*g*]chinolin (Verbindung 264);
1,2-Dihydro-1,2,2,4-tetramethyl-6-trifluormethyl-8-pyranono[5,6-*g*]chinolin (Verbindung 265);
1,2-Dihydro-6-trifluormethyl-2,2,4-trimethyl-9-thiopyran-8-ono[5,6-*g*]chinolin (Verbindung 266);
1,2-Dihydro-1,2,2,4,9-pentamethyl-6-trifluormethyl-8-pyridono[5,6-*g*]chinolin (Verbindung 267),
7-Chlor-1,2-dihydro-2,2,4-trimethyl-6-trifluormethyl-8-pyridono[5,6-*g*]chinolin (Verbindung 268); und
6-Chlor(difluor)methyl-1,2-dihydro-2,2,4-trimethyl-8-pyridono[5,6-*g*]chinolin (Verbindung 269);
6-(3-Furyl)-1,2-dihydro-1,2,2,4-tetramethyl-8-pyranono[5,6-*g*]chinolin (Verbindung 406);
6-(3-Furyl)-1,2-dihydro-1,2,2,4-tetramethyl-8-thiopyranono[5,6-*g*]chinolin (Verbindung 407);
7-Chlor-6-trifluormethyl-1,2-dihydro-1,2,2,4-tetramethyl-8-pyranono[5,6-*g*]chinolin (Verbindung 408);
1,2-Dihydro-2,2-dimethyl-6-trifluormethyl-8-pyranono[5,6-*g*]chinolin (Verbindung 411);
1,2-Dihydro-6-trifluormethyl-1,2,2,4-tetramethyl-8-pyridono[5,6-*f*]chinolin (Verbindung 423);
1,2-Dihydro-1,2,2,4-tetramethyl-6-trifluormethyl-9-thiopyran-8-ono[5,6-*g*]chinolin (Verbindung 430);

der Gruppe der Verbindungen der allgemeinen Formel XVII, bestehend aus:

(*R*/*S*)-1,2,3,4-Tetrahydro-2,2,4-trimethyl-6-trifluormethyl-8-pyranono[5,6-*g*]chinolin (Verbindung 250),
(*R*/*S*)-1,2,3,4-Tetrahydro-2,2,4-trimethyl-6-trifluormethyl-8-thiopyranono[5,6-*g*]chinolin (Verbindung 252);
(*R*/*S*)-6-Chlor(difluor)methyl-1,2,3,4-tetrahydro-2,2,4-trimethyl-8-pyranono[5,6-*g*]chinolin (Verbindung 257);
(*R*/*S*)-7-Chlor-1,2,3,4-tetrahydro-2,2,4-trimethyl-6-trifluormethyl-8-pyranono [5,6-*g*]chinolin (Verbindung 259);
1,2,3,4-Tetrahydro-2,2,4-trimethyl-6-trifluormethyl-8-pyridono[5,6-*g*]chinolin (Verbindung 260);
(*R*/*S*)-1,2,3,4-Tetrahydro-1,2,2,4-tetramethyl-6-trifluormethyl-8-pyranono[5,6-*g*]chinolin (Verbindung 404);
6-(3-Furyl)-1,2,3,4-tetrahydro-2,2,4-trimethyl-8-pyranono[5,6-*g*]chinolin (Verbindung 405);
1,2,3,4-Tetrahydro-2,2,4,10-tetramethyl-6-trifluormethyl-8-pyridono[5,6-*g*]chinolin (Verbindung 409);
(*R*/*S*)-1,2,3,4-Tetrahydro-4-methyl-6-trifluormethyl-8-pyranono[5,6-*g*]chinolin (Verbindung 410);
1,2,3,4-Tetrahydro-2,2-dimethyl-6-trifluormethyl-8-pyranono[5,6-*g*]chinolin (Verbindung 412);
1,2,3,4-Tetrahydro-6-trifluormethyl-8-pyranono[5,6-*g*]chinolin (Verbindung 413);
(*R*/*S*)-4-Ethyl-1,2,3,4-tetrahydro-6-trifluormethyl-8-pyranono [5,6-*g*]chinolin (Verbindung 414);
(*R*/*S*)-1,2,3,4-Tetrahydro-1,4-dimethyl-8-pyranono[5,6-g]chinolin (Verbindung 415);
(*R*/*S*)-4-Ethyl-1,2,3,4-tetrahydro-1-methyl-8-pyranono[5,6-*g*]chinolin (Verbindung 416);
2,2-Dimethyl-1,2,3,4-tetrahydro-6-trifluormethyl-8-pyridono[5,6-*f*]chinolin (Verbindung 417);
5-Trifluormethyl-7-pyridono[5,6-e]indolin (Verbindung 419);
2,2,10-Trimethyl-1,2,3,4-tetramethyl-6-trifluormethyl-8-pyridono[5,6-*g*]chinolin (Verbindung 421);
1,2,3,4-Tetrahydro-6-trifluormethyl-8-pyridono[5,6-*g*]chinolin (Verbindung 422);
3,3-Dimethyl-5-trifluormethyl-7-pyridono[5,6-*f*]indolin (Verbindung 424);
(*R*/*S*)-1,2,3,4-Tetrahydro-4-methyl-6-(trifluormethyl)-8-pyridono[5,6-*g*]chinolin (Verbindung 425);
1,2,2,-Trimethyl-1,2,3,4-tetrahydro-6-trifluormethyl-8-pyranono[5,6-g]chinolin (Verbindung 427);
(*R*/*S*)-1,2,3,4-Tetrahydro-4-propyl-6-trifluormethyl-8-pyranono[5,6-*g*]chinolin (Verbindung 428);
1,2,3,4-Tetrahydro-2,2,4-trimethyl-6-trifluormethyl-9-thiopyran-8-ono[5,6-*g*]chinolin (Verbindung 429);
1,2,3,4-Tetrahydro-1,2,2-trimethyl-6-trifluormethyl-8-pyridono[5,6-*g*]chinolin (Verbindung 431);
1,2,3,4-Tetrahydro-l-methyl-4-propyl-6-trifluormethyl-8-pyranono[5,6-*g*]chinolin (Verbindung 432);
1,2,3,4-Tetrahydro-1,2,2,4-tetramethyl-6-trifluormethyl-9-thiopyran-8-ono[5,6-*g*]chinolin (Verbindung 434);
1,2,3,4-Tetrahydro-2,2,9-trimethyl-6-trifluormethyl-8-pyridono[5,6-*g*]chinolin (Verbindung 435);
(*R*/*S*)-1,2,3,4-Tetrahydro-3-methyl-6-trifluormethyl-8-pyridono[5,6-*g*]chinolin (Verbindung 436);
1,2,3,4-Tetrahydro-3,3-dimethyl-6-trifluormethyl-8-pyridono[5,6-*g*]chinolin (Verbindung 437);
(*R*/*S*)-1,2,3,4-Tetrahydro-2,2,3-trimethyl-6-trifluormethyl-8-pyridono[5,6-*g*]chinolin (Verbindung 438);
(*R*/*S*-2*l*,4*u*)-1,2,3,4-Tetrahydro-2,4-dimethyl-6-trifluormethyl-8-pyridono[5,6-*g*]chinolin (Verbindung 439) ;
(*R*/*S*-2*l*,4*u*)-4-Ethyl-1,2,3,4-tetrahydro-2-methyl-6-trifluormethyl-8-pyranono[5,6-*g*]chinolin (Verbindung 440);
(*R*/*S*-2*l*,3*u*)-1,2,3,4-Tetrahydro-2,3-dimethyl-6-trifluormethyl-8-pyridono [5,6-*g*] chinolin (Verbindung 441);
(*R*/*S*-2*l*,3*l*)-1,2,3,4-Tetrahydro-2,3-dimethyl-6-trifluormethyl-8-pyridono[5,6-*g*]chinolin (Verbindung 442) ;
(*R*/*S*)-1,2,3,4-Tetrahydro-2,3,3-trimethyl-6-trifluormethyl-8-pyridono[5,6-*g*]chinolin (Verbindung 443);
(*R*/*S*)-1,2,3,4-Tetrahydro-2-methyl-6-trifluormethyl-8-pyridono[5,6-*g*]chinolin (Verbindung 444);
(*R*/*S*)-4-Ethyl-1,2,3,4-tetrahydro-6-trifluormethyl-8-pyridono[5,6-*g*]chinolin (Verbindung 445);

(*R*/*S*-2*l*,3*u*)-1,2,3,4-Tetrahydro-2,3,9-trimethyl-6-trifluormethyl-8-pyridono[5,6-*g*]chinolin (Verbindung 446);
(*R*/*S*)-1,2,3,4-Tetrahydro-4-propyl-6-trifluormethyl-8-pyridono[5,6-*g*]chinolin (Verbindung 447);
(*R*/*S*)-3-Ethyl-1,2,3,4-tetrahydro-2,2-dimethyl-6-trifluormethyl-8-pyridono[5,6-*g*]chinolin (Verbindung 448);
(*R*/*S*)-1,2,3,4-Tetrahydro-2,2-dimethyl-6-trifluormethyl-3-propyl-8-pyridono[5,6-*g*]chinolin (Verbindung 449); und
1-Methyl-5-trifluormethyl-7-pyridono[5,6-*f*]indolin (Verbindung 450); und

der Verbindung der allgemeinen Formel XVIII
(*R*/*S*)-1,2,3,4-Tetrahydro-6-trifluormethyl-2,2,4-trimethyl-8-pyridono[5,6-*f*]-3-chinolinon (Verbindung 418).

**3.** Pharmazeutische Zusammensetzung, die eine Verbindung gemäß Anspruch 1 und einen pharmazeutisch akzeptablen Träger umfasst.

**4.** Pharmazeutische Zusammensetzung nach Anspruch 3, wobei die Zusammensetzung für eine orale, topische, intravenöse, suppositorische oder parenterale Verabreichung formuliert ist.

**5.** Pharmazeutische Zusammensetzung nach Anspruch 3, wobei die Verbindung einem Patienten mit einer Dosiseinheit von ungefähr 1 µg/kg an Körpergewicht bis 500 mg/kg an Körpergewicht verabreicht wird.

**6.** Pharmazeutische Zusammensetzung nach Anspruch 3, wobei die Verbindung einem Patienten mit einer Dosiseinheit von ungefähr 10 µg/kg an Körpergewicht bis 250 mg/kg an Körpergewicht verabreicht wird.

**7.** Pharmazeutische Zusammensetzung nach Anspruch 3, wobei die Verbindung einem Patienten mit einer Dosiseinheit von ungefähr 20 µg/kg an Körpergewicht bis 100 mg/kg an Körpergewicht verabreicht wird.

**8.** Verwendung der Verbindung gemäß Anspruch 1 zur Herstellung eines Medikaments zur Behandlung und/oder Modulation der Fertilität beim Menschen, beim Ersatz weiblicher Hormone, einer disfunktionalen Uterusblutung, einer Endometriosie, eines Leiomyoms, von Akne, des Haarausfalls beim Mann, von Osteoporose, von Prostatahyperplasie, von Brustkrebs, von Krebs der Eierstöcke, eines Korpuskarzinoms, von Prostatakrebs, des Kohlenhydrat-, Protein- und Lipidmetabolismus, des Electrolyt- und Wassergleichgewichts und der Funktion des Herzkreislauf-, Nieren-, Zentralnerven-, Immun- und Skelettmuskelsystems.

**9.** Verwendung der Verbindung gemäß Anspruch 1 zur Herstellung eines Medikaments zur Behandlung von Patienten, die eine Androgenrezeptortherapie benötigen; wobei die Verbindung wirksam ist bei der Behandlung und/oder Modulation der Fertilität beim Menschen, beim Ersatz weiblicher Hormone, einer disfunktionalen Uterusblutung, einer Endometriosie, eines Leiomyoms, von Akne, des Haarausfalls beim Mann, von Osteoporose, von Prostatahyperplasie, von Brustkrebs, von Krebs der Eierstöcke, eines Korpuskarzinoms, von Prostatakrebs, des Kohlenhydrat-, Protein- und Lipidmetabolismus, des Electrolyt- und Wassergleichgewichts und der Funktion des Herzkreislauf-, Nieren-, Zentralnerven-, Immun- und Skelettmuskelsystems.

**10.** Verfahren zur Bestimmung des Vorhandenseins von einem oder mehreren Androgenrezeptoren in einer Probe, umfassend das Kombinieren einer Verbindung gemäß Anspruch 1 mit der Probe, die einen oder mehrere unbekannte Androgenrezeptoren enthält, und Bestimmen, ob die Verbindung an einen Rezeptor in der Probe bindet.

**11.** Ligand-Androgenrezeptor-Komplex, der gebildet wird durch das Binden einer Verbindung gemäß Anspruch 1 an einen Androgenrezeptor.

**12.** Verfahren zur Reinigung von Androgenrezeptoren, umfasend das Kombinieren einer Verbindung gemäß Anspruch 1 mit einer Probe, die Androgenrezeptoren enthält, Binden-Lassen der Verbindung an die Androgenrezeptoren und Abtrennen der verbundenen Kombination der Verbindung und der Androgenrezeptoren.

**13.** Verbindung, ausgewählt aus der Gruppe von

1,2-Dihydro-2,2,4-trimethyl-10-isocumarino[4,3-*g*]chinolin (Verbindung 239);
1,2-Dihydro-2,2,4-trimethyl-10-isochinolono[4,3-g]chinolin(Verbindung 240);
(*R*/*S*)-1,2,3,4-Tetrahydro-2,2,4-trimethyl-10-isochinolono[4,3-*g*]chinolin (Verbindung 244);
1,2-Dihydro-2,2,4-trimethyl-10-thioisochinolono[4,3-g]chinolin (Verbindung 245);
(+)-1,2,3,4-Tetrahydro-2,2,4-trimethyl-10-isochinolono[4,3-*g*]chinolin (Verbindung 246);

1,2-Dihydro-10-hydroxy-2,2,4-trimethyl-10*H*-isochromeno[4,3-*g*]chinolin (Verbindung 242);
1,2-Dihydro-2,2,4,6-tetramethyl-8H-pyrano[3,2-*g*]chinolin (Verbindung 243); und
1,2-Dihydro-2,2,4-trimethyl-8-trifluormethyl-6-pyridono[5,6-*g*]chinolin (Verbindung 262);
8-(4-Chlorbenzoyl)-5-trifluormethyl-7-pyridono[5,6-e]indolin (Verbindung 420);
1,2-Dihydro-2,2,4-trimethyl-6-methoxymethyl-8-pyridono[5,6-*g*]chinolin (Verbindung 426); und
1,2,3,4-Tetrahydro-10-hydroxymethyl-2,2,4-trimethyl-6-trifluormethyl-8-pyridono[5,6-*g*]chinolin (Verbindung 433).

## Revendications

**1.** Composé de formules :

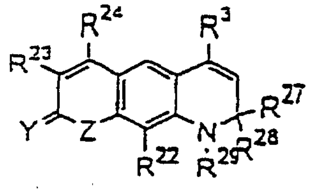

(IX)

OU

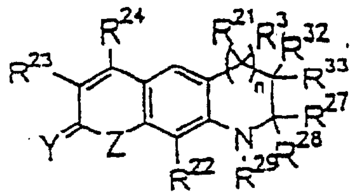

(XVII)

OU

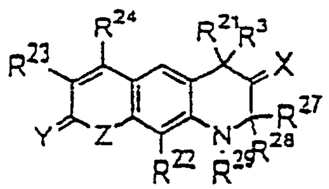

(XVIII)

dans lesquelles :

$R^2$ est un hydrogène, un $C_1$-$C_4$ alkyle ou perfluoroalkyle;
$R^3$ est un hydrogène, un $C_1$-$C_4$ alkyle ou perfluoroalkyle, ou un hydroxyméthyle ;
$R^7$ est un hydrogène, un $C_1$-$C_4$ alkyle ou perfluoroalkyle, $OR^8$ ou $NHR^8$, où $R^8$ est un hydrogène, un $C_1$-$C_6$ alkyle ou perfluoroalkyle, $SO_2R^2$ ou $S(O)R^2$, où $R^2$ a la signification donnée ci-dessus ;
X est $CH_2$, O, S ou $NR^7$, où $R^7$ a la signification donnée ci-dessus ;

$R^{21}$ est un hydrogène, ou un $C_1$-$C_4$ alkyle;
$R^{22}$ est un hydrogène, un $C_1$-$C_4$ alkyle, F, Cl, Br, I, $OR^2$, $NR^2R^7$ ou $SR^2$, où $R^2$ et $R^7$ ont les significations données ci-dessus ;
$R^{23}$ est un hydrogène, Cl, Br, F, ou un $C_1$-$C_4$ alkyle ou perhaloalkyle;
$R^{24}$ est un hydrogène, F, Br, Cl, un $C_1$-$C_4$ alkyle ou perhaloalkyle, un cycle phényle ou un cycle hétérocyclique à cinq chaînons contenant un ou plusieurs hétéroatomes choisis dans le groupe constitué par l'oxygène, l'azote et le soufre, ou un cycle hétérocyclique à six chaînons contenant un ou plusieurs hétéroatomes d'azote, $CF_3$, $CF_2OR^{25}$, $CH_2OR^{25}$, ou $OR^{25}$, où $R^{25}$ est un $C_1$-$C_4$ alkyle, sauf que $R^{24}$ ne peut pas être $CH_3$ lorsque Z est O, $R^{22}$, $R^{23}$, et $R^{29}$ sont tous des hydrogènes et $R^3$, $R^{27}$ et $R^{28}$ sont tous $CH_3$ ;
$R^{27}$ et $R^{28}$ sont chacun indépendamment un hydrogène ou un $C_1$-$C_4$ alkyle ou perfluoroalkyle;
$R^{29}$ est un hydrogène ou un $C_1$-$C_6$ alkyle;
$R^{32}$ et $R^{33}$ sont chacun indépendamment un hydrogène ou un $C_1$-$C_4$ alkyle;
n est 0 ou 1 ;
Y est O ou S ;
Z est O, S, NH, $NR^2$ ou $NCOR^2$, où $R^2$ a la même signification donnée ci-dessus.

**2.** Composé selon la revendication 1, choisi dans le groupe des composés de formule générale IX constitué par :

1,2-Dihydro-2,2,4-triméthyl-6-méthoxyméthyl-8-pyranono[5,6-*g*]quinoline (composé 237) ;
1,2-Dihydro-2,2,4-triméthyl-6-trifluorométhyl-8-pyranone[5,6-*g*]quinoline (composé 238) ;
1,2-Dihydro-2,2,4,6-tétraméthyl-8-pyridono[5,6-*g*]quinoline (composé 241) ;
1,2-Dihydro-2,2,4-triméthyl-6-trifluorométhyl-8-pyridino[5,6-*g*]quinoline (composé 247) ;
1,2-Dihydro-2,2,4-triméthyl-6-trifluorométhyl-8-thiopyranono[5,6-*g*]quinoline (composé 251) ;
6-Chloro(difluoro)méthyl-1,2-dihydro-2,2,4-triméthyl-8-pyranono[5,6-*g*]quinoline (composé 253) ;
9-Acétyl-1,2-dihydro-2,2,4-triméthyl-6-trifluorométhyl-8-pyridono[5,6-*g*]quinoline (composé 254) ;
1,2-Dihydro-2,2,4,10-tétraméthyl-6-trifluorométhyl-8-pyridono[5,6-*g*]quinoline (composé 255) ;
1,2-Dihydro-2,2,4-triméthyl-6-(1,1,2,2,2-pentafluoroéthyl)-8-pyranono[5,6-*g*]quinoline (composé 256) ;
7-Chloro-1,2-dihydro-2,2,4-triméthyl-6-trifluorométhyl-8-pyranono[5,6-*g*]quinoline (composé 258) ;
1,2-Dihydro-2,2,4,9-tétraméthyl-6-trifluorométhyl-8-pyridono[5,6-*g*]quinoline (composé 261) ;
6-[Dichloro(éthoxy)méthyl]-1,2-dihydro-2,2,4-triméthyl-8-pyranono[5,6-*g*]quinoline (composé 263) ;
6-(3-Furyl)-1,2-dihydro-2,2,4-triméthyl-8-pyranono[5,6-*g*]quinoline (composé 264) ;
1,2-Dihydro-1,2,2,4-tétraméthyl-6-trifluorométhyl-8-pyranono[5,6-*g*]quinoline (composé 265) ;
1,2-Dihydro-6-trifluorométhyl-2,2,4-triméthyl-9-thiopyran-8-ono[5,6-*g*]quinoline (composé 266) ;
1,2-Dihydro-1,2,2,4,9-pentaméthyl-6-trifluorométhyl-8-pyridono[5,6-*g*]quinoline (composé 267) ;
7-Chlore-1,2-dihydro-2,2,4-triméthyl-6-trifluorométhyl-8-pyridono[5,6-*g*]quinoline (composé 268) ; et
6-Chloro(difluoro)méthyle-1,2-dihydro-2,2,4-triméthyl-8-pyridono[5,6-*g*]quinoline (composé 269) ;
6-(3-Furyl)-1,2-dihydro-1,2,2,4-tétraméthyl-8-pyranono[5,6-*g*]quinoline (composé 406) ;
6-(3-Furyl)-1,2-dihydro-1,2,2,4-tétraméthyl-8-thiopyranono[5,6-g] quinoline (composé 407) ;
7-Chloro-6-trifluorométhyl-1,2-dihydro-1,2,2,4-tétraméthyl-8-pyranono[5,6-*g*]quinoline (composé 408) ;
1,2-Dihydro-2,2-diméthyl-6-trifluorométhyl-8-pyranono[5,6-*g*]quinoline (composé 411) ;
1,2-Dihydro-6-trifluorométhyl-1,2,2,4-tétraméthyl-8-pyridono[5,6-*f*]quinoline (composé 423) ;
1,2-Dihydro-1,2,2,4-tétraméthyl-6-trifluorométhyl-9-thiopyran-8-ono[5,6-*g*]quinoline (composé 430) ;

le groupe des composés de formule générale XVII constitué par :

(*R*/*S*)-1,2,3,4-Tétrahydro-2,2,4-triméthyl-6-trifluorométhyl-8-pyranono[5,6-*g*]quinoline (composé 250),
(*R*/*S*)-1,2,3,4-Tétrahydro-2;2,4-triméthyl-6-trifluorométhyl-8-thiopyranono[5,6-*g*]quinoline (composé 252) ;
(*R*/*S*)-6-Chloro(difluoro)méthyl-1,2,3,4-tétrahydro-2,2, 4-triméthyl-8-pyranono [5,6-*g*]quinoline (composé 257) ;
(*R*/*S*)-7-Chloro-1,2,3,4-tétrahydro-2,2,4-triméthyl-6-trifluorométhyl-8-pyranono[5,6-*g*]quinoline (composé 259) ;
1,2,3,4-Tétrahydro-2,2,4-triméthyl-6-trifluorométhyl-8-pyridono[5,6-*g*]quinoline (composé 260) ;
(*R*/*S*)-1,2,3,4-Tétrahydro-1,2,2,4-tétraméthyl-6-trifluorométhyl-8-pyranono[5,6-*g*]quinoline (composé 404) ;
6-(3-Furyl)-1,2,3,4-Tétrahydro-2,2,4-triméthyl-8-pyranono[5,6-*g*]quinoline (composé 405) ;
1,2,3,4-Tétrahydro-2,2,4,10-tétraméthyl-6-trifluorométhyl-8-pyridono[5,6-*g*]quinoline (composé 409) ;
(*R*/*S*)-1,2,3,4-Tétrahydro-4-méthyl-6-trifluorométhyl-8-pyranono[5,6-*g*]quinoline (composé 410) ;
1,2,3,4-Tétrahydro-2,2-diméthyl-6-trifluorométhyl-8-pyranono[5,6-*g*]quinoline (composé 412) ;
1,2,3,4-Tétrahydro-6-trifluorométhyl-8-pyranono[5,6-*g*]quinoline (composé 413) ;

(*R*/*S*)-4-Ethyl-1,2,3,4-Tétrahydro-6-trifluorométhyl-8-pyranono[5,6-*g*]quinoline (composé 414) ;
(*R*/*S*)-1,2,3,4-Tétrahydro-1,4-diméthyl-8-pyranono[5,6-*g*]quinoline (composé 415) ;
(*R*/*S*)-4-Ethyl-1,2,3,4-Tétrahydro-1-méthyl-8-pyranono[5,6-*g*]quinoline (composé 416) ;
2,2-Diméthyl-1,2,3,4-Tétrahydro-6-trifluorométhyl-8-pyridono[5,6-*f*]quinoline (composé 417) ;
5-Trifluorométhyl-7-pyridono[5,6-*e*]indoline (composé 419) ;
2,2,10-Triméthyl-1,2,3,4-Tétraméthyl-6-trifluorométhyl-8-pyridono[5,6-*g*]quinoline (composé 421) ;
1,2,3,4-Tétrahydro-6-trifluorométhyl-8-pyridono[5,6-*g*]quinoline (composé 422) ;
3,3-Diméthyl-5-trifluorométhyl-7-pyridono[5,6-*f*]indoline (composé 424) ;
(*R*/*S*)-1,2,3,4-Tétrahydro-4-méthyl-6-(trifluorométhyl)-8-pyridono[5,6-*g*]quinoline (composé 425) ;
1,2,2,-Triméthyl-1,2,3,4-Tétrahydro-6-trifluorométhyl-8-pyranono[5,6-*g*]quinoline (composé 427) ;
(*R*/*S*)-1,2,3,4-Tétrahydro-4-propyl-6-trifluorométhyl-8-pyranono[5,6-*g*]quinoline (composé 428) ;
1,2,3,4-Tétrahydro-2,2,4-triméthyl-6-trifluorométhyl-9-thiopyran-8-ono[5,6-*g*]quinoline (composé 429) ;
1,2,3,4-Tétrahydro-1,2,2-triméthyl-6-trifluorométhyl-8-pyridono[5,6-*g*]quinoline (composé 431) ;
1,2,3,4-Tétrahydro-1-méthyl-4-propyl-6-trifluorométhyl-8-pyranono[5,6-*g*]quinoline (composé 432) ;
1,2,3,4-Tétrahydro-1,2,2,4-Tétraméthyl-6-trifluorométhyl-9-thiopyran-8-ono[5,6-*g*]quinoline (composé 434) ;
1,2,3,4-Tétrahydro-2,2,9-triméthyl-6-trifluorométhyl-8-pyridono[5,6-*g*]quinoline (composé 435) ;
(*R*/*S*)-1,2,3,4-Tétrahydro-3-méthyl-6-trifluorométhyl-8-pyridono[5,6-*g*]quinoline (composé 436) ;
1,2,3,4-Tétrahydro-3,3-diméthyl-6-trifluorométhyl-8-pyridono[5,6-*g*]quinoline (composé 437) ;
(*R*/*S*)-1,2,3,4-Tétrahydro-2,2,3-triméthyl-6-trifluorométhyl-8-pyridono[5,6-*g*]quinoline (composé 438) ;
(*R*/*S*-2*l*,4*u*)-1,2,3,4-Tétrahydro-2,4-diméthyl-6-trifluorométhyl-8-pyridono[5,6-*g*]quinoline (composé 439) ;
(*R*/*S*-2*l*,4*u*)-4-Ethyl-1,2,3,4-Tétrahydro-2-méthyl-6-trifluorométhyl-8-pyranono[5,6-*g*]quinoline (composé 440) ;
(*R*/*S*-2*l*,3*u*)-1,2,3,4-Tétrahydro-2,3-diméthyl-6-trifluorométhyl-8-pyridono[5,6-*g*]quinoline (composé 441) ;
(*R*/*S*-2*l*,3*l*)-1,2,3,4-Tétrahydro-2,3-diméthyl-6-trifluorométhyl-8-pyridono[5,6-*g*]quinoline (composé 442) ;
(*R*/*S*)-1,2,3,4-Tétrahydro-2,3,3-triméthyl-6-trifluorométhyl-8-pyridono[5,6-*g*]quinoline (composé 443) ;
(*R*/*S*)-1,2,3,4-Tétrahydro-2-méthyl-6-trifluorométhyl-8-pyridono[5,6-g]quinoline (composé 444) ;
(*R*/*S*)-4-Ethyl-1,2,3,4-Tétrahydro-6-trifluorométhyl-8-pyridono[5,6-*g*]quinoline (composé 445) ;
(*R*/*S*-2*l*,3*u*)-1,2,3,4-Tétrahydro-2,3,9-triméthyl-6-trifluorométhyl-8-pyridono[5,6-*g*]quinoline (composé 446) ;
(*R*/*S*)-1,2,3,4-Tétrahydro-4-propyl-6-trifluorométhyl-8-pyridono[5,6-*g*]quinoline (composé 447) ;
(*R*/*S*)-3-Ethyl-1,2,3,4-Tétrahydro-2,2-diméthyl-6-trifluorométhyl-8-pyridono[5,6-*g*]quinoline (composé 448) ;
(*R*/*S*)-1,2,3,4-Tétrahydro-2,2-diméthyl-6-trifluorométhyl-3-propyl-8-pyridono[5,6-*g*]quinoline (composé 449) ; et
1-Méthyl-5-trifluorométhyl-7-pyridono[5,6-*f*]indoline (composé 450), et

le composé de formule générale XVIII (*R*/*S*)-1,2,3,4-Tétrahydro-6-trifluorométhyl-2,2,4-triméthyl-8-pyridono [5,6-*f*] -3-quinolinone (composé 418).

**3.** Composition pharmaceutique comprenant un composé selon la revendication 1 et un excipient pharmaceutiquement acceptable.

**4.** Composition pharmaceutique selon la revendication 3, dans laquelle la composition est formulée pour une administration par voie orale, topique, intraveineuse, parentale ou sous forme de suppositoire.

**5.** Composition pharmaceutique selon la revendication 3, dans laquelle le composé est administré à un patient à une unité posologique comprise entre environ 1 µg/kg de poids corporel et 500 mg/kg de poids corporel.

**6.** Composition pharmaceutique selon la revendication 3, dans laquelle le composé est administré à un patient à une unité posologique comprise entre 10 µg/kg de poids corporel et 250 mg/kg de poids corporel.

**7.** Composition pharmaceutique selon la revendication 3, dans laquelle le composé est administré à un patient à une unité posologique comprise entre 20 µg/kg de poids corporel et 100 mg/kg de poids corporel.

**8.** Utilisation des composés selon la revendication 1 pour la préparation d'un médicament destiné au traitement et/ou la modulation de la fertilité humaine, de la substitution d'hormones féminines,"de la ménométrorragie, de l'endométriose, du léiomyome, de l'acné, de la calvitie de type masculine, de l'ostéoporose, de l'hyperplasie prostatique, du cancer du sein, du cancer de l'ovaire, du cancer de l'endomètre, du cancer de la prostate, du métabolisme des glucides, des protéines et des lipides, de l'équilibre des électrolytes et de l'eau, et le fonctionnement des systèmes cardio-vasculaire, rénal, nerveux central, immunitaire et des muscles squelettiques.

**9.** Utilisation d'un composé selon la revendication 1, pour la préparation d'un médicament destiné à traiter des patients requérant une thérapie des récepteurs aux androgènes, dans laquelle le composé est efficace pour traiter et/ou moduler la fertilité humaine, la substitution d'hormones féminines, la ménométrorragie, l'endométriose, le léiomyome, l'acné, la calvitie de type masculine, l'ostéoporose, l'hyperplasie prostatique, le cancer du sein, le cancer de l'ovaire, le cancer de l'endomètre, le cancer de la prostate, le métabolisme des glucides, des protéines et des lipides, l'équilibre des électrolytes et de l'eau, et le fonctionnement des systèmes cardio-vasculaire, rénal, nerveux central, immunitaire et des muscles squelettiques.

**10.** Procédé de détermination de la présence d'un ou plusieurs récepteurs aux androgènes dans un échantillon comprenant l'action de combiner un composé selon la revendication 1 avec un échantillon contenant un ou plusieurs récepteurs aux androgènes inconnus et l'action de déterminer si ledit composé se lie à un récepteur dans l'échantillon.

**11.** Un complexe ligand-récepteur aux androgènes formé par la liaison d'un composé selon la revendication 1 à un récepteur aux androgènes.

**12.** Procédé de purification de récepteurs aux androgènes comprenant l'action de combiner un composé selon la revendication 1 avec un échantillon contenant des récepteurs aux androgènes, l'action de laisser ledit composé se lier auxdits récepteurs aux androgènes et l'action de séparer la combinaison complexée dudit composé et desdits récepteurs aux androgènes.

**13.** Composé choisi dans le groupe comprenant la :

1,2-Dihydro-2,2,4-triméthyl-10-isocoumarino[4,3-g]quinoline (composé 239) ;
1,2-Dihydro-2,2,4-triméthyl-10-isoquinolono[4,3-*g*]quinoline (composé 240) ;
(R/S)-1,2,3,4-Tétrahydro-2,2,4-triméthyl-10-isoquinolono[4,3-g]quinoline (composé 244) ;
1,2-Dihydro-2,2,4-triméthyl-10-thioisoquinolono[4,3-*g*]quinoline (composé 245) ;
(+)-1,2,3,4-Tétrahydro-2,2,4-triméthyl-10-isoquinolono[4,3-*g*]quinoline (composé 246) ;
1,2-Dihydro-10-hydroxy-2,2,4-triméthyl-10*H*-isochroméno[4,3-*g*]quinoline (composé 242) ;
1,2-Dihydro-2,2,4,6-tétraméthyl-8H-pyrano[3,2-*g*]quinoline (composé 243) ; et
1,2 Dihydro-2,2,4-triméthyl-8-trifluorométhyl-6-pyridono[5,6-*g*]quinoline (composé 262) ;
8- (4-Chlorobenzoyl)-5-trifluorométhyl-7-pyridono[5,6-*e*]indoline (composé 420) ;
1,2-Dihydro-2,2,4-triméthyl-6-méthoxyméthyl-8-pyridono[5,6-*g*]quinoline (composé 426) ; et la 1,2,3,4-Tétrahydro-10-hydroxyméthyl-2,2,4-triméthyl-6-trifluorométhyl-8-pyridono[5,6-*g*]quinoline (composé 433).